# EUROPEAN PATENT APPLICATION

(11) **EP 4 212 511 A1**
(43) Date of publication of application: **19.07.2023**
(21) Application number: 21865980.3
(22) Date of filing: 08.09.2021
(51) Int. Cl.: C07C 229/00, C07D 211/00, A61K 31/33, A61K 31/197

(54) **AROMATIC ETHYLENE COMPOUND AND PREPARATION METHOD THEREFOR, AND INTERMEDIATE, PHARMACEUTICAL COMPOSITION, AND APPLICATION THEREOF**

(30) Priority: 09.09.2020 CN 202010939415; 02.12.2020 CN 202011414403; 06.04.2021 CN 202110368880; 27.08.2021 CN 202110995765
(71) Applicant: Guangzhou Maxinovel Pharmaceuticals Co., Ltd., Guangzhou Hitech Park Guangzhou Guangdong 510670 (CN)
(72) Inventor: WANG, Yuguang, Guangzhou, Guangdong 510670 (CN); ZHANG, Nong, Guangzhou, Guangdong 510670 (CN); WU, Tianzhi, Guangzhou, Guangdong 510670 (CN); HE, Min, Guangzhou, Guangdong 510670 (CN); WU, Xinliang, Guangzhou, Guangdong 510670 (CN); ZHANG, Shunli, Guangzhou, Guangdong 510670 (CN)
(74) Representative: Secerna LLP
(86) International application number: PCT/CN2021/117066
(87) International publication number: WO 2022/052926

(57) **Abstract**

An aromatic ethylene compound and preparation method therefor, and intermediate, pharmaceutical composition, and application thereof. The aromatic ethylene compound is represented by formula I-0. The aromatic ethylene compound has a significant inhibitory effect on PD-l/PD-L1, has a higher drug peak concentration, a larger area under the drug concentration time curve, better oral bioavailability, is a type of extremely effective small molecule inhibitor for PD-l/PD-Ll, and can effectively alleviate or treat cancer and a related disease.

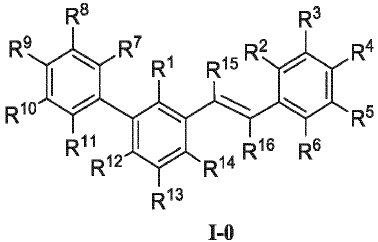

## Description

The present application claims the priority of Chinese patent application 2020109394150 with the filing date of September 09, 2020, the priority of Chinese patent application 2020114144032 with the filing date of December 02, 2020, the priority of the Chinese patent application 2021103688808 with the filing date of April 06, 2021, and the priority of the Chinese patent application 2021109957653 with the filing date of August 27, 2021. The contents of the Chinese patent application are incorporated herein by reference in their entirety.

### TECHNICAL FIELD

The present disclosure relates to an aromatic vinyl compound, a preparation method therefor, an intermediate thereof, a pharmaceutical composition thereof and a use thereof.

### BACKGROUND

PD-1 (programmed death 1) is an important immunosuppressive molecule. It is a member of CD28 superfamily and was originally cloned from the apoptotic mouse T-cell hybridoma 2B4.11. Immunomodulation targeting PD-1 is of great importance to anti-tumor, anti-infection, anti-autoimmune diseases and survival of organ transplantation. Its ligand PD-L1 can also be served as a target, and the corresponding antibodies can also play the same role.

PD-1/PD-L1 plays a role of a negative immunomodulatory effect. When PD-1 on the cell surface is coupled to PD-L1, it can cause the phosphorylation of Tyr of the Immunoreceptor Tyrosine-based Swith motifs (ITSM) domain in T-cell cytoplasmic region. Then the phosphorylated Tyr recruits tyrosine-protein phosphatase 2 and tyrosine-protein phosphatase 1 to block not only the activation of extracellular signal-regulated kinase but also the activation of phosphatidylinositol 3-kinase (PI3K) and serine/threonine protein kinase (Akt), finally inhibits T lymphocyte proliferation and related cytokines secretion. PD-1/PD-L1 signaling can inhibit T cell activation and proliferation, meanwhile the secretion of cytokine interleukin 2 (IL2), interferon γ and IL-10 is also reduced (Eur. J. Immunol., 2002, 32(3), 634-643). In addition, the immune function of PD-1/PD-L1 signaling to the B cell is also similar to that of T cell. After PD-1 binds to B cell antigen receptor, PD-1 cytoplasmic domain interacts with tyrosinase containing the site binding to protein tyrosinase 2, finally blocks B cell activation. The role of negative immunosuppressive molecule PD-1/PD-L1 in tumor immune escape has attracted more and more attention. A lot of studies have confirmed that PD-L1 on the surface of tumor cells in the tumor microenvironment increases, and binds to PD-1 of activated T cells, transmitting a negative regulatory signal and leading to apoptosis or immune incompetence of tumor antigen-specific T cells, thereby inhibiting the immune response and promoting the escape of tumor cells.

Currently PD-1/PD-L1 antibody inhibitors that have been approved for market include Nivolumab (2014) developed by BMS, Lambrolizumab (2014) developed by Merck and Atezolizumab (2016) developed by Roche. PD-1/PD-L1 antibody inhibitors under research include Pidilizumab developed by Cure Tech, AMP-224 developed by GSK and MEDI-4736 developed by AstraZeneca. These are all biomacromolecules, and small molecule PD-1/PD-L1 inhibitors are still in the early stage of development. The PD-L1 small molecule inhibitor AC-170 (WO2012168944, WO2015033299, WO2015033301, WO2015036927, WO2015044900) which is a polypeptide developed by Curis has just entered clinical stage I, a small molecule PD-1/PD-L1 inhibitor which is a benzyl phenyl ether developed by BMS (WO2015034820, WO2015160641, WO2017066227, WO2018009505, WO2018044963, WO2018118848) is still in the preclinical stage, and a series of small molecule PD-1/PD-L1 inhibitors which are developed by Incyte (WO2017070089, WO2017087777, WO2017106634, WO2017112730, WO2017192961, WO2017205464, WO2017222976, WO2018013789, WO2018044783, WO2018119221, WO2018119224, WO2018119263, WO2018219266, WO2018119286) are still in the preclinical stage. Compared to biomacromolecules, small molecule compounds can act on intracellular targets across the cell membrane, so they are widely used. Secondly, small molecules can often have a good bioavailability and compliance after being chemically modified, thus effectively avoiding the decomposition and inactivation by enzymes in the digestive intestinal tract. Finally, the research of small molecule is quite mature in many aspects, e.g., manufacturing process, dosage form design and route of administration.

At present, there is no report on successful marketing of biphenyl compounds as small molecule PD-1/PD-L1 inhibitors in the prior art, and this situation needs to be solved urgently.

### CONTENT OF THE PRESENT INVENTION

The technical problem to be solved by the present disclosure is to provide an aromatic vinyl compound, a preparation method therefor, an intermediate thereof, a pharmaceutical composition thereof and a use thereof, which are completely different from the prior art. The aromatic vinyl compounds of the present disclosure have a significant inhibitory effect on PD-1/PD-L1, as well as a higher drug peak concentration, a larger area under the curve and better oral bioavailability, which is a very effective small molecule inhibitor of PD-1/PD-L1 and can effectively alleviate or treat cancer and other related diseases.

The present disclosure provides an aromatic vinyl compound of formula I-0, a tautomer thereof, a stereoisomer thereof, a racemate thereof, an isotopic derivative thereof, or their (referring to the above aromatic vinyl compound of formula I-0, the tautomer thereof, the stereoisomer thereof, the racemate thereof or the isotopic derivative thereof) pharmaceutically acceptable salts; 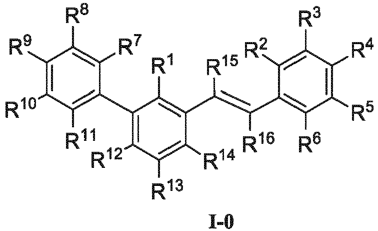 wherein:
R¹ is cyano, C₁-C₄ alkyl, C₁-C₄ alkyl substituted by one or more than one deuterium, halogen, C₁-C₄ alkyl substituted by one or more than one halogen;
R³, R⁶, R¹², R¹³, and R¹⁴ are independently H or deuterium;
R² is hydroxyl, halogen, C₁-C₄ alkyl, C₁-C₄ alkyl substituted by one or more than one R^{A-1}, C₁-C₆ alkoxy or C₁-C₆ alkoxy substituted by one or more than one R^{A-2};
R^{A-1} and R^{A-2} are independently deuterium, hydroxyl, halogen, cyano, C₁-C₄ alkoxy, C₁-C₄ alkoxy substituted by one or more than one deuterium, 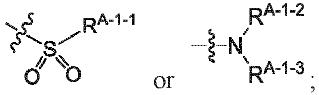 R^{A-1-1} is C₁-C₄ alkyl or C₁-C₄ alkyl substituted by one or more than one deuterium; R^{A-1-2} and R^{A-1-3} are independently H, deuterium, C₁-C₄ alkyl or C₁-C₄ alkyl substituted by one or more than one R^{A-1-1-1}, and R^{A-1-1-1} is deuterium, hydroxyl or COOR^{A-1-1-2};
R⁴ and R⁵ are independently hydroxyl, halogen, C₁-C₄ alkyl, C₁-C₄ alkyl substituted by one or more than one R^{B-1}, C₁-C₆ alkoxy, or C₁-C₆ alkoxy substituted by one or more than one R^{B-2};
R^{B-1} and R^{B-2} are independently deuterium, hydroxyl, halogen, cyano, C₆-C₁₀ aryl, C₆-C₁₀ aryl substituted by one or more than one R^{B-1-3}, 3- to 12-membered heteroaryl, 3- to 12-membered heteroaryl substituted by one or more than one R^{B-1-4}, C₁-C₄ alkoxy, C₁-C₄ alkoxy substituted by one or more than one deuterium, 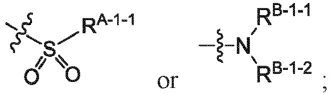 in the heteroaryl, the heteroatom is selected from one or more of N, O and S, and the number of the heteroatom is 1 to 4;
R^{B-1-3} and R^{B-1-4} are independently cyano, halogen, C₁-C₄ alkyl or C₁-C₄ alkoxy;
R^{B-1-1} and R^{B-1-2} are independently H, deuterium, C₁-C₄ alkyl or C₁-C₄ alkyl substituted by one or more than one R^{B-1-1-1}, and R^{B-1-1-1} is deuterium, hydroxyl or COOR^{B-1-1-5};
or, R^{B-1-1}, R^{B-1-2} together with the nitrogen atom to which they are attached form a 5-to 7-membered carbon heterocycle or a 5- to 7-membered carbon heterocycle substituted by one or more than one R^{B-1-1-2;} in the carbon heterocycle, the heteroatom is selected from one or more of N, O and S, and the number of the heteroatom is 1 to 4; R^{B-1-1-2} is deuterium, C₁-C₄ alkyl, C₁-C₄ alkyl substituted by one or more than one deuterium, COOR^{B-1-1-6} or C₁-C₄ amido;
R^{A-1-1-2}, R^{B-1-1-5} and R^{B-1-1-6} are independently H, deuterium, C₁-C₄ alkyl or C₁-C₄ alkyl substituted by one or more than one deuterium;
R⁷, R⁸, R⁹, R¹⁰ and R¹¹ are independently H or deuterium;
R¹⁵ and R¹⁶ are independently H, deuterium or halogen.

In some embodiments, in the aromatic vinyl compound of formula I-0, R^{B-1} and R^{B-2} are independently deuterium, hydroxyl, halogen, cyano, C₁-C₄ alkoxy, C₁-C₄ alkoxy substituted by one or more than one deuterium, 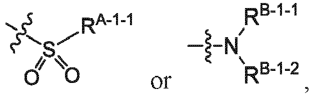 and other variables are defined as in any one of the embodiments of the present disclosure.

The present disclosure provides an aromatic vinyl compound of formula I, a tautomer thereof, a stereoisomer thereof, a racemate thereof, an isotopic derivative thereof, or their (referring to the above aromatic vinyl compound of formula I, the tautomer thereof, the stereoisomer thereof, the racemate thereof or the isotopic derivative thereof) pharmaceutically acceptable salts; 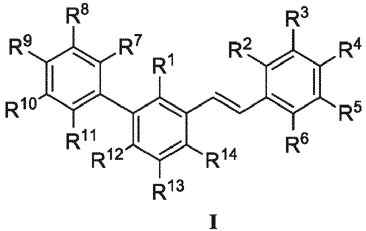 wherein:
R¹ is cyano, C₁-C₄ alkyl, C₁-C₄ alkyl substituted by one or more than one deuterium;
R³, R⁶, R¹², R¹³ and R¹⁴ are independently H or deuterium;
R² is hydroxyl, halogen, C₁-C₄ alkyl, C₁-C₄ alkyl substituted by one or more than one R^{A-1}, C₁-C₆ alkoxy or C₁-C₆ alkoxy substituted by one or more than one R^{A-2};
R^{A-1} and R^{A-2} are independently deuterium, hydroxyl, halogen, cyano, C₁-C₄ alkoxy, C₁-C₄ alkoxy substituted by one or more than one deuterium, 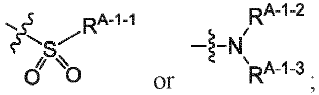 R^{A-1-1} is C₁-C₄ alkyl or C₁-C₄ alkyl substituted by one or more than one deuterium; R^{A-1-2} and R^{A-1-3} are independently H, deuterium, C₁-C₄ alkyl or C₁-C₄ alkyl substituted by one or more than one R^{A-1-1-1}, and R^{A-1-1-1} is deuterium, hydroxyl or COOR^{A-1-1-2};
R⁴ and R⁵ are independently hydroxyl, halogen, C₁-C₄ alkyl, C₁-C₄ alkyl substituted by one or more than one R^{B-1}, C₁-C₆ alkoxy or C₁-C₆ alkoxy substituted by one or more than one R^{B-2};
R^{B-1} and R^{B-2} are independently deuterium, hydroxyl, halogen, cyano, C₁-C₄ alkoxy, C₁-C₄ alkoxy substituted by one or more than one deuterium, 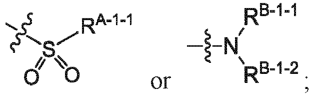
R^{B-1-1} and R^{B-1-2} are independently H, deuterium, C₁-C₄ alkyl or C₁-C₄ alkyl substituted by one or more than one R^{B-1-1-1}, and R^{B-1-1-1} is deuterium, hydroxyl or COOR^{B-1-1-5};
or, R^{B-1-1}, R^{B-1-2} together with the nitrogen atom to which they are attached form a 5-to 7-membered carbon heterocycle or a 5- to 7-membered carbon heterocycle substituted by one or more than one R^{B-1-1-2}; in the carbon heterocycle, the heteroatom is selected from one or more of N, O and S, and the number of the heteroatom is 1 to 4; R^{B-1-1-2} is deuterium, C₁-C₄ alkyl, C₁-C₄ alkyl substituted by one or more than one deuterium, COOR^{B-1-1-6} or C₁-C₄ amido;
R^{A-1-1-2}, R^{B-1-1-5} and R^{B-1-1-6} are independently H, deuterium, C₁-C₄ alkyl or C₁-C₄ alkyl substituted by one or more than one deuterium;
R⁷, R⁸, R⁹, R¹⁰ and R¹¹ are independently H or deuterium.

In some embodiments, in the aromatic vinyl compound of formula I-0 or I, when R¹ is C₁-C₄ alkyl, the C₁-C₄ alkyl is methyl, ethyl, n-propyl, isopropyl, n-butyl, isobutyl or *tert-*butyl, for example methyl, and other variables are defined as in any one of the embodiments of the present disclosure.

In some embodiments, in the aromatic vinyl compound of formula I-0 or I, when R¹ is C₁-C₄ alkyl substituted by one or more than one deuterium, the C₁-C₄ alkyl substituted by one or more than one deuterium is C₁-C₂ alkyl substituted by one or more than one deuterium, for example monodeuterated methyl, dideuterated methyl, trideuterated methyl, monodeuterated ethyl, dideuterated ethyl, trideuterated ethyl, tetradeuterated ethyl or pentadeuterated ethyl, for another example trideuterated methyl, and other variables are defined as in any one of the embodiments of the present disclosure.

In some embodiments, in the aromatic vinyl compound of formula I-0 or I, when R¹ is halogen, the halogen is fluorine, chlorine, bromine, or iodine, for example chlorine, and other variables are defined as in any one of the embodiments of the present disclosure.

In some embodiments, in the aromatic vinyl compound of formula I-0 or I, when R¹ is C₁-C₄ alkyl substituted by one or more than one halogen, the halogen is fluorine, chlorine, bromine or iodine, for example fluorine, and other variables are defined as in any one of the embodiments of the present disclosure.

In some embodiments, in the aromatic vinyl compound of formula I-0 or I, when R¹ is C₁-C₄ alkyl substituted by one or more than one halogen, the C₁-C₄ alkyl is methyl, ethyl, n-propyl, isopropyl, n-butyl, isobutyl or tert-butyl, for example methyl, and other variables are defined as in any one of the embodiments of the present disclosure.

In some embodiments, in the aromatic vinyl compound of formula 1-0 or I, when R² is halogen, the halogen is fluorine, chlorine, bromine or iodine, for example chlorine, and other variables are defined as in any one of the embodiments of the present disclosure.

In some embodiments, in the aromatic vinyl compound of formula I-0 or I, when R² is C₁-C₄ alkyl, the C₁-C₄ alkyl is methyl, ethyl, n-propyl, isopropyl, n-butyl, isobutyl or *tert-*butyl, for example methyl, and other variables are defined as in any one of the embodiments of the present disclosure.

In some embodiments, in the aromatic vinyl compound of formula I-0 or I, when R² is C₁-C₄ alkyl substituted by one or more than one R^{A-1}, the C₁-C₄ alkyl is methyl, ethyl, *n-*propyl, isopropyl, *n*-butyl, isobutyl or *tert*-butyl, for example methyl, and other variables are defined as in any one of the embodiments of the present disclosure.

In some embodiments, in the aromatic vinyl compound of formula I-0 or I, when R² is C₁-C₄ alkyl substituted by one or more than one R^{A-1}, each R^{A-1} is the same or different, and the more than one is 2, 3, 4, or 5, and other variables are defined as in any one of the embodiments of the present disclosure.

In some embodiments, in the aromatic vinyl compound of formula I-0 or I, when R² is C₁-C₆ alkoxy, the C₁-C₆ alkoxy is C₁-C₄ alkoxy, for example methoxy, ethoxy, *n*-propoxy, or *n*-butoxy, for another example, methoxy, and other variables are defined as in any one of the embodiments of the present disclosure.

In some embodiments, in the aromatic vinyl compound of formula 1-0 or I, when R² is C₁-C₆ alkoxy substituted by one or more than one R^{A-2}, the C₁-C₆ alkoxy is C₁-C₄ alkoxy, for example methoxy, ethoxy, *n*-propoxy or *n*-butoxy, and other variables are defined as in any one of the embodiments of the present disclosure.

In some embodiments, in the aromatic vinyl compound of formula I-0 or I, when R² is C₁-C₆ alkoxy substituted by one or more than one R^{A-2}, each R^{A-2} is the same or different, and the more than one is 2, 3, 4 or 5, and other variables are defined as in any one of the embodiments of the present disclosure.

In some embodiments, in the aromatic vinyl compound of formula I-0 or I, when R^{A-1} and R^{A-2} are independently halogen, the halogen is fluorine, chlorine, bromine, or iodine, for example fluorine, and other variables are defined as in any one of the embodiments of the present disclosure.

In some embodiments, in the aromatic vinyl compound of formula I-0 or I, when R^{A-1} and R^{A-2} are independently C₁-C₄ alkoxy, the C₁-C₄ alkoxy is methoxy, ethoxy, *n*-propoxy or *n*-butoxy, for example methoxy, and other variables are defined as in any one of the embodiments of the present disclosure.

In some embodiments, in the aromatic vinyl compound of formula I-0 or I, when R^{A-1} and R^{A-2} are independently C₁-C₄ alkoxy substituted by one or more than one deuterium, the C₁-C₄ alkoxy substituted by one or more than one deuterium is C₁-C₂ alkoxy substituted by one or more than one deuterium, for example monodeuterated methoxy, dideuterated methoxy, trideuterated methoxy, monodeuterated ethoxy, dideuterated ethoxy, trideuterated ethoxy, tetradeuterium ethoxy or pentadeuterium ethoxy, for example trideuterated methoxy, and other variables are defined as in any one of the embodiments of the present disclosure.

In some embodiments, in the aromatic vinyl compound of formula I-0 or I, when R^{A-1-1} is C₁-C₄ alkyl, the C₁-C₄ alkyl is methyl, ethyl, *n*-propyl, isopropyl, *n*-butyl, isobutyl or *tert-*butyl, for example methyl, and other variables are defined as in any one of the embodiments of the present disclosure.

In some embodiments, in the aromatic vinyl compound of formula 1-0 or I, when R^{A-1-1} is C₁-C₄ alkyl substituted by one or more than one deuterium, the C₁-C₄ alkyl substituted by one or more than one deuterium is C₁-C₂ alkyl substituted by one or more than one deuterium, for example monodeuterated methyl, dideuterated methyl, trideuterated methyl, monodeuterated ethyl, dideuterated ethyl, trideuterated ethyl, tetradeuterated ethyl or pentadeuterated ethyl, for another example trideuterated methyl, and other variables are defined as in any one of the embodiments of the present disclosure.

In some embodiments, in the aromatic vinyl compound of formula I-0 or I, when R^{A-1-2} and R^{A-1-3} are independently C₁-C₄ alkyl, the C₁-C₄ alkyl is methyl, ethyl, *n*-propyl, isopropyl, *n*-butyl, isobutyl or *tert*-butyl, for example methyl, ethyl or isopropyl, and other variables are defined as in any one of the embodiments of the present disclosure.

In some embodiments, in the aromatic vinyl compound of formula 1-0 or I, when R^{A-1-2} and R^{A-1-3} are independently C₁-C₄ alkyl substituted by one or more than one R^{A-1-1-1}, the C₁-C₄ alkyl is methyl, ethyl, *n*-propyl, isopropyl, *n*-butyl, isobutyl or *tert*-butyl, for example methyl, ethyl or isopropyl, and other variables are defined as in any one of the embodiments of the present disclosure.

In some embodiments, in the aromatic vinyl compound of formula I-0 or I, when R^{A-1-2} and R^{A-1-3} are independently C₁-C₄ alkyl substituted by one or more than one R^{A-1-1-1}, each R^{A-1-1-1} is the same or different, and the more than one is 2, 3, 4 or 5, and other variables are defined as in any one of the embodiments of the present disclosure.

In some embodiments, in the aromatic vinyl compound of formula I-0 or I, when R⁴ and R⁵ are independently halogen, the halogen is fluorine, chlorine, bromine or iodine, for example chlorine, and other variables are defined as in any one of the embodiments of the present disclosure.

In some embodiments, in the aromatic vinyl compound of formula I-0 or I, when R⁴ and R⁵ are independently C₁-C₄ alkyl, the C₁-C₄ alkyl is methyl, ethyl, *n*-propyl, isopropyl, *n-*butyl, isobutyl or *tert*-butyl, for example methyl, and other variables are defined as in any one of the embodiments of the present disclosure.

In some embodiments, in the aromatic vinyl compound of formula I-0 or I, when R⁴ and R⁵ are independently C₁-C₄ alkyl substituted by one or more than one R^{B-1}, the C₁-C₄ alkyl is methyl, ethyl, *n*-propyl, isopropyl, *n*-butyl, isobutyl or *tert*-butyl, for example methyl, and other variables are defined as in any one of the embodiments of the present disclosure.

In some embodiments, in the aromatic vinyl compound of formula I-0 or I, when R⁴ and R⁵ are independently C₁-C₄ alkyl substituted by one or more than one R^{B-1}, each R^{B-1} is the same or different, and the more than one is 2, 3, 4 or 5, and other variables are defined as in any one of the embodiments of the present disclosure.

In some embodiments, in the aromatic vinyl compound of formula 1-0 or I, when R⁴ and R⁵ are independently C₁-C₆ alkoxy, the C₁-C₆ alkoxy is C₁-C₄ alkoxy, for example methoxy, ethoxy, *n*-propoxy or *n*-butoxy, for another example methoxy, and other variables are defined as in any one of the embodiments of the present disclosure.

In some embodiments, in the aromatic vinyl compound of formula 1-0 or I, when R⁴ and R⁵ are independently C₁-C₆ alkoxy substituted by one or more than one R^{B-2}, the C₁-C₆ alkoxy is C₁-C₄ alkoxy, for example methoxy, ethoxy, n-propoxy or n-butoxy, and other variables are defined as in any one of the embodiments of the present disclosure.

In some embodiments, in the aromatic vinyl compound of formula 1-0 or I, when R⁴ and R⁵ are independently C₁-C₆ alkoxy substituted by one or more than one R^{B-2}, each R^{B-2} is the same or different, and the more than one is 2, 3, 4, or 5, and other variables are defined as in any one of the embodiments of the present disclosure.

In some embodiments, in the aromatic vinyl compound of formula I-0 or I, when R^{B-1} and R^{B-2} are independently halogen, the halogen is fluorine, chlorine, bromine, or iodine, for example fluorine, and other variables are defined as in any one of the embodiments of the present disclosure.

In some embodiments, in the aromatic vinyl compound of formula I-0 or I, when R^{B-1} and R^{B-2} are independently C₆-C₁₀ aryl, the C₆-C₁₀ aryl is phenyl, and other variables are defined as in any one of the embodiments of the present disclosure.

In some embodiments, in the aromatic vinyl compound of formula I-0 or I, when R^{B-1} and R^{B-2} are independently C₆-C₁₀ aryl substituted by one or more than one R^{B-1-3}, the C₆-C₁₀ aryl is phenyl, and other variables are defined as in any one of the embodiments of the present disclosure.

In some embodiments, in the aromatic vinyl compound of formula I-0 or I, when R^{B-1} and R^{B-2} are independently C₆-C₁₀ aryl substituted by one or more than one R^{B-1-3}, each R^{B-1-3} is the same or different, and the more than one is 2, 3, 4 or 5, and other variables are defined as in any one of the embodiments of the present disclosure.

In some embodiments, in the aromatic vinyl compound of formula I-0 or I, when R^{B-1} and R^{B-2} are independently 3- to 12-membered heteroaryl, the 3- to 12-membered heteroaryl is 5- to 7-membered heteroaryl, and other variables are defined as in any one of the embodiments of the present disclosure.

In some embodiments, in the aromatic vinyl compound of formula I-0 or I, when R^{B-1} and R^{B-2} are independently 3- to 12-membered heteroaryl, the heteroatom of the 3- to 12-membered heteroaryl is N, the number of the heteroatom is 1, and other variables are defined as in any one of the embodiments of the present disclosure.

In some embodiments, in the aromatic vinyl compound of formula 1-0 or I, when R^{B-1} and R^{B-2} are independently 3- to 12-membered heteroaryl substituted by one or more than one R^{B-1-4}, the 3- to 12-membered heteroaryl is 5- to 7-membered heteroaryl, and other variables are defined as in any one of the embodiments of the present disclosure.

In some embodiments, in the aromatic vinyl compound of formula I-0 or I, when R^{B-1} and R^{B-2} are independently 3- to 12-membered heteroaryl substituted by one or more than one R^{B-1-4}, the heteroatom of the 3- to 12-membered heteroaryl is N, the number of the heteroatom is 1, and other variables are defined as in any one of the embodiments of the present disclosure.

In some embodiments, in the aromatic vinyl compound of formula I-0 or I, when R^{B-1} and R^{B-2} are independently 3- to 12-membered heteroaryl substituted by one or more than one R^{B-1-4}, each R^{B-1-4} is the same or different, and the more than one is 2, 3, 4 or 5, and other variables are defined as in any one of the embodiments of the present disclosure.

In some embodiments, in the aromatic vinyl compound of formula I-0 or I, when R^{B-1-3} and R^{B-1-4} are independently halogen, the halogen is fluorine, chlorine, bromine or iodine, for example fluorine or iodine, and other variables are defined as in any one of the embodiments of the present disclosure.

In some embodiments, in the aromatic vinyl compound of formula I-0 or I, when R^{B-1} and R^{B-2} are independently C₁-C₄ alkoxy, the C₁-C₄ alkoxy is methoxy, ethoxy, n-propoxy or n-butoxy, for example methoxy, and other variables are defined as in any one of the embodiments of the present disclosure.

In some embodiments, in the aromatic vinyl compound of formula I-0 or I, when R^{B-1} and R^{B-2} are independently C₁-C₄ alkoxy substituted by one or more than one deuterium, the C₁-C₄ alkoxy substituted by one or more than one deuterium is C₁-C₂ alkoxy substituted by one or more than one deuterium, for example monodeuterated methoxy, dideuterated methoxy, trideuterated methoxy, monodeuterated ethoxy, dideuterated ethoxy, trideuterated ethoxy, tetradeuterium ethoxy or pentadeuterium ethoxy, for example trideuterated methoxy, and other variables are defined as in any one of the embodiments of the present disclosure.

In some embodiments, in the aromatic vinyl compound of formula I-0 or I, when R^{B-1-1} and R^{B-1-2} are independently C₁-C₄ alkyl, the C₁-C₄ alkyl is methyl, ethyl, *n*-propyl, isopropyl, *n*-butyl, isobutyl or *tert*-butyl, for example methyl, ethyl or isopropyl, and other variables are defined as in any one of the embodiments of the present disclosure.

In some embodiments, in the aromatic vinyl compound of formula I-0 or I, when R^{B-1-1} and R^{B-1-2} are independently C₁-C₄ alkyl substituted by one or more than one R^{B-1-1-1}, the C₁-C₄ alkyl is methyl, ethyl, *n*-propyl, isopropyl, *n*-butyl, isobutyl or *tert*-butyl, for example methyl, ethyl or isopropyl, and other variables are defined as in any one of the embodiments of the present disclosure.

In some embodiments, in the aromatic vinyl compound of formula I-0 or I, when R^{B-1-1} and R^{B-1-2} are independently C₁-C₄ alkyl substituted by one or more than one R^{B-1-1-1}, each R^{B-1-1-1} is the same or different, and the more than one is 2, 3, 4 or 5, and other variables are defined as in any one of the embodiments of the present disclosure.

In some embodiments, in the aromatic vinyl compound of formula I-0 or I, when R^{B-1-1}, R^{B-1-2} together with the nitrogen atom to which they are attached form the 5- to 7-membered carbon heterocycle, in the carbon heterocycle, the heteroatom is N and/or O, and other variables are defined as in any one of the embodiments of the present disclosure.

In some embodiments, in the aromatic vinyl compound of formula I-0 or I, when R^{B-1-1}, R^{B-1-2} together with the nitrogen atom to which they are attached form the 5- to 7-membered carbon heterocycle, in the carbon heterocycle, the number of the heteroatom is 1 or 2, and other variables are defined as in any one of the embodiments of the present disclosure.

In some embodiments, in the aromatic vinyl compound of formula I-0 or I, when R^{B-1-1}, R^{B-1-2} together with the nitrogen atom to which they are attached form the carbon heterocycle substituted by one or more than one R^{B-1-1-2}, in the carbon heterocycle, the heteroatom is N and/or O, and other variables are defined as in any one of the embodiments of the present disclosure.

In some embodiments, in the aromatic vinyl compound of formula I-0 or I, when R^{B-1-1}, R^{B-1-2} together with the nitrogen atom to which they are attached form the carbon heterocycle substituted by one or more than one R^{B-1-1-2}, in the carbon heterocycle, the number of the heteroatom is 1 or 2, and other variables are defined as in any one of the embodiments of the present disclosure.

In some embodiments, in the aromatic vinyl compound of formula I-0 or I, when R^{B-1-1}, R^{B-1-2} together with the nitrogen atom to which they are attached form the carbon heterocycle substituted by one or more than one R^{B-1-1-2}, each R^{B-1-1-2} is the same or different, and the more than one is 2, 3, 4 or 5, and other variables are defined as in any one of the embodiments of the present disclosure.

In some embodiments, in the aromatic vinyl compound of formula I-0 or I, when R^{B-1-1-2} is C₁-C₄ alkyl, the C₁-C₄ alkyl is methyl, ethyl, *n*-propyl, isopropyl, *n*-butyl, isobutyl or *tert*-butyl, for example methyl, and other variables are defined as in any one of the embodiments of the present disclosure.

In some embodiments, in the aromatic vinyl compound of formula I-0 or I, when R^{B-1-1-2} is C₁-C₄ alkyl substituted by one or more than one deuterium, the C₁-C₄ alkyl substituted by one or more than one deuterium is C₁-C₂ alkyl substituted by one or more than one deuterium, for example monodeuterated methyl, dideuterated methyl, trideuterated methyl, monodeuterated ethyl, dideuterated ethyl, trideuterated ethyl, tetradeuterated ethyl or pentadeuterated ethyl, for another example trideuterated methyl, and other variables are defined as in any one of the embodiments of the present disclosure.

In some embodiments, in the aromatic vinyl compound of formula I-0 or I, when R^{B-1-1-2} is C₁-C₄ amido, the C₁-C₄ amido is 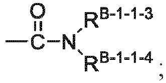 R^{B-1-1-3} and R^{B-1-1-4} are independently H, deuterium, C₁-C₄ alkyl or C₁-C₄ alkyl substituted by one or more than one deuterium; the C₁-C₄ alkyl is, for example, methyl, ethyl, *n*-propyl, isopropyl, *n*-butyl, isobutyl or *tert*-butyl*,* the C₁-C₄ alkyl substituted by one or more than one deuterium is, for example, trideuterated methyl, and other variables are defined as in any one of the embodiments of the present disclosure.

In some embodiments, in the aromatic vinyl compound of formula I-0 or I, when R^{A-1-1-2}, R^{B-1-1-5} and R^{B-1-1-6} are independently C₁-C₄ alkyl, the C₁-C₄ alkyl is methyl, ethyl, *n-*propyl, isopropyl, *n*-butyl, isobutyl or *tert*-butyl, for example methyl, and other variables are defined as in any one of the embodiments of the present disclosure.

In some embodiments, in the aromatic vinyl compound of formula 1-0, when R¹⁵ and R¹⁶ are independently halogen, the halogen is fluorine, chlorine, bromine or iodine, for example fluorine or bromine, and other variables are defined as in any one of the embodiments of the present disclosure.

In some embodiments, in the aromatic vinyl compound of formula I-0 or I, R³, R⁶, R¹², R¹³ and R¹⁴ are independently H, and other variables are defined as in any one of the embodiments of the present disclosure.

In some embodiments, in the aromatic vinyl compound of formula I-0 or I, R² is halogen, C₁-C₄ alkyl, C₁-C₄ alkyl substituted by one or more than one R^{A-1}, and other variables are defined as in any one of the embodiments of the present disclosure.

In some embodiments, in the aromatic vinyl compound of formula I-0 or I, R^{A-1} and R^{A-2} are independently hydroxyl, halogen, cyano, C₁-C₄ alkoxy, 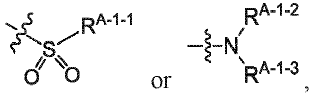 and other variables are defined as in any one of the embodiments of the present disclosure.

In some embodiments, in the aromatic vinyl compound of formula I-0 or I, R^{A-1} is halogen or 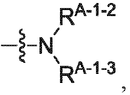 and other variables are defined as in any one of the embodiments of the present disclosure.

In some embodiments, in the aromatic vinyl compound of formula I-0 or I, R^{A-1} is halogen, and other variables are defined as in any one of the embodiments of the present disclosure.

In some embodiments, in the aromatic vinyl compound of formula I-0 or I, R^{A-1-1} is C₁-C₄ alkyl, and other variables are defined as in any one of the embodiments of the present disclosure.

In some embodiments, in the aromatic vinyl compound of formula I-0 or I, R^{A-1-2} and R^{A-1-3} are independently H or C₁-C₄ alkyl substituted by one or more than one R^{A-1-1-1}, and other variables are defined as in any one of the embodiments of the present disclosure.

In some embodiments, in the aromatic vinyl compound of formula I-0 or I, one of R^{A-1-2} and R^{A-1-3} is H, the other is C₁-C₄ alkyl substituted by one or more than one R^{A-1-1-1}, and other variables are defined as in any one of the embodiments of the present disclosure.

In some embodiments, in the aromatic vinyl compound of formula I-0 or I, R^{A-1-1-2}, R^{B-1-1-5} and R^{B-1-1-6} are independently H or C₁-C₄ alkyl, and other variables are defined as in any one of the embodiments of the present disclosure.

In some embodiments, in the aromatic vinyl compound of formula I-0 or I, R^{A-1-1-2}, R^{B-1-1-5} and R^{B-1-1-6} are independently H, and other variables are defined as in any one of the embodiments of the present disclosure.

In some embodiments, in the aromatic vinyl compound of formula I-0 or I, the C₁-C₄ alkyl substituted by one or more than one R^{A-1-1-1} is 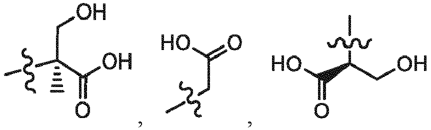 or 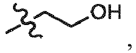 and other variables are defined as in any one of the embodiments of the present disclosure.

In some embodiments, in the aromatic vinyl compound of formula I-0 or I, the C₁-C₄ alkyl substituted by one or more than one R^{A-1-1-1} is 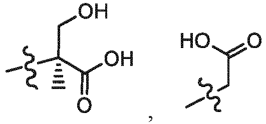 or 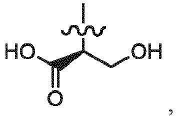 and other variables are defined as in any one of the embodiments of the present disclosure.

In some embodiments, in the aromatic vinyl compound of formula 1-0 or I, R⁴ and R⁵ are independently halogen, C₁-C₄ alkyl, C₁-C₄ alkyl substituted by one or more than one R^{B-1}, C₁-C₆ alkoxy or C₁-C₆ alkoxy substituted by one or more than one R^{B-2}, and other variables are defined as in any one of the embodiments of the present disclosure.

In some embodiments, in the aromatic vinyl compound of formula I-0 or I, R^{B-1} and R^{B-2} are independently deuterium, hydroxyl, cyano, C₆-C₁₀ aryl, C₆-C₁₀ aryl substituted by one or more than one R^{B-1-3}, 3- to 12-membered heteroaryl, 3- to 12-membered heteroaryl substituted by one or more than one R^{B-1-4}, C₁-C₄ alkoxy, C₁-C₄ alkoxy substituted by one or more than one deuterium, 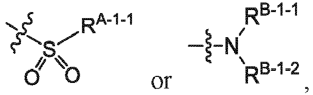 and other variables are defined as in any one of the embodiments of the present disclosure.

In some embodiments, in the aromatic vinyl compound of formula I-0 or I, R^{B-1} and R^{B-2} are independently deuterium, hydroxyl, cyano, C₁-C₄ alkoxy, C₁-C₂ alkoxy substituted by one or more than one deuterium, 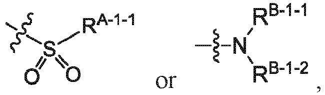 and other variables are defined as in any one of the embodiments of the present disclosure.

In some embodiments, in the aromatic vinyl compound of formula I-0 or I, R^{B-1} is hydroxyl, C₁-C₄ alkoxy, C₁-C₂ alkoxy substituted by one or more than one deuterium, or 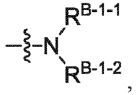 and other variables are defined as in any one of the embodiments of the present disclosure.

In some embodiments, in the aromatic vinyl compound of formula 1-0 or I, R^{B-1} is hydroxyl or 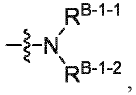 and other variables are defined as in any one of the embodiments of the present disclosure.

In some embodiments, in the aromatic vinyl compound of formula I-0 or I, R^{B-2} is deuterium, cyano, hydroxyl, C₆-C₁₀ aryl, C₆-C₁₀ aryl substituted by one or more than one R^{B-1-3}, 3- to 12-membered heteroaryl, 3- to 12-membered heteroaryl substituted by one or more than one R^{B-1-4} or C₁-C₄ alkoxy, and other variables are defined as in any one of the embodiments of the present disclosure.

In some embodiments, in the aromatic vinyl compound of formula I-0 or I, R^{B-2} is deuterium, cyano, hydroxyl or C₁-C₄ alkoxy, and other variables are defined as in any one of the embodiments of the present disclosure.

In some embodiments, in the aromatic vinyl compound of formula I-0 or I, R^{B-1-3} and R^{B-1-4} are independently cyano or halogen, and other variables are defined as in any one of the embodiments of the present disclosure.

In some embodiments, in the aromatic vinyl compound of formula 1-0 or I, the C₆-C₁₀ aryl substituted by one or more than one R^{B-1-3} is 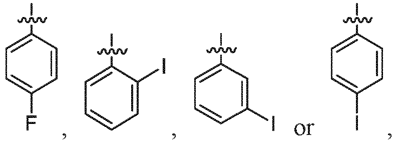 and other variables are defined as in any one of the embodiments of the present disclosure.

In some embodiments, in the aromatic vinyl compound of formula I-0 or I, the 3- to 12-membered heteroaryl is 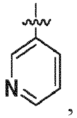 and other variables are defined as in any one of the embodiments of the present disclosure. In some embodiments, in the aromatic vinyl compound of formula I-0 or I, the 3- to 12-membered heteroaryl substituted by one or more than one R^{B-1-4} is 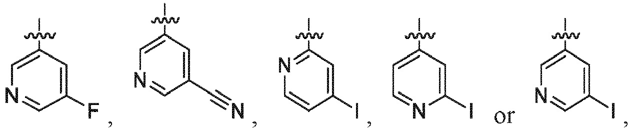 and other variables are defined as in any one of the embodiments of the present disclosure.

In some embodiments, in the aromatic vinyl compound of formula I-0 or I, R^{B-1-1} and R^{B-1-2} are independently H, C₁-C₄ alkyl substituted by one or more than one R^{B-1-1-1}, or, R^{B-1-1}, R^{B-1-2} together with the nitrogen atom to which they are attached form the 5- to 7-membered carbon heterocycle substituted by one or more than one R^{B-1-1-2}, and other variables are defined as in any one of the embodiments of the present disclosure.

In some embodiments, in the aromatic vinyl compound of formula I-0 or I, one of R^{B-1-1} and R^{B-1-2} is H, the other is C₁-C₄ alkyl substituted by one or more than one R^{B-1-1-1}; or, R^{B-1-1}, R^{B-1-2} together with the nitrogen atom to which they are attached form the 5- to 7-membered carbon heterocycle substituted by one or more than one R^{B-1-1-2}, and other variables are defined as in any one of the embodiments of the present disclosure.

In some embodiments, in the aromatic vinyl compound of formula 1-0 or I, the C₁-C₄ alkyl substituted by one or more than one R^{B-1-1-1} is 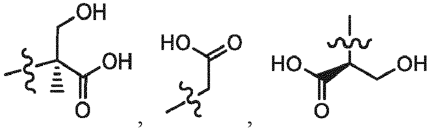 or 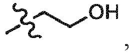 and other variables are defined as in any one of the embodiments of the present disclosure.

In some embodiments, in the aromatic vinyl compound of formula I-0 or I, the C₁-C₄ alkyl substituted by one or more than one R^{B-1-1-1} is 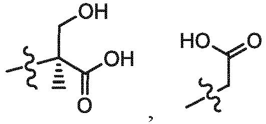 or 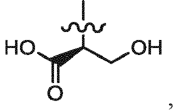 and other variables are defined as in any one of the embodiments of the present disclosure.

In some embodiments, in the aromatic vinyl compound of formula 1-0 or I, R^{B-1-1-2} is C₁-C₄ alkyl, COOR^{B-1-1-6} or C₁-C₄ amido, and other variables are defined as in any one of the embodiments of the present disclosure.

In some embodiments, in the aromatic vinyl compound of formula I-0 or I, R^{B-1-1-2} is methyl, carboxyl or -CONH₂, and other variables are defined as in any one of the embodiments of the present disclosure.

In some embodiments, in the aromatic vinyl compound of formula I-0 or I, the carbon heterocycle is 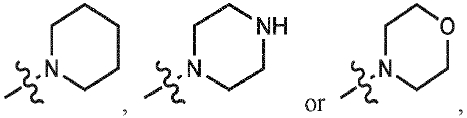 and other variables are defined as in any one of the embodiments of the present disclosure.

In some embodiments, in the aromatic vinyl compound of formula I-0 or I, the carbon heterocycle substituted by one or more than one R^{B-1-1-2} is 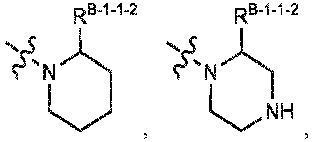 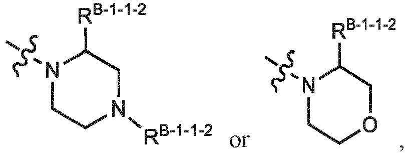 and other variables are defined as in any one of the embodiments of the present disclosure.

In some embodiments, in the aromatic vinyl compound of formula I-0 or I, the carbon heterocycle substituted by one or more than one R^{B-1-1-2} is 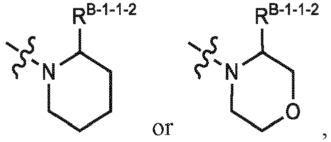 and other variables are defined as in any one of the embodiments of the present disclosure.

In some embodiments, in the aromatic vinyl compound of formula I-0 or I, R⁴ is 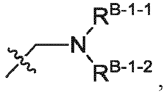 and other variables are defined as in any one of the embodiments of the present disclosure.

In some embodiments, in the aromatic vinyl compound of formula I-0 or I, R⁵ is halogen, C₁-C₄ alkyl, C₁-C₄ alkyl substituted by one or more than one R^{B-1}, C₁-C₆ alkoxy or C₁-C₆ alkoxy substituted by one or more than one R^{B-2}; R^{B-1} and R^{B-2} are independently deuterium, hydroxyl, cyano, C₁-C₄ alkoxy, C₁-C₂ alkoxy substituted by one or more than one deuterium or 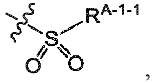 and other variables are defined as in any one of the embodiments of the present disclosure.

In some embodiments, in the aromatic vinyl compound of formula I-0 or I, 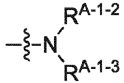 is 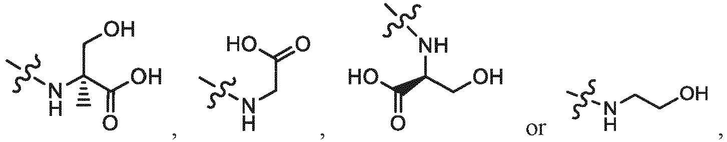 and other variables are defined as in any one of the embodiments of the present disclosure.

In some embodiments, in the aromatic vinyl compound of formula I-0 or I, 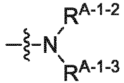 is 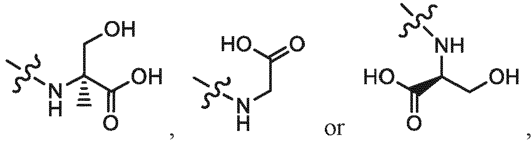 and other variables are defined as in any one of the embodiments of the present disclosure.

In some embodiments, in the aromatic vinyl compound of formula I-0 or I, 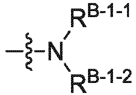 is 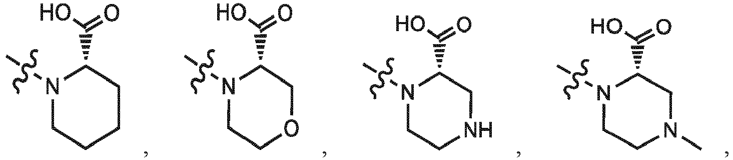 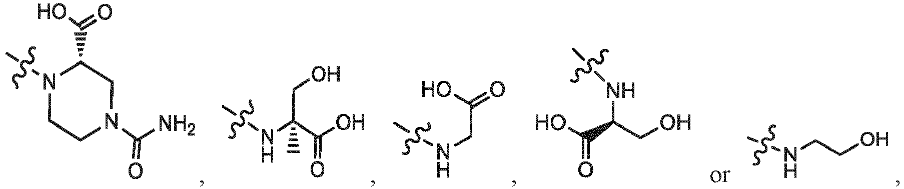 and other variables are defined as in any one of the embodiments of the present disclosure.

In some embodiments, in the aromatic vinyl compound of formula I-0 or I, 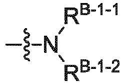 is

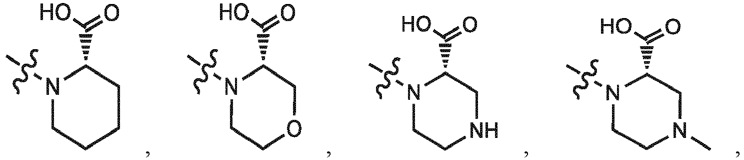

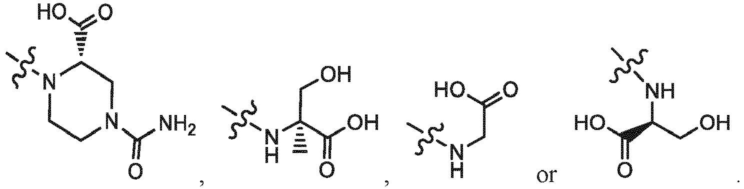

In some embodiments, in the aromatic vinyl compound of formula I-0, at least one of R¹⁵ and R¹⁶ is H or deuterium, and other variables are defined as in any one of the embodiments of the present disclosure.

In some embodiments, in the aromatic vinyl compound of formula I-0, R¹⁵ is H or deuterium; R¹⁶ is H, deuterium or halogen, and other variables are defined as in any one of the embodiments of the present disclosure.

In some embodiments, in the aromatic vinyl compound of formula 1-0, R¹ is halogen, C₁-C₄ alkyl substituted by one or more than one halogen, and other variables are defined as in any one of the embodiments of the present disclosure.

In some embodiments, in the aromatic vinyl compound of formula I-0, R¹⁵ is deuterium or halogen, and other variables are defined as in any one of the embodiments of the present disclosure.

In some embodiments, in the aromatic vinyl compound of formula I-0, R¹⁶ is deuterium or halogen, and other variables are defined as in any one of the embodiments of the present disclosure.

In some embodiments, the aromatic vinyl compound of formula I-0 or I is selected from one of the following schemes, scheme 1: R² is 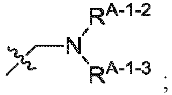 R⁴ and R⁵ are independently halogen, C₁-C₄ alkyl, C₁-C₄ alkyl substituted by one or more than one R^{B-1}, C₁-C₆ alkoxy or C₁-C₆ alkoxy substituted by one or more than one R^{B-2}; R^{B-1} and R^{B-2} are independently deuterium, hydroxyl, cyano, C₁-C₄ alkoxy, C₁-C₂ alkoxy substituted by one or more than one deuterium or 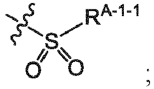 R³, R⁶, R¹², R¹³ and R¹⁴ are H;
scheme 2: R² is halogen, C₁-C₄ alkyl or C₁-C₄ alkyl substituted by one or more than one R^{A-1}; R^{A-1} is halogen; R⁴ is 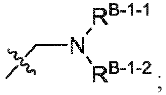 R⁵ is halogen, hydroxyl, C₁-C₄ alkyl, C₁-C₄ alkyl substituted by one or more than one R^{B-1}, C₁-C₆ alkoxy or C₁-C₆ alkoxy substituted by one or more than one R^{B-2}; R^{B-1} and R^{B-2} are independently deuterium, hydroxyl, cyano, C₁-C₄ alkoxy, C₁-C₂ alkoxy substituted by one or more than one deuterium or 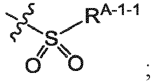 R³, R⁶ R¹², R¹³ and R¹⁴ are H.
scheme 3: R² is halogen, C₁-C₄ alkyl or C₁-C₄ alkyl substituted by one or more than one R^{A-1}; R^{A-1} is halogen; R⁴ is halogen, C₁-C₄ alkyl, C₁-C₄ alkyl substituted by one or more than one R^{B-1}, C₁-C₆ alkoxy or C₁-C₆ alkoxy substituted by one or more than one R^{B-2}; R^{B-1} and R^{B-2} are independently deuterium, hydroxyl, cyano, C₁-C₄ alkoxy, C₁-C₂ alkoxy substituted by one or more than one deuterium or 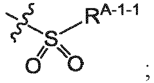 R⁵ is 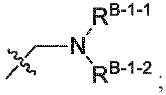 R³, R⁶, R¹², R¹³ and R¹⁴ are H;
scheme 4: R² is 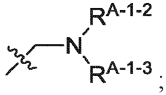 R⁴ and R⁵ are independently halogen, C₁-C₄ alkyl, C₁-C₄ alkyl substituted by one or more than one R^{B-1}, C₁-C₆ alkoxy or C₁-C₆ alkoxy substituted by one or more than one R^{B-2}; R^{B-1} and R^{B-2} are independently deuterium, hydroxyl, cyano, C₁-C₄ alkoxy, C₁-C₂ alkoxy substituted by one or more than one deuterium or 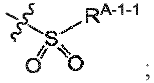 R3, R⁶, R¹², R¹³ and R¹⁴ are H; at least one of R¹⁵ and R¹⁶ is H or deuterium;
scheme 5: R² is halogen, C₁-C₄ alkyl or C₁-C₄ alkyl substituted by one or more than one R^{A-1}; R^{A-1} is halogen; R⁴ is 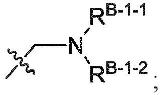 R⁵ is halogen, hydroxyl, C₁-C₄ alkyl, C₁-C₄ alkyl substituted by one or more than one R^{B-1}, C₁-C₆ alkoxy or C₁-C₆ alkoxy substituted by one or more than one R^{B-2}; R^{B-1} and R^{B-2} are independently deuterium, hydroxyl, cyano, C₁-C₄ alkoxy, C₁-C₂ alkoxy substituted by one or more than one deuterium or 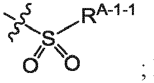 R³, R⁶, R¹², R¹³ and R¹⁴ are H; at least one of R¹⁵ and R¹⁶ is H or deuterium;
scheme 6: R² is halogen, C₁-C₄ alkyl or C₁-C₄ alkyl substituted by one or more than one R^{A-1}; R^{A-1} is halogen; R⁴ is halogen, C₁-C₄ alkyl, C₁-C₄ alkyl substituted by one or more than one R^{B-1}, C₁-C₆ alkoxy or C₁-C₆ alkoxy substituted by one or more than one R^{B-2}; R^{B-1} and R^{B-2} are independently deuterium, hydroxyl, cyano, C₁-C₄ alkoxy, C₁-C₂ alkoxy substituted by one or more than one deuterium or 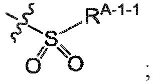 R⁵ is 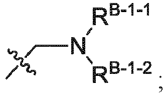 R³, R⁶, R¹², R¹³ and R¹⁴ are H; at least one of R¹⁵ and R¹⁶ is H or deuterium.

In some embodiments, in the aromatic vinyl compound of formula I-0, R¹ is cyano, CH₃, CD₃, Cl, CH₂F, C₁-C₄ alkyl substituted by one or more than one halogen, and other variables are defined as in any one of the embodiments of the present disclosure.

In some embodiments, in the aromatic vinyl compound of formula I-0, 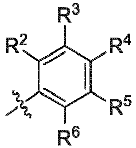 is 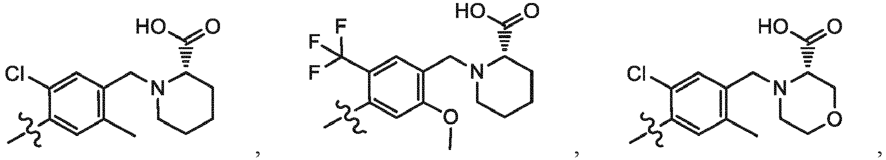 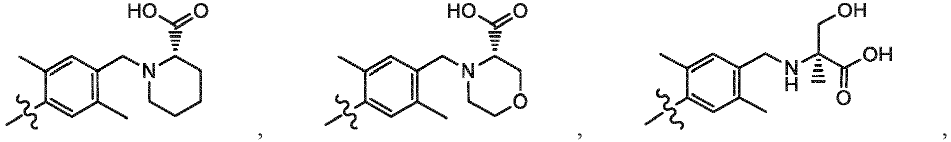 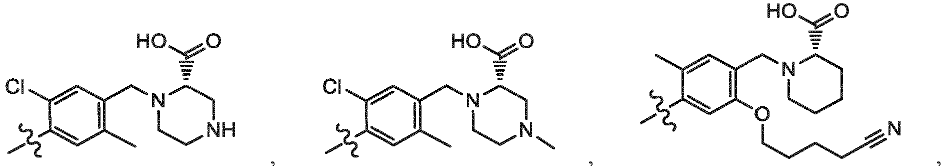 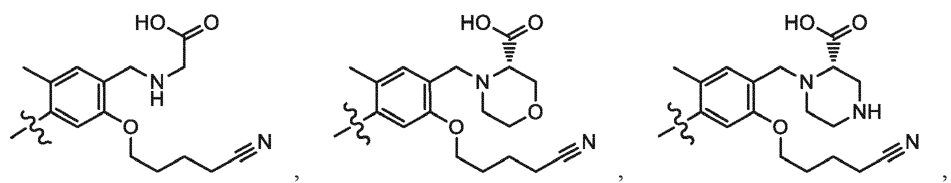 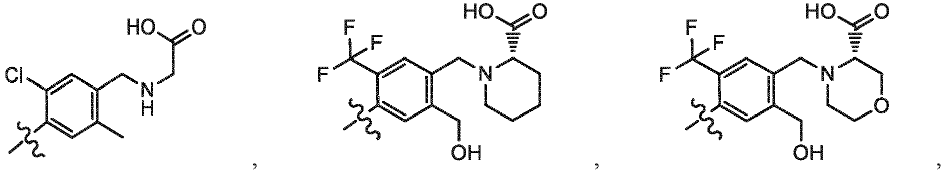 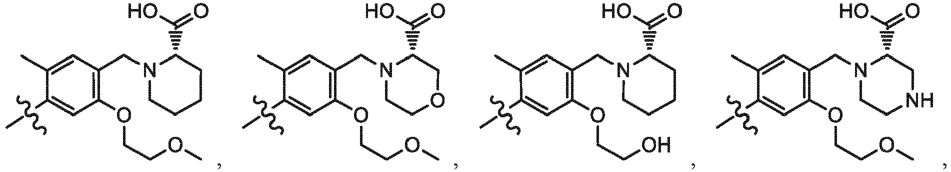 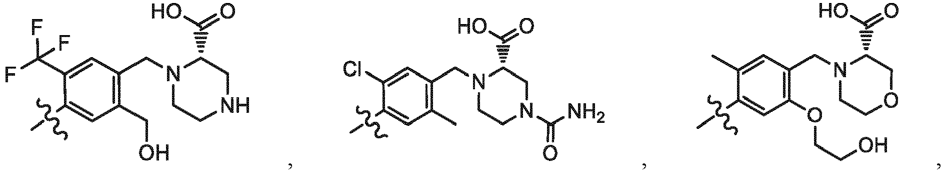 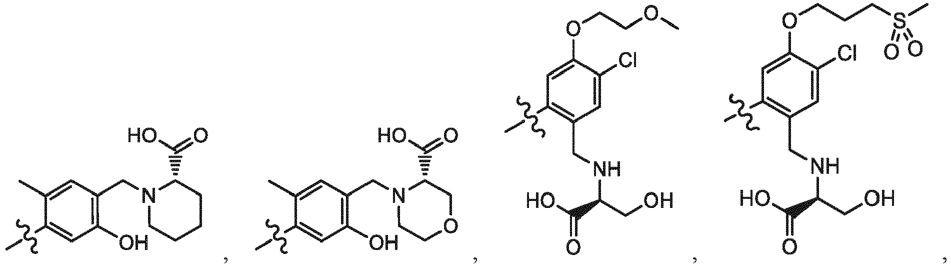 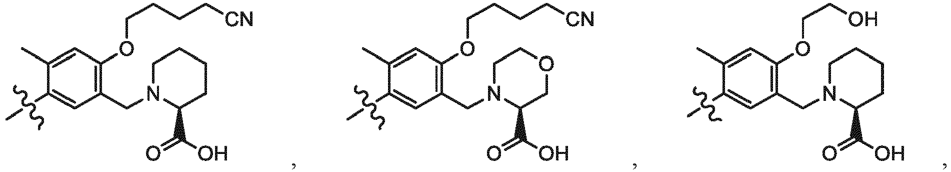 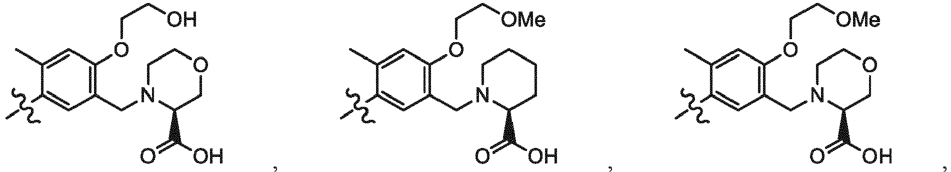 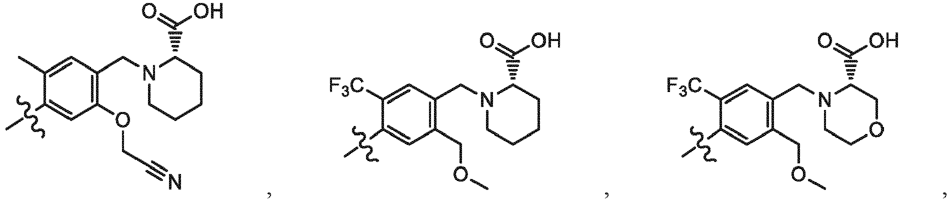 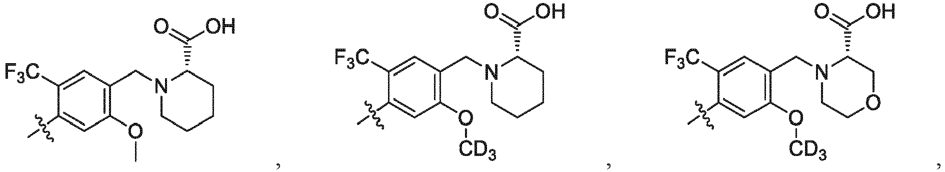 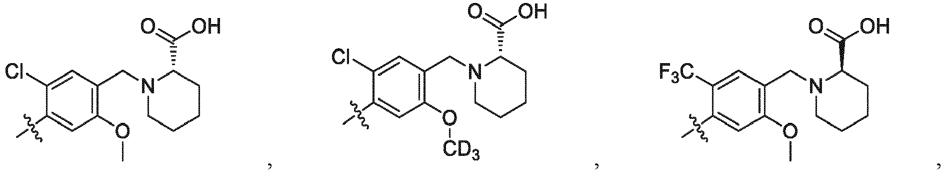 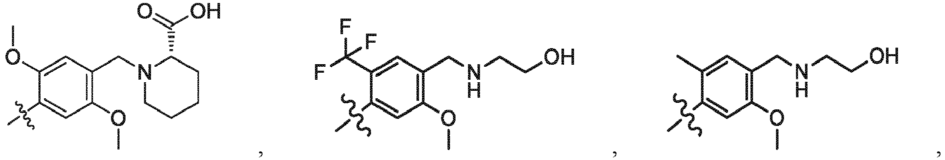 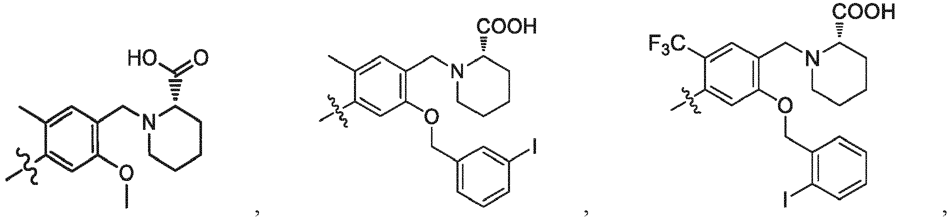 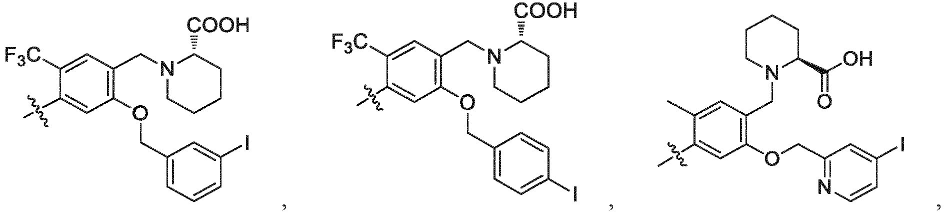 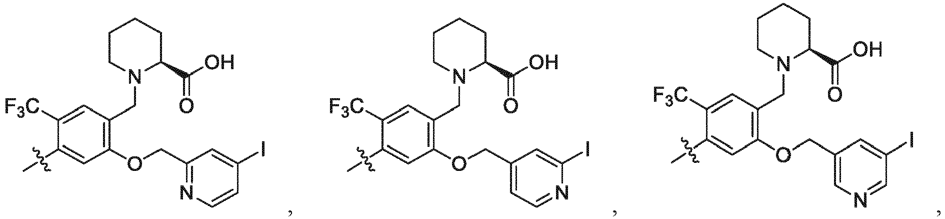 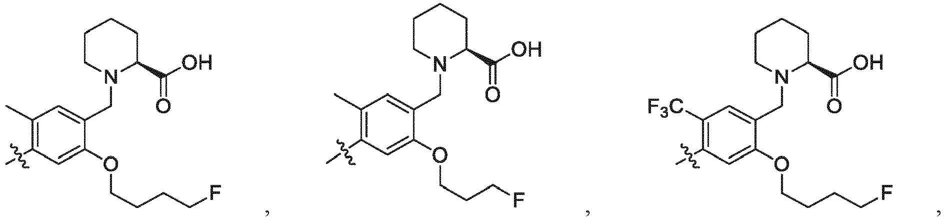 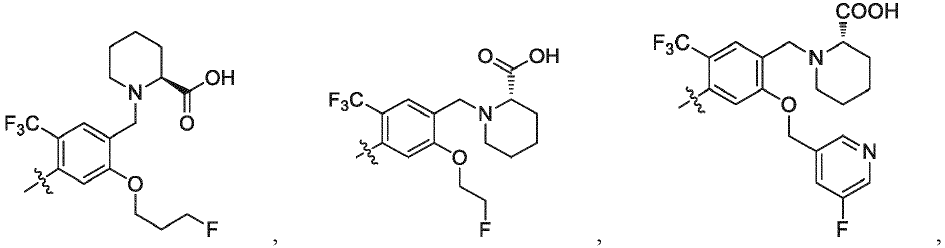 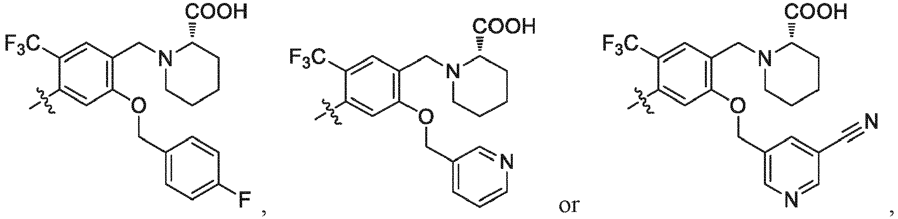 and other variables are defined as in any one of the embodiments of the present disclosure.

In some embodiments, in the aromatic vinyl compound of formula I-0, R¹⁵ and R¹⁶ are independently H, deuterium, Br or F, and other variables are defined as in any one of the embodiments of the present disclosure.

In some embodiments, the aromatic vinyl compound of formula I-0 or I is any one of the following compounds:

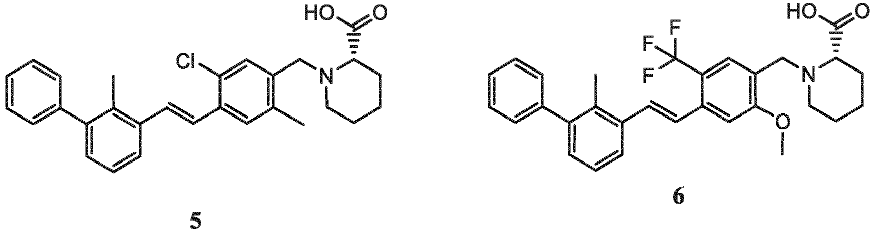

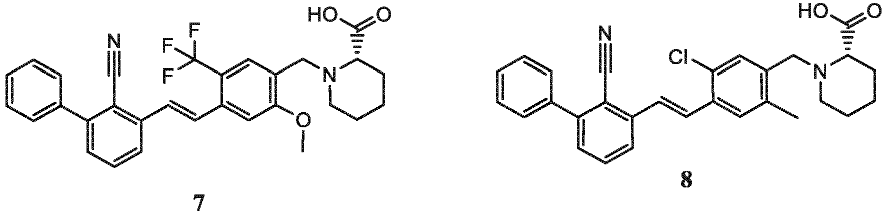

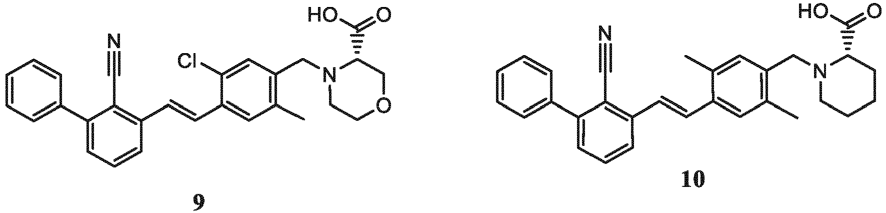

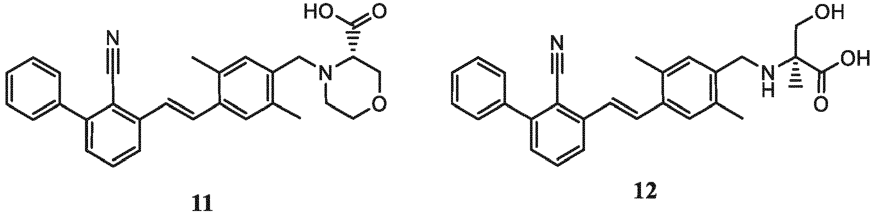

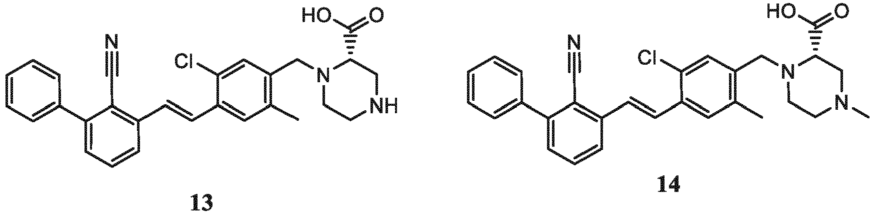

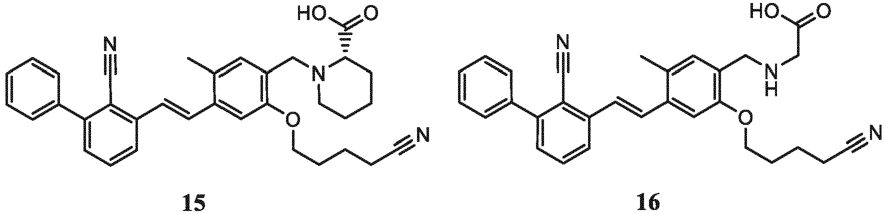

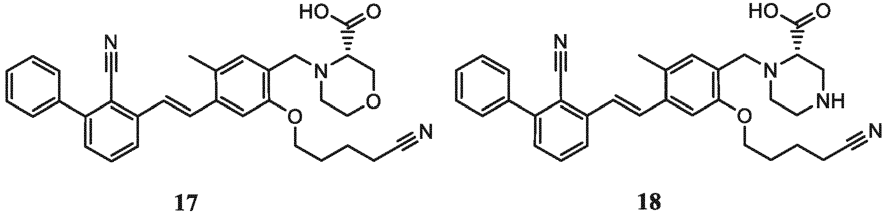

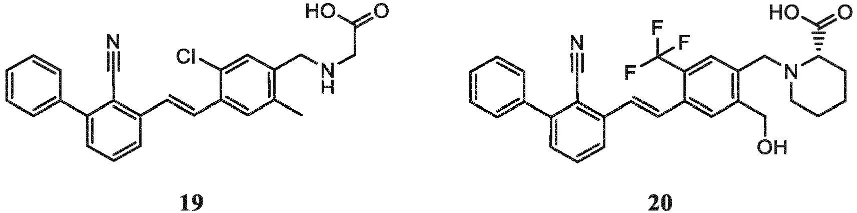

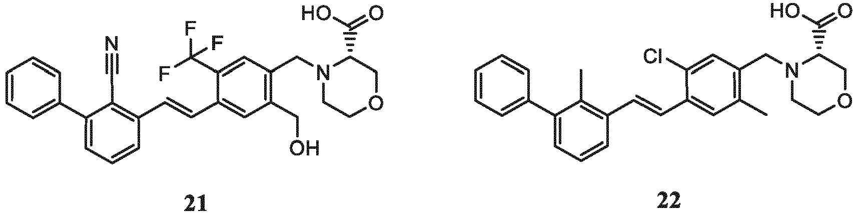

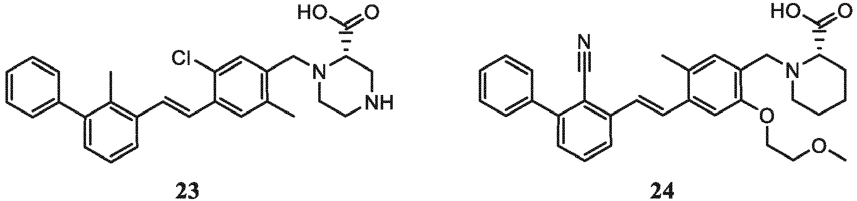

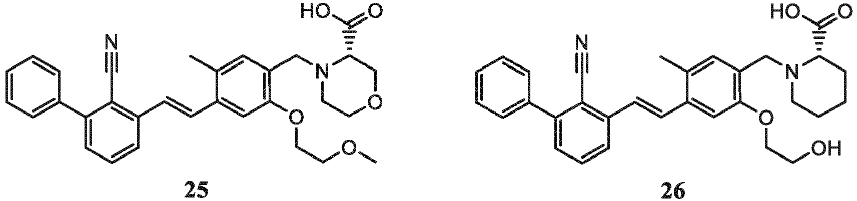

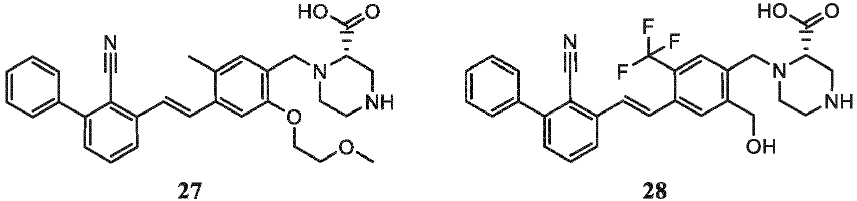

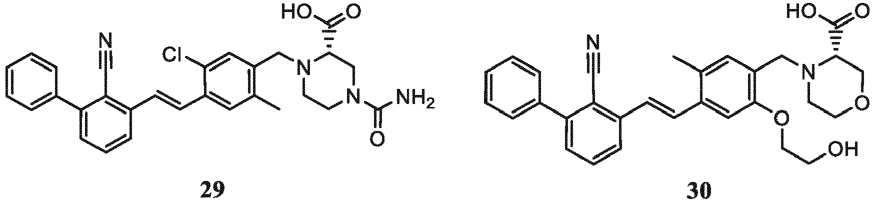

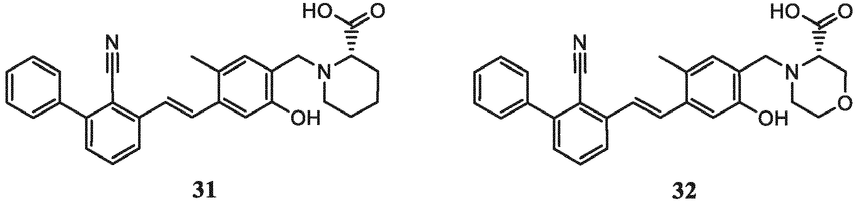

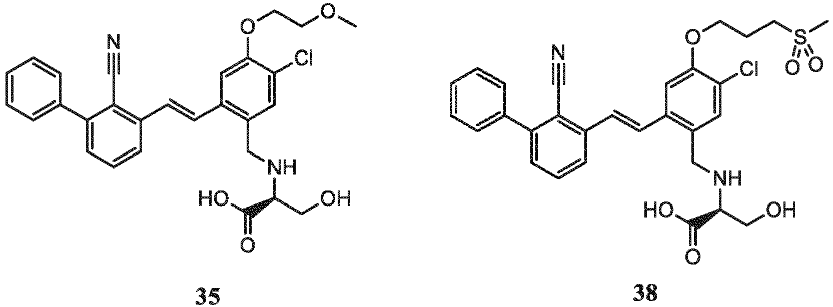

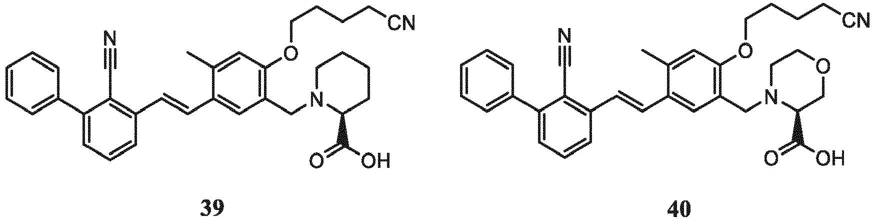

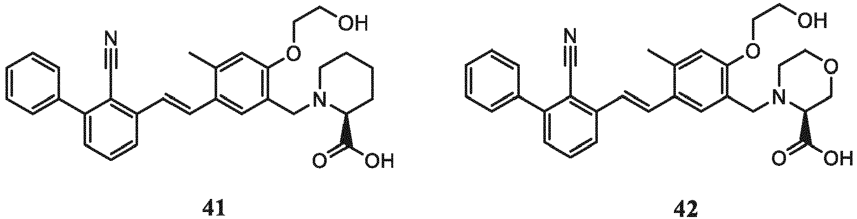

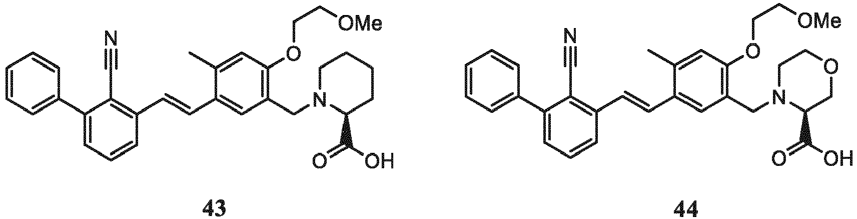

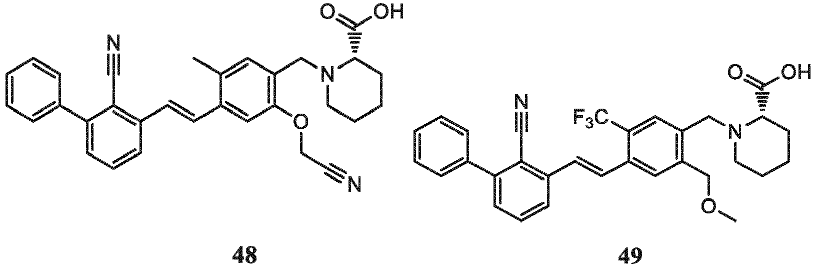

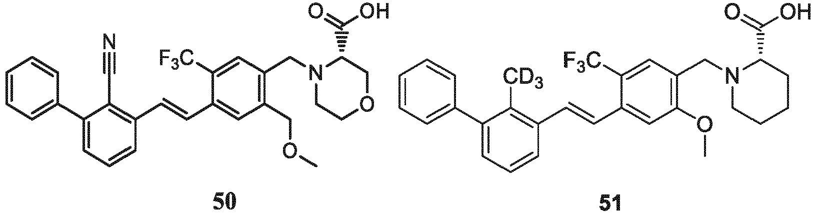

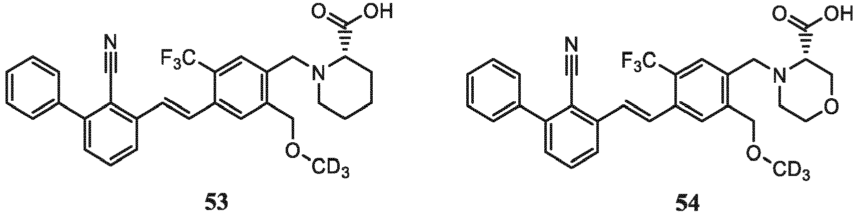

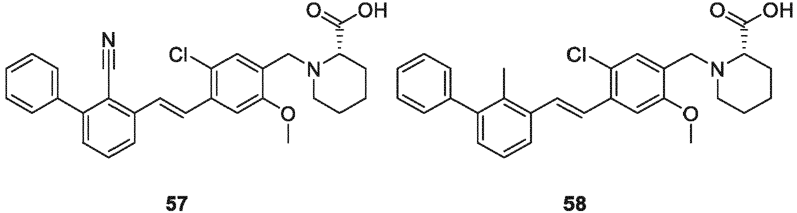

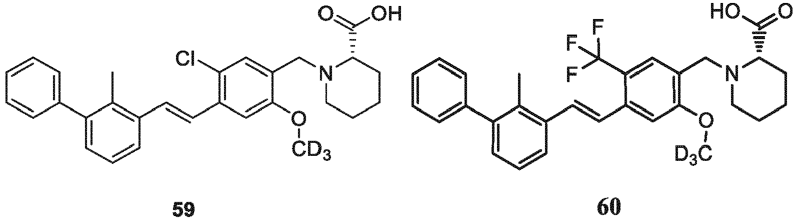

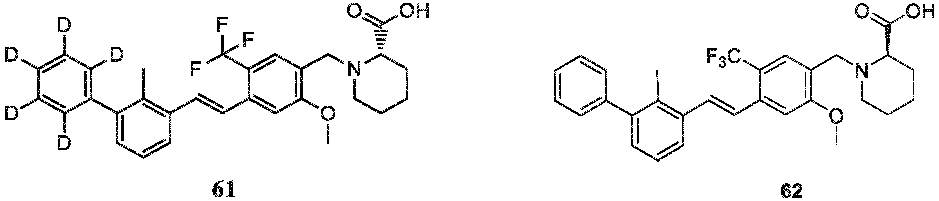

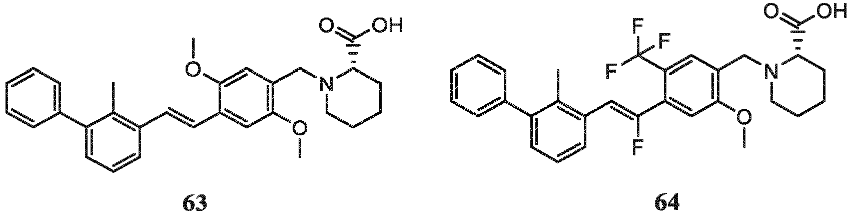

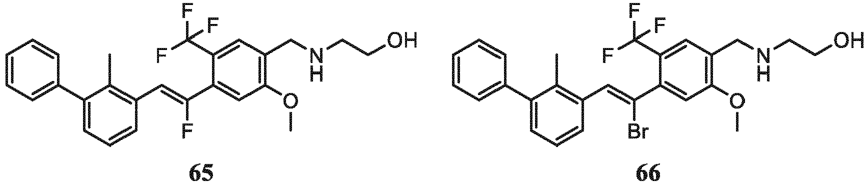

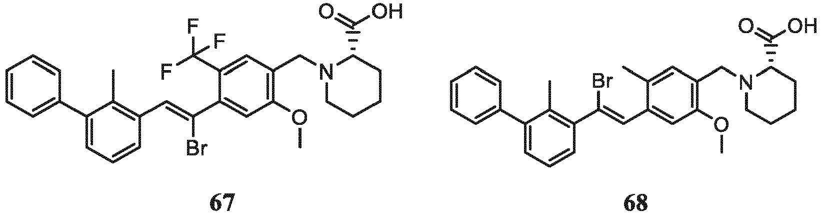

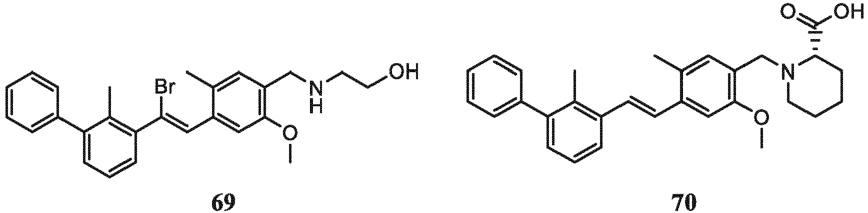

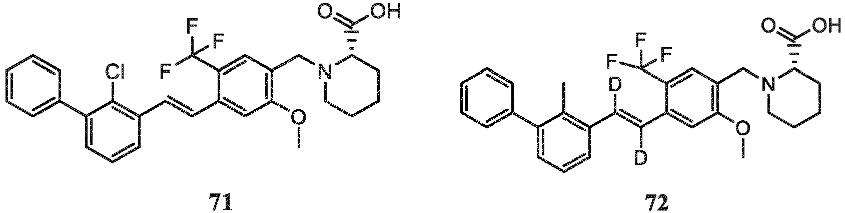

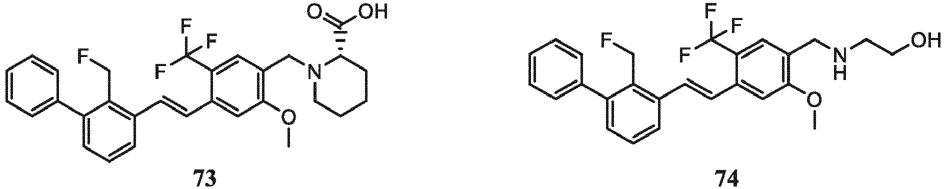

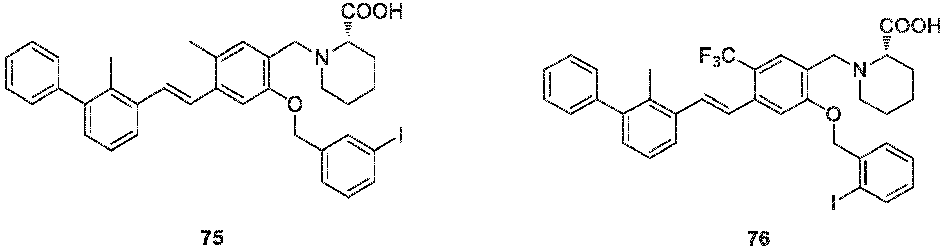

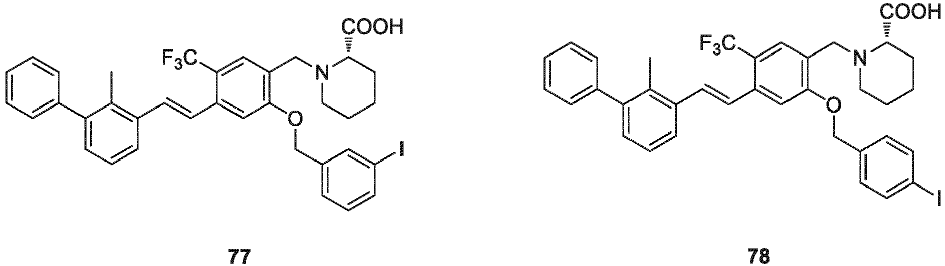

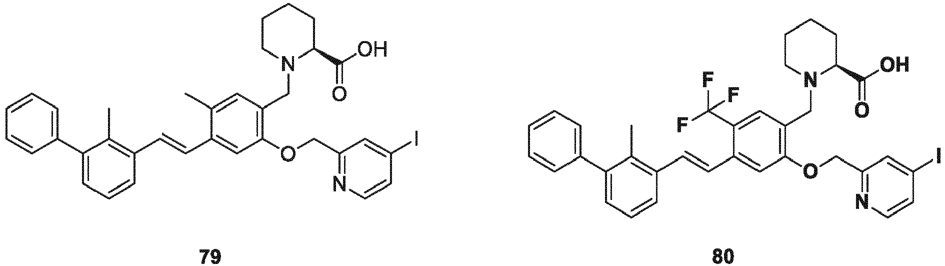

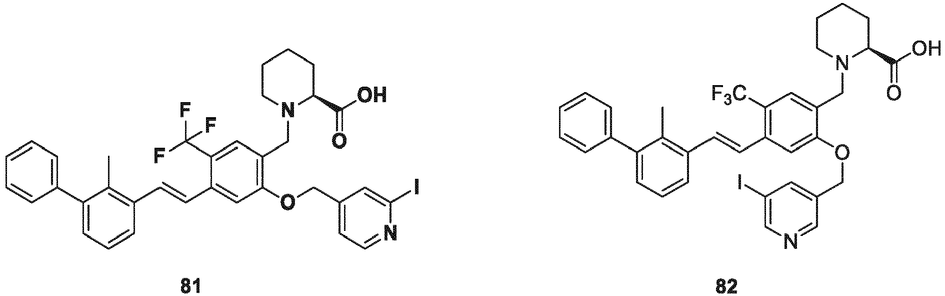

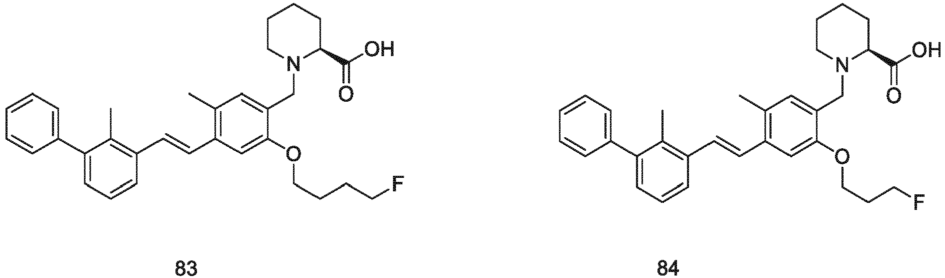

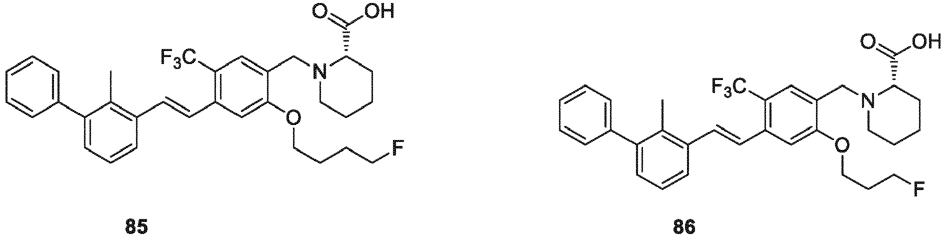

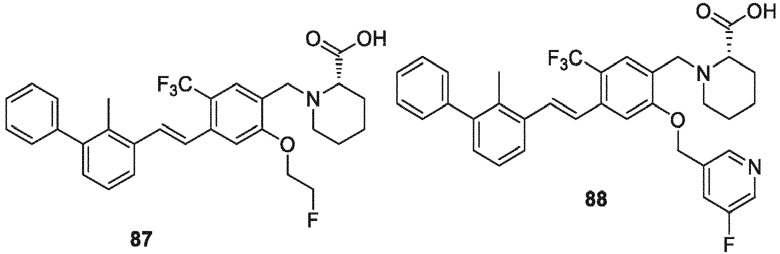

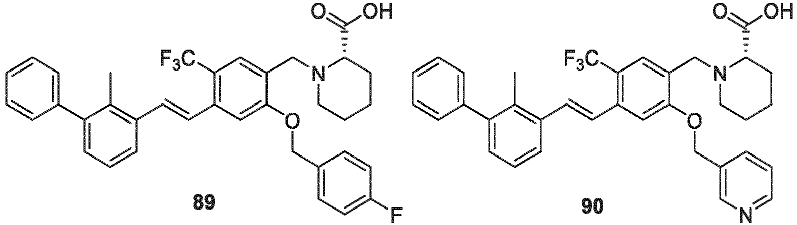

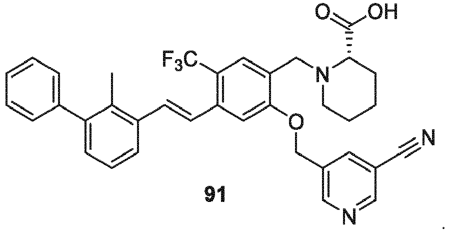

The present disclosure also provides a preparation method for the aromatic vinyl compound of formula I-0, the preparation method for the aromatic vinyl compound of formula I-0 comprises method 1, method 2, method 3, method 4, method 5 or method 6:
method 1 comprises the following steps: in a solvent, in the presence of a reducing agent, subjecting a compound of formula II-a-0 with a compound of formula III-a to a reductive amination reaction as shown below to give the aromatic vinyl compound of formula I-0; 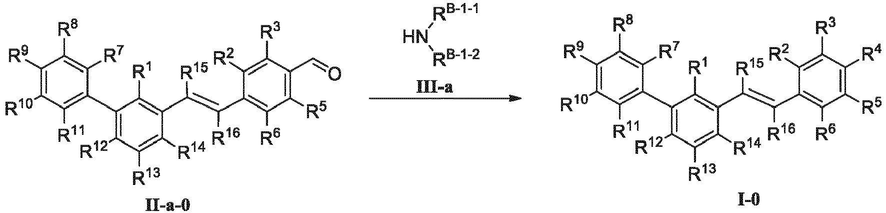
in method 1, R⁴ is 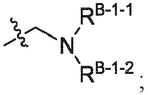 R¹, R², R³, R⁵, R⁶, R⁷, R⁸, R⁹, R¹⁰, R¹¹, R¹², R¹³, R¹⁴, R¹⁵, R¹⁶, R^{B-1-1} and R^{B-1-2} are defined as above;
method 2 comprises the following steps: in a solvent, in the presence of a base, subjecting a compound of formula II-b-0 with a compound of formula III-a to a substitution reaction as shown below to give the aromatic vinyl compound of formula I-0; 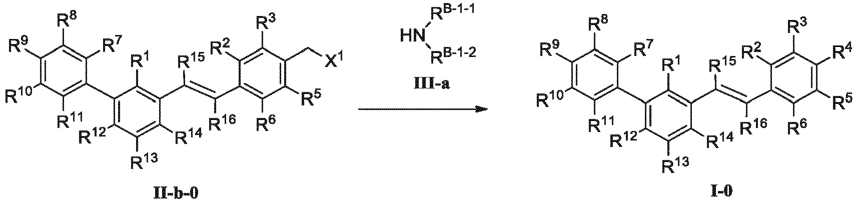
in method 2, R⁴ is 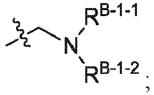 R¹, R², R³, R⁵, R⁶, R⁷, R⁸, R⁹, R¹⁰, R¹¹, R¹², R¹³, R¹⁴, R¹⁵, R¹⁶, R^{B-1-1} and R^{B-1-2} are defined as above, and X¹ is halogen;
method 3 comprises the following steps: in a solvent, in the presence of a reducing agent, subjecting a compound of formula II-c-0 with a compound of formula III-a to a reductive amination reaction as shown below to give the aromatic vinyl compound of formula I-0; 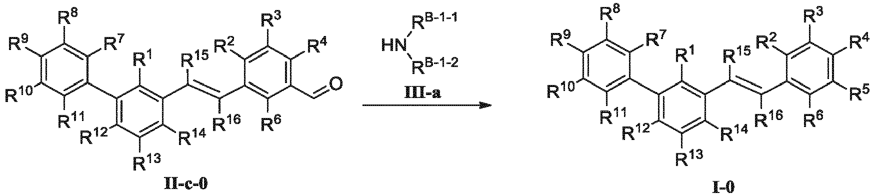
in method 3, R⁵ is 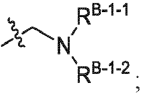 R¹, R², R³, R⁴, R⁶, R⁷, R⁸, R⁹, R¹⁰, R¹¹, R¹², R¹³, R¹⁴, R¹⁵, R¹⁶, R^{B-1-1} and R^{B-1-2} are defined as above;
method 4 comprises the following steps: in a solvent, in the presence of a base, subjecting a compound of formula II-d-0 with a compound of formula III-a to a substitution reaction as shown below to give the aromatic vinyl compound of formula I-0; 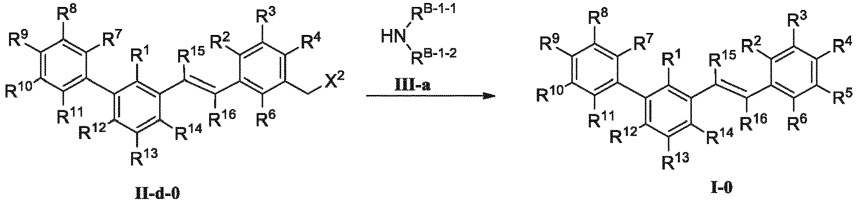
in method 4, R⁵ is 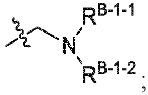 R¹, R², R³, R⁴, R⁶, R⁷, R⁸, R⁹, R¹⁰, R¹¹, R¹², R¹³, R¹⁴, R¹⁵, R¹⁶, R^{B-1-1} and R^{B-1-2} are defined as above, and X² is halogen;
method 5 comprises the following steps: in a solvent, in the presence of a reducing agent, subj ecting a compound of formula II-e-0 with a compound of formula III-b to a reductive amination reaction as shown below to give the aromatic vinyl compound of formula I-0; 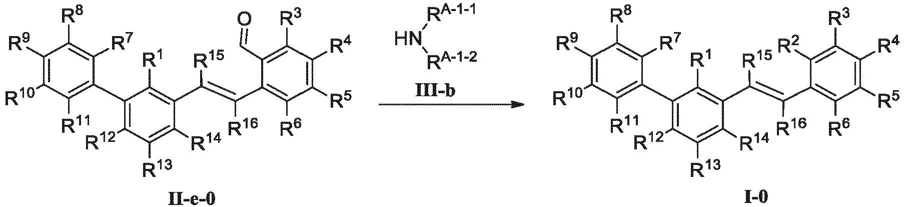
in method 5, R² is 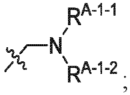 R¹, R³, R⁴ R⁵, R⁶, R⁷ R⁸, R⁹, R¹⁰, R¹¹, R¹², R¹³, R¹⁴, R¹⁵, R¹⁶, R^{A-1-1} and R^{A-1-2} are defined as above;
method 6 comprises the following steps: in a solvent, in the presence of a base, subjecting a compound of formula II-f-0 with a compound of formula III-b to a substitution reaction as shown below to give the aromatic vinyl compound of formula I-0; 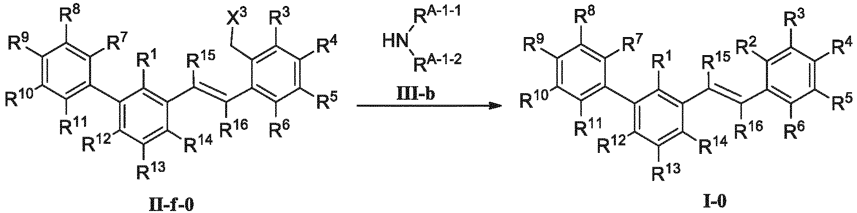
in method 6, R² is 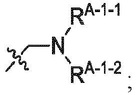 R¹, R³, R⁴, R⁵, R⁶, R⁷, R⁸, R⁹, R¹⁰, R¹¹, R¹², R¹³, R¹⁴, R¹⁵, R¹⁶, R^{A-1-1} and R^{A-1-2} are defined as above, and X³ is halogen.

The present disclosure also provides a preparation method for the aromatic vinyl compound of formula I, the preparation method for the aromatic vinyl compound of formula I comprises method I, method II, method III, method IV, method V or method VI:
method I comprises the following steps: in a solvent, in the presence of a reducing agent, subjecting a compound of formula II-a with a compound of formula III-a to a reductive amination reaction as shown below to give the aromatic vinyl compound of formula I; 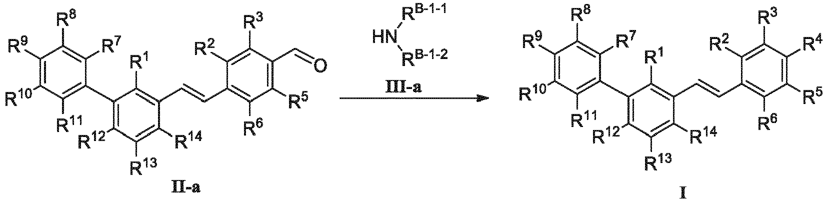
in method I, R⁴ is 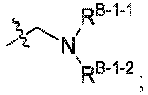 R¹, R², R³, R⁵, R⁶, R⁷, R⁸, R⁹, R¹⁰, R¹¹, R¹², R¹³, R¹⁴, R^{B-1-1} and R^{B-1-2} are defined as above;
method II comprises the following steps: in a solvent, in the presence of a base, subjecting a compound of formula II-b with a compound of formula III-a to a substitution reaction as shown below to give the aromatic vinyl compound of formula I; 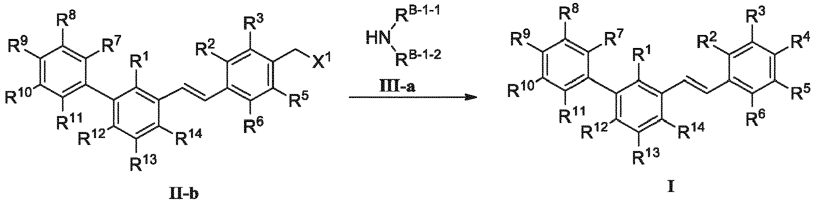
in method II, R⁴ is 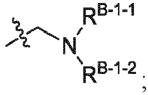 R¹, R², R³, R⁵, R⁶, R⁷, R⁸, R⁹, R¹⁰, R¹¹, R¹², R¹³, R¹⁴, R^{B-1-1} and R^{B-1-2} are defined as above, and X¹ is halogen;
method III comprises the following steps: in a solvent, in the presence of a reducing agent, subjecting a compound of formula II-c with a compound of formula III-a to a reductive amination reaction as shown below to give the aromatic vinyl compound of formula I; 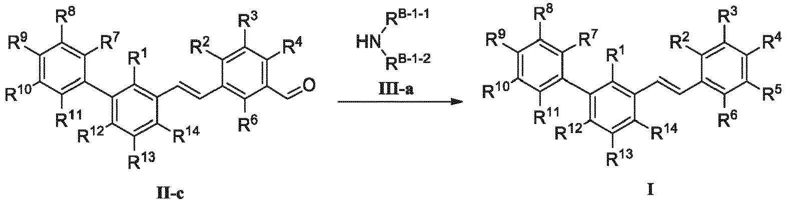
in method III, R⁵ is 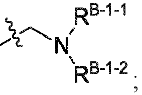 R¹, R², R³, R⁴, R⁶, R⁷, R⁸, R⁹, R¹⁰, R¹¹, R¹², R¹³, R¹⁴, R^{B-1-1} and R^{B-1-2} are defined as above;
method IV comprises the following steps: in a solvent, in the presence of a base, subjecting a compound of formula II-d with a compound of formula III-a to a substitution reaction as shown below to give the aromatic vinyl compound of formula I; 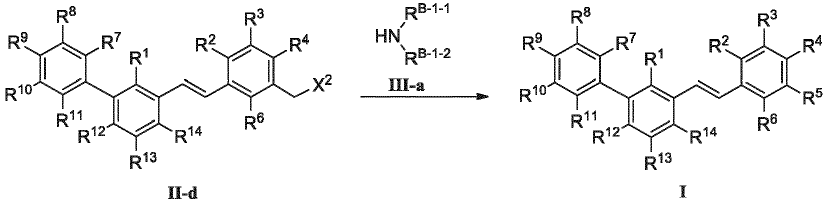
in method IV, R⁵ is 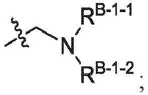 R¹, R², R³, R⁴, R⁶, R⁷, R⁸, R⁹, R¹⁰, R¹¹, R¹², R¹³, R¹⁴, R^{B-1-1} and R^{B-1-2} are defined as above, and X² is halogen;
method V comprises the following steps: in a solvent, in the presence of a reducing agent, subjecting a compound of formula II-e with a compound of formula III-b to a reductive amination reaction as shown below to give the aromatic vinyl compound of formula I; 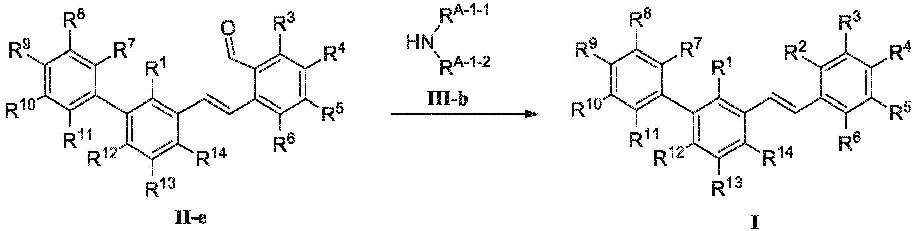
in method V, R² is 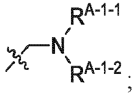 R¹, R³, R⁴, R⁵, R⁶, R⁷, R⁸, R⁹, R¹⁰, R¹¹, R¹², R¹³, R¹⁴, R^{A-1-1} and R^{A-1-2} are defined as above;
method VI comprises the following steps: in a solvent, in the presence of a base, subjecting a compound of formula II-f with a compound of formula III-b to a substitution reaction as shown below to give the aromatic vinyl compound of formula I; 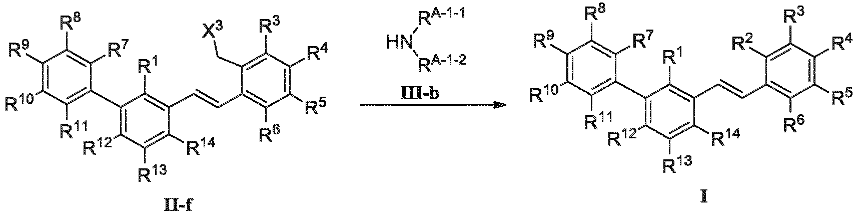
in method VI, R² is 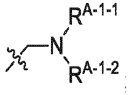 R¹, R³, R⁴, R⁵, R⁶, R⁷, R⁸, R⁹, R¹⁰, R¹¹, R¹², R¹³, R¹⁴, R^{A-1-1} and R^{A-1-2} are defined as above, and X³ is halogen.

In method 1, method 3, method 5, method I, method III or method V, the methods and conditions for the reductive amination reaction may be conventional methods and conditions for such reactions in the art.

In method 2, method 4, method 6, method II, method IV or method VI, the methods and conditions for the substitution reaction may be conventional methods and conditions for such reactions in the art.

The present disclosure also provides a compound of formula II-a-0, II-b-0, II-c-0, II-d-0, II-e-0 or II-f-0, 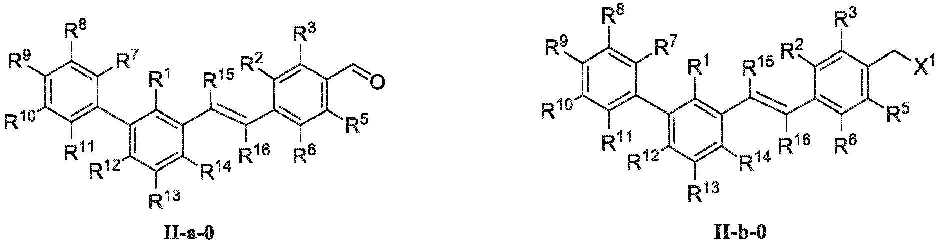 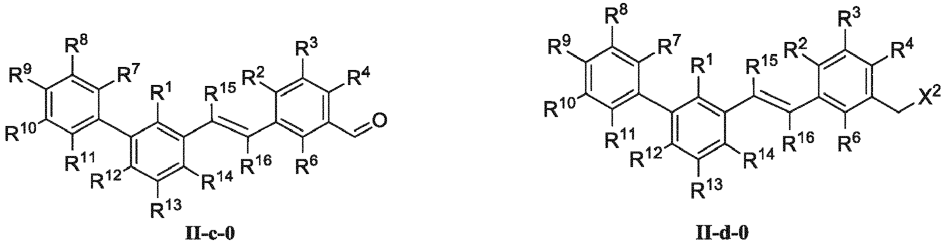 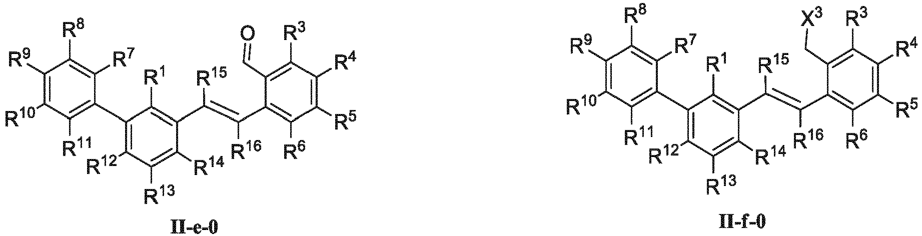 in the compound of the above formulas, R¹, R², R³, R⁴, R⁵, R⁶, R⁷, R⁸, R⁹, R¹⁰, R¹¹, R¹², R¹³, R¹⁴, R¹⁵ and R¹⁶ are defined as above, and X¹, X² and X³ are independently halogen.

The present disclosure also provides a compound of formula II-a, II-b, II-c, II-d, II-e or II-f, 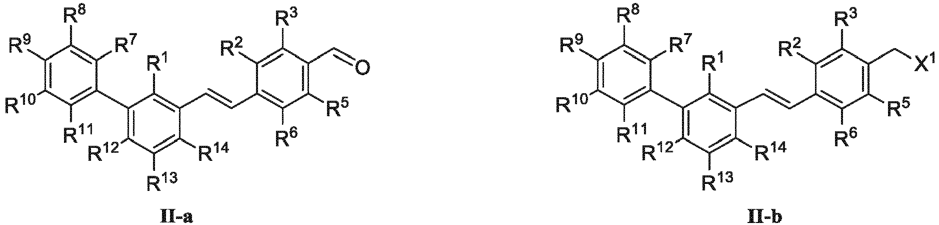 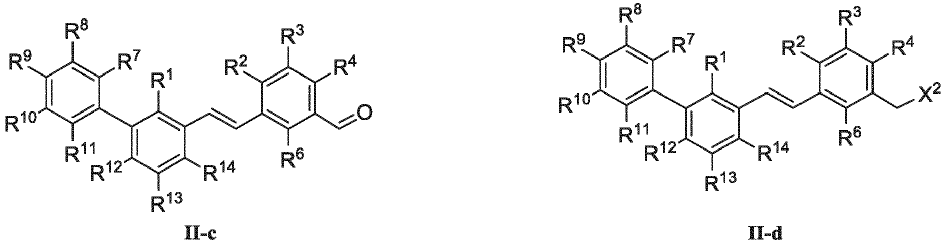 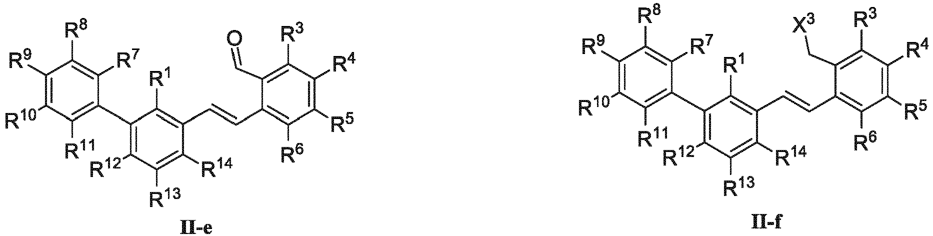 in the compound of the above formulas, R¹, R², R³, R⁴, R⁵, R⁶, R⁷, R⁸, R⁹, R¹⁰, R¹¹, R¹², R¹³ and R¹⁴ are defined as above, and X¹, X² and X³ are independently halogen.

In some embodiments, the compound of formula II-a-0 or II-a is any one of the following compounds:

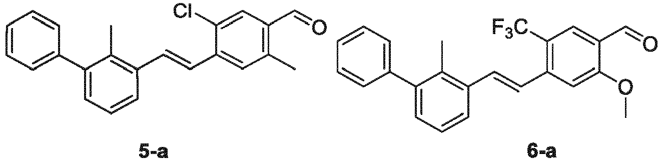

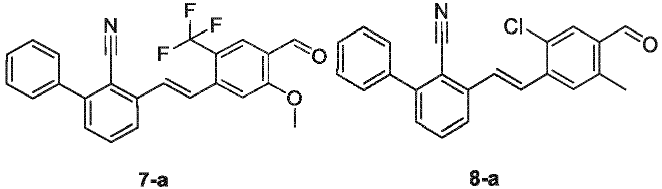

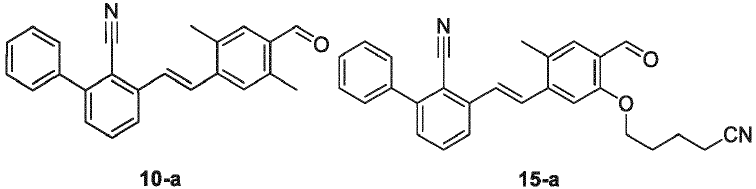

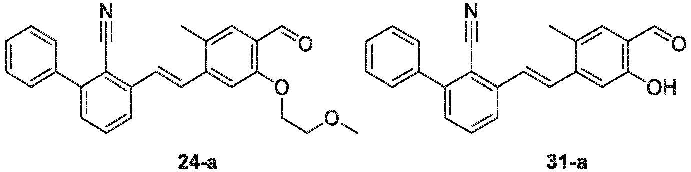

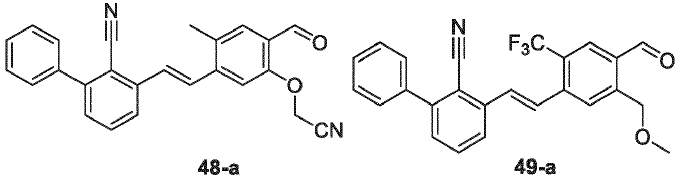

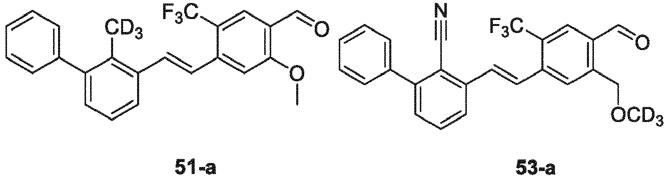

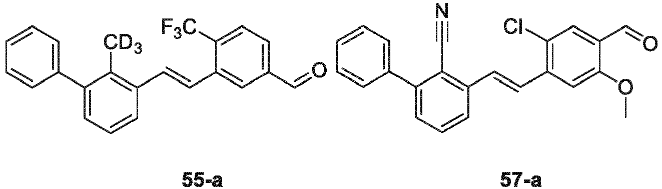

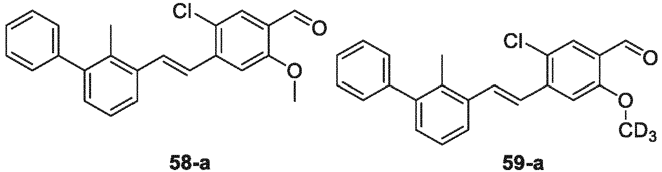

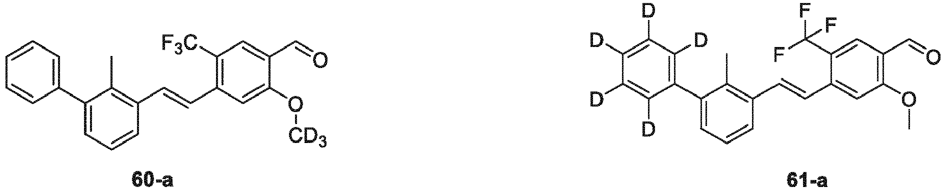

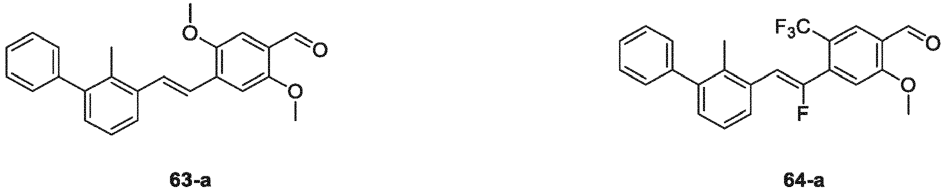

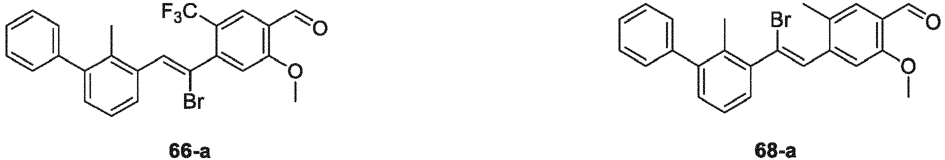

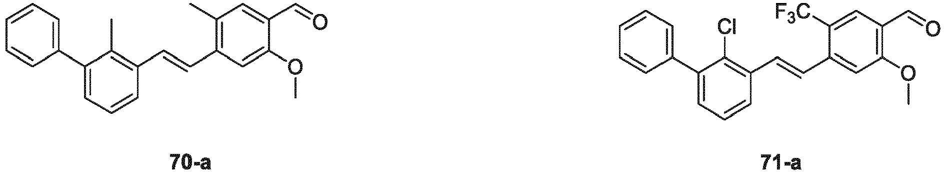

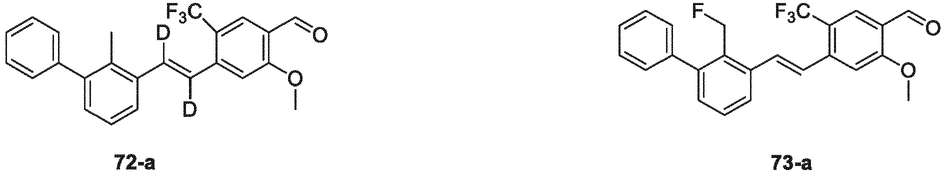

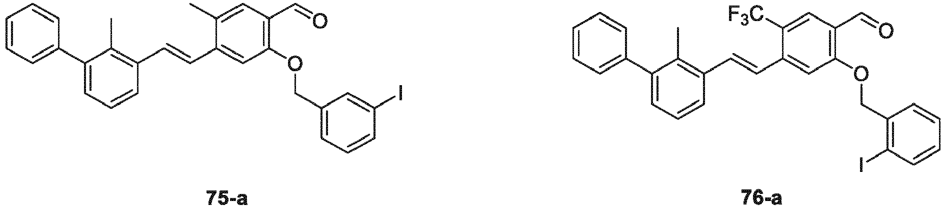

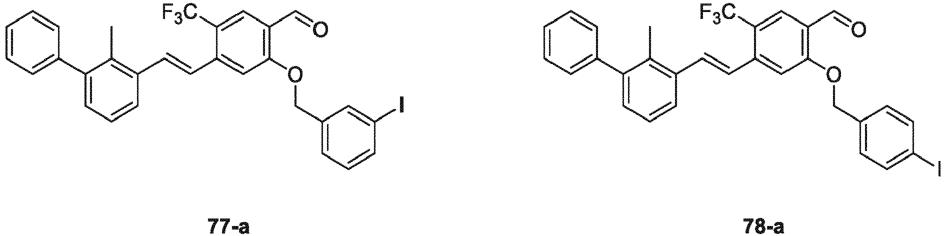

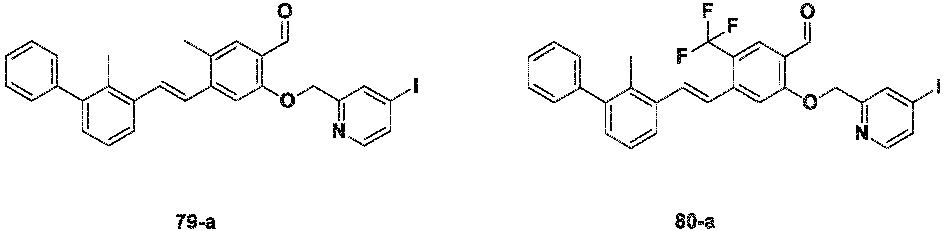

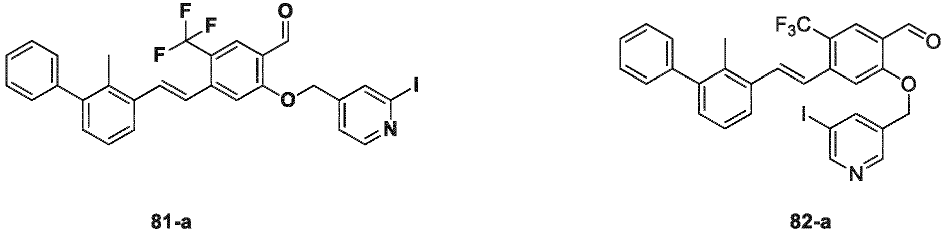

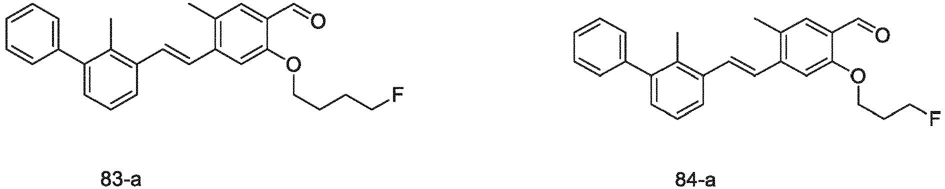

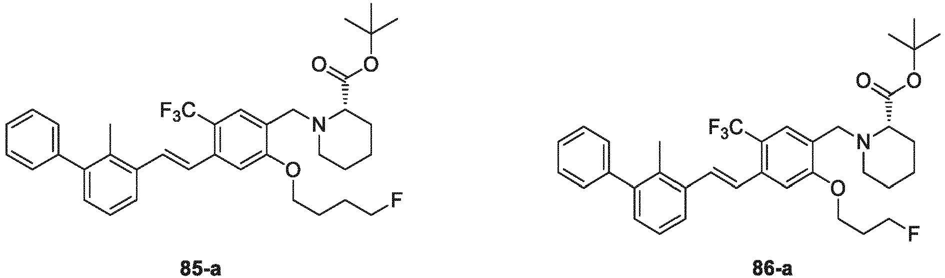

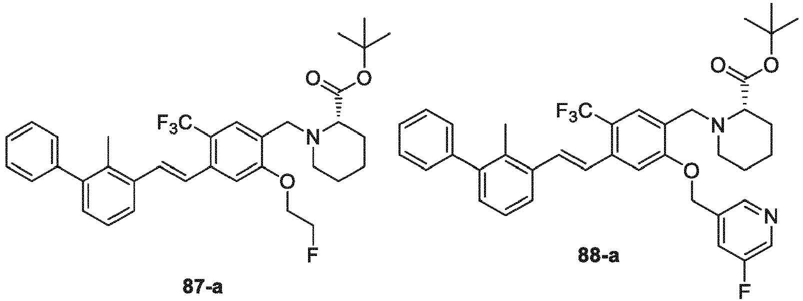

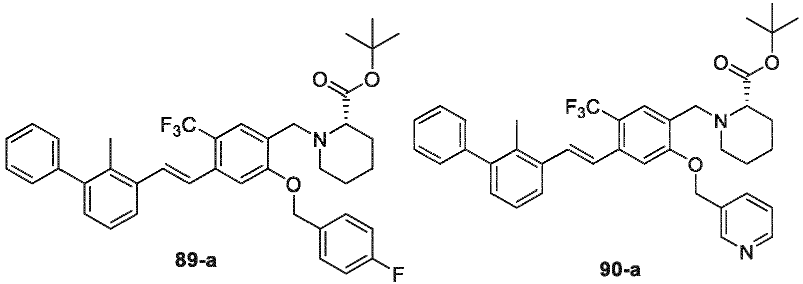

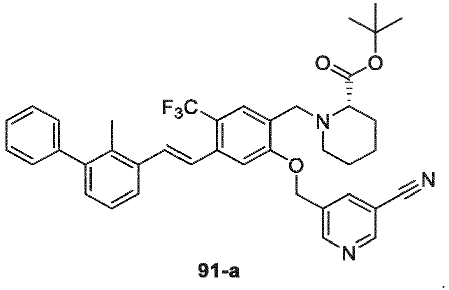

In some embodiments, the compound of formula II-b is the following compound:

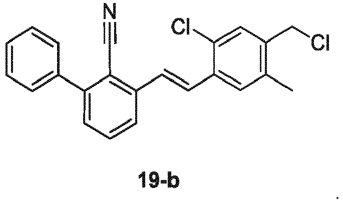

In some embodiments, the compound of formula II-c is any one of the following compounds:

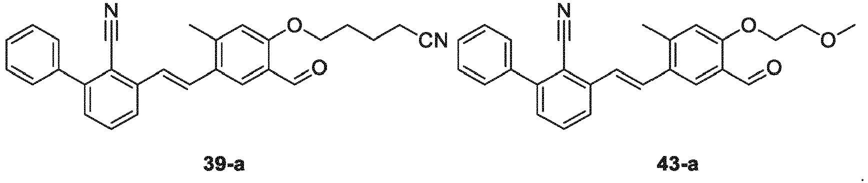

In some embodiments, the compound of formula II-e is any one of the following compounds:

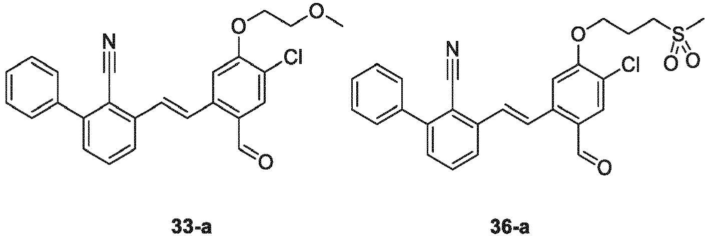

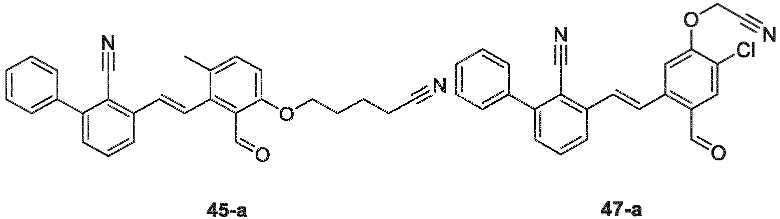

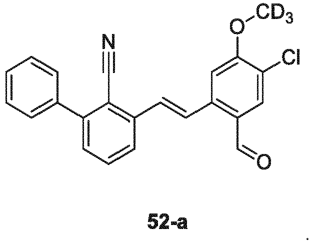

The present disclosure provides any one of the following aromatic vinyl compounds, tautomers thereof, stereoisomers thereof, racemates thereof, isotopic derivatives thereof, or their (referring to the above any one of the following aromatic vinyl compounds, tautomers thereof, stereoisomers thereof, racemates thereof, isotopic derivatives thereof) pharmaceutically acceptable salts;

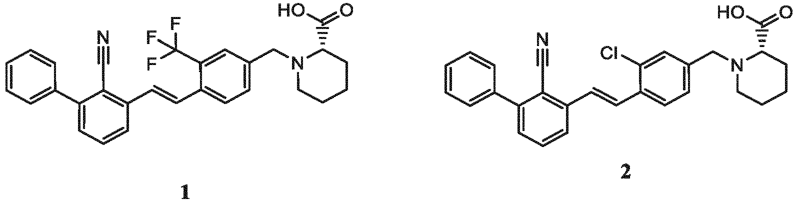

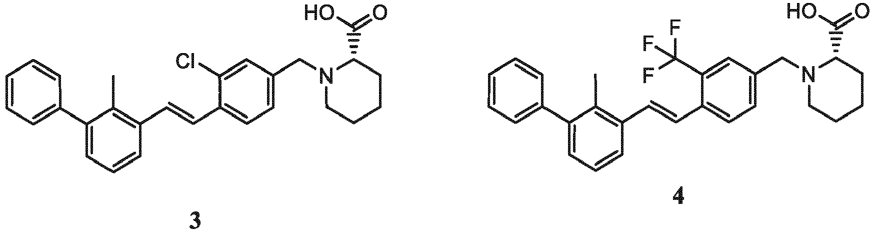

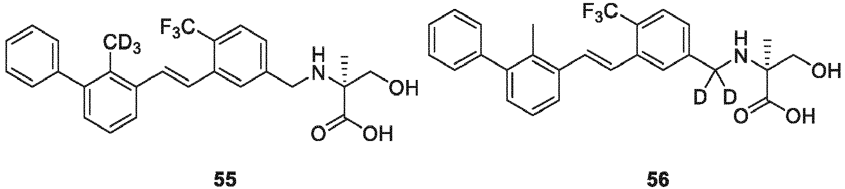

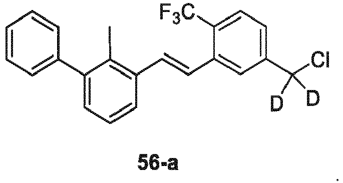

The present disclosure also provides a pharmaceutical composition, the pharmaceutical composition comprises the above aromatic vinyl compound, the tautomer thereof, the stereoisomer thereof, the racemate thereof, the isotopic derivative thereof, or their (referring to the above aromatic vinyl compound, the tautomer thereof, the stereoisomer thereof, the racemate thereof or the isotopic derivative thereof) pharmaceutically acceptable salts, and a pharmaceutical excipient.

The present disclosure also provides a pharmaceutical composition, the pharmaceutical composition comprises the above aromatic vinyl compound, the tautomer thereof, the stereoisomer thereof, the racemate thereof, the isotopic derivative thereof, or their (referring to the above aromatic vinyl compound, the tautomer thereof, the stereoisomer thereof, the racemate thereof or the isotopic derivative thereof) pharmaceutically acceptable salts, and at least one other drug, wherein the other drug is a chemotherapy drug or a targeted drug. The targeted drug is preferably one or more of COX-2 inhibitors, DPP4 inhibitors, CSF-1α inhibitors and A2a antagonists.

In the pharmaceutical composition, the amount of the aromatic vinyl compound, the tautomer thereof, the stereoisomer thereof, the racemate thereof, the isotopic derivative thereof, or their (referring to the above aromatic vinyl compound, the tautomer thereof, the stereoisomer thereof, the racemate thereof or the isotopic derivative thereof) pharmaceutically acceptable salts may be in therapeutically effective amounts.

The pharmaceutical excipients can be those widely used in the field of pharmaceutical production. Excipients are primarily used to provide a safe, stable, and functional pharmaceutical composition, and may also provide methods to enable the active ingredient to dissolve at a desired rate after the subject has received administration or to facilitate effective absorption of the active ingredient after the subject has received administration of the composition. The pharmaceutical excipients can be inert fillers or provide some functions, such as stabilizing the overall pH of the composition or preventing degradation of the active ingredients of the composition. The pharmaceutical excipients may include one or more of the following excipients: binder, suspending agent, emulsifying agent, diluting agent, filler, granulating agent, adhesive agent, disintegrating agent, lubricant, anti-adhesive agent, glidant, wetting agent, gelling agent, absorption retardant, dissolution inhibitor, enhancer, adsorbent, buffering agent, chelating agent, preservative, coloring agent, flavoring agent, and sweetening agent.

The pharmaceutical compositions of the present disclosure can be prepared according to the disclosure using any method known to those skilled in the art. For example, conventional mixing, dissolving, granulating, emulsifying, levigating, encapsulating, embedding, or lyophilizing processes.

The pharmaceutical compositions of the present disclosure can be administered in any form, including injection (intravenous), mucosal, oral (solid and liquid preparations), inhalation, ophthalmic, rectal, topical, or parenteral (infusion, injection, implant, subcutaneous, intravenous, intraarterial, intramuscular) administration. The pharmaceutical compositions of the present disclosure may also be in controlled-release or delayed-release forms (e.g., liposomes or microspheres). Examples of solid oral preparations include, but are not limited to, powders, capsules, caplets, softgels, and tablets. Examples of liquid preparations for oral or mucosal administration include, but are not limited to, suspensions, emulsions, elixirs, and solutions. Examples of topical preparations include, but are not limited to, emulsions, gels, ointments, creams, patches, pastes, foams, lotions, drops, or serum preparations. Examples of preparations for parenteral administration include, but are not limited to, solutions for injection, dry preparations that can be dissolved or suspended in a pharmaceutically acceptable vehicle, suspensions for injection and emulsions for injection. Examples of other suitable preparations of the pharmaceutical composition include, but are not limited to, eye drops and other ophthalmic preparations; aerosols: such as nasal sprays or inhalants; liquid preparations for parenteral administration; suppositories and lozenges.

The present disclosure also provides a use of the above aromatic vinyl compound, the tautomer thereof, the stereoisomer thereof, the racemate thereof, the isotopic derivative thereof, or their (referring to the above aromatic vinyl compound, the tautomer thereof, the stereoisomer thereof, the racemate thereof or the isotopic derivative thereof) pharmaceutically acceptable salts or the above pharmaceutical composition in the manufacture of a PD-1 inhibitor and/or PD-L1 inhibitor.

In the above use, the PD-1 inhibitor or PD-L1 inhibitor can be used in mammal in vivo; it can also be used in vitro, mainly for experimental purposes, for example: as a standard sample or a control sample to provide comparison, or make a kit according to conventional methods in the art to provide rapid detection of PD-1 or PD-L1 inhibitory effect.

The present disclosure also provides a use of the above aromatic vinyl compound, the tautomer thereof, the stereoisomer thereof, the racemate thereof, the isotopic derivative thereof, or their (referring to the above aromatic vinyl compound, the tautomer thereof, the stereoisomer thereof, the racemate thereof or the isotopic derivative thereof) pharmaceutically acceptable salts or the above pharmaceutical composition in the manufacture of a drug for the prevention and/or treatment of diseases related to the PD-1/PD-L1 signaling pathway.

The diseases related to PD-1/PD-L1 signaling pathway are selected from cancer, infectious diseases, autoimmune diseases or related diseases.

The cancer is preferably one or more of lung cancer, esophageal cancer, gastric cancer, colorectal cancer, hematological tumor, lymphoma, head and neck cancer, liver cancer, nasopharyngeal cancer, brain tumor, breast cancer, cervical cancer, blood cancer and bone cancer.

The infectious disease is preferably bacterial infection and/or viral infection.

The autoimmune disease is preferably one or more of rheumatoid arthritis, systemic lupus erythematosus, systemic sclerosis, systemic vasculitis and relapsing polychondritis.

Unless otherwise specified, the terms used in the present disclosure shall have the following meanings:

The term "pharmaceutically acceptable" refers to salts, solvents, excipients, etc. are generally non-toxic, safe, and suitable for patient use. The "patient" is preferred mammal, more preferably human.

The term "pharmaceutically acceptable salt" refers to the salt prepared by the compound of the present disclosure and a relatively nontoxic and pharmaceutically acceptable acid or base. When the compound of the present disclosure contains a relatively acidic functional group, a base addition salt can be obtained by bringing the neutral form of the compound into contact with a sufficient amount of pharmaceutically acceptable base in a pure solution or a suitable inert solvent. Pharmaceutically acceptable base addition salts include, but are not limited to, lithium, sodium, potassium, calcium, aluminum, magnesium, zinc, bismuth, ammonium, diethanolamine salts. When the compound of the present disclosure contains a relatively basic functional group, an acid addition salt can be obtained by bringing the neutral form of the compound into contact with a sufficient amount of pharmaceutically acceptable acid in a pure solution or a suitable inert solvent. The pharmaceutically acceptable acids include inorganic acids, and the inorganic acids include but are not limited to: hydrochloric acid, hydrobromic acid, hydroiodic acid, nitric acid, carbonic acid, phosphoric acid, phosphorous acid, sulfuric acid, etc. The pharmaceutically acceptable acid includes organic acid, and the organic acid includes but is not limited to: acetic acid, propionic acid, oxalic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, salicylic acid, tartaric acid, methanesulfonic acid, isonicotinic acid, acidic citric acid, oleic acid , tannic acid, pantothenic acid, hydrogen tartrate, ascorbic acid, gentisic acid, fumaric acid, gluconic acid, sugar acid, formic acid, ethanesulfonic acid, pamoic acid (i.e. 4,4'-methylene-bis(3-hydroxy-2-naphthoic acid)), amino acids (such as glutamic acid, arginine), etc. When compounds of the present disclosure contain relatively acidic and relatively basic functional groups, they can be converted into base addition salts or acid addition salts. For details, see Berge et al., "Pharmaceutical Salts", Journal of Pharmaceutical Science 66: 1-19 (1977), or, Handbook of Pharmaceutical Salts: Properties, Selection, and Use (P. Heinrich Stahl and Camille G. Wermuth, ed., Wiley-VCH, 2002).

The terms "compound", "tautomer", "stereoisomer", "racemate", "isotope derivative" and their "pharmaceutically acceptable salts" can exist in a crystal form or amorphous form. As used herein, the term "crystal form" refers to that the ions or molecules are strictly and periodically arranged in a three-dimensional space according to a determined method, and are regularly repeated at a certain interval of periods; due to the differences in the periodic arrangement of ions or molecules, more than one crystal form can exist, i.e., polymorphism. The term "amorphous" refers to the disordered distribution of ions or molecules, that is, there is no periodic arrangement between ions and molecules.

The term "stereoisomer" refers to a *cis-trans* isomer or optical isomer. These stereoisomers can be separated, purified and enriched by asymmetric synthesis or chiral separation methods (including but not limited to thin layer chromatography, rotary chromatography, column chromatography, gas chromatography, high pressure liquid chromatography, etc.), and can also be obtained by chiral resolution by bonding (chemical bonding, etc.) or salting (physical bonding, etc.) with other chiral compounds.

The term "isotopic derivative" refers to "compound", "tautomer", "stereoisomer", "racemate" and their "pharmaceutically acceptable salts" in which the atoms can exist in their natural abundance or unnatural abundance. Taking hydrogen atom as an example, its natural abundance form means that about 99.985% is protium and about 0.015% is deuterium; in the form of unnatural abundance, for example, about 95% of which is deuterium. That is, one or more atoms in the terms "compound", "tautomer", "stereoisomer", "racemate" and their "pharmaceutically acceptable salts" may be atoms that exist in unnatural abundance.

When an arbitrary variable (e.g., R^{A-1}) appears many times in the definition of a compound, the definition of each occurrence of the variable has nothing to do with the definitions of other occurrences, and their meanings are independent of each other and have no influence on each other. Therefore, if a group is substituted by 1, 2, 3, 4 or 5 R^{A-1}, that is, the group may be substituted by up to 5 R^{A-1} groups, the definition of this position R^{A-1} is independent of the definition of the remaining position R^{A-1}, which can be different or the same. Additionally, combinations of substituents and/or variables are permissible only if such combinations result in stable compounds.

The term "alkyl" refers to a straight or branched chain alkyl having a specified number of carbon atoms.

The term "alkoxy" refers to the group -O-R^{X}, wherein R^{X} is the alkyl as defined above.

The term "pharmaceutical excipients" refers to the excipients and additives used in the manufacture of drugs and the formulation of prescriptions, which are all substances contained in pharmaceutical preparations except active ingredients. See Pharmacopoeia of the People's Republic of China (2015 Edition) Part IV, or, Handbook of Pharmaceutical Excipients (Raymond C Rowe, 2009 Sixth Edition).

The term "prevention" refers to a reduction in the risk of acquiring or developing a disease or disorder.

The term "treatment" refers to a therapeutic therapy. Regarding to a specific disease, the treatment refers to: (1) alleviation of one or more biological manifestations of a disorder or disease, (2) interfering with (a) one or more points in the biological cascade leading to or causing a disease or (b) one or more biological manifestations of the disease, (3) improvement of one or more symptoms, effects or side effects associated with the disease, or one or more symptoms, effects or side effects associated with the disease or treatment thereof, or (4) slowdown of the progression of a disease or one or more biological manifestations of the disease.

The term "mammal" includes any mammal. Examples of mammals include, but are not limited to, cattle, horses, sheep, pigs, cats, dogs, mice, rats, rabbits, guinea pigs, monkeys, humans, etc., and most preferably humans.

On the basis of not violating the common knowledge in the art, each of the above preferred conditions can be arbitrarily combined to give the one or more preferred examples of the present disclosure.

The reagents and raw materials used in the present disclosure are commercially available.

The positive progress effect of the present disclosure lies in that the aromatic vinyl compound or the pharmaceutically acceptable salt thereof of the present disclosure has a significant inhibitory effect on PD-l/PD-L1, has a higher peak concentration, a larger area under the curve, better oral bioavailability, and can effectively alleviate or treat cancer and related diseases.

### DETAILED DESCRIPTION OF THE EMBODIMENT

The present disclosure will be further described below with reference to embodiments, but the present disclosure is not therefore limited to the scope of the embodiment. Experimental methods without specific conditions in the following embodiments are selected according to conventional methods and conditions, or according to the commercial specification.

In the following embodiments, room temperature refers to 10°C-30°C; overnight refers to 8-24 hours, preferably 12-18 hours.

The structure of the compound was confirmed by nuclear magnetic resonance (NMR) or mass spectrometry (MS), the nuclear magnetic resonance spectrum was determined by a Bruker Avance-500 instrument using deuterated dimethyl sulfoxide, deuterated chloroform, deuterated methanol and the like as a solvent, and tetramethylsilane (TMS) as an internal standard. Mass spectrum was determined by liquid chromatography-mass spectrometry (LC-MS) Agilent Technologies 6110 using an ESI ion source.

The microwave reaction was carried out in an Explorer automatic microwave synthesizer manufactured by CEM company of the United States, the frequency of magnetron was 2450 MHz and the continuous microwave output power was 300 W.

The instrument used for preparative high performance liquid chromatography was Waters 2767, and the preparative column used was XBrige C18, 19 × 150 mm × 5 µm. Acid mobile phase: 1% formic acid (A) + acetonitrile (B); basic mobile phase: 5 mmol/L aqueous ammonium bicarbonate solution. Flow rate: 15 mmL/min. Gradient: 20% to 70% (the initial mobile phase is 80% water/20% acetonitrile, and the final mobile phase is 30% water/70% acetonitrile, wherein % refers to volume percentage). Detection wavelengths: 214 nm and 254 nm.

### Embodiment 1

### (S,E)-1-(4-(2-(2-Cyano-[1,1'-biphenyl]-3-yl)vinyl)-3-trifluoromethylbenzyl)piperidine-2-carboxylic acid (compound 1)

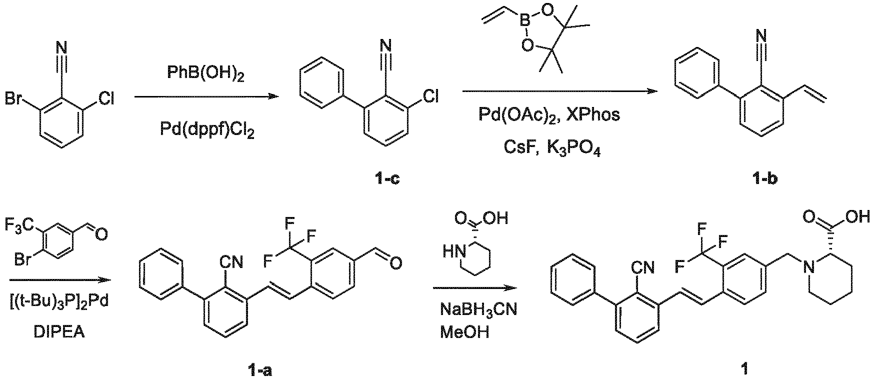

### Synthesis of compound 1-c

Water (4 mL), [1,1'-bis(diphenylphosphino)ferrocene]palladium dichloride (365 mg, 0.5 mmol) and sodium carbonate (2.65 g, 25.0 mmol) were added to a 1,4-dioxane (40 mL) solution of 2-bromo-6-chlorobenzonitrile (2.16 g, 10.0 mmol) and phenylboronic acid (1.33 g, 11.0 mmol). The reaction mixture was heated to 80°C and stirred under nitrogen for 16 hours. The reaction solution was cooled to room temperature, concentrated under reduced pressure, and the residue was purified by silica gel column chromatography (petroleum ether: ethyl acetate = 6:1) to give compound **1-c** (1.55 g, yield: 72%).

### Synthesis of compound 1-b

Water (8 mL), palladium acetate (78 mg, 0.35 mmol), 2-dicyclohexylphosphino-2',4',6'-triisopropylbiphenyl (333 mg, 0.70 mmol), cesium fluoride (2.10 g, 14.0 mmol) and potassium phosphate (2.97 g, 14.0 mmol) were added to a 1,4-dioxane (80 mL) solution of compound **1-c** (1.50 g, 7.0 mmol) and pinacol vinylboronate (2.13 g, 8.4 mmol). The reaction mixture was heated to 80°C, and stirred under nitrogen for 6 hours. The reaction solution was cooled to room temperature, filtered, and the filtrate was concentrated under reduced pressure. The residue was purified by silica gel column chromatography (petroleum ether: ethyl acetate = 6:1) to give compound **1-b** (1.17 g, yield: 82%).

¹H NMR (400 MHz,CD₃Cl): *δ*7.67 (dd, *J*=1.2 Hz, 7.6 Hz, 1H), 7.59 (t, *J*=8.0 Hz, 1H), 7.53-7.56 (m, 2H), 7.44-7.50 (m, 3H), 7.37 (dd, *J*=1.2 Hz, 7.6 Hz, 1H), 7.20 (dd, *J*=10.8 Hz, 17.6 Hz, 1H), 5.96 (d, *J*=17.6 Hz, 1H), 5.57 (d, *J*=10.8 Hz, 1H) ppm

### Synthesis of compound 1-a

Compound **1-b** (100 mg, 0.487 mmol) and 4-bromo-3-trifluoromethyl-benzaldehyde (123 mg, 0.487 mmol) were dissolved in a toluene solution (20 mL), and *N,N'-*diisopropylethylamine (504 mg, 3.896 mmol) and bis(tri-*tert*-butylphosphine)palladium (18 mg, 0.034 mmol) were added. The atmosphere of reaction was replaced with nitrogen three times at room temperature. The reaction solution was heated to 80°C, and stirred for 16 hours. The reaction solution was cooled to room temperature, diluted with ethyl acetate (100 mL), and then washed with water (100 mL×3) and saturated brine (100 mL) in turn. The resulting organic phase was dried with anhydrous sodium sulfate, concentrated under reduced pressure, and the residue was purified by silica gel column chromatography (petroleum ether: ethyl acetate = 20:1 to 5:1) to give compound **1-a** (62 mg, yield: 33.7%).
¹H NMR (400 MHz, CDCl₃): *δ* 10.01 (s, 1H), 8.15 (s, 1H), 8.05-8.00 (m, 2H), 7.77-7.75 (d, *J*= 8.0 Hz, 1H), 7.67-7.60 (m, 3H), 7.51-7.39 (m, 6H) ppm

### Synthesis of compound 1

Compound **1-a** (62 mg, 0.164 mmol) was suspended in methanol (10 mL), and (*S*)-piperidine-2-carboxylic acid (43 mg, 0.329 mmol) and sodium cyanoborohydride (21 mg, 0.329 mmol) were added. The reaction solution was heated to 70°C and stirred for 16 hours. The reaction solution was cooled to room temperature, concentrated under reduced pressure, and the residue was prepared by high performance liquid chromatography to give compound **1** (15 mg, yield: 18.8%).

LC-MS (ESI): m/z = 491.0 [M+H]⁺.

¹H NMR (400 MHz, CD₃OD): *δ* 8.08-8.06 (d, J= 8.0 Hz, 1H), 8.03 (s, 1H), 7.93-7.88 (m, 2H), 7.81-7.77 (t, J= 8.0 Hz, 1H), 7.76-7.71 (m, 1H), 7.66-7.59 (m, 3H), 7.56-7.50 (m, 4H), 4.73-4.70 (d, *J* = 12.8 Hz, 1H), 4.21-4.17 (m, 1H), 3.53-3.50 (m, 1H), 3.39-3.36 (m, 1H), 3.02-2.97 (m, 1H), 2.32-2.29 (m, 1H), 1.88-1.84 (m, 3H), 1.74-1.68 (m, 1H), 1.62-1.56 (m, 1H) ppm.

### Embodiment 2

### (S,E)-1-(3-Chloro-4-(2-(2-cyano-[1,1'-biphenyl]-3-yl)vinyl)benzyl)piperidine-2-carboxylic acid (compound 2)

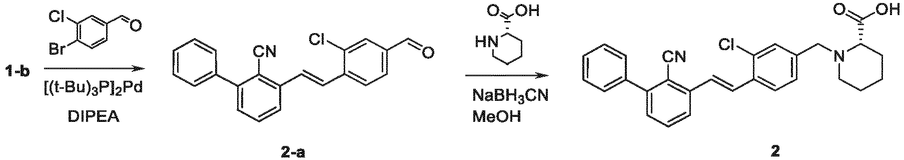

### Synthesis of compound 2-a

Compound **1-b** (205.0 mg, 1.0 mmol) and 4-bromo-3-chlorobenzaldehyde (240.9 mg, 1.1 mmol) were dissolved in a toluene solution (30 mL), and *N*,*N*'-diisopropylethylamine (387 mg, 3.0 mmol) and bis(tri-*tert*-butylphosphine)palladium (51.1 mg, 0.1 mmol) were added. The atmosphere of reaction was replaced with nitrogen three times at room temperature. The reaction solution was heated to 90°C, and stirred for 10 hours. The reaction solution was cooled to room temperature, diluted with ethyl acetate (100 mL), and then washed with water (100 mL×3) and saturated brine (100 mL) in turn. The resulting organic phase was dried with anhydrous sodium sulfate, concentrated under reduced pressure, and the residue was purified by silica gel column chromatography (petroleum ether: ethyl acetate = 20:1 to 5:1) to give compound **2-a** (270 mg, yield: 78%).

### Synthesis of compound 2

Compound **2-a** (172 mg, 0.5 mmol) was suspended in methanol (30 mL), and (S)-piperidine-2-carboxylic acid (43 mg, 0.329 mmol) and sodium cyanoborohydride (63.0 mg, 1.0 mmol) were added. The reaction solution was heated to 60°C and stirred for 2 hours. The reaction solution was cooled to room temperature, concentrated under reduced pressure and the residue was prepared by high performance liquid chromatography to give white solid **2** (75 mg, yield: 32%).

LC-MS (ESI): m/z = 457.3 [M+H]⁺.

¹H NMR (400 MHz, CD₃OD): *δ* 7.97 (d, J = 7.2 Hz, 1H), 7.93 (t, J = 8.0 Hz, 1H), 7.82-7.75 (m, 3H), 7.66-7.47 (m, 3H), 4.62 (d, J = 12.8 Hz, 1H), 4.13 -4.08 (m, 1H), 3.50-3.40 (m, 2H), 3.00-2.94 (m, 1H), 2.32-2.28 (m, 1H), 1.87-1.54 (m, 5H) ppm.

### Embodiment 3

### (S,E)-1-(3-Chloro-4-(2-(2-methyl-[1,1'-biphenyl]-3-yl)vinyl)benzyl)piperidine-2-carboxylic acid (compound 3)

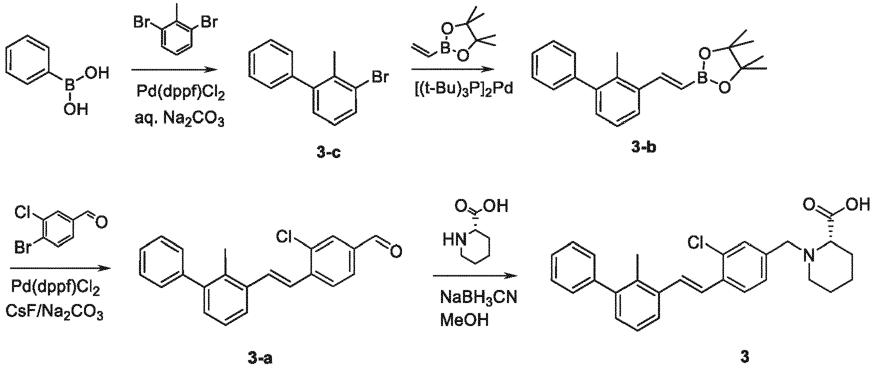

### Synthesis of compound 3-c

Phenylboronic acid (1.626 g, 13.34 mmol) and 2,6-dibromotoluene (5.0 g, 20.0 mmol) were dissolved in a mixture of 1,4-dioxane (60 mL) and water (3 mL), and [1,1'-bis(diphenylphosphino)ferrocene]palladium dichloride (1.154 g, 1.334 mmol) and sodium carbonate (3.535 g, 33.35 mmol) were added. The reaction system was replaced with nitrogen three times and then heated to 80°C and stirred for 16 hours. The reaction solution was cooled to room temperature, diluted with ethyl acetate (100 mL), and then washed with water (100 mL×3) and saturated brine (100 mL) in turn. The resulting organic phase was dried with anhydrous sodium sulfate, concentrated under reduced pressure, and the residue was purified by silica gel column chromatography (petroleum ether) to give compound **3-c** (1.9 g, yield: 57.2%).
¹H NMR (400 MHz, CDCl₃): δ 7.56-7.54 (m, 1H), 7.44-7.35 (m, 3H), 7.28-7.25 (m, 2H), 7.17-7.15 (m, 1H), 7.08 (t, *J* = 8Hz, 1H), 2.31 (s, 3H) ppm

### Synthesis of compound 3-b

Compound **3-c** (1.071 g, 4.33 mmol) and pinacol vinylboronate (800.9 mg, 5.20 mmol) were dissolved in a toluene (50 mL) solution, and bis(tri-*tert*-butylphosphine)palladium (154.8 mg, 0.303 mmol) and triethylamine (3.51 g, 34.64 mmol) were added. The reaction system was replaced with nitrogen three times and then heated to 80°C and stirred for 16 hours. The reaction solution was cooled to room temperature, diluted with ethyl acetate (50 mL), and then washed with water (50 mL×3) and saturated brine (50 mL) in turn. The resulting organic phase was dried with anhydrous sodium sulfate, concentrated under reduced pressure, and the residue was purified by silica gel column chromatography (petroleum ether) to give compound **3-b** (0.89 g, yield: 64.1%).
¹H NMR (400 MHz, CDCl₃): δ 7.75-7.71 (d, *J* = 18Hz, 1H), 7.56-7.54 (m, 1H), 7.41-7.39 (m, 2H), 7.36-7.34 (m, 1H), 7.30-7.28 (m, 2H), 7.23-7.17 (m, 2H), 6.12-6.07 (d, *J* = 18Hz, 1H), 2.82 (s, 3H), 1.32 (s, 12H) ppm

### Synthesis of compound 3-a

Compound **3-b** (349 mg, 1.09 mmol) and 3-chloro-4-bromobenzaldehyde (200 mg, 0.911 mmol) were dissolved in ethylene glycol dimethyl ether (20 mL), and cesium fluoride (277 mg, 1.82 mmol), sodium carbonate (242 mg, 2.28 mmol) and [1,1'-bis(diphenylphosphino)ferrocene]palladium dichloride (79 mg, 0.091 mmol) were added. The atmosphere of the reaction solution was replaced three times with nitrogen at room temperature. The reaction solution was heated to 80°C and stirred for 16 hours. The reaction solution was cooled to room temperature and diluted with ethyl acetate (100 mL), then washed with water (100 mL×3) and saturated brine (100 mL). The resulting organic phase was dried with anhydrous sodium sulfate, concentrated under reduced pressure, and the residue was purified by silica gel column chromatography (petroleum ether: ethyl acetate = 20:1 to 5:1) to give compound **3-a** (175 mg, yield: 57.8%).
¹H NMR (400 MHz, CDCl₃): δ 9.90 (s, 1H), 7.85-7.79 (m, 2H), 7.72-7.70 (d, J= 8.0 Hz, 1H), 7.60-7.57 (m, 1H), 7.52-7.48 (m, 1H), 7.38-7.34 (m, 3H), 7.31-7.21 (m, 5H), 2.26 (s, 3H) ppm

### Synthesis of compound 3

Compound **3-a** (175 mg, 0.526 mmol) was suspended in methanol (15 mL), and (S)-piperidine-2-carboxylic acid (136 mg, 1.052 mmol) and sodium cyanoborohydride (66 mg, 1.052 mmol) were added. The reaction solution was heated to 70°C and stirred for 16 hours. The reaction solution was cooled to room temperature, concentrated under reduced pressure, and the residue was prepared by high performance liquid chromatography to give compound 3 (43 mg, yield: 18.3%).

LC-MS (ESI): m/z = 446.0 [M+H]⁺.

¹H NMR (400 MHz, CD₃OD): *δ* 7.82-7.80 (d, *J*= 8.0 Hz, 1H), 7.58 (s, 1H), 7.52-7.48 (m, 2H), 7.41-7.39 (d, *J*= 7.2 Hz, 1H), 7.34-7.25 (m, 4H), 7.20-7.16 (m, 3H), 7.07-7.06 (d, *J*= 6.8 Hz,1H), 4.50-4.47 (d, *J*= 12.4 Hz,1H), 3.99-3.94 (m, 1H), 3.37-3.34 (d, *J*= 10.4 Hz,1H), 3.28-3.25 (m, 1H), 2.88-2.82 (t, *J*= 12.4 Hz,1H), 2.19 (s, 3H), 2.16 (s, 1H), 1.75-1.72 (m, 3H), 1.63-1.60 (m, 1H), 1.48-1.41 (m, 1H) ppm.

### Embodiment 4

### (S,E)-1-(4-(2-(2-Methyl-[1,1'-biphenyl]-3-yl)vinyl)-3-trifluoromethylbenzyl)piperidine-2-carboxylic acid (compound 4)

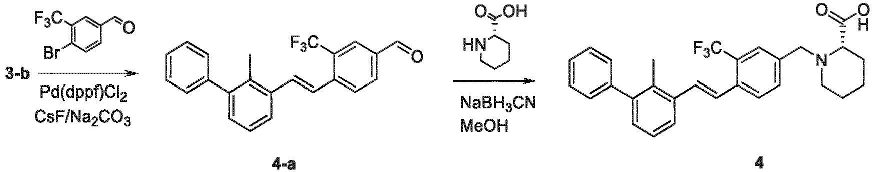

### Synthesis of compound 4-a

Compound **3-b** (304 mg, 0.949 mmol) and 3-trifluoromethyl-4-bromobenzaldehyde (200 mg, 0.791 mmol) were dissolved in ethylene glycol dimethyl ether (20 mL), and cesium fluoride (241 mg, 1.582 mmol), sodium carbonate (210 mg, 1.98 mmol) and [1,1'-bis(diphenylphosphino)ferrocene]palladium dichloride (69 mg, 0.079 mmol) were added. The atmosphere of the reaction solution was replaced three times with nitrogen at room temperature. The reaction solution was heated to 80°C and stirred for 16 hours. The reaction solution was cooled to room temperature and diluted with ethyl acetate (100 mL), then washed with water (100 mL×3) and saturated brine (100 mL). The resulting organic phase was dried with anhydrous sodium sulfate, concentrated under reduced pressure, and the residue was purified by silica gel column chromatography (petroleum ether: ethyl acetate = 20:1 to 5:1) to give compound **4-a** (132 mg, yield: 45.7%).

¹H NMR (400 MHz, CDCl₃): δ 9.98 (s, 1H), 8.12 (s, 1H), 8.01-7.98 (d, J= 8.4 Hz,1H), 7.92-7.90 (d, J= 8.4 Hz, 1H), 7.54-7.48 (m, 2H), 7.38-7.22 (m, 8H), 2.26 (s, 3H) ppm

### Synthesis of compound 4

Compound **4-a** (132 mg, 0.36 mmol) was suspended in methanol (15 mL), and (S)-piperidine-2-carboxylic acid (93 mg, 0.72 mmol) and sodium cyanoborohydride (46 mg, 0.72 mmol) were added. The reaction solution was heated to 70°C and stirred for 16 hours. The reaction solution was cooled to room temperature, concentrated under reduced pressure, and the residue was prepared by high performance liquid chromatography to give compound **4** (39 mg, yield: 22.7%).

LC-MS (ESI): m/z = 480.0 [M+H]⁺.

¹H NMR (400 MHz, CD₃OD): δ 7.99-7.97 (d, J= 8.0 Hz, 1H), 7.88 (s, 1H), 7.75-7.73 (d, J= 8.0 Hz, 1H), 7.56-7.52 (d, J= 16.0 Hz, 1H), 7.49-7.47 (d, J= 8.0 Hz, 1H), 7.37-7.34 (t, J= 7.2 Hz, 2H), 7.30-7.19 (m, 5H), 7.12-7.10 (d, J= 7.2 Hz, 1H), 4.63-4.59 (d, J= 12.8 Hz, 1H), 4.10-4.07 (m, 1H), 3.41-3.39 (d, J= 10.4 Hz, 1H), 2.93-2.87 (t, J= 10.8 Hz, 1H), 2.22 (s, 3H), 2.20 (m, 1H), 1.78-1.76 (m, 3H), 1.65-1.62 (m, 1H), 1.52-1.46 (m, 1H) ppm.

### Embodiment 5

### (S,E)-1-(5-Chloro-2-methyl-4-(2-(2-methyl-[1,1'-biphenyl]-3-yl)vinyl)benzyl)piperidine-2-carboxylic acid (compound 5)

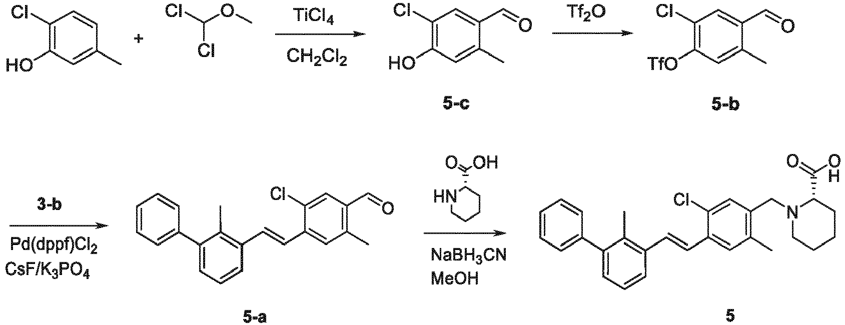

### Synthesis of compound 5-c

5-Chloro-2-methylphenol (2.85 g, 20.0 mmol) was dissolved in anhydrous dichloromethane (100 mL), then titanium tetrachloride (11.38 g, 60.0 mmol) was added, and the reaction solution was cooled to 0°C. Dichloromethoxymethane (6.90 g, 60.0 mmol) was added dropwise under nitrogen. After the dropwise addition was completed, the reaction solution was raised to room temperature and continued stirring for 2 hours. Then, the reaction solution was poured into crushed ice to quench the reaction, extracted with ethyl acetate (100 mL×2), and the organic phase was combined and washed with water (100 mL) and saturated brine (100 mL) in turn. The resulting organic phase was dried with anhydrous sodium sulfate, concentrated under reduced pressure, and the residue was purified by silica gel column chromatography (petroleum ether: ethyl acetate = 10:1 to 3:1) to give compound **5-c** (1.569 g, yield: 45.9%).

### LC-MS (ESI): m/z = 171.0 [M+H]⁺.

### Synthesis of compound 5-b

Compound **5-c** (1.05 g, 6.16 mmol) was dissolved in a anhydrous dichloromethane solution (100 mL), and triethylamine (1.25 g, 12.32 mmol) was added. The reaction solution was cooled to -78°C under nitrogen, and trifluoromethanesulfonic anhydride (2.61 g, 9.23 mmol) was added dropwise. After the dropwise addition was completed, the reaction solution was raised to room temperature and continued stirring for 2 hours. The reaction solution was cooled to room temperature and diluted with ethyl acetate (100 mL), then washed with water (100 mL) and saturated brine (100 mL). The resulting organic phase was dried with anhydrous sodium sulfate, concentrated under reduced pressure, and the residue was purified by silica gel column chromatography (petroleum ether: ethyl acetate = 50:1 to 10:1) to give compound **5-b** (1.50 g, yield: 80.2%).

### Synthesis of compound 5-a

Compound **3-b** (634.7 mg, 1.982 mmol) and compound **5-b** (500 mg, 1.652 mmol) were dissolved in a toluene solution (20 mL) at room temperature, and potassium phosphate (701.4 mg, 3.304 mmol), cesium fluoride (501.9 mg, 3.304 mmol) and [1,1'-bis(diphenylphosphino)ferrocene]palladium dichloride (142.7 mg, 0.165 mmol) were added. The reaction solution was heated to 80°C and stirred under nitrogen for 16 hours. The reaction solution was cooled to room temperature, diluted with ethyl acetate (100 mL) and washed with water (100 mL) and saturated brine (100 mL). The resulting organic phase was dried with anhydrous sodium sulfate, concentrated under reduced pressure, and the residue was purified by silica gel column chromatography (petroleum ether: ethyl acetate = 50:1 to 20:1) to give compound **5-a** (501 mg, yield: 87.6%).

### Synthesis of compound 5

Dichloromethane (10 mL) was added to a methanol (10 mL) solution of compound **5-a** (70 mg, 0.20 mmol) and L-piperidine-2-carboxylic acid (52 mg, 0.40 mmol). The mixture was stirred at room temperature for 1 hour, and sodium cyanoborohydride (32 mg, 0.50 mmol) was added. After stirring the reaction for 16 hours at room temperature, the reaction solution was concentrated under reduced pressure. The residue was washed with water (100 mL), filtered and prepared by high performance liquid chromatography to give white solid **5** (34 mg, yield: 37%).

LC-MS (ESI): m/z = 460 [M+H]⁺.

¹H NMR (400 MHz, CD₃Cl): *δ* 7.77 (s, 1H), 7.70 (s, 1H), 7.62-7.63 (m, 1H), 7.60 (d, *J*=18.0Hz, 1H), 7.43-7.47 (m, 2H), 7.34-7.39 (m, 2H), 7.27-7.32 (m, 3H), 7.17-7.19 (m, 1H), 4.88 (d, *J*=12.8Hz, 1H), 4.67 (d, *J*=12.8Hz, 1H), 4.05-4.09 (m, 1H), 3.53-3.57 (m, 1H), 3.01-3.06 (m, 1H), 2.53 (s, 3H), 2.32 (s, 3H), 2.27-2.31 (m, 1H), 1.82-1.89 (m, 3H), 1.72-1.75 (m, 1H), 1.57-1.64 (m, 1H) ppm.

### Embodiment 6

### (S,E)-1-(2-Methoxy-4-(2-(2-methyl-[1,1'-biphenyl]-3-yl)vinyl)-5-trifluoromethylbenzyl)piperidine-2-carboxylic acid (compound 6)

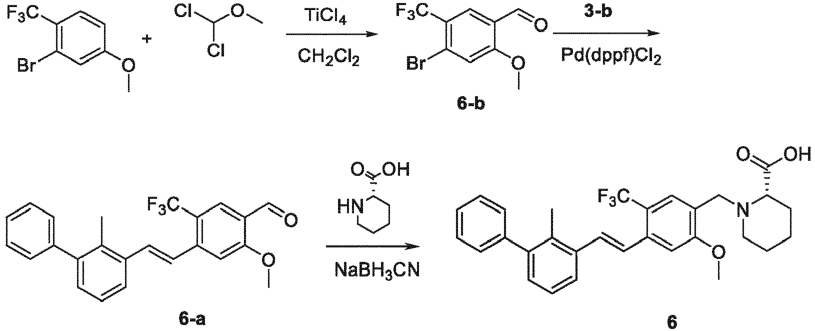

### Synthesis of compound 6-b

Titanium tetrachloride (2.84 g, 15.0 mmol) was added to a anhydrous dichloromethane (30 mL) solution of 2-bromo-4-methoxy-1-(trifluoromethyl)benzene (1.28 g, 5.0 mmol) at 0°C. 1,1'-Dichloromethyl ether (1.15 g, 10.0 mmol) was slowly added dropwise to the mixture at 0°C with stirring. After the addition was completed, the reaction was continued at 0°C for 3 hours. Ice water (30 mL) was slowly added to quench the reaction. The mixture was extracted with ethyl acetate (30 mL×3) and washed with water (30 mL×2) and saturated brine (30 mL×2) in turn. The organic phase was concentrated under reduced pressure, and the residue was purified by silica gel column chromatography (petroleum ether: ethyl acetate = 15:1) to give compound **6-b** (254 mg, yield: 18%).
¹H NMR (400 MHz, CD₃Cl): *δ* 10.40 (s, 1H), 8.14 (s, 1H), 7.35 (s, 1H), 4.01 (s, 3H) ppm

### Synthesis of compound 6-a

[1,1'-Bis(diphenylphosphino)ferrocene]palladium dichloride (48 mg, 0.12 mmol), potassium phosphate (212 mg, 1.0 mmol) and cesium fluoride (150 mg, 1.0 mmol) were added to a mixture of compound **6-b** (141 mg, 0.50 mmol), compound **3-b** (200 mg, 0.62 mmol) and toluene (25 mL), and the mixture was stirred at 80°C for 16 hours under nitrogen. The reaction solution was cooled to room temperature, filtered, and the filtrate was concentrated under reduced pressure. The residue was purified by silica gel column chromatography (petroleum ether: ethyl acetate = 6:1 to 4:1) to give compound **6-a** (107 mg, yield: 54%).

### LC-MS (ESI): m/z = 397 [M+H]⁺.

### Synthesis of compound 6

Dichloromethane (10 mL) was added to a methanol (10 mL) solution of compound **6-a** (80 mg, 0.20 mmol) and L-piperidine-2-carboxylic acid (52 mg, 0.40 mmol). The mixture was stirred at room temperature for 1 hour, and sodium cyanoborohydride (32 mg, 0.50 mmol) was added. The mixture was stirred for 16 hours at room temperature, concentrated under reduced pressure. The residue was washed with water (30 mL×3), filtered, concentrated under reduced pressure, and prepared by high performance liquid chromatography to give white solid product 6 (41 mg, yield: 40%).

LC-MS (ESI): m/z = 510 [M+H]⁺.

¹H NMR (400 MHz, CD₃OD): *δ* 7.81 (s, 1H), 7.54 (d, *J*=16.0Hz, 1H), 7.45 (d, *J*=16.0Hz, 1H), 7.44 (s, 1H), 7.32-7.35 (m, 2H), 7.24-7.28 (m, 2H), 7.17-7.21 (m, 3H), 7.09 (d, *J*=7.6Hz, 1H), 4.45 (d, *J*=12.8Hz, 1H), 4.31 (d, *J*=12.8Hz, 1H), 3.98 (s, 3H), 3.40-3.44 (m, 1H), 2.87-2.94 (m, 1H), 2.21 (s, 3H), 2.14-2.17 (m, 1H), 1.67-1.77 (m, 3H), 1.45-1.51 (m, 1H) ppm.

### Embodiment 7

### (S,E)-1-(4-(2-(2-Cyano-[1,1'-biphenyl]-3-yl)vinyl)-2-methoxy-5-trifluoromethylbenzyl)piperidine-2-carboxylic acid (compound 7)

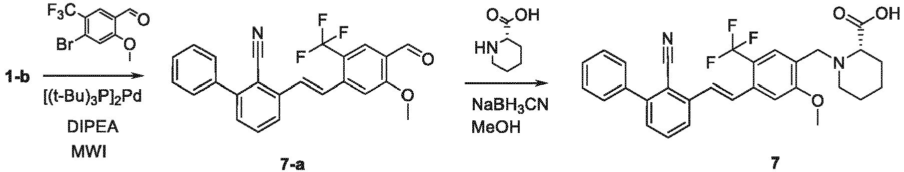

### Synthesis of compound 7-a

Compound **1-b** (100 mg, 0.487 mmol) and 4-bromo-2-methoxy-5-trifluoromethylbenzaldehyde (138 mg, 0.487 mmol) were dissolved in a toluene solution (20 mL), and *N*,*N*'-diisopropylethylamine (504 mg, 3.896 mmol) and bis(tri-tert-butylphosphine)palladium (25 mg, 0.049 mmol) were added. The atmosphere of the reaction was replaced with nitrogen for one minute at room temperature. The reaction solution was heated to 110°C under microwave, and stirred for 30 minutes. The reaction solution was cooled to room temperature, diluted with ethyl acetate (100 mL), and then washed with water (100 mL×3) and saturated brine (100 mL) in turn. The resulting organic phase was dried with anhydrous sodium sulfate, concentrated under reduced pressure, and the residue was purified by silica gel column chromatography (petroleum ether: ethyl acetate = 20:1 to 5:1) to give compound **7-a** (17 mg, yield: 8.6%).

LC-MS (ESI): m/z = 408.0 [M+H]⁺.

### Synthesis of compound 7

Compound **7-a** (17 mg, 0.042 mmol) was suspended in methanol (5 mL), and (S)-piperidine-2-carboxylic acid (11 mg, 0.084 mmol) and sodium cyanoborohydride (6.0 mg, 0.084 mmol) were added. The reaction solution was heated to 70°C, and stirred for 16 hours. The reaction solution was cooled to room temperature, concentrated under reduced pressure, and the residue was prepared by high performance liquid chromatography to give compound 7 (3.7 mg, yield: 7.0%).

LC-MS (ESI): m/z = 521.0 [M+H]⁺.

¹H NMR (400 MHz, CD₃OD): δ 7.86 (s, 1H), 7.80 (d, J= 7.6 Hz, 1H), 7.70-7.66 (t, J= 8.0 Hz, 1H), 7.62-7.52 (m, 2H), 7.50-7.47 (m, 2H), 7.44-7.37 (m, 5H), 4.46-4.43 (d, J= 13.2 Hz, 2H), 4.32-4.29 (d, J= 12.8 Hz, 1H), 3.98 (s, 3H), 3.43-3.41 (m, 1H), 2.93-2.87 (m, 1H), 2.17-2.14 (m, 1H), 1.79-1.61 (m, 4H), 1.48-1.45 (m, 1H) ppm.

### Embodiment 8

### (S,E)-1-(5-Chloro-4-(2-(2-cyano-[1,1'-biphenyl]-3-yl)vinyl)-2-methylbenzyl)piperidine-2-carboxylic acid (compound 8)

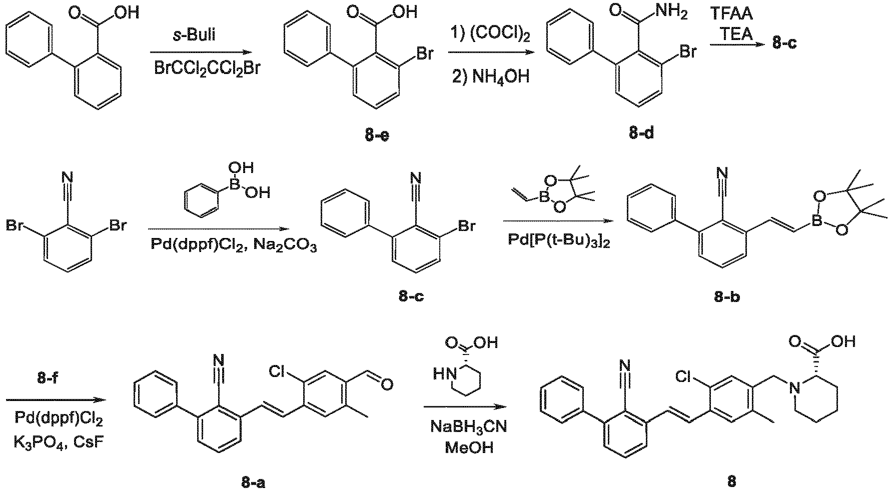

### Synthesis of compound 8-c

### Method I:

Biphenyl-2-carboxylic acid (5 g, 25.22 mmol) was dissolved in anhydrous tetrahydrofuran (180 mL), stirred under nitrogen and cooled to -78°C with a dry ice-acetone bath, and 1.3 M of s-butyllithium hexane solution (44 mL, 57.2 mmol) was added dropwise to the mixture within 30 min under nitrogen. After the dropwise addition was completed, the mixture was stirred for 2.5 hours at -78°C under nitrogen. 1,2-Dibromotetrachloroethane (24.6 g, 75.6 mmol) was added to the resulting orange-red solution. The dry ice acetone bath was removed, and the reaction was allowed to return to room temperature, and stirred for 16 hours. Water (30 mL) was added to the reaction solution, and the layers were separated. The aqueous phase was washed with diethyl ether (40 mL), and then adjusted to pH=1 with 4N hydrochloric acid. The aqueous phase was extracted with diethyl ether (20 mL×4), and the organic phases were combined and washed with brine (20 mL), concentrated under reduced pressure, and the residue was recrystallized with petroleum ether/ethyl acetate to give compound **8-e** (4.9 g, yield: 71 %). LC-MS (ESI): m/z = 276 [M-H]⁺.

Compound **8-e** (2.75 g, 10 mmol) was dissolved in dichloromethane (50 mL) and two drops of *N*,*N*'-dimethylformamide were added as a catalyst. Oxalyl chloride (5.54 g, 20 mmol) was added dropwise with stirring and stirred for one hour after dropwise addition. The mixture was evaporated to dryness to give crude acyl chloride. The acyl chloride was dissolved in dichloromethane (50 mL) and the solution was then added slowly dropwise to stirred concentrated ammonia water (50 mL). After the dropwise addition was completed, the mixture was stirred for one hour. The aqueous phase was extracted with ethyl acetate (20 mL×3), and the organic phases were combined, washed with brine (20 mL), and concentrated under reduced pressure to give compound **8-d** (2.56 g, yield: 93%), which was directly used in the next reaction step. LC-MS (ESI): m/z = 277 [M+H]⁺.

Compound **8-d** (2.50 g, 9.1 mmol) was dissolved in dichloromethane (50 mL), and triethylamine (2.30 g, 23 mmol) was added. Trifluoroacetic anhydride (2.5 g, 11.8 mmol) was added dropwise at 0°C and stirred for two hours. The reaction solution was diluted with ethyl acetate (200 mL), washed with brine (20 mL×3), and concentrated under reduced pressure to give white solid **8-c** (2.10 g, yield: 92%), which was directly used in the next reaction step.

### Method II:

Phenylboronic acid (3.06 g, 10 mmol) and 2-bromo-6-iodobenzonitrile (3.0 g, 12 mmol) were dissolved in a mixture of 1,4-dioxane (40 mL) and water (4 mL), and [1,1'-bis(diphenylphosphino)ferrocene]palladium dichloride dichloromethane complex (731 mg, 1.0 mmol) and sodium carbonate (4.08 g, 30 mmol) were added. The reaction system was replaced with nitrogen three times and then heated to 40°C and stirred for 16 hours. The reaction solution was cooled to room temperature, concentrated under reduced pressure, and the residue was purified by silica gel column chromatography (petroleum ether: dichloromethane = 7:1) to give compound **8-c** (1.65 g, yield: 67.5%).

### Synthesis of compound 8-b

Compound **8-c** (516 mg, 2 mmol) and pinacol vinylboronate (462 mg, 3 mmol) were dissolved in toluene (20 mL), and bis(tri-tert-butylphosphine)palladium (102 mg, 0.2 mmol) and triethylamine (2.02 g, 20 mmol) were added. The reaction system was replaced with nitrogen three times and then heated to 80°C and stirred for 12 hours. The reaction solution was cooled to room temperature, concentrated under reduced pressure, and the residue was purified by silica gel column chromatography (petroleum ether: ethyl acetate = 10: 1) to give compound **8-b** (364 mg, yield: 55%).
¹H NMR (400 MHz, DMSO-*d*6): *δ* 8.01 (d, J=7.6Hz, 1H), 7.79-7.75 (m, 1H), 7.60-7.50 (m, 7H), 6.50 (d, J=18Hz, 1H), 2.28 (s, 3H), 1.27 (s, 12H) ppm

### Synthesis of compound 8-a

Compound **8-b** (110 mg, 0.332 mmol) and compound **8-f** (84 mg, 0.277 mmol) were dissolved in toluene solution (20 mL), and potassium phosphate (118 mg, 0.554 mmol), cesium fluoride (84 mg, 0.554 mmol) and [1,1'-bis(diphenylphosphino)ferrocene]palladium dichloride (24 mg, 0.028 mmol) were added. The atmosphere of reaction was replaced with nitrogen three times. Then, the reaction solution was heated to 90°C and stirred for 16 hours. The reaction solution was cooled to room temperature, diluted with ethyl acetate (100 mL) and washed with water (100 mL) and saturated brine (100 mL) in turn. The resulting organic phase was dried with anhydrous sodium sulfate, concentrated under reduced pressure, and the residue was purified by silica gel column chromatography (petroleum ether: ethyl acetate = 20:1 to 5:1) to give compound **8-a** (56 mg, yield: 56.6%).

### Synthesis of compound 8

Compound **8-a** (56 mg, 0.156 mmol) was suspended in methanol (10 mL), and (S)-piperidine-2-carboxylic acid (41 mg, 0.313 mmol) and sodium cyanoborohydride (20 mg, 0.313 mmol) were added. The reaction solution was heated to 70°C and stirred for 16 hours. The reaction solution was cooled to room temperature, concentrated under reduced pressure, and the residue was prepared by high performance liquid chromatography to give compound **8** (21.9 mg, yield: 29.7%).

LC-MS (ESI): m/z = 471.0 [M+H]⁺.

¹H NMR (400 MHz, CD₃OD): *δ* 7.85-7.84 (d, J= 7.6 Hz, 1H), 7.67-7.61 (m, 4H), 7.52-7.46 (m, 3H), 7.42-7.38 (m, 4H), 4.57-4.47 (m, 2H), 3.99-3.96 (m, 1H), 3.47-3.44 (m, 1H), 2.95-2.88 (m, 1H), 2.42 (s, 3H), 2.20-2.15 (m, 1H), 1.77-1.61 (m, 4H), 1.52-1.46 (m, 1H) ppm.

### Embodiment 9

### (S,E)-4-(5-Chloro-4-(2-(2-cyano-[1,1'-biphenyl]-3-yl)vinyl)-2-methylbenzyl)morpholine-3-carboxylic acid (compound 9)

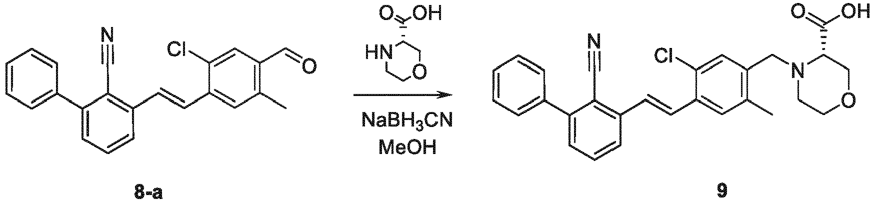

### Synthesis of compound 9

Compound **8-a** (100 mg, 0.279 mmol) and (*S*)-morpholine-3-carboxylic acid (73 mg, 0.559 mmol) were suspended in methanol (10 mL), and sodium cyanoborohydride (36 mg, 0.559 mmol) was added. The reaction solution was heated to 70°C and stirred for 16 hours. After the reaction was completed, the solution was evaporated to dryness, and the obtained white residue was dissolved in ethyl acetate, and washed once with water and once with saturated brine. The reaction solution was cooled to room temperature, diluted with ethyl acetate (100 mL), and washed with water (100 mL) and saturated brine (100 mL) in turn. The obtained organic phase was dried with anhydrous sodium sulfate, concentrated under reduced pressure, and the residue was prepared by high performance liquid chromatography to give compound **9** (7.3 mg, yield: 5.5%).

LC-MS (ESI): m/z = 473.0 [M+H]⁺.

¹H NMR (400 MHz, CD₃OD): *δ* 7.81-7.79 (d, J =7.6Hz, 1H), 7.62-7.57 (m, 2H), 7.53 (s, 1H), 7.45-7.33 (m, 8H), 4.18-4.14 (d, *J*=13.2Hz, 1H), 3.92-3.89 (dd, *J*=3.6Hz, *J*=11.6Hz, 1H), 3.73-3.64 (m, 3H), 3.60-3.55 (m, 1H), 3.35-3.33 (m, 1H), 2.98-2.93 (m, 1H), 2.60-2.56 (m, 1H), 2.33 (s, 3H) ppm.

### Embodiment 10

### (S,E)-1-(4-(2-(2-Cyano-[1,1'-biphenyl]-3-yl)vinyl)-2,5-dimethylbenzyl)pyridine-3-carboxylic acid (compound 10)

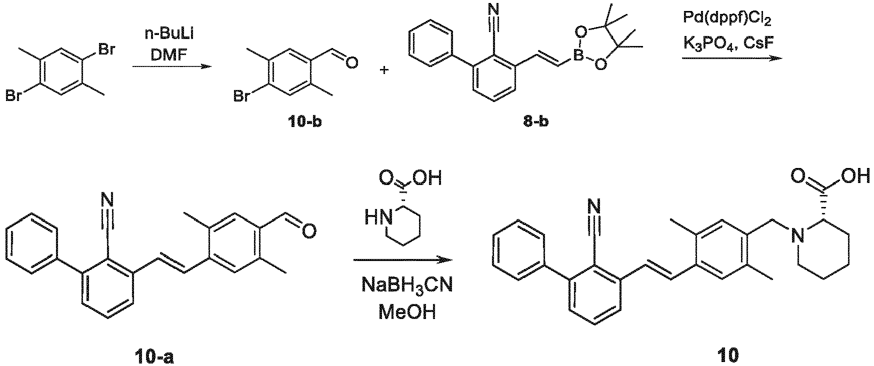

### Synthesis of compound 10-b

1,4-Dibromo-2,5-dimethylbenzene (2.64 g, 10.0 mmol) was dissolved in anhydrous tetrahydrofuran (60 mL), and the solution was cooled to -78°C. Under nitrogen and stirring, 2.4 M n-butyllithium solution (5.0 mL, 12.0 mmol) was added slowly dropwise. After the dropwise addition, the temperature of the reaction solution was slowly raised to -20°C. After ten minutes, the mixture was brought back down to -78°C and anhydrous *N,N'-*dimethylformamide (876 mg, 12.0 mmol) was added dropwise. After stirring for 30 min, the ice water bath was removed, and the temperature of the reaction system was naturally raised to room temperature and continued stirring for 10 hours. The reaction solution was quenched by pouring into an ice-water mixture, then extracted with ethyl acetate (200 mL). The organic phase was washed with water (200 mL) and saturated brine (200 mL), dried with anhydrous sodium sulfate, concentrated under reduced pressure, and the residue was purified by silica gel column chromatography (petroleum ether: ethyl acetate = 100: 1 to 10: 1) to give compound 10-**b** (1.2 g, yield: 56%).
¹H NMR: (400 MHz DMSO-*d*6): *δ* 10.17(s, 3H), 7.75 (s, 1H), 7.62 (s, 1H), 2.56 (s, 3H), 2.38 (s, 3H) ppm

### Synthesis of compound 10-a

Compound **10-b** (106.5 mg, 0.5 mmol) and compound **8-b** (198.6 mg, 0.6 mmol) were dissolved in anhydrous toluene (30 mL), and then [1,1'-bis(diphenylphosphino)ferrocene]dichloropalladium(II) dichloromethane complex (73.1 mg, 0.1 mmol), potassium phosphate (424 mg, 2.0 mmol) and cesium fluoride (304 mg, 2.0 mmol) were added to the mixture. The reaction mixture was stirred at 100°C for 16 h under nitrogen. The reaction solution was cooled to room temperature, concentrated under reduced pressure, and the residue was purified by silica gel column chromatography (petroleum ether: ethyl acetate = 15:1 to 5:1) to give yellow solid **10-a** (80 mg, yield: 48%).

### Synthesis of compound 10

Compound **10-a** (80.0 mg, 0.237 mmol) and (S)-piperidine-2-carboxylic acid (61.2 mg, 0.474 mmol) were dissolved in methanol (20 mL), and sodium cyanoborohydride (37.3 mg, 0.59 mmol) was added. The reaction solution was heated to 60°C, and stirred for 2 hours. The reaction solution was cooled to room temperature, concentrated under reduced pressure, and the residue was prepared by high performance liquid chromatography to give white solid 10 (36.1 mg, yield: 34%).

LC-MS (ESI): m/z = 451.3 [M+H]⁺.

¹H NMR (400 MHz CD₃OD): *δ* 7.96 (d, *J* = 8.0 Hz, 1H), 7.74 (t, *J* = 8.0 Hz, 1H), 7.67 (d, *J* = 16.0 Hz, 1H), 7.59-7.45 (m, 9H), 4.67 (d, *J* = 12.8 Hz, 1H), 4.06 (d, *J* = 12.8 Hz, 1H), 3.56-3.53 (m, 1H), 3.33-3.32 (m, 1H), 3.01-2.98 (m, 1H), 2.50 (s, 3H), 2.49 (s, 3H), 2.31-2.27 (m, 1H), 1.88-1.60 (m, 5H) ppm.

### Embodiment 11

### (S,E)-4-(4-(2-(2-Cyano-[1,1'-biphenyl]-3-yl)vinyl)-2,5-dimethylbenzyl)morpholine-3-carboxylic acid (compound 11)

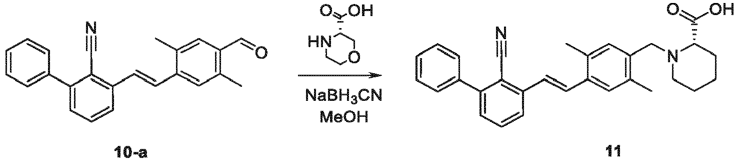

### Synthesis of compound 11

Compound **10-a** (80.0 mg, 0.237 mmol) and (S)-piperidine-3-carboxylic acid (62 mg, 0.474 mmol) were dissolved in methanol (25 mL), and sodium cyanoborohydride (37.3 mg, 0.59 mmol) was added. The reaction was heated to 60°C, and stirred for 2 hours. The reaction solution was cooled to room temperature, concentrated under reduced pressure, and the residue was prepared by high performance liquid chromatography to give white solid **11** (50 mg, yield: 46%).

LC-MS (ESI): m/z = 453.3 [M+H]⁺.

¹H NMR (400 MHz CD₃OD): *δ* 7.95 (d, *J* = 7.6 Hz, 1H), 7.73 (t, *J* = 8.0 Hz, 1H), 7.66 (d, *J*= 16.4 Hz, 1H), 7.59-7.47 (m, 7H), 7.44 (d, *J* = 7.2 Hz, 1H), 7.38 (s, 1H), 4.55 (d, *J*= 12.8 Hz, 1H), 4.16 (dd, *J*₁ = 3.6 Hz, *J*₂ = 12.0 Hz, 1H), 3.94 (d, *J* = 12.8 Hz, 1H), 3.90-3.85 (m, 1H), 3.79-3.61 (m, 3H), 3.13-3.10 (m, 1H), 3.00-2.94 (m, 1H), 2.50 (s, 3H), 2.48 (s, 3H) ppm.

### Embodiment 12

### (S,E)-2-((4-(2-(2-Cyano-[1,1'-biphenyl]-3-yl)vinyl)-2,5-dimethylbenzyl)amino)-3-hydroxy-2-methylpropionic acid (compound 12)

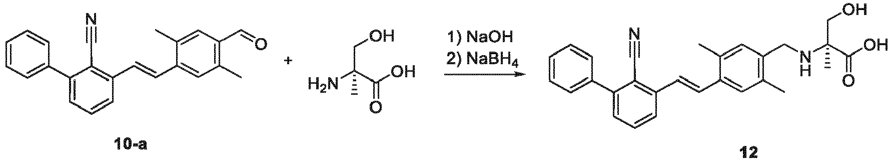

### Synthesis of compound 12

Methyl serine (127 mg, 1.06 mmol) was dissolved in methanol (10 mL), then 0.53 M sodium hydroxide aqueous solution (2 mL, 1.06 mmol) was added dropwise with stirring, and the reaction solution was stirred for 10 min. The reaction solution was cooled to 0°C, and a solution of compound **10-a** (120.0 mg, 0.35 mmol) in tetrahydrofuran (6 mL) was added slowly dropwise. The reaction solution was raised to room temperature and stirred for 16 hours. Sodium borohydride (27.0 mg, 0.71 mmol) was added to the reaction solution, and the reaction solution was continued stirring for 2 hours. The reaction solution was concentrated under reduced pressure, and the residue was prepared by high performance liquid chromatography to give white solid **12** (12 mg, yield: 7%).

LC-MS (ESI): m/z = 441.4 [M+H]⁺.

¹H NMR (400 MHz DMSO-*d₆*): *δ* 8.07 (d, *J* = 7.6 Hz, 1H), 7.77 (t, *J* = 8.0 Hz, 1H), 7.68 (d, *J* = 16.0 Hz, 1H), 7.61-7.47 (m, 6H), 7.43 (s, 2H), 7.32 (d, *J* = 16.0 Hz, 1H), 7.32 (s, 1H), 6.09 (bs, 1H), 3.63 (s, 2H), 3.44-3.38 (m, 2H), 2.40 (s, 3H), 2.32 (s, 3H), 1.15 (s, 3H) ppm.

### Embodiment 13

### (S,E)-1-(5-Chloro-4-(2-(2-cyano-[1,1'-biphenyl]-3-yl)vinyl)-2-methylbenzyl)piperazine-2-carboxylic acid (compound 13)

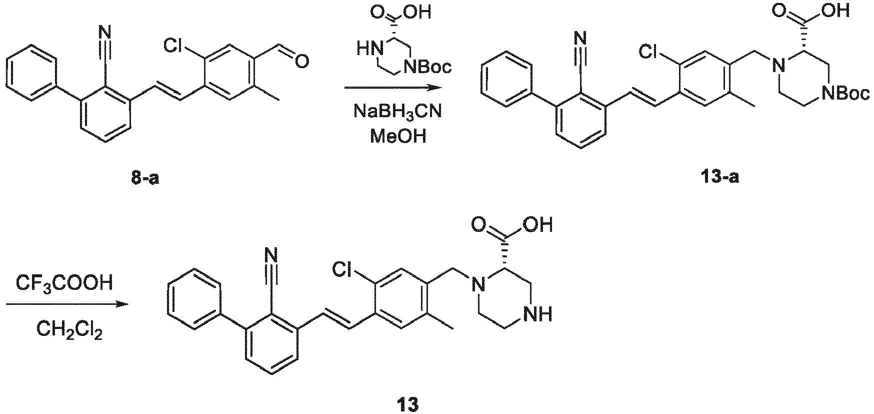

### Synthesis of compound 13-a

Compound **8-a** (100 mg, 0.279 mmol) and (S)-4-Boc-piperazine-2-carboxylic acid (129 mg, 0.559 mmol) were dissolved in methanol (20 mL), and sodium cyanoborohydride (36 mg, 0.559 mmol) was added. The reaction was heated to 70°C, and stirred for 16 hours. The reaction solution was cooled to room temperature, concentrated under reduced pressure, and the residue was dissolved with ethyl acetate (100 mL) and washed with water (100 mL) and saturated brine (100 mL) in turn. The obtained organic phase was dried with anhydrous sodium sulfate, concentrated under reduced pressure, and the residue was prepared by high performance liquid chromatography to give compound **13-a** (28 mg, yield: 17.5%).

LC-MS (ESI): m/z = 572.0 [M+H]⁺.

### Synthesis of compound 13

Compound **13-a** (28 mg, 0.049 mmol) was dissolved in a dichloromethane solution (10 mL), and trifluoroacetic acid (2 mL) was added. The reaction solution was stirred at room temperature for 2 h, concentrated under reduced pressure, and the residue was prepared by high performance liquid chromatography to give compound **13** (10.9 mg, yield: 47.4%).

LC-MS (ESI): m/z = 472.0 [M+H]⁺.

¹H NMR (400 MHz, CD₃OD): *δ* 7.93-7.91 (d, *J* =8.0Hz, 1H), 7.74-7.70 (m, 2H), 7.61-7.55 (m, 4H), 7.51-7.46 (m, 5H), 4.59 (s, 1H), 3.79 (s, 2H), 3.41-3.37 (m, 1H), 3.24-3.20 (m, 2H), 3.13-3.04 (m, 2H), 2.63-2.59 (m, 1H), 2.41 (s, 3H) ppm.

### Embodiment 14

### (S,E)-1-(5-Chloro-4-(2-(2-cyano-[1,1'-biphenyl]-3-yl)vinyl)-2-methylbenzyl)-4-methylpiperazine-2-carboxylic acid (compound 14)

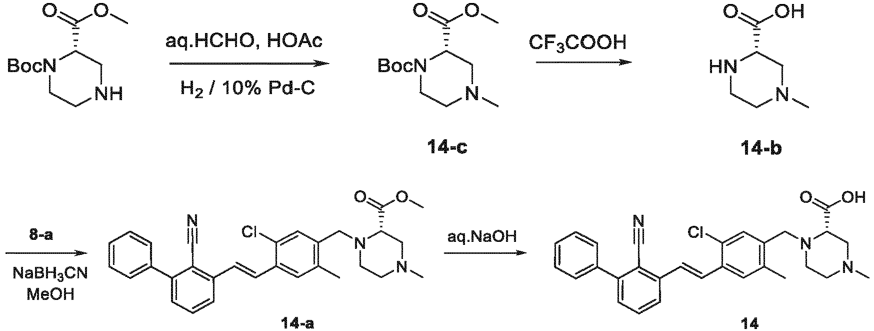

### Synthesis of compound 14-c

(*S*)-1-Boc-piperazine-2-carboxylic acid methyl ester (200 mg, 0.819 mmol) was dissolved in a methanol solution (20 mL), and then 37% formaldehyde aqueous solution (0.5 mL, 4.10 mmol), glacial acetic acid (99 mg, 1.64 mmol) and 10% Pd-C (50 mg) were added. The reaction solution was stirred at room temperature for 16 hours under hydrogen. The reaction solution was filtered through diatomite, washed with methanol (30 mL×3). The filtrate was combined, concentrated under reduced pressure, and the residue was purified by silica gel column chromatography (petroleum ether: ethyl acetate = 1:1) to give compound **14-c** (204 mg, yield: 96.7%).

LC-MS (ESI): m/z = 159.0 [M-Boc+H]⁺.

### Synthesis of compound 14-b

Compound **14-c** (204 mg, 0.79 mmol) was dissolved in dichloromethane (30 mL), and trifluoroacetic acid (6 mL) was added. The reaction solution was stirred at room temperature for 2 h, concentrated under reduced pressure. The residue was added with dichloromethane (30 mL), then concentrated under reduced pressure, and repeated once. The residue was added with toluene (30 mL), then concentrated under reduced pressure, and repeated once. The residue was vacuum dried to give compound **14-b** (123 mg, yield: 99.0%), which was used directly in the next reaction step.

LC-MS (ESI): m/z = 159.0 [M+H]⁺.

### Synthesis of compound 14-a

Compound **8-a** (136 mg, 0.38 mmol) and compound **14-b** (195 mg, 0.76 mmol) were dissolved in a methanol solution (20 mL), and then sodium cyanoborohydride (48 mg, 0.76 mmol) and sodium acetate (155 mg, 1.14 mmol) were added. The reaction solution was heated to 70°C and stirred for 16 hours. The reaction solution was cooled to room temperature, concentrated under reduced pressure, and the residue was dissolved with dichloromethane (100 mL), washed with water (100 mL) and saturated brine (100 mL) in turn. The obtained organic phase was dried with anhydrous sodium sulfate, concentrated under reduced pressure, and the residue was prepared by high performance liquid chromatography to give compound **14-a** (30 mg, yield: 15.8%).

LC-MS (ESI): m/z = 500.0 [M+H]⁺.

### Synthesis of compound 14

Compound **14-a** (70 mg, 0.185 mmol) was dissolved in a mixture of methanol (5 mL), tetrahydrofuran (5 mL) and water (1 mL), and sodium hydroxide (23 mg, 0.371 mmol) was added. The reaction solution was stirred at room temperature for 16 hours. After the reaction was completed, the reaction was concentrated under reduced pressure, and the residue was diluted with water (50 mL). The pH of the solution was adjusted to 3 to 5 with dilute hydrochloric acid, and a white solid was precipitated. After filtration, the filter cake was prepared by high performance liquid chromatography to give compound **14** (11.0 mg, yield: 37.9%).

LC-MS (ESI): m/z = 486.0 [M+H]⁺.

¹H NMR (400 MHz, CD₃OD): *δ* 7.92-7.90 (d, *J* =8.0Hz, 1H), 7.74-7.69 (m, 2H), 7.60-7.55 (m, 3H), 7.54-7.52 (m, 1H), 7.51-7.41 (m, 5H), 3.82 (s, 2H), 3.40 (s, 2H), 3.22-3.20 (d, *J* =8.4Hz, 2H), 3.07-3.05 (d, *J*=9.2Hz, 1H), 2.79 (s, 3H), 2.69-2.65 (m, 1H), 2.40 (s, 3H) ppm.

### Embodiment 15

### (S,E)-1-(4-(2-(2-Cyano-[1,1'-biphenyl]-3-yl)vinyl)-2-(4-cyanobutoxy)-5-methylbenzyl)piperidine-2-carboxylic acid (compound 15)

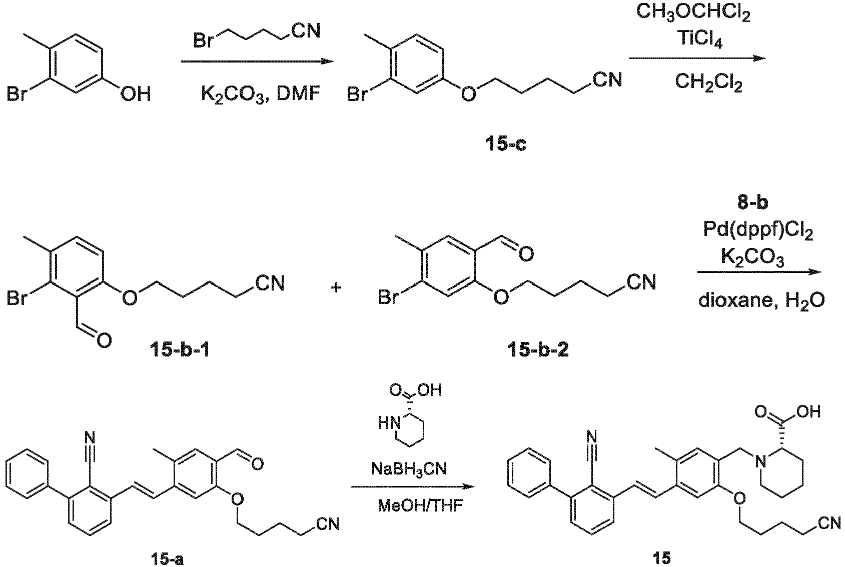

### Synthesis of compound 15-c

A mixture of 3-bromo-4-methylphenol (374 mg, 1.0 mmol), 5-bromovaleronitrile (486 mg, 3.0 mmol), potassium carbonate (553 mg, 4 mmol), and *N*,*N*'-dimethylformamide (5 mL) was heated to 60°C and stirred for 16 h. The reaction solution was cooled to room temperature and diluted with water (20 mL). The resulting mixture was extracted with ethyl acetate (20 mL^{∗}2). The organic phase was combined, washed with saturated brine (20 mL), dried with anhydrous sodium sulfate, and concentrated under reduced pressure. The resulting residue was purified by silica gel column chromatography (petroleum ether: ethyl acetate = 10:1) to give a white solid **15-c** (536 mg, yield: 100%).

¹H NMR (400 MHz, CDCl₃): *δ* 7.12 (d, *J* = 8.4 Hz, 1H), 7.08 (d, *J* = 2.6 Hz, 1H), 6.74 (dd, *J* = 8.4, 2.6 Hz, 1H), 3.96 (t, *J* = 5.7 Hz, 2H), 2.44 (t, *J* = 6.8 Hz, 2H), 2.32 (s, 3H), 1.97 - 1.82 (m, 4H) ppm

### Synthesis of compounds 15-b-1 and 15-b-2

Compound **15-c** (268 mg, 1 mmol) and 1,2-dichloromethyl methyl ether (138 mg, 1.2 mmol) were dissolved in dichloromethane (5 mL). Titanium tetrachloride (569 mg, 3.0 mmol) was added dropwise at 0°C and stirred for 2 h. The reaction solution was quenched with ice water (20 mL), and the resulting mixture was extracted with dichloromethane (20 mL). The organic phase was dried with anhydrous sodium sulfate, concentrated under reduced pressure, and the resulting residue was purified by silica gel column chromatography (petroleum ether: ethyl acetate = 3:1) to give white solid **15-b-1** (141 mg, yield: 48%) and pale yellow solid **15-b-2** (100 mg, yield: 34%).

### Compound 15-b-1:

¹H NMR (400 MHz, CDCl₃): *δ* 10.39 (s, 1H), 7.67 (s, 1H), 7.18 (s,1H), 4.11 (t, *J* = 5.9 Hz, 2H), 2.47 (t, *J* = 6.9 Hz, 2H), 2.36 (s, 3H), 2.03 (dt, *J* = 8.8, 5.8 Hz, 2H), 1.96 - 1.84 (m, 2H) ppm

### Compound 15-b-2:

¹H NMR (400 MHz, CDCl₃): *δ* 10.41 (s, 1H), 7.34 (d, *J* = 8.5 Hz, 1H), 6.85 (d, *J* = 8.5 Hz, 1H), 4.07 (t, *J* = 5.7 Hz, 2H), 2.48 (t, *J* = 6.8 Hz, 2H), 2.08 - 1.85 (m, 4H) ppm

### Synthesis of compound 15-a

A mixture of compound **15-b-2** (135 mg, 0.46 mmol), compound **8-b** (181 mg, 0.55 mmol), [1,1'-bis(diphenylphosphino)ferrocene]palladium dichloride (37 mg, 0.05 mmol), potassium carbonate (126 mg, 0.91 mmol), 1,4-dioxane (2 mL) and water (0.2 mL) was stirred for 16 h at 90°C under nitrogen. The reaction solution was cooled to room temperature and diluted with saturated brine (20 mL). The resulting mixture was extracted with dichloromethane (50 mL×2). The organic phase was combined, dried with anhydrous sodium sulfate, filtered, and concentrated under reduced pressure. The resulting residue was purified by silica gel column chromatography (petroleum ether: ethyl acetate = 2:1) to give compound **15-a** (130 mg, yield: 68%).

LC-MS (ESI): m/z = 421.4[M+H]⁺.
¹H NMR (400 MHz, CDCl₃): *δ* 10.45 (s, 1H), 7.80 (d, *J* = 7.6 Hz, 1H), 7.70 - 7.63 (m, 2H), 7.60 - 7.42 (m, 8H), 7.21 (s, 1H), 4.22 (t, *J* = 5.9 Hz, 2H), 2.48 (t, *J* = 7.0 Hz, 2H), 2.42 (s, 3H), 2.12 - 2.02 (m, 2H), 2.00 - 1.89 (m, 2H) ppm

### Synthesis of compound 15

Sodium cyanoborohydride (15 mg, 0.24 mmol) was added to a mixture of compound **15-a** (50 mg, 0.12 mmol), (*S*)-piperidine-2-carboxylic acid (31 mg, 0.24 mmol), methanol (1 mL) and tetrahydrofuran (1 mL). The reaction solution was heated to 70°C and stirred for 2 hours. The reaction solution was cooled to room temperature, concentrated under reduced pressure, and the residue was prepared by high performance liquid chromatography to give white solid **15** (32 mg, yield: 50%).

LC-MS (ESI): m/z = 534.5 [M+H]⁺.

¹H NMR (400 MHz, CD₃OD): *δ* 7.96 (d, *J* = 7.8 Hz, 1H), 7.73 (t, *J* = 7.9 Hz, 1H), 7.67 (d, *J* = 16.1 Hz, 1H), 7.60 - 7.54 (m, 2H), 7.54 - 7.44 (m, 5H), 7.40 (s, 1H), 7.33 (s, 1H), 4.50 (d, *J* = 12.9 Hz, 1H), 4.34 (d, *J* = 12.7 Hz, 1H), 4.22 (t, *J* = 6.1 Hz, 2H), 3.53 (d, *J* = 7.5 Hz, 1H), 3.32 (s, 1H), 2.99 (t, *J* = 12.3 Hz, 1H), 2.57 (t, *J* = 7.0 Hz, 2H), 2.45 (s, 3H), 2.29 - 2.16 (m, 1H), 2.09 - 1.99 (m, 2H), 1.95 - 1.65 (m, 6H), 1.63 - 1.50 (m, 1H) ppm.

### Embodiment 16

### (E)-(4-(2-(2-Cyano-[1,1'-biphenyl]-3-yl)vinyl)-2-(4-cyanobutoxy)-5-methylbenzyl)glycine (compound 16)

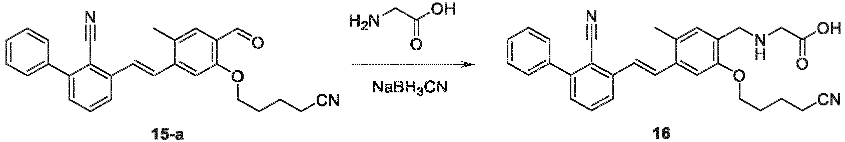

### Synthesis of compound 16

Sodium cyanoborohydride (60 mg, 0.95 mmol) was added to a mixture of compound **15-a** (80 mg, 0.19 mmol), glycine (57 mg, 0.76 mmol), methanol (1 mL) and tetrahydrofuran (1 mL). The reaction solution was heated to 70°C and stirred for 1 h, after that, sodium cyanoborohydride (60 mg, 0.95 mmol) was added, and the reaction solution was continued stirring for 1 h. The reaction solution was cooled to room temperature, concentrated under reduced pressure, and the residue was prepared by high performance liquid chromatography to give white solid **16** (19.6 mg, yield: 21%).

LC-MS (ESI): m/z = 480.5 [M+H]⁺.

¹H NMR (400 MHz, DMSO-*d₆*): *δ* 8.05 (d, *J* = 7.9 Hz, 1H), 7.79 (t, *J* = 7.9 Hz, 1H), 7.67 (d, *J* = 16.0 Hz, 1H), 7.62 - 7.47 (m, 6H), 7.40 (d, *J* = 16.0 Hz, 1H), 7.26 (d, *J* = 5.6 Hz, 2H), 4.12 (t, *J* = 6.0 Hz, 2H), 3.94 (s, 2H), 3.13 (s, 2H), 2.60 (t, *J* = 7.0 Hz, 2H), 2.38 (s, 3H), 1.93 - 1.84 (m, 2H), 1.83 - 1.74 (m, 2H) ppm.

### Embodiment 17

### (S,E)-4-(4-(2-(2-Cyano-[1,1'-biphenyl]-3-yl)vinyl)-2-(4-cyanobutoxy)-5-methylbenzyl)morpholine-3-carboxylic acid (compound 17)

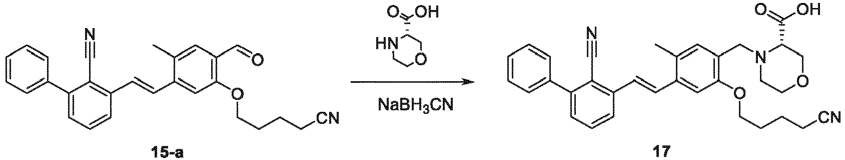

### Synthesis of compound 17

Sodium cyanoborohydride (38 mg, 0.60 mmol) was added to a mixture of compound **15-a** (63 mg, 0.15 mmol), (S)-morpholine-3-carboxylic acid (39 mg, 0.30 mmol), methanol (1 mL) and tetrahydrofuran (1 mL). The reaction solution was heated to 65°C and stirred for 1 hour. The reaction solution was cooled to room temperature, concentrated under reduced pressure, and the residue was prepared by high performance liquid chromatography to give white solid **17** (46.1 mg, yield: 57%).

LC-MS (ESI): m/z = 536.4 [M+H]⁺.

¹H NMR (400 MHz, CD₃OD): δ 7.95 (d, *J* = 7.9 Hz, 1H), 7.72 (t, *J* = 7.9 Hz, 1H), 7.66 (d, *J* = 16.1 Hz, 1H), 7.59 - 7.54 (m, 2H), 7.54 - 7.43 (m, 5H), 7.40 (s, 1H), 7.32 (s, 1H), 4.50 (d, *J* = 12.8 Hz, 1H), 4.33 (d, *J* = 12.7 Hz, 1H), 4.26 - 4.13 (m, 3H), 3.93 (d, *J* = 12.8 Hz, 1H), 3.82 - 3.64 (m, 3H), 3.25 (d, *J* = 12.6 Hz, 1H), 3.09 (t, *J* = 9.8 Hz, 1H), 2.57 (t, *J* = 7.0 Hz, 2H), 2.44 (s, 3H), 2.09 - 1.98 (m, 2H), 1.95 - 1.83 (m, 2H) ppm.

### Embodiment 18

### (S,E)-1-(4-(2-(2-Cyano-[1,1'-biphenyl]-3-yl)vinyl)-2-(4-cyanobutoxy)-5-methylbenzyl)piperazine-2-carboxylic acid (compound 18)

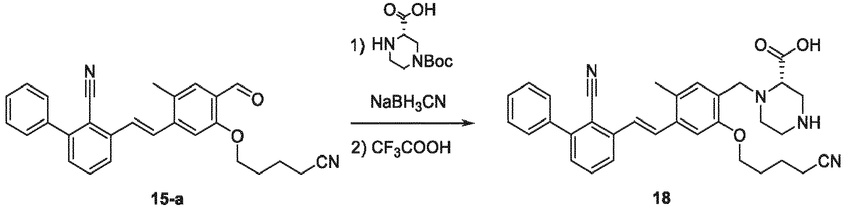

### Synthesis of compound 18

Sodium cyanoborohydride (38 mg, 0.60 mmol) was added to a mixture of compound **15-a** (63 mg, 0.15 mmol), (*S*)-4-(*tert*-butoxycarbonyl)piperazine-2-carboxylic acid (69 mg, 0.30 mmol), methanol (1 mL) and tetrahydrofuran (1 mL). The reaction solution was heated to 60°C and stirred for 1 hour, cooled to room temperature, concentrated under reduced pressure. The obtained residue was dissolved in dichloromethane (1 mL) and trifluoroacetic acid (1 mL), and stirred at room temperature for 16 hours. The reaction solution was concentrated under reduced pressure, and the residue was prepared by high performance liquid chromatography to give white solid **18** (36.5 mg, yield: 45%).

LC-MS (ESI): m/z = 535.5 [M+H]⁺.

¹H NMR (400 MHz, CD₃OD): *δ* 7.94 (d, *J* = 8.0 Hz, 1H), 7.71 (t, *J* = 7.9 Hz, 1H), 7.65 (d, *J* = 16.0 Hz, 1H), 7.59 - 7.39 (m, 7H), 7.31 (s, 1H), 7.22 (s, 1H), 4.12 (t, *J* = 5.8 Hz, 2H), 3.92 (s, 2H), 3.42 (t, *J* = 4.9 Hz, 1H), 3.29 (s, 1H), 3.19 - 3.05 (m, 3H), 2.69 (m, 1H), 2.56 (t, *J* = 6.9 Hz, 2H), 2.42 (s, 3H), 2.03 - 1.83 (m, 4H) ppm.

### Embodiment 19

### (E)-(5-Chloro-4-(2-(2-cyano-[1,1'-biphenyl]-3-yl)vinyl)-2-methylbenzyl)glycine (compound 19 )

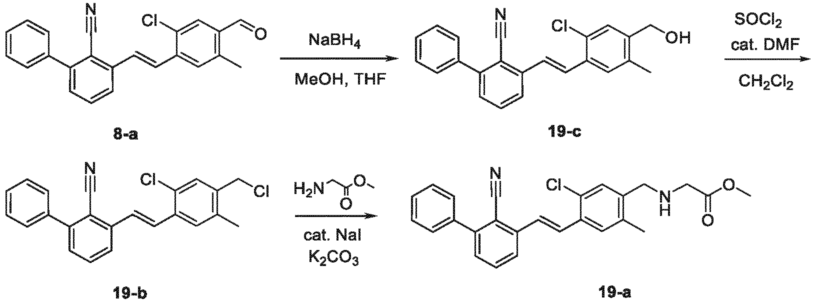

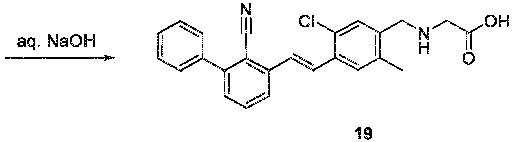

### Synthesis of compound 19-c

Compound **8-a** (260 mg, 0.727 mmol) was dissolved in a mixed solution of methanol (5 mL) and tetrahydrofuran (5 mL), and sodium borohydride (55 mg, 1.454 mmol) was added, and the mixture was stirred at room temperature for 2 hours. The reaction solution was concentrated under reduced pressure, and the residue was dissolved with ethyl acetate (50 mL), washed with water (50 mL) and saturated brine (50 mL) in turn. The organic phase was dried with anhydrous sodium sulfate, concentrated under reduced pressure, and the residue was purified by silica gel column chromatography (petroleum ether: ethyl acetate = 20:1 to 3:1) to give compound **19-c** (218 mg, yield: 83.8%).

### Synthesis of compound 19-b

Compound **19-c** (116 mg, 0.322 mmol) was dissolved in dichloromethane (10 mL), and one drop of *N*,*N*'-dimethylformamide was added, and then sulfoxide chloride (0.5 mL) was added dropwise, and the reaction solution was stirred for 2 h at room temperature. The reaction solution was concentrated under reduced pressure, and the residue was concentrated under reduced pressure after adding dichloromethane (50 mL×2). The residue was vacuum dried to give compound **19-b** (122 mg, yield: 99.0%), which was used directly in the next reaction step.

### Synthesis of compound 19-a

Methyl serine hydrochloride (41 mg, 0.322 mmol) and compound **19-b** (122 mg, 0.322 mmol) were dissolved in acetonitrile (20 mL), and then potassium carbonate (223 mg, 1.61 mmol) and sodium iodide (10 mg, 0.065 mmol) were added. The reaction solution was heated to 80°C and stirred for 16 hours. The reaction solution was cooled to room temperature, concentrated under reduced pressure, and the residue was dissolved with dichloromethane (50 mL), then washed with water (50 mL) and saturated brine (50 mL) in turn. The resulting organic phase was dried with anhydrous sodium sulfate, concentrated under reduced pressure, and the residue was purified by silica gel column chromatography (petroleum ether: ethyl acetate = 10:1 to 1:2) to give compound **19-a** (63 mg, yield: 45.5%).

LC-MS (ESI): m/z = 431.0 [M+H]⁺.

### Synthesis of compound 19

Compound **19-a** (63 mg, 0.146 mmol) was dissolved in a mixture of methanol (5 mL), tetrahydrofuran (5 mL) and water (1 mL), then sodium hydroxide (12 mg, 0.292 mmol) was added, and the reaction solution was stirred at room temperature for 16 h. After the reaction was completed, the reaction solution was concentrated under reduced pressure. The residue was diluted with water (50 mL), and the pH of the solution was adjusted to 3 to 5 with dilute hydrochloric acid to precipitate a white solid. The mixture was filtered, and the filter cake was prepared by high performance liquid chromatography to give compound **19** (10.0 mg, yield: 16.4%).

LC-MS (ESI): m/z = 417.0 [M+H]⁺.

¹H NMR (400 MHz, CD₃OD): *δ*7.97-7.95 (d, *J* =8.0Hz, 1H), 7.79-7.72 (m, 3H), 7.64-7.57 (m, 4H), 7.52-7.47 (m, 4H), 4.35 (s, 2H), 4.05 (s, 2H), 2.52 (s, 3H) ppm.

### Embodiment 20

### (S,E)-1-(4-(2-(2-Cyano-[1,1'-biphenyl]-3-yl)vinyl)-2-hydroxymethyl-5-trifluoromethylbenzyl)piperidine-2-carboxylic acid (compound 20)

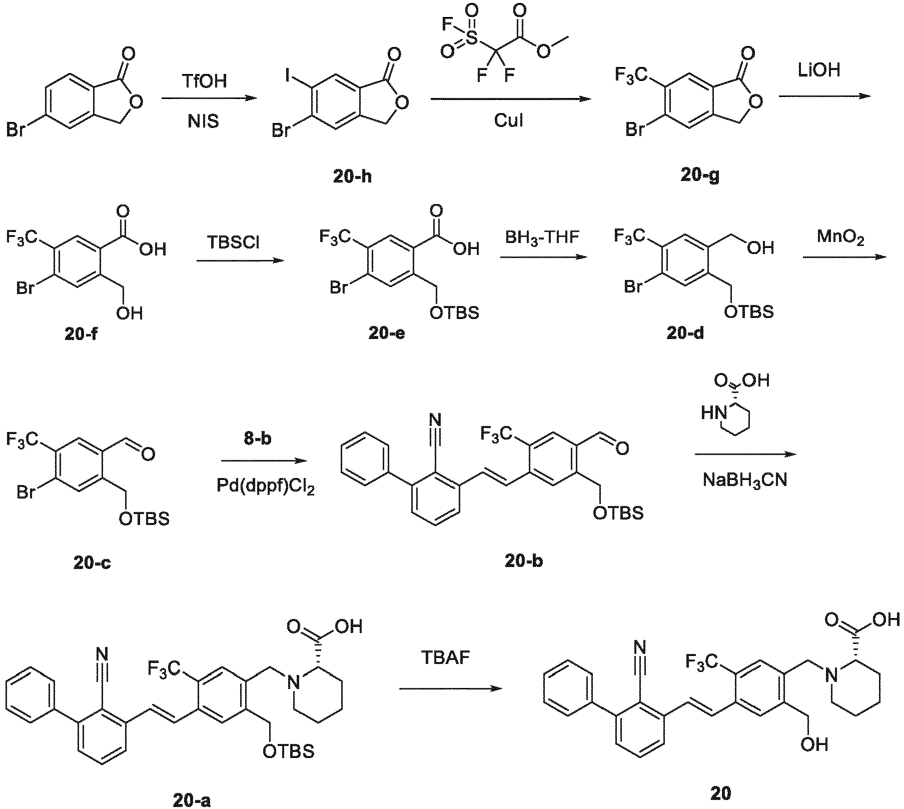

### Synthesis of compound 20-h

5-Bromophthalide (6.3 g, 30 mmol) was dissolved in trifluoromethanesulfonic acid (60 mL) and cooled to 0°C with stirring. N-Iodosuccinimide (16.8 g, 75 mmol) powder was slowly added to the reaction system. Subsequently, the ice bath was removed, and the mixture was stirred at room temperature for 16 hours. The reaction mixture was poured into ice water (200 mL) and a large amount of dark yellow solid was precipitated, filtered, and the solid was washed with water (500 mL×3). The solid was dissolved in dichloromethane (500 mL), and washed with saturated sodium thiosulfate solution (100 mL×2). The organic phase was washed again with water (200 mL), dried with anhydrous sodium sulfate, and concentrated under reduced pressure to give dark yellow solid **20-h** (9.0 g, yield: 89.1%). The product was used directly in the next reaction step without further purification.

### Synthesis of compound 20-g

Compound **20-h** (6.7 g, 20 mmol), methyl fluorosulfonyl difluoroacetate (19 g, 100 mmol) were mixed with cuprous iodide (760 mg, 2 mmol) in *N*,*N*-dimethylformamide (50 mL), and the reaction was stirred at 90°C for 16 h under nitrogen. The reaction solution was cooled to room temperature, filtered, and the filtrate was added with ethyl acetate (300 mL), washed with water (50 mL×3) and saturated brine (50 mL) in turn, dried with anhydrous sodium sulfate, concentrated under reduced pressure, and the residue was purified by silica gel column chromatography (petroleum ether: ethyl acetate = 5:1) to give white solid **20-g** (900 mg, yield: 16.0%).

LC-MS (ESI): m/z = 282 [M+H]⁺.

### Synthesis of compound 20-f

Compound **20-g** (800 mg, 2.8 mmol) was dissolved in a mixture of tetrahydrofuran (18 mL) and water (2 mL). Lithium hydroxide monohydrate (420 mg, 10 mmol) was added thereto, and stirred at room temperature for 16 hours. The pH was adjusted to 1 with 1N hydrochloric acid, and the mixture was concentrated under reduced pressure and purified by reversed-phase chromatography (Biotage Flash) to give white solid **20-f** (650 mg, yield: 75.6%).

LC-MS (ESI): m/z = 299 [M-H]⁺.

### Synthesis of compound 20-e

Compound **20-f** (600 mg, 2 mmol) was dissolved in *N*,*N*-dimethylformamide (10 mL), then imidazole (260 mg, 4 mmol) and *tert*-butyldimethylchlorosilane (450 mg, 3 mmol) were added. The reaction solution was stirred at room temperature for 16 hours. Ethyl acetate (100 mL) was added to the reaction solution. The reaction solution was washed with water (20 mL×3) and saturated brine (50 mL) in turn, dried with anhydrous sodium sulfate, concentrated under reduced pressure, and the residue was purified by silica gel column chromatography (petroleum ether: ethyl acetate = 10:1) to give compound **20-e** (600 mg, yield: 72.6%).

¹H NMR (400 MHz, DMSO-*d₆*): *δ* 8.19 (s, 1H), 8.11 (s, 1H), 5.10 (s, 2H), 5.10 (s, 9H), 0.11 (s, 6H) ppm

### Synthesis of compound 20-d

Compound **20-e** (413 mg, 1 mmol) was dissolved in anhydrous tetrahydrofuran (10 mL), then a solution of borane in tetrahydrofuran (5 mL, 5 mmol) was added dropwise at 0°C under nitrogen, and the mixture was refluxed for 16 h after the dropwise addition. The reaction solution was cooled to room temperature, and methanol (10 mL) was slowly added dropwise. The reaction solution was refluxed for one hour after the dropwise addition. The reaction solution was cooled to room temperature, concentrated under reduced pressure, and the residue was purified by silica gel column chromatography (petroleum ether: ethyl acetate = 10:1) to give compound **20-d** (260 mg, yield: 65.0%).

LC-MS (ESI): m/z = 399 [M-H]⁺.

### Synthesis of compound 20-c

Compound **20-d** (210 mg, 0.52 mmol) was dissolved in dichloromethane (10 mL), and manganese dioxide (450 mg, 5.2 mmol) was added. The reaction was stirred at room temperature for 16 hours. The reaction solution was filtered and washed with dichloromethane (10 mL×3). The filtrate was concentrated under reduced pressure, and the residue was purified by silica gel column chromatography (petroleum ether: ethyl acetate = 10:1) to give compound **20-c** (150 mg, yield: 71.5%). The product required no further purification.

### Synthesis of compound 20-b

A mixture of compound **20-c** (120 mg, 0.42 mmol), compound **8-b** (141 mg, 0.42 mmol), sodium carbonate (132 mg, 1.26 mmol), [1,1'-bis(diphenylphosphino)ferrocene]palladium dichloride (31 mg, 0.042 mmol), 1,4-dioxane (5 mL) and water (0.5 mL) was stirred at 80°C for 16 hours under nitrogen. The reaction solution was cooled to room temperature, concentrated under reduced pressure, and the residue was purified by silica gel column chromatography (petroleum ether: ethyl acetate = 10:1) to give compound **20-b** (65 mg, yield: 29.8%).

LC-MS (ESI): m/z = 522 [M+H]⁺.

### Synthesis of compound 20-a

A mixture of compound **20-b** (50 mg, 0.096 mmol), L-piperidine-2-carboxylic acid (24 mg, 0.19 mmol), sodium cyanoborohydride (12 mg, 0.19 mmol) and methanol (3 mL) was stirred at 80°C for 3 h under nitrogen. The reaction solution was cooled to room temperature, concentrated under reduced pressure, and the residue was purified by reversed-phase chromatography (Biotage Flash) to give compound **20-a** (35 mg, yield: 57.6%).

LC-MS (ESI): m/z = 635 [M+H]⁺.

### Synthesis of compound 20

Compound **20-a** (30 mg, 0.047 mmol) was dissolved in tetrahydrofuran (5 mL), and 1.0 M tetrabutylammonium fluoride solution (0.5 mL, 0.5 mmol) was added. The reaction solution was stirred at room temperature for 16 h, concentrated under reduced pressure, and the residue was prepared by high performance liquid chromatography to give compound 20 (8 mg, yield: 33.3%).

LC-MS (ESI): m/z = 521 [M+H]⁺.

¹H NMR (400 MHz, CD₃OD): *δ* 8.04 (s, 1H), 7.93 (s, 1H), 7.89 (d, *J*=8.0Hz, 1H), 7.77 (t, *J*=8.0Hz, 1H), 7.72-7.62 (m, 2H), 7.59-7.46 (m, 6H), 5.04 (d, *J*=12.4Hz, 1H), 4.92-4.91 (m, 1H), 4.81-4.80 (m, 1H), 4.21 (d, *J*=12.8Hz, 1H), 3.56-3.53 (m, 1H), 3.21-3.18 (m, 1H), 2.95-2.89 (m, 1H), 2.26-2.23 (m, 1H), 1.94-1.76 (m, 3H), 1.63-1.58 (m, 2H) ppm.

### Embodiment 21

### (S,E)-4-(4-(2-(2-Cyano-[1,1'-biphenyl]-3-yl)vinyl)-2-hydroxymethyl-5-trifluoromethylbenzyl)morpholine-3-carboxylic acid (compound 21)

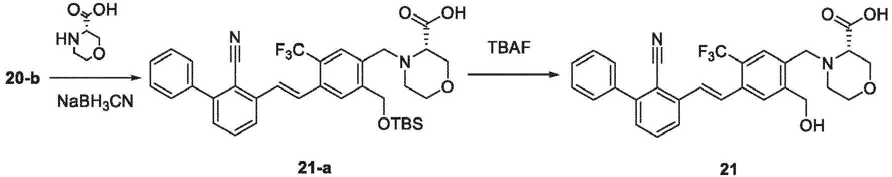

### Synthesis of compound 21-a

A mixture of compound **20-b** (50 mg, 0.096 mmol), L-morpholine-3-carboxylic acid (24 mg, 0.19 mmol), sodium cyanoborohydride (12 mg, 0.19 mmol) and methanol (3 mL) was stirred at 80°C for 3 h under nitrogen. The reaction solution was cooled to room temperature, concentrated under reduced pressure, and the residue was purified by reversed-phase chromatography (Biotage Flash) to give compound **21-a** (35 mg, yield: 57.6%).

LC-MS (ESI): m/z = 635 [M+H]⁺.

### Synthesis of compound 21

Compound **21-a** (30 mg, 0.047 mmol) was dissolved in tetrahydrofuran (5 mL), then 1.0 M tetrabutylammonium fluoride solution (0.5 mL, 0.5 mmol) was added. The reaction was stirred at room temperature for 16 h, concentrated under reduced pressure, and the residue was prepared by high performance liquid chromatography to give compound **21** (8 mg, yield: 33.3%).

LC-MS (ESI): m/z = 523 [M+H]⁺.

¹H NMR (400 MHz, CD₃OD): *δ* 8.03(s, 1H), 7.87 (d, *J*=8.0Hz, 1H), 7.81 (s, 1H), 7.75(t, *J*=8.0Hz, 1H), 7.67-7.47 (m, 8H), 4.98 (d, *J*=13.2Hz, 1H), 4.81-4.80 (m, 1H), 4.51 (d, *J*=12.8Hz, 1H), 4.03-3.99 (m, 1H), 3.88-3.74 (m, 3H), 3.66-3.61 (m,1H), 3.43-3.42 (m, 1H), 3.00-2.96 (m, 1H), 2.63-2.59 (m, 1H) ppm.

### Embodiment 22

### (S,E)-4-(5-Chloro-2-methyl-4-(2-(2-methyl-[1,1'-biphenyl]-3-yl)vinyl)benzyl)morpholine-3-carboxylic acid (compound 22)

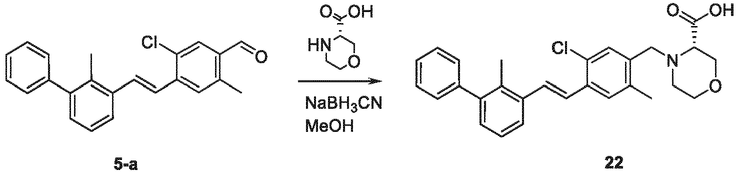

### Synthesis of compound 22

Compound **5-a** (100 mg, 0.288 mmol) and (*S*)-morpholine-3-carboxylic acid (75.7 mg, 0.577 mmol) were dissolved in methanol (10 mL), and sodium cyanoborohydride (36.3 mg, 0.577 mmol) was added, then the reaction solution was heated to 70°C and stirred for 16 hours. The reaction solution was cooled to room temperature, concentrated under reduced pressure, and the residue was prepared by high performance liquid chromatography to give white solid **22** (37.2 mg, yield: 27.8%).

LC-MS (ESI): m/z = 462.0 [M+H]⁺.

¹H NMR (400 MHz, CD₃OD): *δ* 7.69 (s, 1H), 7.60-7.52 (m, 3H), 7.44-7.40 (m, 2H), 7.36-7.24 (m, 5H), 7.16-7.14 (d, *J* = 7.6Hz, 2H), 4.44-4.40 (d, *J* = 13.2Hz, 2H), 4.11-4.07 (dd, J = 4.0Hz, *J* = 12.4Hz, 1H), 3.93-3.90 (d, *J* = 13.2Hz, 1H), 3.86-3.79 (m, 2H), 3.74-3.68 (m, 1H), 3.61-3.58 (m, 1H), 3.15-3.12 (m, 1H), 2.91-2.86 (m, 1H), 2.48 (s, 3H), 2.29 (s, 3H) ppm.

### Embodiment 23

### (S,E)-1-(5-Chloro-2-methyl-4-(2-(2-methyl-[1,1'-biphenyl]-3-yl)vinyl)benzyl)piperazine-2-carboxylic acid (compound 23)

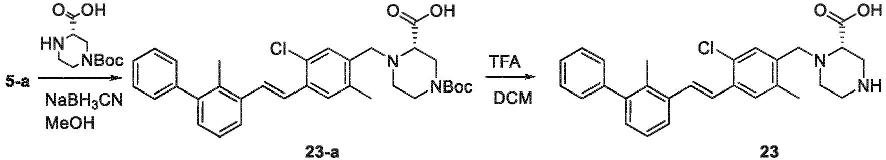

### Synthesis of compound 23-a

Compound **5-a** (100 mg, 0.288 mmol) and (*S*)-4-(*tert*-butoxycarbonyl)piperazine-2-carboxylic acid (133 mg, 0.577 mmol) were dissolved in methanol (10 mL), and sodium cyanoborohydride (36 mg, 0.577 mmol) was added, then the reaction solution was heated to 70°C and stirred for 16 hours. The reaction solution was cooled to room temperature, concentrated under reduced pressure, and the residue was prepared by high performance liquid chromatography to give compound **23-a** (76 mg, yield: 46.9%).

LC-MS (ESI): m/z = 561.0 [M+H]⁺.

### Synthesis of compound 23

Compound **23-a** (76 mg, 0.135 mmol) was dissolved in dichloromethane (10 mL), then trifluoroacetic acid (2 mL) was added, and the reaction solution was stirred for 2 h at room temperature. The reaction solution was concentrated under reduced pressure, and the residue was prepared by high performance liquid chromatography to give compound **23** (29.4 mg, yield: 46.8%).

LC-MS (ESI): m/z = 461.0 [M+H]⁺.

¹H NMR (400 MHz, CD₃OD): *δ* 7.63 (s, 1H), 7.59-7.57 (d, *J* = 7.6Hz, 1H), 7.51-7.47 (d, *J* = 16.0Hz, 1H), 7.44-7.41 (m, 3H), 7.37-7.24 (m, 5H), 7.15-7.13 (d, *J* = 7.2Hz, 1H), 3.96-3.85 (q, *J* = 13.6Hz, *J* = 16.4Hz, 2H), 3.75-3.73 (t, *J* = 4.0Hz, 1H), 3.59-3.55 (dd, *J* = 4.4Hz, *J* = 13.2Hz, 1H), 3.38-3.34 (dd, *J* = 4.0Hz, *J* = 12.8Hz, 1H), 3.25-3.19 (m, 2H), 3.12-3.06 (m, 1H), 2.84-2.80(m, 1H), 2.41 (s, 3H), 2.29 (s, 3H) ppm.

### Embodiment 24

### (S,E)-1-(4-(2-(2-Cyano-[1,1'-biphenyl]-3-yl)vinyl)-2-(2-methoxyethoxy)-5-methylbenzyl)piperidine-2-carboxylic acid (compound 24)

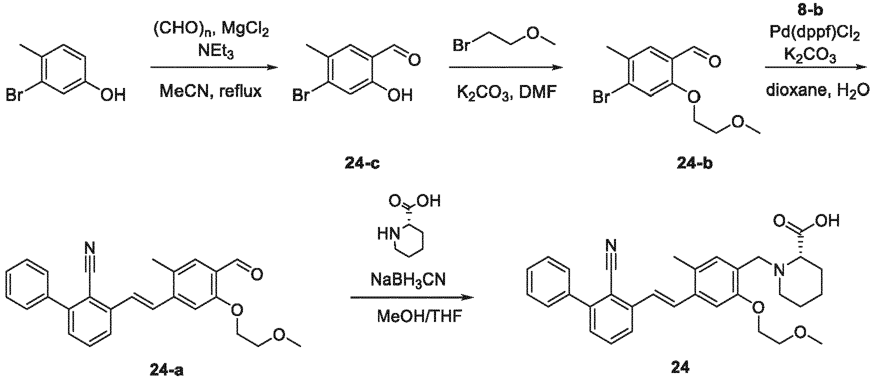

### Synthesis of compound 24-c

A mixture of 3-bromo-4-methylphenol (3.74 g, 20 mmol), paraformaldehyde (4.41 g, 152 mmol), magnesium chloride (2.86 g, 30 mmol), triethylamine (7.56 g, 75 mmol) and acetonitrile (150 mL) was refluxed for 4 hours. The reaction solution was cooled to room temperature, diluted with water (500 mL), and pH was adjusted to 2-3 with 1M hydrochloric acid. The resulting mixture was extracted with ethyl acetate (500 mL×2). The organic phase was combined, washed with saturated brine (200 mL), concentrated under reduced pressure, and the residue was purified by silica gel column chromatography (petroleum ether: ethyl acetate = 50:1) to give white solid **24-c** (2.45 g, yield: 57%).

### Synthesis of compound 24-b

A mixture of compound **24-c** (645 mg, 3.0 mmol), 1-bromo-2-methoxyethane (625 mg, 4.5 mmol), potassium carbonate (829 mg, 6.0 mmol) and *N,N*'-dimethylformamide (5 mL) was heated to 60°C and stirred for 4 h. The reaction solution was cooled to room temperature and diluted with water (50 mL). The resulting mixture was extracted with ethyl acetate (50 mL) and the organic phase was washed with water (50 mL) and saturated brine (20 mL) in turn, dried with anhydrous sodium sulfate, and concentrated under reduced pressure. The resulting residue was purified by silica gel column chromatography (petroleum ether: ethyl acetate = 5:1) to give white solid **24-b** (624 mg, yield: 76%).
¹H NMR (400 MHz, CDCl₃): *δ* 10.43 (s, 1H), 7.67 (s, 1H), 7.21 (s, 1H), 4.21 (t, *J* = 6.0, 2H), 3.79 (t, *J* = 4.0, 2H), 3.45 (s, 3H), 2.36 (s, 3H) ppm

### Synthesis of compound 24-a

A mixture of compound **24-b** (273 mg, 1.0 mmol), compound **8-b** (397 mg, 1.2 mmol), [1,1'-bis(diphenylphosphino)ferrocene]palladium dichloride (73 mg, 0.1 mmol), potassium carbonate (276 mg, 2.0 mmol), 1,4-dioxane (5 mL) and water (0.5 mL) was stirred for 16 h at 90°C under nitrogen. The reaction solution was cooled to room temperature, diluted with saturated brine (20 mL), and the resulting mixture was extracted with ethyl acetate (40 mL×2). The organic phase was combined, dried with anhydrous sodium sulfate, filtered, and concentrated under reduced pressure. The residue was washed with ethyl acetate (50 mL) to give light green solid **24-a** (330 mg, yield: 83%).

LC-MS (ESI): m/z = 398.4 [M+H]⁺.

### Synthesis of compound 24

Sodium cyanoborohydride (38 mg, 0.60 mmol) was added to a mixture of compound **24-a** (60 mg, 0.15 mmol), (*S*)-piperidine-2-carboxylic acid (39 mg, 0.30 mmol), methanol (1 mL) and tetrahydrofuran (1 mL), and the reaction solution was heated to 65°C and stirred for 1 h. The reaction solution was cooled to room temperature, concentrated under reduced pressure, and the residue was prepared by high performance liquid chromatography to give white solid **24** (35.2 mg, yield: 46%).

LC-MS (ESI): m/z = 511.5 [M+H]⁺.

¹H NMR (400 MHz, CD₃OD): *δ* 7.96 (d, *J* = 7.9 Hz, 1H), 7.73 (t, *J* = 7.9 Hz, 1H), 7.66 (d, *J* = 16.1 Hz, 1H), 7.60 - 7.54 (m, 2H), 7.54 - 7.44 (m, 5H), 7.34 (s, 2H), 4.82 - 4.77 (m, 1H), 4.60 (d, *J* = 12.8 Hz, 1H), 4.36 - 4.26 (m, 2H), 4.22 (d, *J* = 13.1 Hz, 1H), 3.94 - 3.74 (m, 2H), 3.57 - 3.48 (m, 1H), 3.45 (s, 3H), 2.95 (t, *J* = 12.0 Hz, 1H), 2.44 (s, 3H), 2.34 - 2.20 (m, *J* = 15.8 Hz, 1H), 1.94 - 1.76 (m, 3H), 1.74 - 1.62 (m, 1H), 1.61 - 1.47 (m, 1H) ppm.

### Embodiment 25

### (S,E)-4-(4-(2-(2-Cyano-[1,1'-biphenyl]-3-yl)vinyl)-2-(2-methoxyethoxy)-5-methylbenzyl)morpholine-3-carboxylic acid (compound 25)

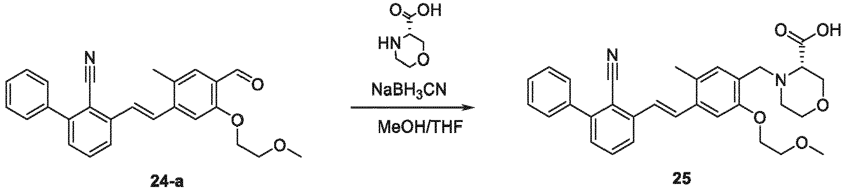

### Synthesis of compound 25

Sodium cyanoborohydride (38 mg, 0.60 mmol) was added to a mixture of compound **24-a** (60 mg, 0.15 mmol), (*S*)-morpholine-3-carboxylic acid (39 mg, 0.30 mmol), methanol (1 mL) and tetrahydrofuran (1 mL), and the reaction solution was heated to 65°C and stirred for 1 h. The reaction solution was cooled to room temperature, concentrated under reduced pressure, and the residue was prepared by high performance liquid chromatography to give white solid **25** (41.0 mg, yield: 53%).

LC-MS (ESI): m/z = 513.5 [M+H]⁺.

¹H NMR (400 MHz, CD₃OD): *δ* 7.96 (d, *J* = 7.9 Hz, 1H), 7.73 (t, *J* = 7.9 Hz, 1H), 7.66 (d, *J* = 16.1 Hz, 1H), 7.59 - 7.54 (m, *J* = 8.0, 1.6 Hz, 2H), 7.54 - 7.44 (m, 5H), 7.36 (s, 1H), 7.34 (s, 1H), 4.65 (d, *J* = 12.7 Hz, 1H), 4.36 - 4.20 (m, 4H), 3.98 - 3.86 (m, 2H), 3.85 - 3.77 (m, 1H), 3.77 - 3.64 (m, 3H), 3.46 (s, 3H), 3.26 - 3.10 (m, 2H), 2.44 (s, 3H) ppm.

### Embodiment 26

### (S,E)-4-(4-(2-(2-Cyano-[1,1'-biphenyl]-3-yl)vinyl)-2-(2-hydroxyethoxy)-5-methylbenzyl)piperidine-2-carboxylic acid (compound 26)

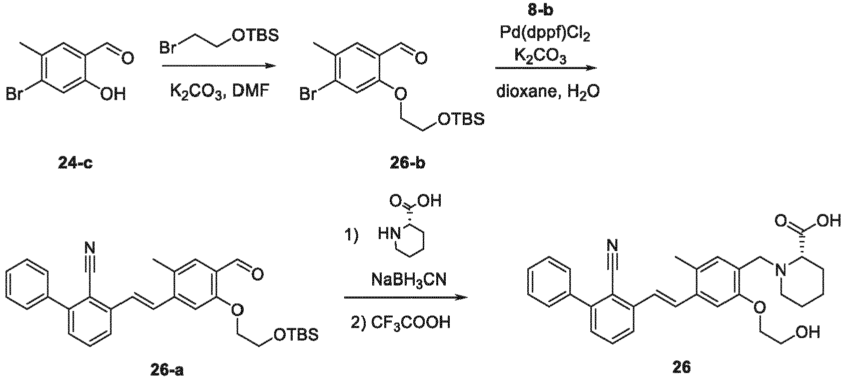

### Synthesis of compound 26-b

A mixture of compound **24-c** (645 mg, 3.0 mmol), (2-bromoethoxy)(tert-butyl)dimethylsilane (1.08 g, 4.5 mmol), potassium carbonate (829 mg, 6.0 mmol) and *N,N'-*dimethylformamide (5 mL) was heated to 60°C and stirred for 16 h. The reaction solution was cooled to room temperature and diluted with water (50 mL). The resulting mixture was extracted with ethyl acetate (50 mL). The organic phase was washed with water (50 mL) and saturated brine (20 mL), respectively, dried with anhydrous sodium sulfate, concentrated under reduced pressure, and the residue was purified by silica gel column chromatography (petroleum ether: ethyl acetate = 20:1) to give white solid **26-b** (767 mg, yield: 68%).

### Synthesis of compound 26-a

A mixture of compound **26-b** (373 mg, 1.0 mmol), compound **8-b** (397 mg, 1.2 mmol), [1,1'-bis(diphenylphosphino)ferrocene]palladium dichloride (73 mg, 0.1 mmol), potassium carbonate (276 mg, 2.0 mmol), 1,4-dioxane (5 mL) and water (0.5 mL) was stirred for 16 h at 90°C under nitrogen. The reaction solution was cooled to room temperature, and diluted with saturated brine (20 mL). The resulting mixture was extracted with dichloromethane (30 mL×3). The organic phase was combined, dried with anhydrous sodium sulfate, filtered and concentrated under reduced pressure, and the residue was purified by silica gel column chromatography (petroleum ether: ethyl acetate = 5:1) to give yellow solid **26-a** (355 mg, yield: 71%).

LC-MS (ESI): m/z = 498.5 [M+H]⁺.

### Synthesis of compound 26

Sodium cyanoborohydride (38 mg, 0.60 mmol) was added to a mixture of compound **26-a** (75 mg, 0.15 mmol), (*S*)-piperidine-2-carboxylic acid (39 mg, 0.30 mmol), methanol (1 mL) and tetrahydrofuran (1 mL). The reaction solution was heated to 60°C and stirred for 1 hour, then concentrated under reduced pressure. The obtained residue was added with a mixture of tetrahydrofuran (1 mL), water (one drop) and trifluoroacetic acid (0.5 mL), and stirred at room temperature for 1 hour. The reaction solution was concentrated under reduced pressure, and the residue was prepared by high performance liquid chromatography to give white solid **26** (48.3 mg, yield: 53%).

LC-MS (ESI): m/z = 497.5 [M+H]⁺.

¹H NMR (400 MHz, DMSO-*d₆*): *δ* 8.05 (d, *J* = 8.0 Hz, 1H), 7.79 (t, *J* = 7.9 Hz, 1H), 7.67 (d, *J* = 16.0 Hz, 1H), 7.63 - 7.47 (m, 6H), 7.40 (d, *J* = 16.0 Hz, 1H), 7.27 (s, 2H), 7.07 (s, 1H), 4.13 - 3.90 (m, 5H), 3.75 (t, *J* = 4.6 Hz, 2H), 3.28 - 3.21 (m, 1H), 3.08 - 3.00 (m, 1H), 2.38 (s, 3H), 1.97 - 1.86 (m, 1H), 1.78 - 1.66 (m, 1H), 1.63 - 1.48 (m, 3H), 1.45 - 1.31 (m, 1H) ppm.

### Embodiment 27

### (S,E)-1-(4-(2-(2-Cyano-[1,1'-biphenyl]-3-yl)vinyl)-2-(2-methoxyethoxy)-5-methylbenzyl)piperazine-2-carboxylic acid (compound 27)

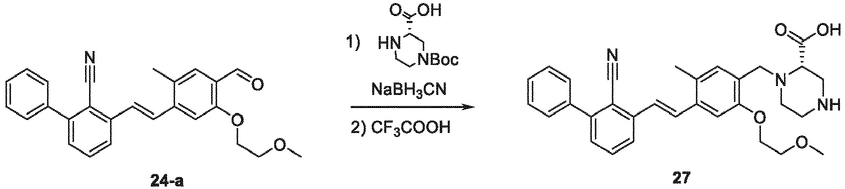

### Synthesis of compound 27

Sodium cyanoborohydride (38 mg, 0.60 mmol) was added to a mixture of compound **24-a** (60 mg, 0.15 mmol), (*S*)-4-(*tert*-butoxycarbonyl)piperazine-2-carboxylic acid (69 mg, 0.30 mmol), methanol (1 mL) and tetrahydrofuran (1 mL), and the reaction solution was heated to 60°C and stirred for 1 hour, then cooled to room temperature, concentrated under reduced pressure. The obtained residue was dissolved in dichloromethane (1.5 mL) and trifluoroacetic acid (0.5 mL), and stirred at room temperature for 16 hours. The reaction solution was concentrated under reduced pressure, and the residue was prepared by high performance liquid chromatography to give white solid 27 (66.7 mg, yield: 87%).

LC-MS (ESI): m/z = 512.4 [M+H]⁺.

¹H NMR (400 MHz, CD₃OD): *δ* 7.95 (d, *J* = 7.9 Hz, 1H), 7.72 (t, *J* = 7.8 Hz, 1H), 7.65 (d, *J* = 16.1 Hz, 1H), 7.59 - 7.54 (m, *J* = 8.0, 1.5 Hz, 2H), 7.53 - 7.41 (m, 5H), 7.30 (s, 1H), 7.27 (s, 1H), 4.22 (t, *J* = 4.4 Hz, 2H), 4.06 (d, *J* = 13.1 Hz, 1H), 3.97 (d, *J* = 13.4 Hz, 1H), 3.85 - 3.75 (m, 2H), 3.49 (dd, *J* = 7.0, 3.5 Hz, 1H), 3.44 (s, 3H), 3.35 (dd, *J* = 12.9, 3.5 Hz, 1H), 3.25 (dd, *J* = 13.1, 6.9 Hz, 1H), 3.18 - 3.08 (m, 3H), 2.79 - 2.71 (m, 1H), 2.42 (s, 3H) ppm.

### Embodiment 28

### (S,E)-1-(4-(2-(2-Cyano-[1,1'-biphenyl]-3-yl)vinyl)-2-hydroxymethyl-5-trifluoromethylbenzyl)piperazine-2-carboxylic acid (compound 28)

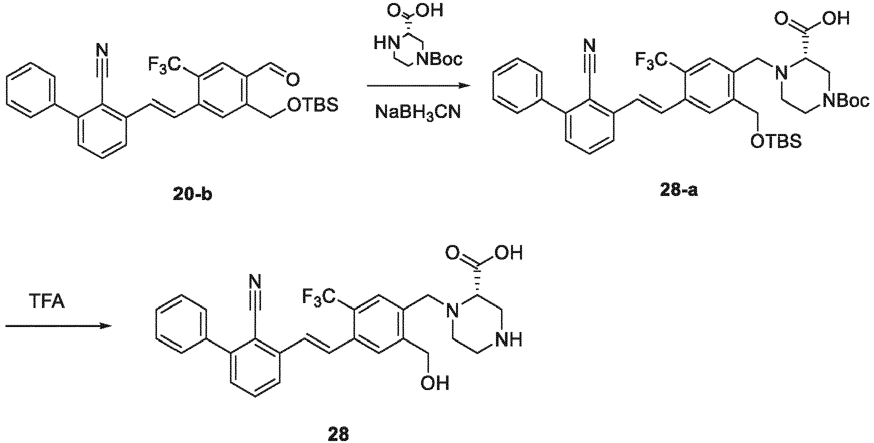

### Synthesis of compound 28-a

A mixture of compound **20-b** (50 mg, 0.096 mmol), (*S*)-4-*tert*-butoxycarbonyl-2-piperazinecarboxylic acid (44 mg, 0.19 mmol), sodium cyanoborohydride (12 mg, 0.19 mmol) and methanol (3 mL) was stirred at 80°C for 3 h under nitrogen. The mixture was cooled to room temperature, concentrated under reduced pressure, and the residue was purified by reversed-phase chromatography (Biotage Flash) to give compound **28-a** (42 mg, yield: 60%).

LC-MS (ESI): m/z = 736 [M+H]⁺.

### Synthesis of compound 28

Compound **28-a** (34 mg, 0.047 mmol) was dissolved in dichloromethane (5 mL), then trifluoroacetic acid (1 mL) was added, and the reaction was stirred at room temperature overnight. The reaction solution was concentrated under reduced pressure, and the residue was prepared by high performance liquid chromatography to give compound **28** (11 mg, yield: 45%).

LC-MS (ESI): m/z = 522 [M+H]⁺.

¹H NMR (400 MHz, DMSO-*d₆*): *δ* 8.01(s, 1H), 7.98 (d, *J*=8.0Hz, 1H), 7.82 (t, *J*=8.0Hz, 1H), 7.77 (s, 1H), 7.67-7.47 (m, 8H), 4.75 (dd, *J*=36.8Hz, 14.8Hz, 2H), 3.81 (dd, *J*=38.4 Hz, 13.6Hz, 2H), 3.16-3.12 (m, 2H), 3.01-2.98 (m, 2H), 2.93-2.90 (m, 1H), 2.86-2.84 (m, 2H) ppm.

### Embodiment 29

### (S,E)-4-Carbamoyl-1-(5-chloro-4-(2-(2-cyano-[1,1'-biphenyl]-3-yl)vinyl)-2-methylbenzyl)piperazine-2-carboxylic acid (compound 29)

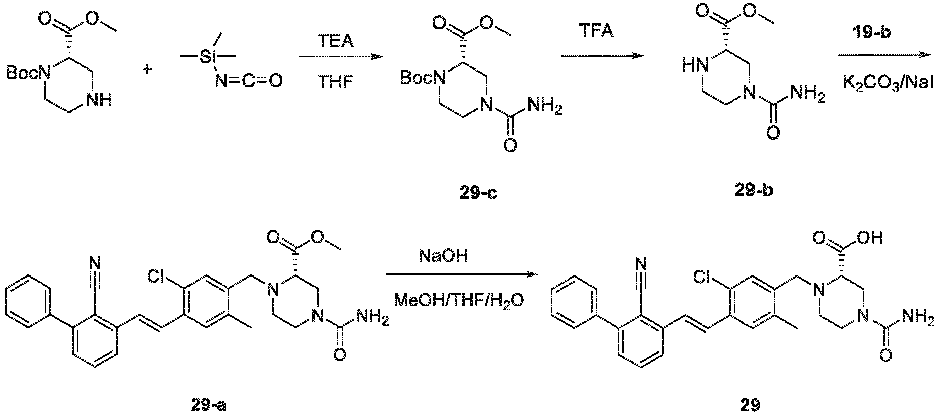

### Synthesis of compound 29-c

(*S*)-1-Boc-piperazine-2-carboxylic acid methyl ester (733 mg, 3.0 mmol) and triethylamine (3.036 g, 30.0 mmol) were dissolved in a tetrahydrofuran solution (50 mL) and cooled to 0°C in an ice bath, and trimethylsilyl isocyanate (3.456 g, 30.0 mmol) was added dropwise. After the dropwise addition was completed, the reaction solution was stirred at room temperature for 2 hours. After the reaction was completed, the reaction solution was diluted with ethyl acetate (50 mL) and washed with water (50 mL) and saturated brine (50 mL) in turn. The resulting organic phase was dried with anhydrous sodium sulfate, concentrated under reduced pressure, and the residue was purified by silica gel column chromatography (petroleum ether: ethyl acetate = 1000:3) to give compound **29-c** (663 mg, yield: 76.9%).

LC-MS (ESI): m/z = 288.0 [M+H]⁺.

### Synthesis of compound 29-b

Compound **29-c** (663 mg, 2.308 mmol) was dissolved in dichloromethane (15 mL), then trifluoroacetic acid (3 mL) was added, and the reaction solution was stirred for 2 h at room temperature. The reaction solution was concentrated under reduced pressure, and the residue was added with toluene (20 mL), concentrated under reduced pressure, then the residue was added with toluene (20 mL) again, concentrated under reduced pressure, and the residue was vacuum dried to give compound **29-b** (1.132 g, yield: 99%), which was directly used in the next reaction step.

### Synthesis of compound 29-a

Compound **29-b** (79 mg, 0.208 mmol) and compound **19-b** (59 mg, 0.208 mmol) were dissolved in acetonitrile (20 mL), and potassium carbonate (144 g, 1.04 mmol) and sodium iodide (7 mg, 0.042 mmol) were added. The reaction solution was heated to 80°C and stirred for 16 hours. The reaction solution was cooled to room temperature, concentrated under reduced pressure, and the residue was dissolved with ethyl acetate (50 mL) and washed with water (50 mL) and saturated brine (50 mL) in turn. The obtained organic phase was dried with anhydrous sodium sulfate, concentrated under reduced pressure, and the residue was prepared by high performance liquid chromatography to give compound **29-a** (56 mg, yield: 50.9%).

LC-MS (ESI): m/z = 529.0 [M+H]⁺.

### Synthesis of compound 29

Compound **29-a** (56 mg, 0.106 mmol) was dissolved in methanol (5 mL), tetrahydrofuran (5 mL) and water (1 mL), and then sodium hydroxide (9 mg, 0.212 mmol) was added, and the reaction solution was stirred for 3 h at room temperature. The pH was adjusted to 4-5 with 1N hydrochloric acid, and the reaction solution was concentrated under reduced pressure. The residue was prepared by high performance liquid chromatography to give compound **29** (14.9 mg, yield: 27.3%).

LC-MS (ESI): m/z = 515.0 [M+H]⁺.

¹H NMR (400 MHz, CD₃OD): *δ* 7.95-7.93 (d, *J* = 8.0Hz, 1H), 7.76-7.72 (m, 2H), 7.66 (s, 1H), 7.59-7.46 (m, 8H), 4.21-4.17 (d, *J* = 13.2Hz, 1H), 3.83-3.76 (m, 2H), 3.70-3.64 (m, 1H), 3.59-3.55 (m, 1H), 3.42-3.37 (m, 2H), 3.13-3.06 (m, 1H), 2.62-2.57 (m, 1H), 2.45 (s, 3H) ppm.

### Embodiment 30

### (S,E)-4-(4-(2-(2-Cyano-[1,1'-biphenyl]-3-yl)vinyl)-2-(2-hydroxyethoxy)-5-methylbenzyl)morpholine-3-carboxylic acid (compound 30)

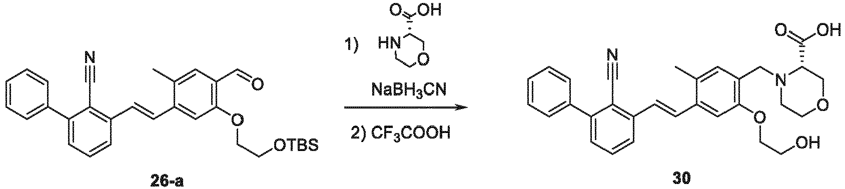

### Synthesis of compound 30

Sodium cyanoborohydride (38 mg, 0.60 mmol) was added to a mixture of compound **26-a** (75 mg, 0.15 mmol), (*S*)-morpholine-3-carboxylic acid (39 mg, 0.30 mmol), methanol (1 mL) and tetrahydrofuran (1 mL). The reaction solution was heated to 65°C and stirred for 1 hour, then cooled to room temperature, concentrated under reduced pressure. The residue was added with tetrahydrofuran (1.0 mL), water (0.5 mL) and trifluoroacetic acid (0.5 mL), and stirred for 16 hours at room temperature. The reaction solution was concentrated under reduced pressure, and the residue was prepared by high performance liquid chromatography to give white solid **30** (66 mg, yield: 88%).

LC-MS (ESI): m/z = 499.5 [M+H]⁺.

¹H NMR (400 MHz, DMSO-*d₆*): *δ* 8.05 (d, *J* = 7.8 Hz, 1H), 7.78 (t, *J* = 7.9 Hz, 1H), 7.66 (d, *J* = 16.0 Hz, 1H), 7.63 - 7.47 (m, 6H), 7.37 (d, *J* = 16.0 Hz, 1H), 7.23 (s, 1H), 7.20 (s, 1H), 4.06 (t, *J* = 5.1 Hz, 2H), 3.85 (d, *J* = 13.9 Hz, 1H), 3.81 - 3.67 (m, 5H), 3.59 (bs, 2H), 3.24 (t, *J* = 4.6 Hz, 1H), 3.02 - 2.94 (m, 1H), 2.38 (s, 3H), 2.36 - 2.31 (m, 1H) ppm.

### Embodiment 31

### (S,E)-1-(4-(2-(2-Cyano-[1,1'-biphenyl]-3-yl)vinyl)-2-hydroxy-5-methylbenzyl)piperidine-2-carboxylic acid (compound 31)

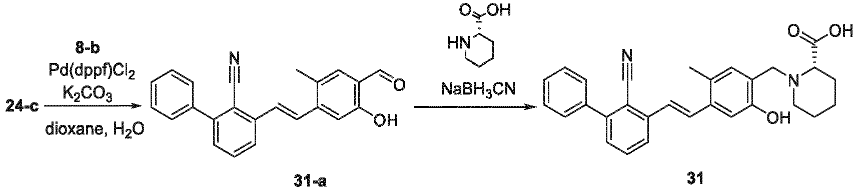

### Synthesis of compound 31-a

A mixture of compound **24-c** (215 mg, 1.0 mmol), compound **8-b** (397 mg, 1.2 mmol), [1,1'-bis(diphenylphosphino)ferrocene]palladium dichloride (73 mg, 0.1 mmol), potassium carbonate (276 mg, 2.0 mmol), 1,4-dioxane (5 mL) and water (1 mL) was stirred for 16 h at 90°C under nitrogen. The reaction solution was cooled to room temperature, diluted with saturated brine (20 mL), and the pH was adjusted to about 3 with 1 M dilute hydrochloric acid. The resulting mixture was extracted with dichloromethane (50 mL×2). The organic phase was combined, dried with anhydrous sodium sulfate, filtered, and concentrated under reduced pressure. The residue was washed with ethyl acetate (50 mL) to give brown solid **31-a** (210 mg, yield: 62%).

LC-MS (ESI): m/z = 340.3 [M+H]⁺.

### Synthesis of compound 31

Sodium cyanoborohydride (38 mg, 0.60 mmol) was added to a mixture of compound **31-a** (51 mg, 0.15 mmol), (*S*)-piperidine-2-carboxylic acid (39 mg, 0.30 mmol), methanol (1 mL) and tetrahydrofuran (1 mL). The reaction solution was heated to 65°C, stirred for 1 h. The reaction solution was cooled to room temperature, concentrated under reduced pressure, and the residue was prepared by high performance liquid chromatography to give white solid **31** (21.6 mg, yield: 32%).

LC-MS (ESI): m/z = 453.4 [M+H]⁺.

¹H NMR (400 MHz, DMSO-*d₆*): *δ* 8.08 (d, *J* = 8.0 Hz, 1H), 7.78 (t, *J* = 7.9 Hz, 1H), 7.64 (d, *J* = 16.0 Hz, 1H), 7.61 - 7.47 (m, 6H), 7.30 (d, *J* = 16.0 Hz, 1H), 7.06 (s, 1H), 7.02 (s, 1H), 4.01 (d, *J* = 13.6 Hz, 1H), 3.48 (d, *J* = 13.5 Hz, 1H), 3.09 (d, *J* = 6.1 Hz, 1H), 2.93 (d, *J* = 11.8 Hz, 1H), 2.33 (s, 3H), 2.28 (t, *J* = 9.6 Hz, 1H), 1.96 - 1.85 (m, 1H), 1.73 - 1.51 (m, 3H), 1.51 - 1.30 (m, 2H) ppm.

### Embodiment 32

### (S,E)-4-(4-(2-(2-Cyano-[1,1'-biphenyl]-3-yl)vinyl)-2-hydroxy-5-methylbenzyl)morpholine-3-carboxylic acid (compound 32)

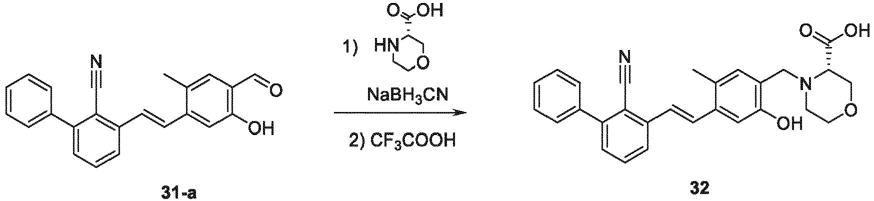

### Synthesis of compound 32

Sodium cyanoborohydride (38 mg, 0.60 mmol) was added to a mixture of compound **31-a** (75 mg, 0.15 mmol), (*S*)-morpholine-3-carboxylic acid (39 mg, 0.30 mmol), methanol (1 mL) and tetrahydrofuran (1 mL). The reaction solution was heated to 65°C, and stirred for 1 h. The reaction solution was cooled to room temperature, concentrated under reduced pressure, and the residue was prepared by high performance liquid chromatography to give white solid **32** (19.8 mg, yield: 29%).

LC-MS (ESI): m/z = 455.4 [M+H]⁺.

¹H NMR (400 MHz, DMSO-*d₆*): *δ* 8.09 (d, *J* = 8.0 Hz, 1H), 7.77 (t, *J* = 7.8 Hz, 1H), 7.65 (d, *J* = 16.0 Hz, 1H), 7.62 - 7.46 (m, 6H), 7.29 (d, *J* = 16.0 Hz, 1H), 7.09 (s, 1H), 7.04 (s, 1H), 3.93 (d, *J* = 13.9 Hz, 1H), 3.83 (dd, *J* = 11.2, 3.5 Hz, 1H), 3.70 (dd, *J* = 11.2, 6.6 Hz, 1H), 3.66 - 3.53 (m, 3H), 3.29 - 3.27 (m, 1H), 2.96 - 2.87 (m, 1H), 2.38 - 2.29 (m, 4H) ppm.

### Embodiment 33

### (S,E)-1-(5-Chloro-2-(2-(2-cyano-[1,1'-biphenyl]-3-yl)vinyl)-4-(2-methoxyethoxy)benzyl)piperidine-2-carboxylic acid (compound 33)

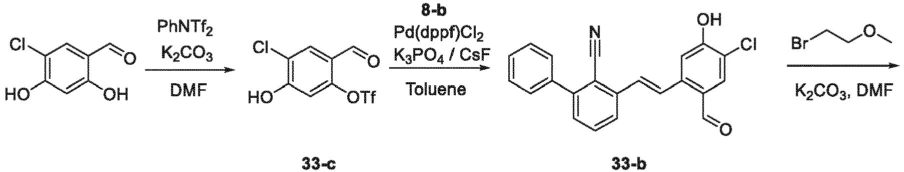

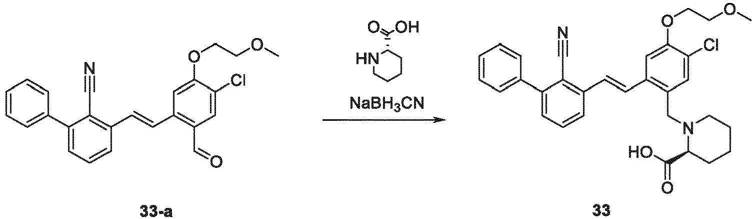

### Synthesis of compound 33-c

Potassium carbonate (1.66 g, 12.0 mmol) was added to a solution of 2,4-dihydroxy-5-chlorobenzaldehyde (1.73 g, 10.0 mmol) in *N,N*-dimethylformamide (16 mL). The mixture was stirred at 0°C for 15 minutes. The temperature was cooled to -10°C, and a solution of *N-*phenyl(bistrifluoromethanesulfonyl)imide (3.56 g, 10.0 mmol) in *N,N*-dimethylformamide (20 mL) was added dropwise to the mixture. After the addition, the reaction was carried out at - 10°C for 2 hours. 100 mL of water was added and the mixture was extracted with ethyl acetate (30 mL×3). The organic phase was combined, and washed with water (30 mL×1) and saturated brine (30 mL×1) in turn. The organic phase was concentrated under reduced pressure, and the residue was purified by silica gel column chromatography (petroleum ether: ethyl acetate = 4:1) to give compound **33-c** (2.51 g, yield: 82%).
¹H NMR (400 MHz, CD₃Cl): *δ* 10.07 (s, 1H), 7.99 (s, 1H), 7.07 (s, 1H) ppm

### Synthesis of compound 33-b

[1,1'-Bis(diphenylphosphino)ferrocene]palladium dichloride (88 mg, 0.12 mmol), potassium phosphate (733 mg, 3.46 mmol) and cesium fluoride (519 mg, 3.46 mmol) were added to a mixture of compound **33-c** (524 mg, 1.73 mmol), compound **8-b** (662 mg, 2.00 mmol) and toluene (25 mL), and stirred at 80°C for 16 h under nitrogen. The reaction solution was concentrated under reduced pressure, and the residue was diluted with water. The pH value of the residue was adjusted to less than 3 with 1M hydrochloric acid, and then the mixture was extracted with dichloromethane (30 mL×3). The organic phase was combined, and washed with saturated brine (30 mL×1). The organic phase was concentrated under reduced pressure, and the residue was purified by silica gel column chromatography (petroleum ether: ethyl acetate = 4:1) to give compound **33-b** (447 mg, yield: 72%).

LC-MS (ESI): m/z = 358 [M-H]⁻.

### Synthesis of compound 33-a

Potassium carbonate (276 mg, 2.00 mmol) and 2-bromoethyl methyl ether (210 mg, 1.50 mmol) were added to a solution of compound **33-b** (358 mg, 1.00 mmol) in *N,N-*dimethylformamide (15 mL). The mixture was stirred at 60°C for 6 hours. The mixture was cooled to room temperature, diluted with water (100 mL), and extracted with ethyl acetate (30 mL×3). The organic phase was combined, and washed with water (30 mL×2) and saturated brine (30 mL). The organic phase was concentrated under reduced pressure, and the residue was purified by silica gel column chromatography (petroleum ether: ethyl acetate = 4:1) to give compound **33-a** (347 mg, yield: 83%).

LC-MS (ESI): m/z = 418 [M+H]⁺.

### Synthesis of compound 33

(*S*)-Piperidine-2-carboxylic acid (39 mg, 0.30 mmol) and methanol (10 mL) were added to a solution of compound **33-a** (84 mg, 0.20 mmol) in dichloromethane (10 mL) and the mixture was stirred at room temperature for 1 h, then sodium cyanoborohydride (32 mg, 0.50 mmol) was added. After the addition, the reaction solution was stirred at room temperature for 16 hours. The reaction solution was concentrated under reduced pressure, and the residue was prepared by high performance liquid chromatography to give compound **33** (35 mg, yield: 28%).

LC-MS (ESI): m/z = 53 1 [M+H]⁺.

¹H NMR (400 MHz, CD₃Cl): *δ* 8.30 (d, *J*=8.0Hz, 1H), 8.05 (d, *J*=16.0Hz, 1H), 7.77(t, *J*=8.0Hz, 1H), 7.66 (s, 1H), 7.56-7.60 (m, 3H), 7.47-7.53 (m, 5H), 4.32-4.34 (m, 2H), 4.06-4.11 (m, 1H), 3.81-3.84 (m, 2H), 3.54-3.57 (m, 1H), 3.44-3.46 (m, 2H), 3.40 (s, 3H) , 2.94-3.00 (m, 1H) , 2.29-2.33 (m, 1H) , 1.80-1.87 (m, 3H), 1.68-1.71 (m, 1H) , 1.54-1.61 (m, 1H) ppm.

### Embodiment 34

### (S,E)-4-(5-Chloro-2-(2-(2-cyano-[1,1'-biphenyl]-3-yl)vinyl)-4-(2-methoxyethoxy)benzyl)morpholine-3-carboxylic acid (compound 34)

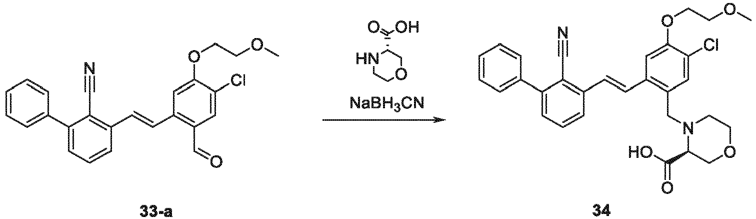

### Synthesis of compound 34

(*S*)-Morpholine-3-carboxylic acid (39 mg, 0.30 mmol) and methanol (10 mL) were added to a solution of compound **33-a** (84 mg, 0.20 mmol) in dichloromethane (10 mL). The mixture was stirred for 1 h at room temperature, then sodium cyanoborohydride (32 mg, 0.50 mmol) was added, and stirred for 16 h at room temperature. The reaction solution was concentrated under reduced pressure, and the residue was prepared by high performance liquid chromatography to give compound **34** (36 mg, yield: 28%).

LC-MS (ESI): m/z = 533 [M+H]⁺.

¹H NMR (400 MHz, DMSO-*d₆*): *δ* 8.09 (d, *J*=7.6Hz, 1H), 8.05 (d, *J*=18.0Hz, 1H), 7.78(t, *J*=8.0Hz, 1H), 7.58-7.61 (m, 2H), 7.49-7.55 (m, 4H), 7.46(s, 1H), 7.43 (d, *J*=18.0Hz, 1H), 7.39 (s, 1H) , 4.29 (t, *J*=4.8Hz, 2H), 4.02 (d, *J*=12.6Hz, 1H), 3.71-3.73 (m, 4H), 3.50-3.57 (m, 3H), 3.35 (s, 3H) , 3.19 (t, *J*=4.8Hz, 1H), 2.73-2.80 (m, 1H) , 2.23-2.28 (m, 1H) ppm.

### Embodiment 35

### (S,E)-2-((5-Chloro-2-(2-(2-cyano-[1,1'-biphenyl]-3-yl)vinyl)-4-(2-methoxyethoxy)benzyl)amino)-3-hydroxy-2-methylpropanoic acid (compound 35)

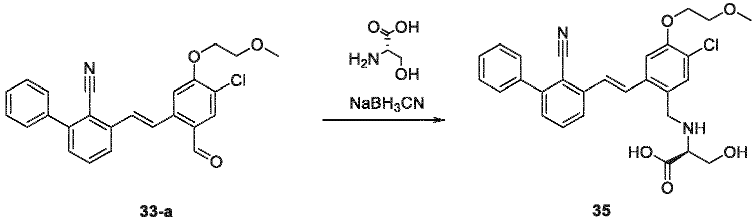

### Synthesis of compound 35

1 M sodium hydroxide aqueous solution (1 mL, 1.0 mmol) was added to a suspension of (*S*)-methyl serine (60 mg, 0.50 mmol) in methanol (10 mL), then the mixture was stirred, and the solid dissolved. A solution of compound **33-a** (63 mg, 0.15 mmol) in tetrahydrofuran (5 mL) was added to this mixture, and the mixture was stirred for 16 h after the addition. Sodium borohydride (19 mg, 0.50 mmol) was added, and then the reaction solution was stirred for 1 hour. The reaction solution was concentrated under reduced pressure. The residue was washed with water, extracted with ethyl acetate, and concentrated under reduced pressure. Then, the residue was prepared by high performance liquid chromatography to give compound **35** (21 mg, yield: 27%).

LC-MS (ESI): m/z = 521 [M+H]⁺.

¹H NMR (400 MHz, DMSO-*d₆*): *δ* 8.21 (d, *J*=7.6Hz, 1H), 8.06 (d, *J*=16.0Hz, 1H), 7.79(t, *J*=8.0Hz, 1H), 7.56-7.61 (m, 3H), 7.52-7.55 (m, 3H), 7.51(s, 1H), 7.47 (d, *J*=16.0Hz, 1H), 7.47 (s, 1H) , 4.30-4.32 (m, 2H), 4.00 (dd, *J*=24.0 Hz ,12.6Hz, 2H), 3.72-3.79 (m, 2H), 3.66 (d, *J*=10.8Hz, 1H), 3.57(d, *J*=10.8Hz, 1H), 3.35(s, 3H), 1.32 (s, 3H) ppm.

### Embodiment 36

### (S,E)-1-(5-Chloro-2-(2-(2-cyano-[1,1'-biphenyl]-3-yl)vinyl)-4-(3-methylsulfonyl)propoxy)benzyl)piperidine-2-carboxylic acid (compound 36)

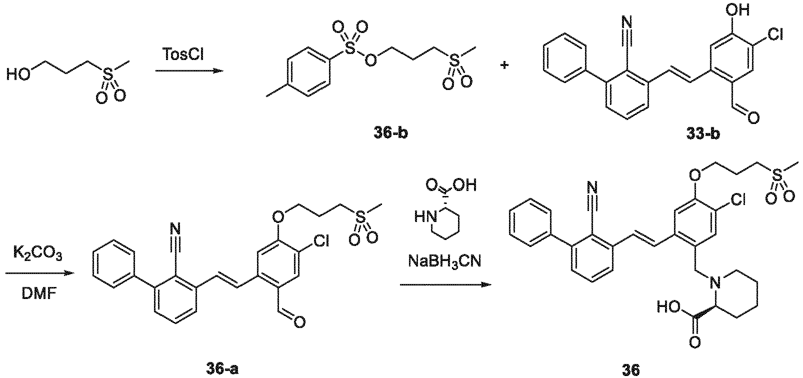

### Synthesis of compound 36-b

3-Methylsulfonyl-1-propanol (1.38 g, 10 mmol), 1,4-diazabicyclo[2.2.2]octane (1.68 g, 15 mmol), and anhydrous dichloromethane (40 mL) were added to a 100 mL reaction flask*. p*-Toluenesulfonyl chloride (2.29 g, 12 mmol) was added in portions to this mixture at 0°C. After the addition, the reaction solution was stirred at room temperature for 16 hours. Water (60 mL) was added to the mixture, then the organic phase was separated, and the aqueous phase was extracted with dichloromethane (40 mL×2). The organic phase was combined, concentrated under reduced pressure, and the residue was purified by silica gel column chromatography (petroleum ether: ethyl acetate = 2:1) to give compound **36-b** (2.69 g, yield: 92%).

### Synthesis of compound 36-a

Compound **33-b** (80.0 mg, 0.22 mol) and compound **33-b** (92.9 mg, 0.33 mol) were dissolved in anhydrous *N,N*-dimethylformamide (20 mL), and potassium carbonate (91.0 mg, 0.66 mmol) was added to the solution. The reaction mixture was stirred under nitrogen at 50°C for 16 hours, then cooled to room temperature. Water (100 mL) was added to form a suspension, and 4 N dilute hydrochloric acid was added dropwise to adjust the suspension to pH = 7. The mixture was extracted with ethyl acetate (150 mL), and the organic phase was washed with water (60 mL) and saturated brine (50 mL), dried with anhydrous sodium sulfate, concentrated under reduced pressure, and the residue was purified by silica gel column chromatography (petroleum ether: ethyl acetate = 10-3:1) to give yellow solid **36-a** (80 mg, yield: 77%).

LC-MS (ESI): m/z = 480.1 [M+H]⁺.

### Synthesis of compound 36

Compound **36-a** (80.0 mg, 0.17 mmol) and (*S*)-piperidine-2-carboxylic acid (56.7 mg, 0.44 mmol) were dissolved in methanol (20 mL), and sodium cyanoborohydride (27.7 mg, 0.44 mmol) was added, then the reaction solution was heated to 60°C, and stirred for 2 hours. The reaction solution was cooled to room temperature, concentrated under reduced pressure, and the residue was prepared by high performance liquid chromatography to give white solid **36** (28.1 mg, yield: 28%).

LC-MS (ESI): m/z = 593.2 [M+H]⁺.

¹H NMR (400 MHz, DMSO-*d₆*): *δ* 8.17-7.81 (m, 2H), 7.79 (t, *J=* 8.0 Hz, 1H), 7.62-7.50 (m, 6H), 7.45 (s, 1H), 7.44-7.41 (m, 2H), 4.30 (t, *J* = 6.4 Hz, 2H), 4.02 (d, *J =* 12.8 Hz, 1H), 3.34-3.30 (m, 3H), 3.10-3.06 (bs, 1H), 3.05 (s, 3H), 2.79-2.74 (m, 1H), 2.26-2.21 (m, 3H), 1.81-1.47 (m, 4H), 1.38-1.36 (m, 2H) ppm.

### Embodiment 37

### (S,E)-4-(5-Chloro-2-(2-(2-cyano-[1,1'-biphenyl]-3-yl)vinyl)-4-(3-methylmethylsulfonyl)propoxy)benzyl)morpholine-3-carboxylic acid (compound 37)

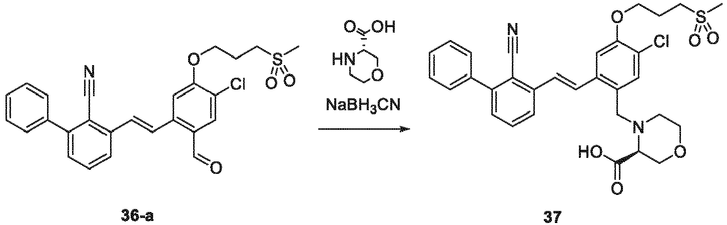

### Synthesis of compound 37

Compound **36-a** (150 mg, 0.31 mmol) and and (*S*)-morpholine-3-carboxylic acid (131 mg, 1.0 mmol) were dissolved in methanol (20 mL), and sodium cyanoborohydride (63.0 mg, 1.0 mmol) was added, then the reaction was heated to 60°C, and stirred for 2 hours. Then, the reaction solution was cooled to room temperature, concentrated under reduced pressure, and the residue was prepared by high performance liquid chromatography to give white solid **37** (15.0 mg, yield: 5.0%).

LC-MS (ESI): m/z = 595.2 [M+H]⁺.

¹H NMR (400 MHz, DMSO-*d₆*): *δ* 8.10 (d, *J* = 8.0 Hz, 1H), 8.06 (d, *J* = 16.0 Hz, 1H), 7.80 (t, *J* = 8.0 Hz, 1H), 7.62-7.51 (m, 6H), 7.46-7.42 (m, 3H), 4.29 (t, *J* = 6.0 Hz, 2H), 4.03 (d, *J* = 12.8 Hz, 1H), 3.75-3.72 (m, 2H), 3.59-3.52 (m, 3H), 3.31-3.28 (m, 3H), 3.22-3.20 (m, 1H), 3.05 (s, 3H), 2.82-2.78 (m, 1H), 2.29-2.20 (m, 3H) ppm.

### Embodiment 38

### (S,E)-2-((5-Chloro-2-(2-(2-cyano-[1,1'-biphenyl]-3-yl)vinyl)-4-(3-methylsulfonyl)propoxy)benzyl)amino)-3-hydroxy-2-methylpropionic acid (compound 38)

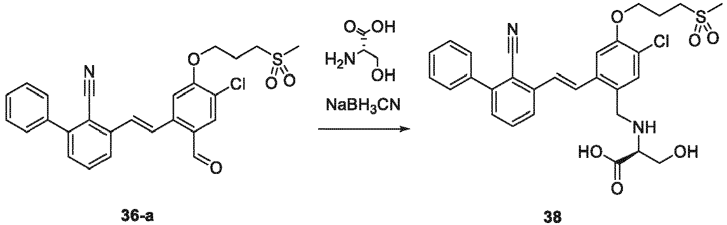

### Synthesis of compound 38

(*S*)-Methyl serine (74.5 mg, 0.626 mmol) was dissolved in methanol (8 mL), and 0.313 M sodium hydroxide aqueous solution (2 mL, 0.626 mmol) was added dropwise with stirring, and then the reaction solution was stirred at room temperature for 10 min after the addition. The reaction solution was cooled to 0°C, and a solution of compound **36-a** (120.0 mg, 0.35 mmol) in tetrahydrofuran (6 mL) was added slowly dropwise. After the dropwise addition, the temperature was raised to room temperature and the mixture was stirred for 16 hours. Sodium borohydride (27.0 mg, 0.71 mmol) was added to the reaction solution, and the mixture was stirred at room temperature for 2 hours. The reaction solution was concentrated under reduced pressure, and the residue was prepared by high performance liquid chromatography to give white solid **38** (27.0 mg, yield: 22%).

LC-MS (ESI): m/z = 441.4 [M+H]⁺.

¹H NMR (400 MHz, DMSO-*d₆*): *δ* 8.21 (d, *J* = 8.0 Hz, 1H), 8.05 (d, *J* = 6.0 Hz, 1H), 7.77 (t, *J* = 8.0 Hz, 1H), 7.60-7.44 (m, 9H), 4.30 (t, *J* = 6.4 Hz, 2H), 4.05-4.00 (m, 2H), 3.70-3.58 (m, 2H), 3.31-3.29 (m, 3H), 3.03 (s, 3H), 2.25-2.20 (m, 2H), 1.32 (s, 3H) ppm.

### Embodiment 39

### (S,E)-1-(5-(2-(2-Cyano-[1,1'-biphenyl]-3-yl)vinyl)-2-(4-cyanobutoxy)-4-methylbenzyl)piperidine-2-carboxylic acid (compound 39)

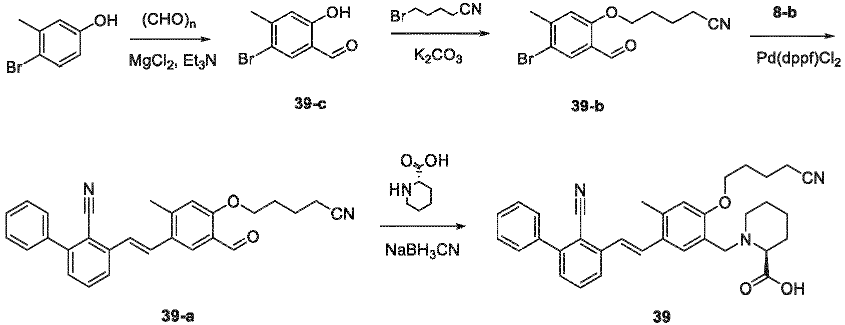

### Synthesis of compound 39-c

A mixture of 4-bromo-3-methylphenol (1.87 g, 10.0 mmol), paraformaldehyde (2.21 g, 76.2 mmol), magnesium chloride (1.43 g, 15.0 mmol), triethylamine (3.78 g, 37.4 mmol) and acetonitrile (70 mL) was refluxed for 3 hours. The reaction solution was cooled to room temperature, diluted with water (100 mL). The pH was adjusted to about 3 with 1 M hydrochloric acid. The mixture was extracted with ethyl acetate (100 mL×2). The organic phase was combined, washed with water (100 mL) and saturated brine (50 mL), respectively, concentrated under reduced pressure, and the residue was purified by silica gel column chromatography (petroleum ether: ethyl acetate = 50:1) to give white solid **39-c** (1.4 g, yield: 66%).

### Synthesis of compound 39-b

A mixture of compound **39-c** (500 mg, 2.33 mmol), 5-bromovaleronitrile (452 mg, 2.79 mmol), potassium carbonate (482 mg, 3.49 mmol) and *N,N*-dimethylformamide (5 mL) was heated at 60°C for 16 h. The reaction solution was cooled to room temperature and diluted with water (50 mL). The resulting mixture was extracted with ethyl acetate (50 mL). The organic phase was washed with water (50 mL) and saturated brine (20 mL), respectively, dried with anhydrous sodium sulfate, concentrated under reduced pressure, and the residue was purified by silica gel column chromatography (petroleum ether: ethyl acetate = 10:1) to give white solid **39-b** (550 mg, yield: 80%).
¹H NMR (400 MHz, CDCl₃): *δ* 10.33 (s, 1H), 7.95 (s, 1H), 6.86 (s, 1H), 4.12 (t, *J* = 5.9 Hz, 2H), 2.47 (t, *J* = 6.9 Hz, 2H), 2.44 (s, 3H), 2.09 - 2.00 (m, 2H), 1.96 - 1.86 (m, 2H) ppm

### Synthesis of compound 39-a

A mixture of compound **39-b** (296 mg, 1.0 mmol), compound **8-b** (397 mg, 1.2 mmol), [1,1'-bis(diphenylphosphino)ferrocene]palladium dichloride (73 mg, 0.1 mmol), potassium carbonate (276 mg, 2.0 mmol), 1,4-dioxane (5 mL) and water (0.5 mL) was stirred for 16 h at 90°C under nitrogen. The reaction solution was diluted with saturated brine (20 mL). The resulting mixture was extracted with ethyl acetate (20 mL×2). The organic phase was combined, dried with anhydrous sodium sulfate, filtered, and concentrated under reduced pressure. The residue was purified by silica gel column chromatography (petroleum ether: ethyl acetate = 2:1) to give compound **39-a** (260 mg, yield: 62%).

LC-MS (ESI): m/z = 421.3 [M+H]⁺.

### Synthesis of compound 39

Sodium cyanoborohydride (30 mg, 0.24 mmol) was added to a mixture of compound **39-a** (50 mg, 0.12 mmol), (*S*)-piperidine-2-carboxylic acid (31 mg, 0.24 mmol), methanol (1 mL) and tetrahydrofuran (1 mL). The reaction solution was stirred at 65°C for 1 hour. The reaction solution was cooled to room temperature, concentrated under reduced pressure, and the residue was prepared by high performance liquid chromatography to give white solid 39 (37.7 mg, yield: 56%).

LC-MS (ESI): m/z = 534.6 [M+H]⁺.

¹H NMR (400 MHz, CD₃OD): *δ* 7.88 (d, *J* = 7.9 Hz, 1H), 7.82 (s, 1H), 7.70 (t, *J=* 8.0 Hz, 1H), 7.61 (d, *J* = 16.1 Hz, 1H), 7.58 - 7.45 (m, 5H), 7.43 (d, *J* = 7.6 Hz, 1H), 7.39 (d, *J* = 16.1 Hz, 1H), 7.00 (s, 1H), 4.57 (d, *J* = 12.8 Hz, 1H), 4.37 (d, *J* = 12.7 Hz, 1H), 4.17 (t, *J=* 6.1 Hz, 2H), 3.56 (d, *J* = 6.5 Hz, 1H), 3.32 (s, 1H), 3.03 (t, *J* = 10.3 Hz, 1H), 2.58 (t, *J* = 7.0 Hz, 2H), 2.50 (s, 3H), 2.23 (d, *J* = 13.3 Hz, 1H), 2.09 - 1.98 (m, 2H), 1.98 - 1.81 (m, 4H), 1.80 - 1.51 (m, 3H) ppm.

### Embodiment 40

### (S,E)-4-(5-(2-(2-Cyano-[1,1'-biphenyl]-3-yl)vinyl)-2-(4-cyanobutoxy)-4-methylbenzyl)morpholine-3-carboxylic acid (compound 40)

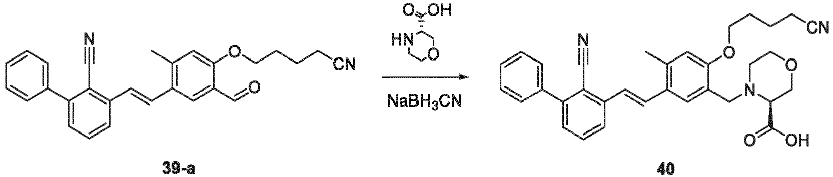

### Synthesis of compound 40

Sodium cyanoborohydride (30 mg, 0.24 mmol) was added to a mixture of compound 39-a (50 mg, 0.12 mmol), (*S*)-morpholine-3-carboxylic acid (31 mg, 0.24 mmol), methanol (1 mL) and tetrahydrofuran (1 mL). The reaction solution was stirred at 65°C for 1 hour. The reaction solution was cooled to room temperature, concentrated under reduced pressure, and the residue was prepared by high performance liquid chromatography to give white solid **40** (39 mg, yield: 61%).

LC-MS (ESI): m/z = 536.5 [M+H]⁺.

¹H NMR (400 MHz, DMSO-*d₆*): *δ* 8.08 (d, *J* = 8.0 Hz, 1H), 7.76 (t, *J* = 7.9 Hz, 1H), 7.71 (s, 1H), 7.68 (d, *J* = 16.0 Hz, 1H), 7.63 - 7.48 (m, 5H), 7.46 (d, *J* = 7.7 Hz, 1H), 7.28 (d, *J=* 16.0 Hz, 1H), 6.88 (s, 1H), 4.05 (t, *J* = 5.9 Hz, 2H), 3.91 - 3.81 (m, 2H), 3.77 - 3.62 (m, 3H), 3.61 - 3.54 (m, 1H), 3.29 (t, J= 3.8 Hz, 1H), 3.10 - 3.02 (m 1H), 2.60 (t, J= 7.0 Hz, 2H), 2.44 (s, 3H), 2.38 - 2.30 (m, 1H), 1.88 - 1.80 (m, 2H), 1.79 - 1.70 (m, 2H) ppm.

### Embodiment 41

### (S,E)-1-(5-(2-(2-Cyano-[1,1'-biphenyl]-3-yl)vinyl)-2-(2-hydroxyethoxy)-4-methylbenzyl)piperidine-2-carboxylic acid (compound 41)

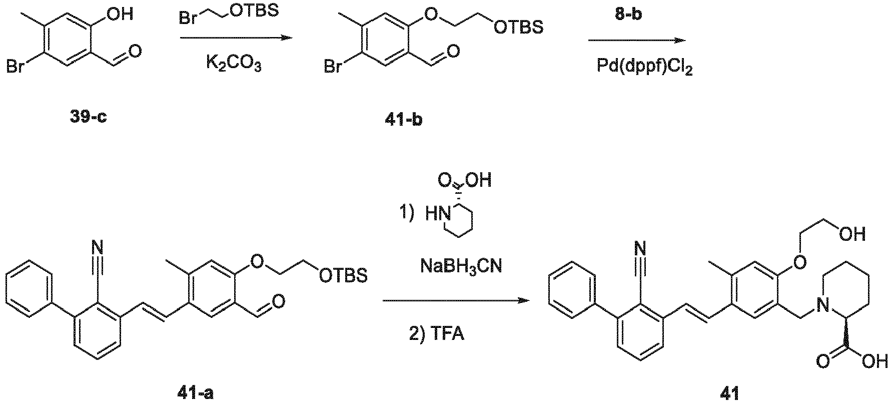

### Synthesis of compound 41-b

A mixture of compound 39-c (400 mg, 1.86 mmol), (2-bromoethoxy)(tert-butyl)dimethylsilane (534 mg, 2.23 mmol), potassium carbonate (514 mg, 3.72 mmol) and *N,N*-dimethylformamide (4 mL) was heated to 60°C for 16 h. The reaction solution was cooled to room temperature and diluted with water (50 mL). The resulting mixture was extracted with ethyl acetate (50 mL×2). The organic phase was combined, washed with water (50 mL) and saturated brine (20 mL), respectively, dried with anhydrous sodium sulfate, concentrated under reduced pressure, and the residue was purified by silica gel column chromatography (petroleum ether: ethyl acetate = 30:1) to give white solid **41-b** (526 mg, yield: 76%).

### Synthesis of compound 41-a

A mixture of compound **41-b** (373 mg, 1.0 mmol), compound **8-b** (397 mg, 1.2 mmol), [1,1'-bis(diphenylphosphino)ferrocene]palladium dichloride (73 mg, 0.1 mmol), potassium carbonate (276 mg, 2.0 mmol), 1,4-dioxane (5 mL) and water (0.5 mL) was stirred for 16 h at 90°C under nitrogen. The reaction solution was cooled to room temperature, diluted with saturated brine (20 mL), and the resulting mixture was extracted with dichloromethane (50 mL×2). The organic phase was combined, dried with anhydrous sodium sulfate, filtered, concentrated under reduced pressure, and the residue was recrystallized with ethyl acetate to give yellow solid **41-a** (420 mg, yield: 84%).

LC-MS (ESI): m/z = 498.5 [M+H]⁺.

### Synthesis of compound 41

Sodium cyanoborohydride (38 mg, 0.60 mmol) was added to a mixture of compound **41-a** (75 mg, 0.15 mmol), (*S*)-piperidine-2-carboxylic acid (39 mg, 0.30 mmol), methanol (1 mL) and tetrahydrofuran (1 mL). The reaction solution was stirred at 65°C for 1 hour. The reaction solution was cooled to room temperature, and concentrated under reduced pressure. The residue was added with tetrahydrofuran (1 mL), water (0.5 mL) and trifluoroacetic acid (0.5 mL), and stirred at room temperature for 16 hours. The reaction solution was concentrated under reduced pressure, and the residue was prepared by high performance liquid chromatography to give white solid **41** (40.2 mg, yield: 54%).

LC-MS (ESI): m/z = 495.5 [M-H]⁺.

¹H NMR (400 MHz, DMSO-*d₆*): *δ* 8.06 (d, *J* = 7.9 Hz, 1H), 7.76 (t, *J* = 7.9 Hz, 1H), 7.74 (s, 1H), 7.66 (d, *J* = 16.0 Hz, 1H), 7.62 - 7.58 (m, 2H), 7.57 - 7.48 (m, 3H), 7.47 (d, *J* = 7.6 Hz, 1H), 7.27 (d, *J* = 16.0 Hz, 1H), 6.91 (s, 1H), 4.10 - 3.97 (m, 4H), 3.81 (d, *J* = 13.7 Hz, 1H), 3.73 (t, *J* = 4.6 Hz, 2H), 3.12 (dd, *J=* 8.2, 3.9 Hz, 1H), 3.07 - 3.00 (m, 1H), 2.45 (s, 3H), 2.42 - 2.31 (m, 1H), 1.90 - 1.80 (m, 1H), 1.77 - 1.65 (m 1H), 1.61 - 1.45 (m, 3H), 1.43 - 1.22 (m, 1H) ppm.

### Embodiment 42

### (S,E)-4-(5-(2-(2-Cyano-[1,1'-biphenyl]-3-yl)vinyl)-2-(2-hydroxyethoxy)-4-methylbenzyl)morpholine-3-carboxylic acid (compound 42)

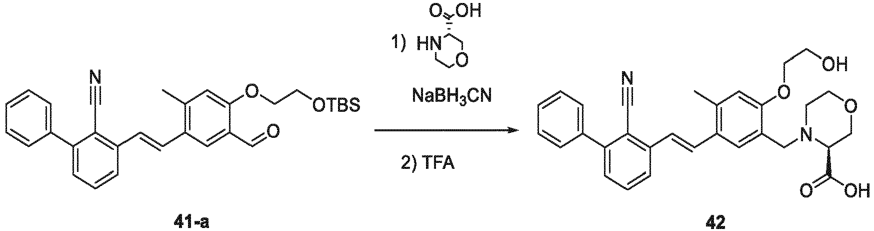

### Synthesis of compound 42

Sodium cyanoborohydride (38 mg, 0.60 mmol) was added to a mixture of compound **41-a** (75 mg, 0.15 mmol), (*S*)-morpholine-3-carboxylic acid (39 mg, 0.30 mmol), methanol (1 mL) and tetrahydrofuran (1 mL). The reaction solution was stirred at 65°C for 1 hour. The reaction solution was cooled to room temperature, and concentrated under reduced pressure. The residue was added with tetrahydrofuran (1 mL), water (0.5 mL) and trifluoroacetic acid (0.5 mL), and stirred at room temperature for 16 hours. The reaction solution was concentrated under reduced pressure, and the residue was prepared by high performance liquid chromatography to give white solid **42** (53.3 mg, yield: 58%).

LC-MS (ESI): m/z = 499.5 [M+H]⁺.

¹H NMR (400 MHz, DMSO-*d₆*): *δ* 8.08 (d, *J* = 7.9 Hz, 1H), 7.76 (t, *J* = 7.9 Hz, 1H), 7.72 (s, 1H), 7.68 (d, *J=* 16.0 Hz, 1H), 7.63 - 7.50 (m, 5H), 7.46 (d, *J* = 7.7 Hz, 1H), 7.28 (d, *J* = 16.0 Hz, 1H), 6.88 (s, 1H), 4.03 (t, *J* = 4.1 Hz, 2H), 3.92 (d, *J* = 14.4 Hz, 1H), 3.80 - 3.71 (m, 4H), 3.67 (d, *J* = 14.5 Hz, 1H), 3.61 (t, *J* = 4.7 Hz, 2H), 3.22 (t, *J* = 4.5 Hz, 1H), 3.02 - 2.95 (m, 1H), 2.44 (s, 3H), 2.37 - 2.28 (m, 1H) ppm.

### Embodiment 43

### (S,E)-1-(5-(2-(2-Cyano-[1,1'-biphenyl]-3-yl)vinyl)-2-(2-methoxyethoxy)-4-methylbenzyl)piperidine-2-carboxylic acid (compound 43)

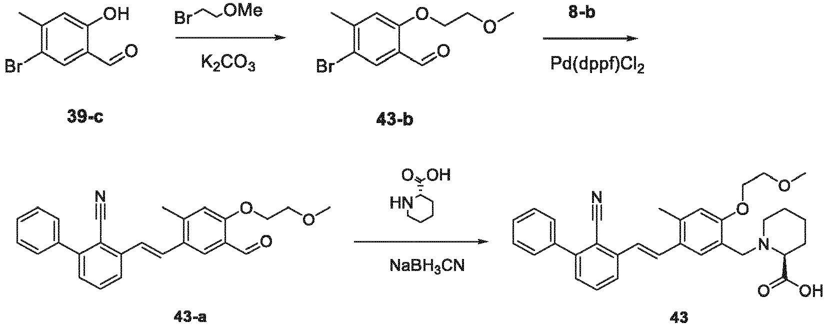

### Synthesis of compound 43-b

A mixture of compound **39-c** (400 mg, 1.86 mmol), 1-bromo-2-methoxyethane (310 mg, 2.23 mmol), potassium carbonate (514 mg, 3.72 mmol) and *N,N*-dimethylformamide (4 mL) was heated at 60°C for 20 hours. The reaction solution was cooled to room temperature, diluted with water (50 mL), and the resulting mixture was extracted with ethyl acetate (50 mL×2). The organic phase was combined, washed with water (50 mL) and saturated brine (20 mL), respectively, dried with anhydrous sodium sulfate, concentrated under reduced pressure, and the residue was purified by silica gel column chromatography (petroleum ether: ethyl acetate = 3:1) to give white solid **43-b** (433 mg, yield: 85%).

### Synthesis of compound 43-a

A mixture of compound **43-b** (273 mg, 1.0 mmol), compound **8-b** (397 mg, 1.2 mmol), [1,1'-bis(diphenylphosphino)ferrocene]palladium dichloride (73 mg, 0.1 mmol), potassium carbonate (276 mg, 2.0 mmol), 1,4-dioxane (5 mL) and water (0.5 mL) was stirred for 16 h at 90°C under nitrogen. The reaction solution was diluted with saturated brine (20 mL). The resulting mixture was extracted with dichloromethane (50 mL×2). The organic phase was combined, dried with anhydrous sodium sulfate, filtered, and concentrated under reduced pressure. The residue was washed with ethyl acetate (20 mL) to give off-white solid 43-a (284 mg, yield: 71%).

LC-MS (ESI): m/z = 398.4 [M+H]⁺.

### Synthesis of compound 43

Sodium cyanoborohydride (25 mg, 0.4 mmol) was added to a mixture of compound 43-a (40 mg, 0.1 mmol), (*S*)-piperidine-2-carboxylic acid (26 mg, 0.2 mmol), methanol (1 mL) and tetrahydrofuran (1 mL). The reaction solution was stirred at 65°C for 1 hour. The reaction solution was cooled to room temperature, concentrated under reduced pressure and the obtained residue was prepared by high performance liquid chromatography to give white solid **43** (33.2 mg, yield: 65%).

LC-MS (ESI): m/z = 511.4 [M+H]⁺.

¹H NMR (400 MHz, CD₃OD): *δ* 7.89 (d, J= 8.0 Hz, 1H), 7.76 (s, 1H), 7.70 (t, *J* = 7.8 Hz, 1H), 7.61 (d, *J* = 16.1 Hz, 1H), 7.58 - 7.45 (m, 5H), 7.43 (d, *J* = 7.6 Hz, 1H), 7.38 (d, *J* = 16.1 Hz, 1H), 6.99 (s, 1H), 4.68 (d, *J* = 12.7 Hz, 1H), 4.35 - 4.16 (m, 3H), 3.97 - 3.86 (m, 1H), 3.84 - 3.73 (m, 1H), 3.56 (d, 1H), 3.45 (s, 3H), 3.29 - 3.21 (m, 1H), 3.00 (t, *J* = 11.6 Hz, 1H), 2.50 (s, 3H), 2.25 (s, 1H), 1.99 - 1.49 (m, 5H) ppm.

### Embodiment 44

### (S,E)-4-(5-(2-(2-Cyano-[1,1'-biphenyl]-3-yl)vinyl)-2-(2-methoxyethoxy)-4-methylbenzyl)morpholine-3-carboxylic acid (compound 44)

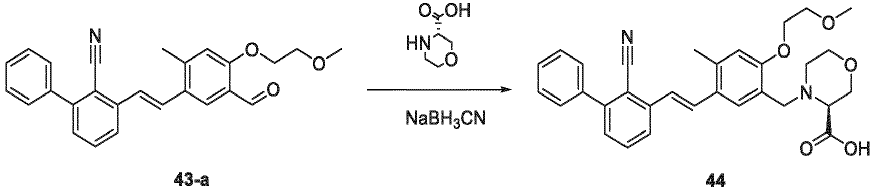

### Synthesis of compound 44

Sodium cyanoborohydride (25 mg, 0.4 mmol) was added to a mixture of compound **43-a** (40 mg, 0.1 mmol), (*S*)-morpholine-3-carboxylic acid (26 mg, 0.2 mmol), methanol (1 mL) and tetrahydrofuran (1 mL). The reaction solution was stirred at 65°C for 1 hour. The reaction solution was cooled to room temperature, concentrated under reduced pressure, and the residue was prepared by high performance liquid chromatography to give white solid **44** (26 mg, yield: 51%).

LC-MS (ESI): m/z = 513.4 [M+H]⁺.

¹H NMR (400 MHz, DMSO-*d₆*): *δ* 8.08 (d, *J* = 8.0 Hz, 1H), 7.76 (t, *J* = 7.8 Hz, 1H), 7.74 (s, 1H), 7.68 (d, *J* = 16.0 Hz, 1H), 7.64 - 7.48 (m, 5H), 7.46 (d, *J* = 7.6 Hz, 1H), 7.29 (d, *J* = 16.1 Hz, 1H), 6.89 (s, 1H), 4.18 - 4.10 (m, 2H), 3.90 - 3.81 (m, 2H), 3.78 - 3.55 (m, 7H), 3.33 (s, 3H), 3.06 (t, *J* = 10.8 Hz, 1H), 2.44 (s, 3H), 2.36 (d, *J* = 12.0 Hz, 1H) ppm.

### Embodiment 45

### (S,E)-1-(2-(2-(2-Cyano-[1,1'-biphenyl]-3-yl)vinyl)-6-(4-cyanobutoxy)-3-methylbenzyl)piperidine-2-carboxylic acid (compound 45)

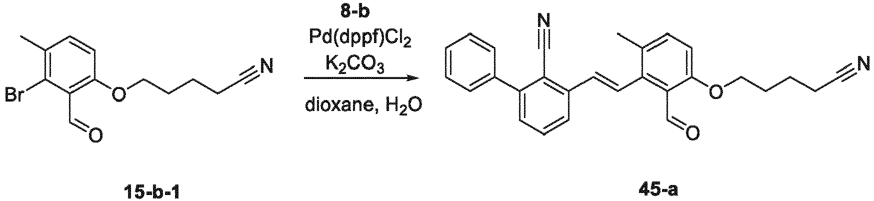

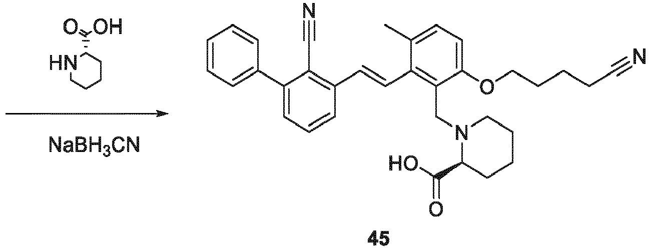

### Synthesis of compound 45-a

A mixture of compound **15-b-1** (250 mg, 0.84 mmol), compound **8-b** (334 mg, 1.01 mmol), [1,1'-bis(diphenylphosphino)ferrocene]palladium dichloride (58 mg, 0.08 mmol), potassium carbonate (232 mg, 1.68 mmol), 1,4-dioxane (5 mL) and water (0.5 mL) was stirred for 16 h at 90°C under nitrogen. The reaction solution was cooled to room temperature, and diluted with saturated brine (20 mL). The resulting mixture was extracted with dichloromethane (50 mL×2). The organic phase was combined, dried with anhydrous sodium sulfate, filtered, and concentrated under reduced pressure. The resulting residue was washed with acetonitrile (20 mL) and a mixed solution of dioxane and water (5:1) (20 mL) to give off-white solid **45-a** (280 mg, yield: 79%).

LC-MS (ESI): m/z = 421.4 [M+H]⁺.

### Synthesis of compound 45

Sodium cyanoborohydride (25 mg, 0.4 mmol) was added to a mixture of compound **45-a** (42 mg, 0.1 mmol), (*S*)-piperidine-2-carboxylic acid (26 mg, 0.2 mmol), methanol (1 mL) and tetrahydrofuran (1 mL). The reaction solution was stirred at 65°C for 1 hour. The reaction solution was cooled to room temperature, concentrated under reduced pressure, and the residue was prepared by high performance liquid chromatography to give white solid **45** (30.8 mg, yield: 58%).

LC-MS (ESI): m/z = 534.5 [M+H]⁺.

¹H NMR (400 MHz, CD₃OD): δ 8.11 (d, *J* = 7.9 Hz, 1H), 7.79 (t, *J* = 7.9 Hz, 1H), 7.62 (d, *J* = 16.8 Hz, 1H), 7.59-7.45 (m, 6H), 7.39 (d, *J* = 8.5 Hz, 1H), 7.06 (d, *J* = 6.8 Hz, 1H), 7.03 (d, *J* = 14.9 Hz, 1H), 4.76 (d, 1H), 4.59 (d, *J* = 13.9 Hz, 1H), 4.27-4.14 (m, 2H), 3.63 (s, 1H), 3.28 - 3.22 (m, 1H), 3.18-3.04 (m, 1H), 2.58 (t, *J* = 7.0 Hz, 2H), 2.39 (s, 3H), 2.17-1.99 (m, *J* = 9.0, 5.9 Hz, 4H), 1.94-1.85 (m, 2H), 1.85-1.75 (m, 1H), 1.75-1.54 (m, 3H) ppm.

### Embodiment 46

### (S,E)-4-(2-(2-(2-Cyano-[1,1'-biphenyl]-3-yl)vinyl)-6-(4-cyanobutoxy)-3-methylbenzyl)morpholine-3-carboxylic acid (compound 46)

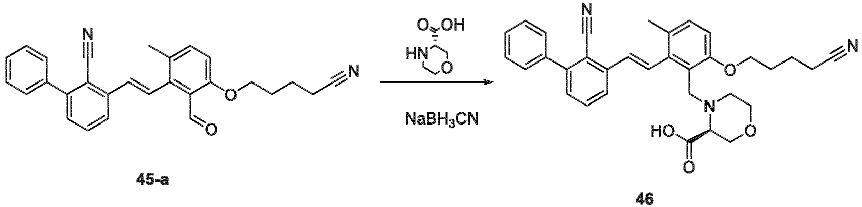

### Synthesis of compound 46

Sodium cyanoborohydride (25 mg, 0.4 mmol) was added to a mixture of compound **45-a** (42 mg, 0.1 mmol), (*S*)-morpholine-3-carboxylic acid (26 mg, 0.2 mmol), methanol (1 mL) and tetrahydrofuran (1 mL). The reaction solution was stirred at 65°C for 1 hour. The reaction solution was cooled to room temperature, concentrated under reduced pressure, and the residue was prepared by high performance liquid chromatography to give white solid **46** (25.3 mg, yield: 47%).

LC-MS (ESI): m/z = 536.5 [M+H]⁺.
¹H NMR (400 MHz, CD₃OD): δ 8.18 (d, *J* = 7.9 Hz, 1H), 7.78 (t, *J =* 7.9 Hz, 1H), 7.71 (d, *J* = 16.5 Hz, 1H), 7.60-7.54 (m, 2H), 7.54-7.44 (m, 4H), 7.37 (d, *J* = 8.6 Hz, 1H), 7.06 (d, *J* = 16.9 Hz, 1H), 7.02 (d, *J* = 9.2 Hz, 1H), 4.71 (d, *J* = 13.0 Hz, 1H), 4.55 (d, *J* = 13.2 Hz, 1H), 4.22-4.13 (m, 2H), 4.10 (dd, *J* = 12.6, 3.8 Hz, 1H), 3.97-3.83 (m, 2H), 3.80-3.66 (m, 2H), 3.29-3.22 (m, 1H), 3.15-3.02 (m, 1H), 2.58 (t, *J* = 7.0 Hz, 2H), 2.40 (s, 3H), 2.10-1.98 (m, 2H), 1.94-1.82 (m, 2H) ppm

### Embodiment 47

### (S,E)-1-(5-Chloro-2-(2-(2-cyano-[1,1'-biphenyl]-3-yl)vinyl)-4-(cyanomethoxy)benzyl)piperidine-2-carboxylic acid (compound 47)

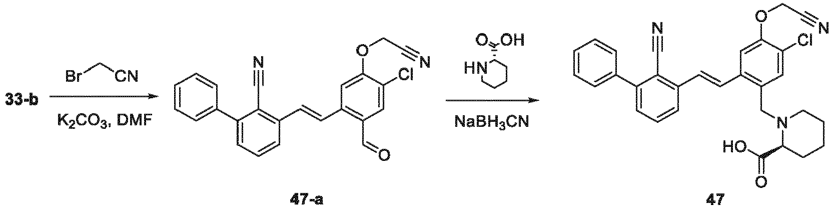

### Synthesis of compound 47-a

Compound **33-b** (155 mg, 0.43 mol) and bromoacetonitrile (102.7 mg, 0.86 mol) were dissolved in anhydrous *N,N*-dimethylformamide (15 mL), and potassium carbonate (178 mg , 1.29 mmol) was added to the solution. The reaction mixture was stirred at 30°C under nitrogen for about 5 hours, then cooled to room temperature. Water (100 mL) was added to form a suspension, and the pH value was adjusted to 7 with 4N hydrochloric acid. The mixture was extracted with ethyl acetate (150 mL), washed with water (60 mL) and saturated brine (50 mL), dried with anhydrous sodium sulfate, concentrated under reduced pressure, and the residue was purified by silica gel column chromatography (petroleum ether: ethyl acetate = 10-3:1) to give compound **47-a** (70 mg, yield: 41%).

LC-MS (ESI): m/z = 399.1 [M+H]⁺.

### Synthesis of compound 47

Compound **47-a** (70.0 mg, 0.175 mmol) and raw material (*S*)-piperidine-2-carboxylic acid (45.3 mg, 0.35 mmol) were dissolved in methanol (12 mL), and sodium cyanoborohydride (22.1 mg, 0.35 mmol) was added, then the reaction was heated to 60°C, and stirred for 2 hours. The reaction solution was cooled to room temperature, concentrated under reduced pressure, and the residue was prepared by high performance liquid chromatography to give white solid **47** (25.0 mg, yield: 28%).

LC-MS (ESI): m/z = 512.3 [M+H]⁺.

¹H NMR: (400 MHz DMSO-*d₆*): *δ* 8.14 (s, 1H), 8.11 (d, *J* = 9.6 Hz, 1H), 7.79 (t, *J* = 8.0 Hz, 1H), 7.61-7.44 (m, 9H), 5.40 (s, 2H), 7.44-7.41 (m, 2H), 3.98 (d, *J* = 13.2 Hz, 1H), 3.43 (d, *J* = 13.2 Hz, 1H), 3.08-3.05 (m, 1H), 2.76-2.73 (m, 1H), 2.15-2.11 (m, 1H), 1.78-1.68 (m, 2H), 1.54-1.36 (m, 4H) ppm.

### Embodiment 48

### (S,E)-1-(4-(2-(2-Cyano-[1,1'-biphenyl]-3-yl)vinyl)-2-(cyanomethoxy)-5-methylbenzyl)piperidine-2-carboxylic acid (compound 48)

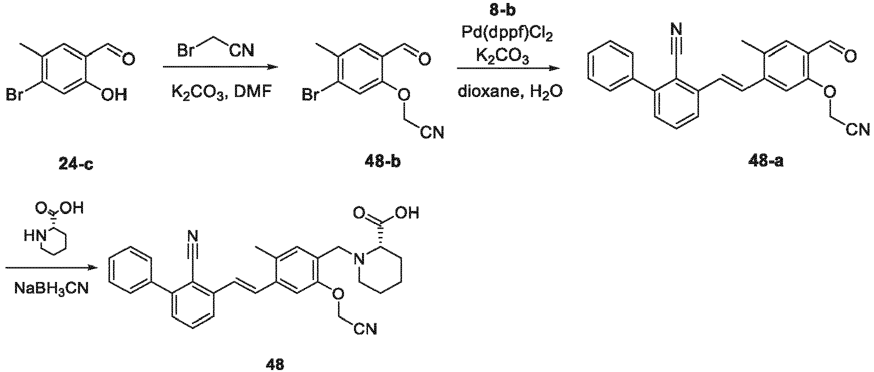

### Synthesis of compound 48-b

A mixture of compound **24-c** (215 mg, 1.0 mmol), bromoacetonitrile (120 mg, 1.2 mmol), potassium carbonate (276 mg, 2.0 mmol) and *N,N*-dimethylformamide (3 mL) was stirred at room temperature for 16 hours. The reaction solution was diluted with water (20 mL). The precipitated solid was filtered out and dried to give white solid **48-b** (213 mg, yield: 84%).

### Synthesis of compound 48-a

A mixture of compound **48-b** (210 mg, 0.826 mmol), compound **8-b** (328 mg, 0.992 mmol), [1,1'-bis(diphenylphosphino)ferrocene]palladium dichloride (61 mg, 0.083 mmol), potassium carbonate (228 mg, 1.65 mmol), 1,4-dioxane (5 mL) and water (0.5 mL) was stirred for 16 h at 90°C under nitrogen. The reaction solution was cooled to room temperature, and diluted with saturated brine (20 mL). The resulting mixture was extracted with dichloromethane (50 mL×2). The organic phase was combined, dried with anhydrous sodium sulfate, filtered, and concentrated under reduced pressure. The resulting residue was washed with ethyl acetate (20 mL×2) to give light green solid **48-a** (300 mg, yield: 96%).

LC-MS (ESI): m/z = 379.3 [M+H]⁺.

### Synthesis of compound 48

Sodium cyanoborohydride (38 mg, 0.60 mmol) was added to a mixture of compound **48-a** (57 mg, 0.15 mmol), (*S*)-piperidine-2-carboxylic acid (39 mg, 0.30 mmol), methanol (1 mL) and tetrahydrofuran (1 mL). The reaction solution was stirred at 65°C for 1 hour. The reaction solution was cooled to room temperature, concentrated under reduced pressure, and the residue was prepared by high performance liquid chromatography to give white solid **48** (14.7 mg, yield: 20%).

LC-MS (ESI): m/z = 492.4 [M+H]⁺.

¹H NMR (400 MHz, CD₃OD): *δ* 7.97 (d, *J* = 8.0 Hz, 1H), 7.75 (t, *J* = 7.9 Hz, 1H), 7.69 (d, *J* = 16.1 Hz, 1H), 7.62-7.44 (m, 9H), 5.20 (s, 2H), 4.49 (d, *J* = 12.9 Hz, 1H), 4.34 (d, *J* = 12.8 Hz, 1H), 3.50 (d, *J* = 8.4 Hz,1H), 3.42-3.33 (m, 1H), 2.97 (t, *J* = 10.5 Hz, 1H), 2.49 (s, 3H), 2.26 (d, *J* = 12.2 Hz, 1H), 1.95-1.66 (m,4H), 1.63-1.46 (m, 1H) ppm.

### Embodiment 49

### (S,E)-1-(4-(2-(2-Cyano-[1,1'-biphenyl]-3-yl)vinyl)-2-(methoxymethyl)-5-trifluoromethylbenzyl)piperidine-2-carboxylic acid (compound 49)

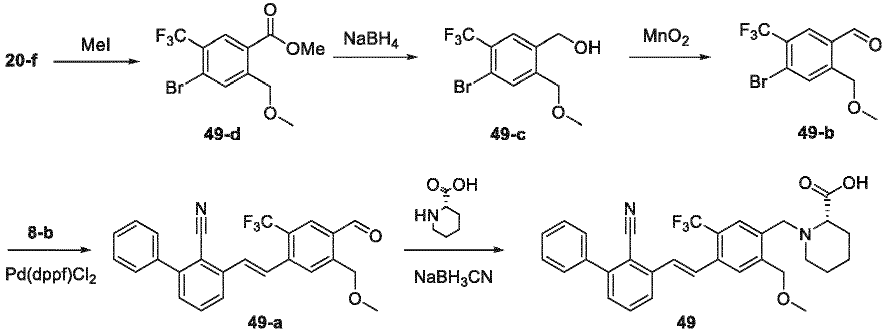

### Synthesis of compound 49-d

Compound **20-f** (600 mg, 2 mmol) was dissolved in anhydrous *N,N-*dimethylformamide (10 mL), and sodium hydride (80 mg, 4 mmol) was added under nitrogen at 0°C. After the addition, the mixture was raised to room temperature and stirred for 30 min. Iodomethane (570 mg, 4 mmol) was added and the reaction was stirred for 3 h at room temperature. The reaction was quenched with water (1 mL), extracted with ethyl acetate (100 mL), washed with water (20 mL×3) and saturated brine (50 mL) in turn, dried with anhydrous sodium sulfate, and concentrated under reduced pressure. The residue was purified by silica gel column chromatography (petroleum ether: ethyl acetate = 10:1) to give compound **49-d** (292 mg, yield: 45.0%).

¹H NMR (400 MHz, DMSO-*d₆*): *δ* 8.22 (s, 1H), 8.09 (s, 1H), 4.87 (s, 2H), 3.92 (s, 3H), 3.46 (s, 3H) ppm.

### Synthesis of compound 49-c

Compound **49-d** (290 mg, 0.88 mmol) was dissolved in anhydrous methanol (10 mL), and sodium borohydride (100 mg, 2.66 mmol) was added under nitrogen at 0°C. The mixture was refluxed for 16 h after the addition. The reaction solution was cooled to room temperature, concentrated under reduced pressure, and the residue was purified by silica gel column chromatography (petroleum ether: ethyl acetate = 10:1) to give compound **49-c** (170 mg, yield: 65.0%).

LC-MS (ESI): m/z = 299 [M-H]⁺.

### Synthesis of compound 49-b

Compound **49-c** (170 mg, 0.56 mmol) was dissolved in dichloromethane (10 mL), then manganese dioxide (493 mg, 5.6 mmol) was added. The reaction solution was stirred at room temperature for 16 hours. The reaction solution was filtered and the filter cake was washed with dichloromethane (20 mL×3). The filtrate was concentrated under reduced pressure, and the residue was purified by silica gel column chromatography (petroleum ether: ethyl acetate = 10:1) to give compound **49-b** (114 mg, yield: 68%).

### Synthesis of compound 49-a

Compound **49-b** (110 mg, 0.36 mmol), compound **8-b** (141 mg, 0.42 mmol), sodium carbonate (132 mg, 1.26 mmol) and [1,1'-bis(diphenylphosphino)ferrocene]palladium dichloride (31 mg, 0.042 mmol) were added to a mixture of 1,4-dioxane (5 mL) and water (0.5 mL), and the mixture was stirred at 80°C for 16 hours under nitrogen. The reaction solution was concentrated under reduced pressure, and the residue was purified by silica gel column chromatography (petroleum ether: ethyl acetate = 10:1) to give compound **49-a** (65 mg, yield: 43.0%).

LC-MS (ESI): m/z = 422 [M+H]⁺.

### Synthesis of compound 49

A mixture of compound **49-a** (30 mg, 0.07 mmol), (5)-piperidine-2-carboxylic acid (24 mg, 0.19 mmol), sodium cyanoborohydride (12 mg, 0.19 mmol) and methanol (3 mL) was stirred at 80°C for 3 h under nitrogen. The reaction solution was cooled to room temperature, concentrated under reduced pressure, and the residue was prepared by high performance liquid chromatography to give compound **49** (10 mg, yield: 26.3%).

LC-MS (ESI): m/z = 535 [M+H]⁺.

¹H NMR (400 MHz, DMSO-*d₆*): *δ* 7.97 (d, *J*=8.0 Hz, 1H), 7.93(s, 1H), 7.83 (d, *J*=8.0 Hz, 1H), 7.80 (s,1H), 7.66-7.46 (m,8H), 4.67 (s, 2H), 3.87(d, *J*=14.4 Hz, 1H), 3.57 (d, *J*=14.4 Hz, 1H), 3.38 (s, 3H), 3.17 (t, *J*=5.6 Hz, 1H), 2.81-2.78 (m, 1H), 2.20-2.19 (m, 1H), 1.78-1.76 (m, 2H), 1.45-1.37 (m, 4H) ppm.

### Embodiment 50

### (S,E)-4-(4-(2-(2-Cyano-[1,1'-biphenyl]-3-yl)vinyl)-2-(methoxymethyl)-5-trifluoromethylbenzyl)morpholine-3-carboxylic acid (compound 50)

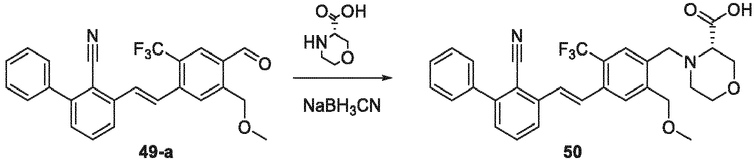

### Synthesis of compound 50

A mixture of compound **49-a** (30 mg, 0.07 mmol), (*S*)-morpholine-3-carboxylic acid (24 mg, 0.19 mmol), sodium cyanoborohydride (12 mg, 0.19 mmol) and methanol (3 mL) was stirred at 80°C for 3 h under nitrogen. The reaction solution was cooled to room temperature, concentrated under reduced pressure, and the residue was prepared by high performance liquid chromatography to give compound **50** (10 mg, yield: 33.3%).

LC-MS (ESI): m/z = 537 [M+H]⁺.

¹H NMR (400 MHz, DMSO-*d₆*): *δ* 7.90 (d, *J*=8.0Hz,1H), 7.87(s,1H), 7.83 (t, *J*=8.0 Hz, 1H), 7.69 (s,1H), 7.59-7.39 (m,8H), 4.61 (s, 2H), 3.91 (d, *J*=14.4 Hz, 1H), 3.80-3.76 (m, 1H), 3.69-3.64 (m, 2H), 3.56-3.52 (m, 1H), 3.48-3.42 (m, 2H), 3.32 (s, 3H), 2.87-2.83 (m, 1H), 2.21-2.18 (m, 1H) ppm.

### Embodiment 51

### (S,E)-1-(2-Methoxy-4-(2-(2-methyl-d₃)-[1,1'-biphenyl]-3-yl)vinyl)-5-trifluoromethylbenzyl)piperidine-2-carboxylic acid (compound 51)

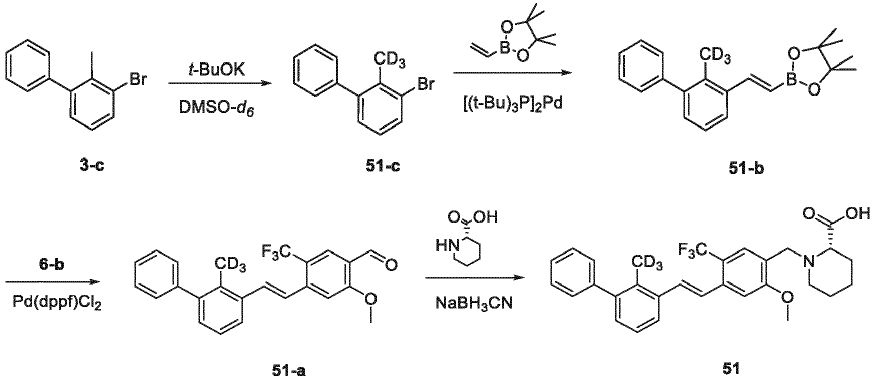

### Synthesis of compound 51-c

Potassium tert-butoxide (84 mg, 0.75 mmol) was added to a mixture of compound **3-c** (618 mg, 2.5 mmol) and deuterated dimethylsulfoxide (3 mL). The reaction solution was stirred at room temperature for 20 hours, and then diluted with water (20 mL). The mixture was extracted with petroleum ether (50 mL). The obtained organic phase was washed with water (20 mL) and saturated brine (20 mL), respectively, dried with anhydrous sodium sulfate, concentrated under reduced pressure, and the residue was purified by silica gel column chromatography (petroleum ether) to give compound **51-c** (550 mg, yield: 88%).

¹H NMR (400 MHz, DMSO-*d₆*): δ 7.67-7.59 (m, 1H), 7.49 - 7.37 (m, 3H), 7.35 -7.28 (m, 2H), 7.23-7.17 (m, 2H) ppm

### Synthesis of compound 51-b

A mixture of compound **51-c** (500 mg, 2.0 mmol), pinacol vinylboronate (462 mg, 3.0 mmol), bis(tri-*tert*-butylphosphine)palladium (102 mg, 0.2 mmol), triethylamine (2.02 g, 20 mmol) and toluene (10 mL) was stirred at 80°C for 16 h under nitrogen. The reaction solution was cooled to room temperature, concentrated under reduced pressure, and the residue was purified by silica gel column chromatography (petroleum ether: ethyl acetate = 50:1) to give compound **51-b** (450 mg, yield: 70%).

### Synthesis of compound 51-a

A mixture of compound **6-b** (113 mg, 0.4 mmol), compound **51-b** (155 mg, 0.48 mmol), [1,1'-bis(diphenylphosphino)ferrocene]palladium dichloride (29 mg, 0.04 mmol), potassium carbonate (110 mg, 0.8 mmol), 1,4-dioxane (3 mL) and water (0.5 mL) was stirred for 5 h at 90°C under nitrogen. The reaction solution was cooled to room temperature, and diluted with saturated brine (20 mL). The resulting mixture was extracted with dichloromethane (40 mL×2). The organic phase was combined, dried with anhydrous sodium sulfate, filtered, concentrated under reduced pressure, and the residue was purified by silica gel column chromatography (petroleum ether: ethyl acetate = 50:1). The resulting product was washed with methanol (1.5 mL×2) to give white solid **51-a** (75 mg, yield: 47%).

LC-MS (ESI): m/z = 400.4 [M+H]⁺.
¹H NMR (400 MHz, CDCl₃): *δ* 10.44 (s, 1H), 8.16 (s, 1H), 7.59 (dd, *J* = 7.6, 1.0 Hz, 1H), 7.51-7.28 (m, 10H), 7.25-7.23 (m, 1H), 4.07 (s, 3H) ppm

### Synthesis of compound 51

Sodium cyanoborohydride (25 mg, 0.4 mmol) was added to a mixture of compound **51-a** (40 mg, 0.1 mmol), (*S*)-piperidine-2-carboxylic acid (26 mg, 0.2 mmol), methanol (1 mL) and tetrahydrofuran (1 mL). The reaction solution was stirred at 65°C for 1 hour. The reaction solution was cooled to room temperature, concentrated under reduced pressure, and the residue was prepared by high performance liquid chromatography to give white solid **51** (16.2 mg, yield: 32%).

LC-MS (ESI): m/z = 513.5 [M+H]⁺.
¹H NMR (400 MHz, CD₃OD): *δ* 7.89 (s, 1H), 7.62 (d, *J* = 15.8 Hz, 1H), 7.56 - 7.51 (m, 2H), 7.42 (t, *J* = 7.3 Hz, 2H), 7.37-7.24 (m, 5H), 7.20-7.15 (m, 1H), 4.53 (d, *J* = 12.9 Hz, 1H), 4.40 (d, *J* = 12.9 Hz,1H), 4.06 (s, 3H), 3.57-3.46 (m, 1H), 3.31 (s, 1H), 3.00 (t, *J* = 10.7 Hz, 1H), 2.24 (d, *J* = 14.8 Hz, 1H), 1.97-1.64 (m, 4H), 1.63-1.48 (m, 1H) ppm

### Embodiment 52

### (S,E)-1-(5-Chloro-2-(2-(2-cyano-[1,1'-biphenyl]-3-yl)vinyl)-4-(methoxy-d₃) benzyl)piperidine-2-carboxylic acid (compound 52)

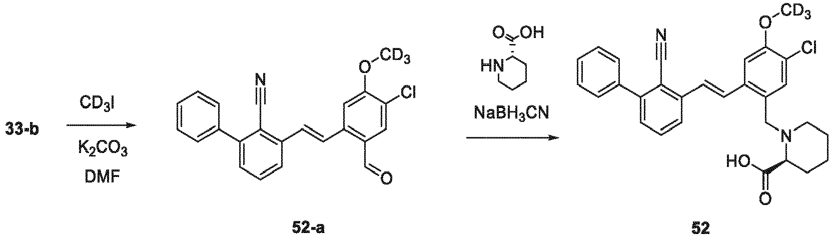

### Synthesis of compound 52-a

Compound **33-b** (140 mg, 0.39 mol) and deuterated iodomethane (565.5 mg, 3.9 mol) were dissolved in anhydrous *N,N*-dimethylformamide (15 mL), then potassium carbonate (269.1 mg, 1.95 mmol) was added. The reaction mixture was stirred at 30°C for 12 hours under nitrogen, and then returned to room temperature. The reaction solution was added water (100 mL) to form a suspension, and then 4N hydrochloric acid solution was added dropwise to adjust the suspension to neutral. The mixture was extracted with ethyl acetate (150 mL×3), and the organic phase was washed with water (60 mL) and saturated brine (50 mL), dried with anhydrous sodium sulfate, filtered, and concentrated under reduced pressure. The residue was purified by silica gel column chromatography (petroleum ether: ethyl acetate = 20-10:1) to give white solid **52-a** (110 mg, yield: 75%).

LC-MS (ESI): m/z = 377.4 [M+H]⁺.

### Synthesis of compound 52

Compound **52-a** (110.0 mg, 0.292 mmol) and (*S*)-piperidine-2-carboxylic acid (75.3 mg, 0.58 mmol) were dissolved in methanol (20 mL), and sodium cyanoborohydride (36.8 mg, 0.58 mmol) was added. The reaction was heated to 60°C, and stirred for 2 hours. The reaction solution was cooled to room temperature, concentrated under reduced pressure, and the residue was prepared by high performance liquid chromatography to give white solid **52** (19.0 mg, yield: 13%).

LC-MS (ESI): m/z = 490.4 [M+H]⁺.

¹H NMR (400 MHz, DMSO-*d₆*): *δ* 8.17-8.13 (m, 2H), 7.78 (t, *J* = 8.0 Hz, 1H), 7.62-7.41 (m, 9H), 4.00 (d, *J=* 12.4 Hz, 1H), 3.41 (d, *J=* 12.8 Hz, 1H), 3.12-3.03 (m, 1H), 2.78-2.74 (m, 1H), 2.16-2.12 (m, 1H), 1.82-1.36 (m, 6H) ppm.

### Embodiment 53

### (S,E)-1-(4-(2-(2-Cyano-[1,1'-biphenyl]-3-yl)vinyl)-2-((methoxy-d₃)methyl)-5-trifluoromethylbenzyl)piperidine-2-carboxylic acid (compound 53)

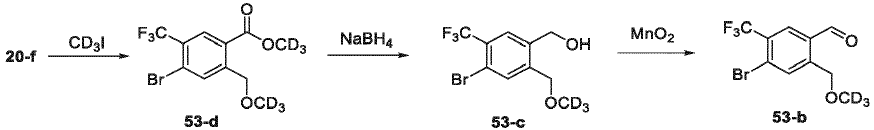

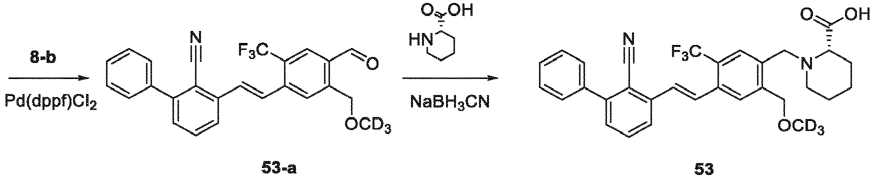

### Synthesis of compound 53-d

Compound **20-f** (600 mg, 2 mmol) was dissolved in anhydrous *N,N-*dimethylformamide (10 mL), and sodium hydride (80 mg, 4 mmol) was added under ice bath cooling and nitrogen, and the reaction was returned to room temperature after the addition and stirred for 30 min. Deuterated iodomethane (570 mg, 4 mmol) was added to the reaction mixture, and the reaction was stirred at room temperature for 3 hours. The reaction was quenched with water (1 mL), extracted with ethyl acetate (100 mL), washed with water (20 mL×3) and saturated brine (50 mL), dried with anhydrous sodium sulfate, and concentrated under reduced pressure. The residue was purified by silica gel column chromatography (petroleum ether: ethyl acetate = 10:1) to give compound **53-d** (292 mg, yield: 45.0%).

### Synthesis of compound 53-c

Compound **53-d** (290 mg, 0.88 mmol) was dissolved in anhydrous methanol (10 mL), and sodium borohydride (100 mg, 2.66 mmol) was added under nitrogen at 0°C, and the reaction mixture was refluxed for 16 h. The reaction solution was cooled to room temperature, concentrated under reduced pressure, and the residue was purified by silica gel column chromatography (petroleum ether: ethyl acetate = 10:1) to give compound **53-c** (170 mg, yield: 65.0%).

LC-MS (ESI): m/z = 301 [M-H]⁺.

### Synthesis of compound 53-b

Compound **53-c** (170 mg, 0.56 mmol) was dissolved in dichloromethane (10 mL), then manganese dioxide (493 mg, 5.6 mmol) was added, and the reaction mixture was stirred for 16 h at room temperature. The reaction mixture was filtered and manganese dioxide was washed with dichloromethane. The filtrate was concentrated under reduced pressure, and the residue was purified by silica gel column chromatography (petroleum ether: ethyl acetate = 10:1) to give compound **53-b** (114 mg, yield: 68%).
¹H NMR (400 MHz, DMSO-*d₆*): *δ* 10.17 (s, 1H), 8.31 (s, 1H), 8.08 (s, 1H), 4.88 (s, 2H) ppm

### Synthesis of compound 53-a

A mixture of compound **53-b** (110 mg, 0.36 mmol), compound **8-b** (141 mg, 0.42 mmol), sodium carbonate (132 mg, 1.26 mmol), [1,1'-bis(diphenylphosphino)ferrocene]palladium dichloride (31 mg, 0.042 mmol), 1,4-dioxane (5 mL) and water (0.5 mL) was stirred at 80°C for 16 hours under nitrogen. The reaction solution was cooled to room temperature, concentrated under reduced pressure, and the residue was purified by silica gel column chromatography (petroleum ether: ethyl acetate = 10:1) to give compound **53-a** (65 mg, yield: 43.0%).

LC-MS (ESI): m/z = 425 [M+H]⁺.

### Synthesis of compound 53

A mixture of compound **53-a** (30 mg, 0.07 mmol), (*S*)-piperidine-2-carboxylic acid (24 mg, 0.19 mmol), sodium cyanoborohydride (12 mg, 0.19 mmol) and methanol (3 mL) was stirred at 80°C for 3 h under nitrogen. The reaction solution was cooled to room temperature, concentrated under reduced pressure, and the residue was prepared by high performance liquid chromatography to give compound **53** (10 mg, yield: 26.3%).

LC-MS (ESI): m/z = 538 [M+H]⁺.
¹H NMR (400 MHz, DMSO-*d₆*): *δ* 7.97 (d, *J*=8.0 Hz, 1H), 7.94 (s,1H), 7.82 (t, *J*=8.0 Hz, 1H), 7.80 (s,1H), 7.66-7.46 (m, 8H), 4.66 (s, 2H), 3.91(d, *J*=14.0 Hz,1H), 3.61 (d, *J*=14.4Hz, 1H), 3.23-3.21 (m, 1H), 2.83-2.80 (m, 1H), 2.26-2.23 (m, 1H), 1.81-1.78 (m, 2H), 1.54-1.40 (m, 4H) ppm

### Embodiment 54

### (S,E)-4-(4-(2-(2-Cyano-[1,1'-biphenyl]-3-yl)vinyl)-2-((methoxy-d₃)methyl)-5-trifluoromethylbenzyl)morpholine-3-carboxylic acid (compound 54)

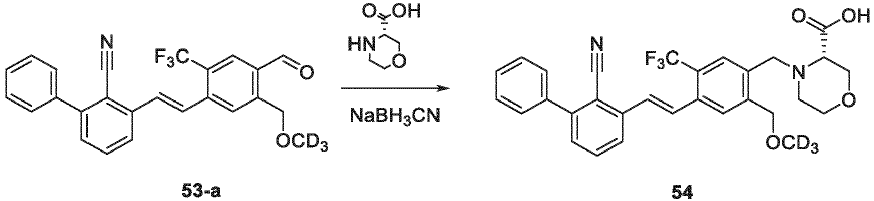

### Synthesis of compound 54

A mixture of compound **53-a** (30 mg, 0.07 mmol), (*S*)-morpholine-3-carboxylic acid (24 mg, 0.19 mmol), sodium cyanoborohydride (12 mg, 0.19 mmol) and methanol (3 mL) was stirred at 80°C for 3 h under nitrogen. The reaction solution was cooled to room temperature, concentrated under reduced pressure, and the residue was prepared by high performance liquid chromatography to give compound **54** (10 mg, yield: 33.3%).

LC-MS (ESI): m/z = 540 [M+H]⁺.
¹H NMR (400 MHz, DMSO-*d₆*): δ 7.97 (d, *J*=8.0 Hz, 1H), 7.93 (s,1H), 7.82 (t, *J*=8.0 Hz, 1H), 7.79 (s, 1H), 7.61-7.49 (m, 8H), 4.71 (s, 2H), 4.06 (d, *J*=14.0 Hz, 1H), 3.74-3.73 (m, 2H), 3.58-3.52 (m, 3H), 3.06 (d, *J*=14.4 Hz, 1H), 2.82-2.79 (m, 1H), 2.20-2.15 (m, 1H) ppm

### Embodiment 55

### (S,E)-3-Hydroxy-2-methyl-((3-(2-(2-methyl-d₃)-[1,1'-biphenyl]-3-yl)vinyl)-4-trifluoromethylbenzyl)amino)propionic acid (compound 55)

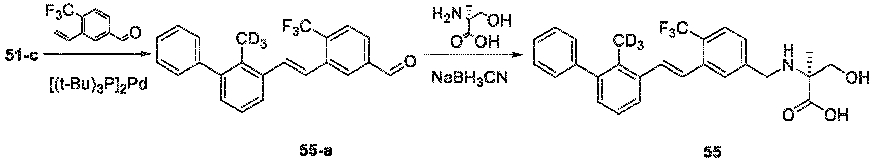

### Synthesis of compound 55-a

A mixture of compound 51-c (125 mg, 0.5 mmol), 4-(trifluoromethyl)-3-vinylbenzaldehyde (150 mg, 0.75 mmol), bis(tri-tert-butylphosphine)palladium (26 mg, 0.05 mmol), triethylamine (506 mg, 5.0 mmol) and toluene (2 mL) was stirred at 80°C for 16 h under nitrogen. The reaction solution was cooled to room temperature, concentrated under reduced pressure, and the residue was purified by silica gel column chromatography (petroleum ether: ethyl acetate = 0-0.04:1) to give compound **55-a** (92 mg, yield: 50%).

### Synthesis of compound 55

Sodium cyanoborohydride (25 mg, 0.4 mmol) was added to a mixture of compound **55-a** (37 mg, 0.1 mmol), (*S*)-2-amino-3-hydroxy-2-methylpropionic acid (24 mg, 0.2 mmol), methanol (1 mL) and tetrahydrofuran (1 mL). The reaction solution was heated to 65°C and stirred for 1 h. The reaction solution was cooled to room temperature, concentrated under reduced pressure, and the residue was prepared by high performance liquid chromatography to give white solid **55** (18.4 mg, yield: 39%).

LC-MS (ESI): m/z = 473.5 [M+H]⁺.
¹H NMR (400 MHz, CD₃OD): *δ* 8.15 (s, 1H), 7.80 (d, *J* = 8.1 Hz, 1H), 7.67 - 7.59 (m, 2H), 7.55 (dd, *J* = 7.7 Hz, 1H), 7.46 - 7.39 (m, 2H), 7.39 - 7.25 (m, 5H), 7.18 (dd, *J* = 7.6, 1.2 Hz, 1H), 4.35 (q, *J* = 12.5 Hz, 2H), 4.06 (d, *J* = 12.2 Hz, 1H), 3.86 (d, *J* = 12.1 Hz, 1H), 1.60 (s, 3H) ppm

### Embodiment 56

### (S,E)-3-Hydroxy-2-methyl-2-(((3-(2-(2-methyl-[1,1'-biphenyl]-3-yl)vinyl)-4-trifluoromethylphenyl)methylene-d₂)amino)propionic acid (compound 56)

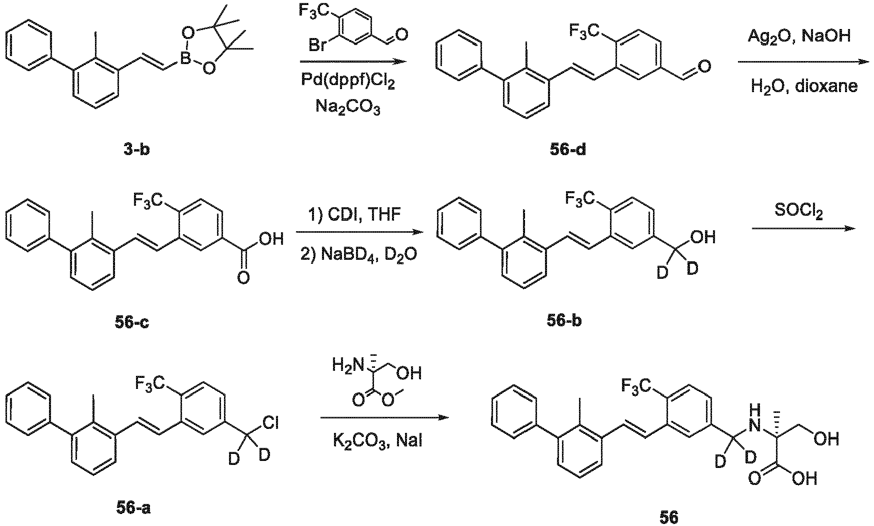

### Synthesis of compound 56-d

[1,1'-Bis(diphenylphosphino)ferrocene]palladium dichloride (57.8 mg, 0.079 mmol) and sodium carbonate (216.2 g, 2.4 mmol) were added to a mixture of 3-bromo-4-trifluoromethylbenzaldehyde (253 mg, 0.79 mmol) and 3-b (300 mg, 0.79 mmol) in 1,4-dioxane (20 mL) and water (2 mL) at room temperature, and the reaction solution was heated to 80°C and stirred under nitrogen for 16 h. The reaction solution was cooled to room temperature, concentrated under reduced pressure, and the residue was purified by silica gel column chromatography (petroleum ether: ethyl acetate = 15:1) to give compound **56-d** (136 mg, yield: 47%).

### Synthesis of compound 56-c

Sodium hydroxide (360.0 mg, 9.0 mmol) was added to a mixture of silver oxide (510.4 mg, 2.2 mmol) in water (20 mL) and dioxane (50 mL) at room temperature, and compound **56-d** (732.0 mg, 2.0 mmol) was added in batches. The reaction mixture was stirred at 70°C and stirred for 12 h, cooled to room temperature, concentrated under reduced pressure. The residue was dissolved in water (40 mL), and adjusted to pH = 4-5 with 4 M hydrochloric acid solution. A large amount of white solid was precipitated, then the mixture was filtered under reduced pressure, and the filter cake was dried to give white solid **56-c** (740 mg, yield: 96%), which was used without further purification.
¹H NMR: (400 MHz, DMSO-*d6*): *δ* 13.34 (s, 1H), 8.44 (s, 1H), 8.03 (d, *J* = 8.0 Hz, 1H), 7.92 (d, *J* = 8.4 Hz, 1H), 7.66-7.59 (m, 2H), 7.50-7.46 (m, 2H), 7.42-7.25 (m, 5H), 7.22 (d, *J* = 6.8 Hz, 1H), 2.30 (s, 3H) ppm

### Synthesis of compound 56-b

Compound **56-c** (740 mg, 1.93 mol) was dissolved in anhydrous tetrahydrofuran (40 mL), and *N,N*-carbonyldiimidazole (333.5 mg, 2.03 mmol) was added at room temperature, and the mixture was stirred for 16 hours under nitrogen at room temperature. A solution of sodium deuterated borohydride (203.3 mg, 4.84 mmol) in deuterium oxide (8 mL) was slowly added dropwise to the reaction mixture, and the reaction solution was continued stirring at room temperature for 16 hours. The mixture was concentrated under reduced pressure, and the residue was added with water (50 mL) and adjusted to neutral with 4 M hydrochloric acid solution. The mixture was extracted with ethyl acetate (150 mL×3), and the organic phase was washed with water (50 mL) and saturated brine (50 mL), dried with anhydrous sodium sulfate, filtered, and concentrated under reduced pressure. The residue was purified by silica gel column chromatography (petroleum ether: ethyl acetate = 10-2:1) to give white solid **56-b** (630 mg, yield: 88%).

¹H NMR (400 MHz, DMSO-*d₆*): *δ*7.95 (s, 1H), 7.73 (d, *J* = 8.4 Hz, 1H), 7.58 (d, *J* = 2.8 Hz, 1H), 7.56 (d, *J* = 11.6 Hz, 1H),7.49-7.45 (m, 3H), 7.40-7.24 (m, 5H), 7.19 (d, *J* = 7.2 Hz, 1H), 5.40 (s, 1H), 2.28 (s, 3H) ppm

### Synthesis of compound 56-a

Compound **56-b** (630 mg, 1.70 mmol) was dissolved in anhydrous dichloromethane (50 mL), and thionyl chloride (2.02 g, 17.0 mmol) and anhydrous *N,N*-dimethylformamide ( 0.5 mL) were added, and the reaction solution was heated to 70°C and stirred for 6 hours. The reaction solution was cooled to room temperature, concentrated under reduced pressure, and the residue was purified by silica gel column chromatography (petroleum ether: ethyl acetate = 30:10-1) to give compound **56-a** (600 mg, yield: 90%).
¹H NMR (400 MHz, DMSO-*d₆*): *δ* 8.15 (s, 1H), 7.80 (d, *J* = 8.0 Hz, 1H), 7.65-7.59 (m, 3H), 7.50-7.23 (m, 7H), 7.21 (d, *J* = 7.2 Hz, 1H), 2.31 (s, 3H) ppm

### Synthesis of compound 56

Compound **56-a** (116.4 mg, 0.30 mmol) and raw material (*S*)-2-methylserine methyl ester hydrochloride (183.0 mg, 0.60 mmol) were dissolved in acetonitrile (20 mL), and potassium carbonate (207 mg, 1.5 mmol) and sodium iodide (45.0 mg, 0.30 mmol) were added, and the reaction solution was heated to 80°C and stirred for 5 hours. The reaction solution was cooled to room temperature, concentrated under reduced pressure, and the residue was prepared by high performance liquid chromatography to give compound **56** (13.5 mg, yield: 9.5%).

LC-MS (ESI): m/z = 572.5 [M+H]⁺.
¹H NMR (400 MHz, DMSO-*d₆*) : *δ* 8.05 (s, 1H), 7.77 (d, *J* = 8.4 Hz, 1H), 7.65-7.58 (m, 2H), 7.52-7.46 (m, 3H), 7.42-7.32 (m, 4H), 7.29-7.24 (m, 1H), 7.21 (d, *J* = 6.8 Hz, 1H), 5.06 (t, *J* = 6.0 Hz, 1H), 3.61-3.57 (dd, *J₁* = 5.6 Hz, *J₂* = 10.0 Hz, 1H), 3.41 3.37 (dd, *J₁* = 5.2 Hz, *J₂* = 10.0 Hz, 1H), 2.29 (s, 3H), 1.94 (bs, 1H), 1.20 (s, 3H) ppm

### Embodiment 57

### (S,E)-1-(5-Chloro-4-(2-(2-cyano-[1,1'-biphenyl]-3-yl)vinyl)-2-methoxybenzyl)piperidine-2-carboxylic acid (compound 57)

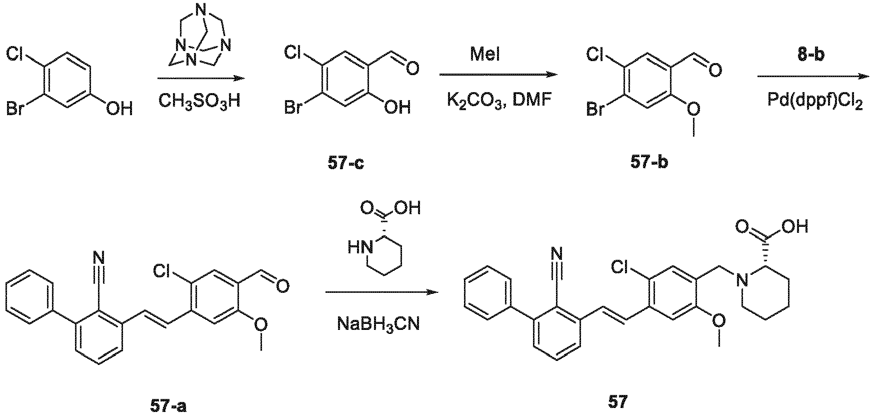

### Synthesis of compound 57-c

Urotropine (1.54 g, 11.0 mmol) was slowly added to a mixture of 3-bromo-4-chlorophenol (2.07 g, 10.0 mmol) and methanesulfonic acid (15 mL) in portions. The mixture was stirred at 105°C for 1 hour. The reaction solution was cooled to room temperature, and an ice-water mixture (100 mL) was added. The reaction solution was extracted with ethyl acetate (30 mL×3). The organic phase was combined, washed with water (30 mL) and saturated brine (30 mL) in turn. The organic phase was concentrated under reduced pressure, and the residue was purified by silica gel column chromatography (petroleum ether: ethyl acetate = 10: 1) to give compound **57-c** (750 mg, yield: 32%).
¹H NMR (400 MHz, CDCl₃): δ 10.90 (brs, 1H), 9.83 (s, 1H), 7.62 (s, 1H), 7.34 (s, 1H) ppm

### Synthesis of compound 57-b

Iodomethane (362 mg, 2.55 mmol) was added to a mixture of compound **57-c** (200 mg, 0.85 mmol), cesium carbonate (552 mg, 1.70 mmol) and *N*,*N*-dimethylformamide (10 mL), and the reaction solution was stirred for 4 h at room temperature. The mixture was diluted with water (100 mL), extracted with ethyl acetate (30 mL×3), and the organic phase was combined and washed with water (30 mL) and saturated brine (30 mL) in turn. The organic phase was concentrated under reduced pressure, and the residue was purified by silica gel column chromatography (petroleum ether: ethyl acetate = 10:1) to give compound **57-b** (170 mg, yield: 80%).

### Synthesis of compound 57-a

[1,1'-Bis(diphenylphosphino)ferrocene]palladium dichloride (44 mg, 0.06 mmol), potassium phosphate (318 mg, 1.5 mmol) and cesium fluoride (225 mg, 1.5 mmol) were added to a mixture of compound **57-b** (150 mg, 0.60 mmol), compound **8-b** (260 mg, 0.78 mmol) and toluene (25 mL), and the mixture was stirred at 90°C for 16 h under nitrogen. The reaction solution was cooled to room temperature, concentrated under reduced pressure, and the residue was purified by silica gel column chromatography (petroleum ether: ethyl acetate = 5:1) to give compound **57-a** (210 mg, yield: 94%).
¹H NMR (400 MHz, CDCl₃): *δ* 10.41 (s, 1H), 7.88 (d, *J*=7.6Hz, 1H), 7.87 (s, 1H), 7.65-7.68 (m, 3H), 7.45-7.58 (m, 6H), 7.34 (s, 1H), 4.03 (s, 3H) ppm

### Synthesis of compound 57

(*S*)-Piperidine-2-carboxylic acid (59 mg, 0.46 mmol) and methanol (10 mL) were added to a solution of compound **57-a** (112 mg, 0.30 mmol) in dichloromethane (10 mL). The mixture was stirred for 1 h at room temperature, then sodium cyanoborohydride (32 mg, 0.50 mmol) was added. The mixture was stirred for 16 h at room temperature. The reaction solution was concentrated under reduced pressure, and the residue was prepared by high performance liquid chromatography to give compound **57** (56 mg, yield: 38%).

LC-MS (ESI): m/z = 487 [M+H]⁺.
¹H NMR (400 MHz, CD₃OD): *δ* 7.96 (d, J=7.6Hz, 1H), 7.77 (t, *J*=8.0Hz, 1H), 7.74 (d, *J*=16.0Hz, 1H), 7.66 (s, 1H), 7.65 (d, *J*=16.0Hz, 1H), 7.56-7.58 (m, 2H), 7.46-7.53 (m, 5H), 4.47 (d, *J*=12.8Hz, 1H), 4.34 (d, *J*=12.8Hz, 1H), 4.00 (s, 3H), 3.48-3.53 (m, 1H), 2.96-3.03 (m, 1H), 2.22-2.26 (m, 1H), 1.73-1.85 (m, 5H), 1.54-1.59 (m, 1H) ppm

### Embodiment 58

### (S,E)-1-(5-Chloro-2-methoxy-4-(2-(2-methyl-[1,1'-biphenyl]-3-yl)vinyl)benzyl)piperidine-2-carboxylic acid (compound 58)

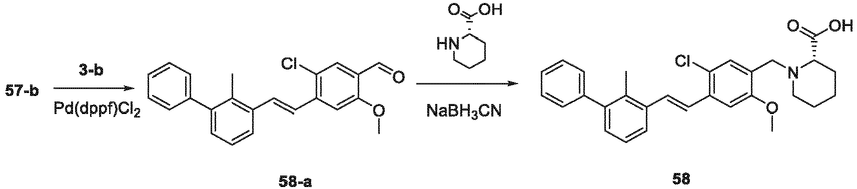

### Synthesis of compound 58-a

[1,1'-Bis(diphenylphosphino)ferrocene]palladium dichloride (73 mg, 0.10 mmol), potassium phosphate (424 mg, 2.0 mmol) and cesium fluoride (300 mg, 2.0 mmol) were added to a mixture of compound **57-b** (250 mg, 1.00 mmol), compound **3-b** (400 mg, 1.25 mmol) and toluene (20 mL). The mixture was stirred at 90°C for 16 h under nitrogen. The reaction mixture was cooled to room temperature, filtered, and the filtrate was concentrated under reduced pressure. The residue was purified by silica gel column chromatography (petroleum ether: ethyl acetate = 5:1) to give compound **58-a** (272 mg, yield: 75%).
¹H NMR (400 MHz, CDCl₃): *δ* 10.39 (s, 1H), 7.85 (s, 1H), 7.63 (d, *J*=8.0Hz, 1H), 7.49 (d, *J*=16.0Hz, 1H), 7.36-7.45 (m, 4H), 7.28-7.31 (m, 3H), 7.23-7.25 (m, 2H), 4.01 (s, 3H), 2.33 (s, 3H) ppm

### Synthesis of compound 58

(*S*)-Piperidine-2-carboxylic acid (59 mg, 0.46 mmol) and methanol (10 mL) were added to a solution of compound **58-a** (109 mg, 0.30 mmol) in dichloromethane (10 mL). The mixture was stirred for 1 h at room temperature, then sodium cyanoborohydride (32 mg, 0.50 mmol) was added, and stirred for 16 h at room temperature. The reaction solution was concentrated under reduced pressure, and the residue was prepared by high performance liquid chromatography to give compound **58** (66 mg, yield: 46%).

LC-MS (ESI): m/z = 476 [M+H]⁺.
¹H NMR (400 MHz, CD₃OD): *δ* 7.59-7.65 (m, 3H), 7.40-7.44 (m, 3H), 7.33-7.37 (m, 2H), 7.25-7.30 (m, 3H), 7.16 (d, *J*=7.2Hz, 1H), 4.46 (d, *J*=12.8Hz, 1H), 4.34 (d, *J*=12.8Hz, 1H), 4.00 (s, 3H), 3.48-3.53 (m, 1H), 2.96-3.03 (m, 1H), 2.30 (s, 1H), 2.22-2.26 (m, 1H), 1.76-1.86 (m, 5H), 1.54-1.59 (m, 1H) ppm

### Embodiment 59

### (S,E)-1-(5-Chloro-2-(methoxy-d₃)-4-(2-(2-methyl-[1,1'-biphenyl]-3-yl)vinyl)benzyl)piperidine-2-carboxylic acid (compound 59)

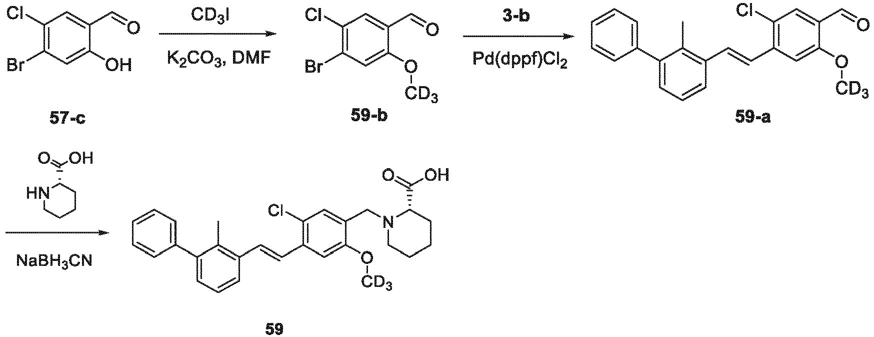

### Synthesis of compound 59-b

Deuterated iodomethane (362 mg, 2.55 mmol) was added to a mixture of compound **57-c** (150 mg, 0.60 mmol), cesium carbonate (390 mg, 1.20 mmol) and *N,N-*dimethylformamide (8 mL), and the reaction solution was stirred for 16 h at room temperature. The mixture was diluted with water (50 mL), extracted with ethyl acetate (30 mL×3), and the organic phase was combined and washed with water (30 mL) and saturated brine (30 mL) in turn. The organic phase was concentrated under reduced pressure, and the residue was purified by silica gel column chromatography (petroleum ether: ethyl acetate = 10: 1) to give compound **59-b** (136 mg, yield: 85%).
¹H NMR (400 MHz, CDCl₃): *δ* 10.35 (s, 1H), 7.87 (s, 1H), 7.28 (s, 1H) ppm

### Synthesis of compound 59-a

[1,1'-Bis(diphenylphosphino)ferrocene]palladium dichloride (65 mg, 0.089 mmol), potassium phosphate (212 mg, 1.0 mmol) and cesium fluoride (150 mg, 1.0 mmol) were added to a mixture of compound **59-b** (133 mg, 0.50 mmol), compound **3-b** (200 mg, 0.62 mmol) and toluene (20 mL), and the mixture was stirred at 90°C for 16 h under nitrogen. The reaction mixture was cooled to room temperature, filtered, and the filtrate was concentrated under reduced pressure, and the residue was purified by silica gel column chromatography (petroleum ether: ethyl acetate = 7:1) to give compound **59-a** (123 mg, yield: 67%).
¹H NMR (400 MHz, CDCl₃): *δ* 10.39 (s, 1H), 7.85 (s, 1H), 7.63 (d, *J*=8.0Hz, 1H), 7.49 (d, *J* = 16.0Hz, 1H), 7.35-7.43 (m, 4H), 7.28-7.30 (m, 3H), 7.23-7.25 (m, 2H), 4.01 (s, 3H), 2.33 (s, 3H) ppm

### Synthesis of compound 59

(*S*)-Piperidine-2-carboxylic acid (109 mg, 0.78 mmol) and methanol (10 mL) were added to a solution of compound **59-a** (120 mg, 0.33 mmol) in dichloromethane (10 mL). The mixture was stirred for 1 h at room temperature, then sodium cyanoborohydride (100 mg, 1.58 mmol) was added. The mixture was stirred for 16 h at room temperature. The reaction solution was concentrated under reduced pressure, and the residue was prepared by high performance liquid chromatography to give compound **59** (62 mg, yield: 43%).

LC-MS (ESI): m/z = 479 [M+H]⁺.

¹H NMR (400 MHz, CD₃OD): *δ* 7.59-7.65 (m, 3H), 7.40-7.44 (m, 3H), 7.33-7.37 (m, 2H), 7.25-7.30 (m, 3H), 7.16 (d, *J*=7.2Hz, 1H), 4.46 (d, *J*=12.8Hz, 1H), 4.34 (d, *J*=12.8Hz, 1H), 3.50-3.53 (m, 1H), 2.96-3.02 (m, 1H), 2.30(s, 1H), 2.22-2.26 (m, 1H), 1.76-1.86 (m, 5H), 1.54-1.60 (m, 1H) ppm.

### Embodiment 60

### (S,E)-1-(2-(Methoxy-d₃)-4-(2-(2-methyl-[1,1'-biphenyl]-3-yl)vinyl)-5-trifluoromethylbenzyl)piperidine-2-carboxylic acid (compound 60)

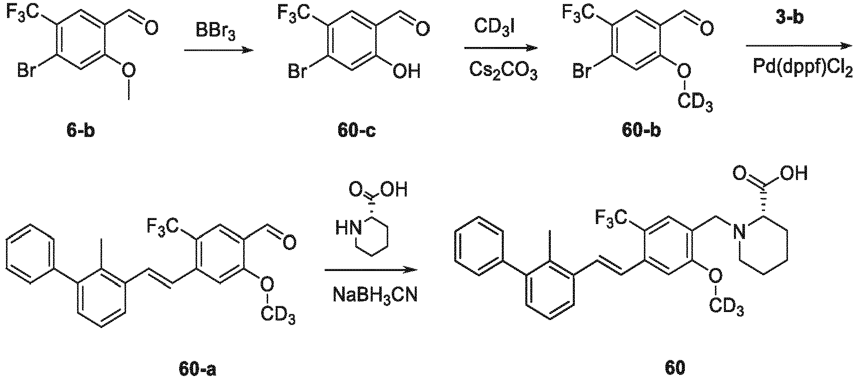

### Synthesis of compound 60-c

1.0 M dichloromethane solution of boron tribromide (2.5 mL, 2.5 mmol) was slowly added dropwise to a solution of 4-bromo-2-methoxy-5-trifluoromethyl-benzaldehyde (283 mg, 1.0 mmol) in dichloromethane (10 mL). After the addition, the ice bath was removed, and the mixture was stirred at room temperature for 16 hours. The mixture was slowly added with 50 mL of saturated sodium bicarbonate aqueous solution and extracted with ethyl acetate (30 mL×3). The organic phase was combined and washed with water (30 mL) and saturated brine (30 mL) in turn. The organic phase was concentrated under reduced pressure, and the residue was purified by silica gel column chromatography (petroleum ether: ethyl acetate = 10: 1) to give compound **60-c** (142 mg, yield: 53%).

LC-MS (ESI): m/z = 267 [M-H]⁻.

### Synthesis of compound 60-b

Deuterated iodomethane (290 mg, 2.00 mmol) was added to a mixture of compound **60-c** (130 mg, 0.48 mmol), cesium carbonate (500 mg, 1.54 mmol) and *N,N-*dimethylformamide (8 mL), and the reaction solution was stirred for 16 h at room temperature. The mixture was diluted with water (100 mL), extracted with ethyl acetate (30 mL×3). The organic phase was combined, and washed with water (30 mL) and saturated brine (30 mL) in turn. The organic phase was concentrated under reduced pressure, and the residue was purified by silica gel column chromatography (petroleum ether: ethyl acetate = 10: 1) to give compound **60-b** (106 mg, yield: 78%).

### Synthesis of compound 60-a

[1,1'-Bis(diphenylphosphino)ferrocene]palladium dichloride (25 mg, 0.035 mmol), potassium phosphate (148 mg, 0.7 mmol) and cesium fluoride (105 mg, 0.7 mmol) were added to a mixture of compound **60-b** (100 mg, 0.35 mmol), compound **3-b** (145 mg, 0.45 mmol) and toluene (15 mL), and the mixture was stirred at 90°C for 16 h under nitrogen. The reaction mixture was cooled to room temperature, concentrated under reduced pressure, and the residue was purified by silica gel column chromatography (petroleum ether: ethyl acetate = 6:1) to give compound **60-a** (95 mg, yield: 68%).

MS (ESI): m/z = 400 [M+H]⁺.

### Synthesis of compound 60

L-Piperidine-2-carboxylic acid (39 mg, 0.3 mmol) and methanol (10 mL) were added to a solution of compound **60-a** (40 mg, 0.10 mmol) in dichloromethane (10 mL). The mixture was stirred for 1 h at room temperature, then sodium cyanoborohydride (16 mg, 0.25 mmol) was added. The mixture was stirred for 16 h at room temperature after the addition. The reaction mixture was concentrated under reduced pressure, and the residue was purified by preparative liquid chromatography to give compound **60** (20 mg, yield: 39%).

LC-MS (ESI): m/z = 513 [M+H]⁺.

¹H NMR (400 MHz, CD₃OD): δ 7.90 (s, 1H), 7.63 (d, *J*=16.0Hz, 1H), 7.54 (d, *J*=8.0Hz, 1H), 7.53 (s, 1H), 7.41-7.44 (m, 2H), 7.35 (d, *J*=8.0Hz, 1H), 7.26-7.33 (m, 4H), 7.18 (d, *J*=7.6Hz, 1H), 4.53 (d, *J*=12.8Hz, 1H), 4.40 (d, *J*=12.8Hz, 1H), 3.49-3.52 (m, 1H), 2.93-3.03 (m, 1H), 2.30 (s, 3H), 2.22-2.26 (m, 1H), 1.67-1.85 (m, 5H), 1.54-1.59 (m, 1H) ppm.

### Embodiment 61

### (S,E)-1-(2-Methoxy-4-(2-(2-methyl-[1,1'-biphenyl]-3-yl-2',')',4',5',6'-d₅)vinyl)-5-trifluoromethylbenzyl)piperidine-2-carboxylic acid (compound 61)

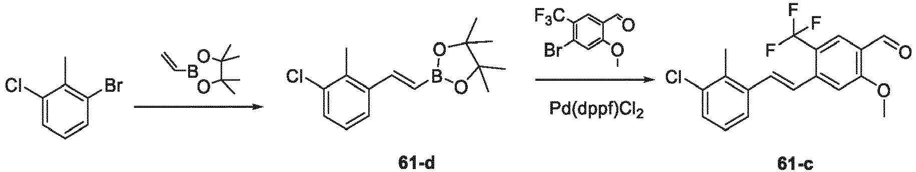

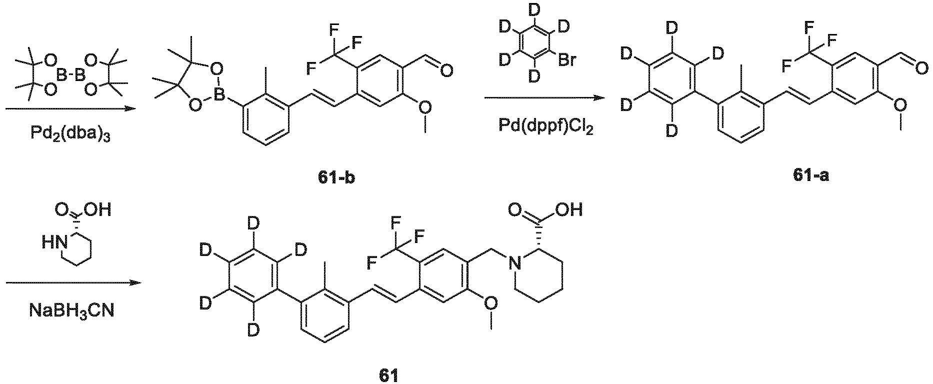

### Synthesis of compound 61-d

Bis(tri-*tert*-butylphosphine)palladium (400 mg, 0.8 mmol) and triethylamine (3.03 g, 30.0 mmol) were added to a solution of 1-bromo-3-chloro-2-methylbenzene (2.05 g, 10.0 mmol) and pinacol vinylboronate (2.00 g, 13.0 mmol) in toluene (40 mL), and the mixture was stirred at 80°C for 16 hours under nitrogen. The reaction mixture was cooled to room temperature, filtered, and the filtrate was concentrated under reduced pressure, and the residue was purified by silica gel column chromatography (petroleum ether: ethyl acetate = 10:1) to give compound **61-d** (2.36 g, yield: 85%).

### Synthesis of compound 61-c

[1,1'-Bis(diphenylphosphino)ferrocene]palladium dichloride (73 mg, 0.10 mmol), potassium phosphate (424 mg, 2.0 mmol) and cesium fluoride (300 mg, 2.0 mmol) were added to a mixture of compound **61-d** (279 mg, 1.00 mmol), 4-bromo-2-methoxy-5-trifluoromethylbenzaldehyde (300 mg, 1.06 mmol) and toluene (15 mL), and stirred at 90°C for 16 h under nitrogen. The reaction mixture was cooled to room temperature, concentrated under reduced pressure, and the residue was purified by silica gel column chromatography (petroleum ether: ethyl acetate = 8:1) to give compound **61-c** (205 mg, yield: 58%).

### Synthesis of compound 61-b

Tris(dibenzylideneacetone)dipalladium (37 mg, 0.04 mmol), 2-dicyclohexylphosphino-2',4',6'-triisopropylbiphenyl (76 mg, 0.16 mmol) and potassium acetate (165 mg, 1.68 mmol) were added to a mixture of compound **61-c** (200 mg, 0.56 mmol), bis(pinacolato)diboron (200 mg, 0.78 mmol) and toluene (15 mL). The mixture was stirred at 90°C for 16 h under nitrogen. The reaction mixture was cooled to room temperature, concentrated under reduced pressure, and the residue was purified by silica gel column chromatography (petroleum ether: ethyl acetate = 7:1) to give compound **61-c** (103 mg, yield: 41%).
¹H NMR (400 MHz, CD₃Cl): δ 10.44 (s, 1H), 8.15 (s, 1H), 7.77 (d, *J*=7.6Hz, 1H), 7.62 (d, *J*=7.6Hz, 1H), 7.46 (d, *J*=16.0Hz, 1H), 7.23-7.30 (m, 3H), 4.07(s, 3H), 2.64 (s, 3H), 1.37 (s, 12H) ppm

### Synthesis of compound 61-a

[1,1'-Bis(diphenylphosphino)ferrocene]palladium dichloride (32 mg, 0.044 mmol), potassium phosphate (93 mg, 0.44 mmol) and cesium fluoride (66 mg, 0.44 mmol) were added to a solution of compound **61-b** (100 mg, 0.22 mmol), pentadeuterated bromobenzene (54 mg, 0.33 mmol) in toluene (10 mL), and the mixture was stirred at 90°C for 16 h under nitrogen. The reaction mixture was cooled to room temperature, concentrated under reduced pressure, and the residue was purified by silica gel column chromatography (petroleum ether: ethyl acetate = 5:1) to give compound **61-a** (60 mg, yield: 68%).
¹H NMR (400 MHz, CD₃Cl): δ 10.44 (s, 1H), 8.16 (s, 1H), 7.59 (d, J=7.6Hz, 1H), 7.48 (d, J=16.0Hz, 1H), 7.33-7.38 (m, 3H), 7.24-7.28 (m, 1H), 4.07(s, 3H), 2.33 (s, 3H) ppm

### Synthesis of compound 61

L-Piperidine-2-carboxylic acid (40 mg, 0.3 mmol) and methanol (10 mL) were added to a solution of compound **61-a** (60 mg, 0.15 mmol) in dichloromethane (10 mL). The mixture was stirred for 1 h at room temperature, then sodium cyanoborohydride (28 mg, 0.45 mmol) was added. The mixture was stirred for 16 h at room temperature after the addition. The reaction mixture was concentrated under reduced pressure, and the residue was purified by preparative liquid chromatography to give compound **61** (21 mg, yield: 27%).

LC-MS (ESI): m/z = 515 [M+H]⁺.

¹H NMR (400 MHz, CD₃OD): *δ* 7.90 (s, 1H), 7.63 (d, *J*=16.0Hz, 1H), 7.54 (d, *J*=8.0Hz, 1H), 7.53 (s, 1H), 7.26-7.33 (m, 2H), 7.18 (d, *J*=7.6Hz, 1H), 4.53 (d, *J*=12.8Hz, 1H), 4.40 (d, *J*=12.8Hz, 1H), 4.07 (s, 3H), 3.49-3.52 (m, 1H), 2.97-3.03 (m, 1H), 2.30(s, 3H), 2.23-2.27 (m, 1H), 1.67-1.85 (m, 5H), 1.54-1.59 (m, 1H) ppm.

### Embodiment 62

### (R,E)-1-(2-Methoxy-4-(2-(2-methyl-[1,1'-biphenyl]-3-yl)vinyl)-5-trifluoromethylbenzyl)piperidine-2-carboxylic acid (compound 62)

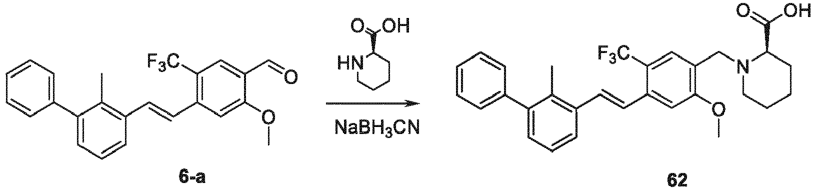

### Synthesis of compound 62

Sodium cyanoborohydride (63 mg, 1.0 mmol) was added to a solution of compound **6-a** (198 mg, 0.5 mmol) and D-piperidine-2-carboxylic acid (161.2 mg, 1.25 mmol) in methanol (30 mL). The mixture was heated to 60°C and stirred for 2 hours, cooled to room temperature, and concentrated under reduced pressure. The residue was washed with water (30 mL×3), filtered, and the filtrate was concentrated under reduced pressure, and prepared by high performance liquid chromatography to give white solid product **62** (110 mg, yield: 43%).

LC-MS (ESI): m/z = 510.5 [M+H]⁺.

¹H NMR: (400 MHz DMSO-*d6*): *δ* 7.78 (s, 1H), 7.65 (d, *J=* 16.0 Hz, 1H), 7.58 (d, *J* = 7.6 Hz, 1H), 7.50-7.46 (m, 3H), 7.42-7.32 (m, 4H), 7.25-7.19 (m, 2H), 3.97 (s, 3H), 3.82-3.67 (m, 2H), 2.94-2.90 (m, 1H), 2.30 (s, 3H), 2.29-2.27 (m, 1H), 1.85-1.78 (m, 2H), 1.52-1.41 (m, 4H) ppm.

### Embodiment 63

### (S,E)-1-(2,5-Dimethoxy-4-(2-(2-methyl-[1,1'-biphenyl]-3-yl)vinyl)benzyl)piperidine-2-carboxylic acid (compound 63)

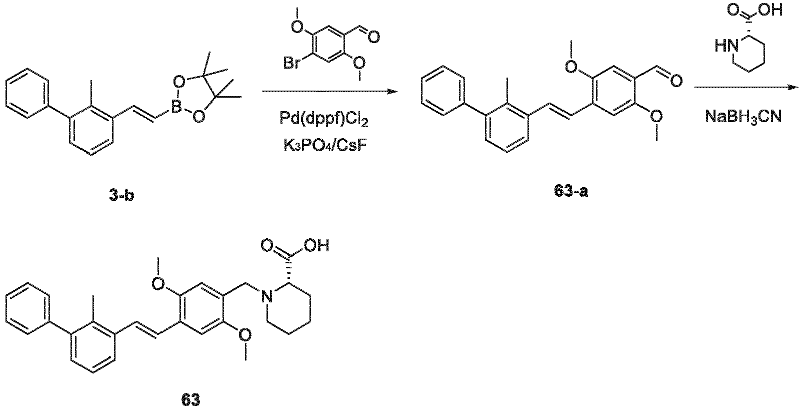

### Synthesis of compound 63-a

[1,1'-Bis(diphenylphosphino)ferrocene]palladium dichloride (36.6 mg, 0.05 mmol), potassium phosphate (318 mg, 1.5 mmol) and cesium fluoride (231 mg, 1.5 mmol) were added to a mixture of compound **3-b** (160 mg, 0.5 mmol), 4-bromo-2,5-dimethoxybenzaldehyde (122.5 mg, 0.5 mmol) and toluene (25 mL), and the mixture was stirred at 110°C for 12 h under nitrogen. The reaction mixture was cooled to room temperature, filtered, and the filtrate was concentrated under reduced pressure. The residue was purified by silica gel column chromatography (petroleum ether: ethyl acetate = 20 - 3:1) to give compound **63-a** (140 mg, yield: 78%).

LC-MS (ESI): m/z = 359.2 [M+H]⁺.

### Synthesis of compound 63

(S)-Piperidine-2-carboxylic acid (126.1 mg, 0.97 mmol) and sodium cyanoborohydride (49.1 mg, 0.78 mmol) were added to a solution of compound **63-a** (140.0 mg, 0.39 mmol) in methanol (20 mL), and the mixture was heated to 60°C and stirred for 2 h. The reaction solution was cooled to room temperature, concentrated under reduced pressure, and the residue was prepared by high performance liquid chromatography to give compound **63** (110 mg, yield: 59%).

LC-MS (ESI): m/z = 472.3 [M+H]+.
¹H NMR (400 MHz, CD₃OD): *δ* 7.60 (d, *J* = 12.0 Hz, 1H), 7.56 (d, *J=* 2.0 Hz, 1H), 7.43-7.40 (m, 2H), 7.39-7.21 (m, 6H), 7.11 (d, *J* = 7.2 Hz, 1H), 4.47 (d, *J* = 12.4 Hz, 1H), 4.36 (d, *J* = 12.4 Hz, 1H), 3.97 (s, 3H), 3.91 (s, 3H), 3.50-3.47 (m, 1H), 3.38-3.30 (m, 1H), 2.95-2.90 (m, 1H), 2.27 (s, 3H), 2.26-2.23 (m, 1H), 1.90-1.53 (m, 5H)ppm

### Embodiment 64

### (S,Z)-1-(4-(1-Fluoro-2-(2-methyl-[1,1'-biphenyl]-3-yl)vinyl)2-methoxy-5-trifluoromethylbenzyl)piperidine-2-carboxylic acid (compound 64)

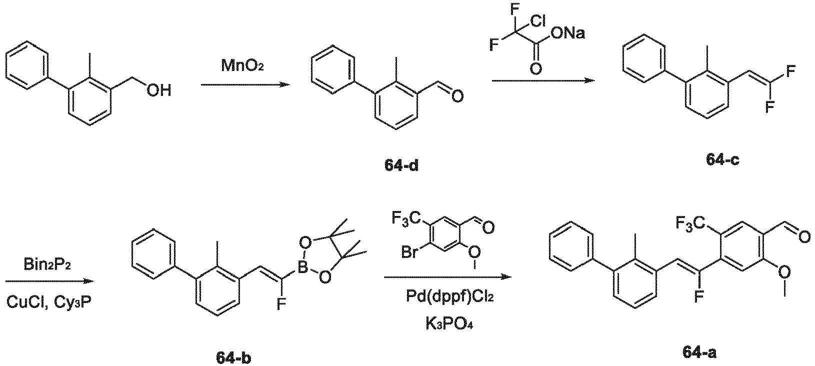

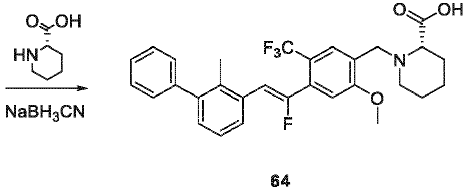

### Synthesis of compound 64-d

(2-Methyl-[1,1'-biphenyl]-3-yl)methanol (1.98 g, 10.0 mmol) was dissolved in dichloromethane (100 mL), and manganese dioxide (2.64 g, 30.0 mmol) was added to the solution. The reaction mixture was stirred at room temperature for 16 hours, filtered, and the filtrate was concentrated under reduced pressure, and the residue was purified by silica gel column chromatography (petroleum ether: ethyl acetate = 10-5:1) to give colorless crystal **64-d** (1.9 g, yield: 96.9%)

LC-MS (ESI): m/z = 197.2 [M+H]⁺.

### Synthesis of compound 64-c

Compound **64-d** (3.92 g, 20 mmol) was dissolved in *N*,*N*-dimethylformamide (39 mL), then triphenylphosphine (6.1 g, 23.28 mmol) was added, and the mixture was heated to 100°C, and then a 2.0 M solution of sodium chlorodifluoroacetate (4.4 g, 29.1 mmol) in *N,N-*dimethylformamide was added dropwise, and the reaction was continued stirring at 100°C for 1 h. The mixture was poured into water (200 mL), and extracted with ethyl acetate (100 mL×2). The organic phase was washed with water (100 mL) and saturated brine (100 mL) in turn, dried with anhydrous sodium sulfate, and the filtrate was concentrated under reduced pressure. The residue was purified by silica gel column chromatography (petroleum ether) to give compound **64-c** (2.67 g, yield: 58.1%).

### Synthesis of compound 64-b

Compound **64-c** (2.3 g, 10 mmol) was dissolved in tetrahydrofuran (30 mL), and bis(pinacolato)diboron (4.04 g, 15.9 mmol), cuprous chloride (10.5 mg, 0.106 mmol), tricyclohexylphosphine (59.4 mg, 0.212 mmol) and potassium acetate (1.25 g, 12.72 mmol) were added. The reaction was stirred at 40°C for 16 hours. After cooling to room temperature, the reaction mixture was concentrated under reduced pressure and the residue was purified by silica gel column chromatography (petroleum ether: ethyl acetate = 9:1) to give compound **64-b** (3.0 g, yield: 90%)

### Synthesis of compound 64-a

A mixture of compound **64-b** (200 mg, 0.59 mmol), 4-bromo-2-methoxy-5-trifluoromethylbenzaldehyde (168 mg, 0.59 mmol), potassium carbonate (163 mg, 1.18 mmol), [1,1'-bis(diphenylphosphino)ferrocene]palladium dichloride (43 mg, 0.059 mmol) in a mixed solvent of 1,4-dioxane (5 mL) and water (0.5 mL) was stirred at 80°C under nitrogen for 16 hours, and then cooled to room temperature. The reaction mixture was concentrated under reduced pressure, and the residue was purified by silica gel column chromatography (petroleum ether: ethyl acetate = 10:1) to give compound **64-a** (154 mg, yield: 63.0%).

LC-MS (ESI): m/z = 415 [M+H]⁺.

### Synthesis of compound 64

A mixture of compound **64-a** (29 mg, 0.07 mmol), (*S*)-piperidine-2-carboxylic acid (24 mg, 0.19 mmol), sodium cyanoborohydride (12 mg, 0.19 mmol) and methanol (3 mL) was stirred at 80°C for 3 h under nitrogen, and then cooled to room temperature. The reaction mixture was concentrated under reduced pressure, and the residue was prepared by high performance liquid chromatography to give compound **64** (15 mg, yield: 40.6%).

LC-MS (ESI): m/z = 528 [M+H]⁺.
¹H NMR (400 MHz, DMSO-*d₆*): δ 7.87(s, 1H), 7.62 (d, *J*=7.6Hz, 1H), 7.46 (d, *J*=7.6 Hz, 2H), 7.39 (t, *J*=7.2Hz, 1H), 7.35-7.31 (m, 4H), 7.18 (d, *J*=7.6Hz,1H), 6.50 (d, *J*=36.8 Hz, 1H), 3.95 (s, 3H), 3.81-3.67 (m, 2H), 3.24-3.22 (m, 1H), 2.90-2.88 (m, 1H), 2.28-2.25 (m, 1H), 2.21 (s, 3H), 1.88- 1.73 (m,2H), 1.50 - 1.41 (m, 4H) ppm

### Embodiment 65

### (Z)-2-((4-(1-Fluoro-2-(2-methyl-[1,1'-biphenyl]-3-yl)vinyl)-2-methoxy-5-trifluoromethylbenzyl)amino)-1-ethanol (compound 65)

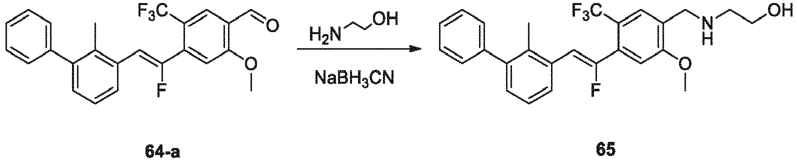

### Synthesis of compound 65

A mixture of compound **64-a** (29 mg, 0.07 mmol), ethanolamine (11 mg, 0.19 mmol), sodium cyanoborohydride (12 mg, 0.19 mmol) and methanol (3 mL) was stirred at 80°C for 3 h under nitrogen, and then cooled to room temperature. The reaction mixture was concentrated under reduced pressure, and the residue was prepared by high performance liquid chromatography to give compound **64** (18 mg, yield: 50.1%).

LC-MS (ESI): m/z = 460 [M+H]⁺.
¹H NMR (400 MHz, DMSO-*d₆*): *δ* 7.83 (s, 1H), 7.62 (d, *J*=7.6 Hz,1H), 7.46 (d, *J*=7.6 Hz, 2H), 7.39 (t, *J*=7.2 Hz, 1H), 7.35-7.31 (m, 4H), 7.17 (d, *J*=7.6 Hz, 1H), 6.50 (d, *J*=36.8 Hz, 1H), 4.51 (t, *J*=5.2Hz, 1H), 3.96 (s, 3H), 3.77 (s, 3H), 3.49 (q, *J*=5.6 Hz, 2H), 2.60 (t, *J*=5.6Hz, 2H), 2.20 (s, 3H) ppm

### Embodiment 66

### (Z)-2-((4-(1-Bromo-2-(2-methyl-[1,1'-biphenyl]-3-yl)vinyl)-2-methoxy-5-trifluoromethylbenzyl)amino)-1-ethanol (compound 66)

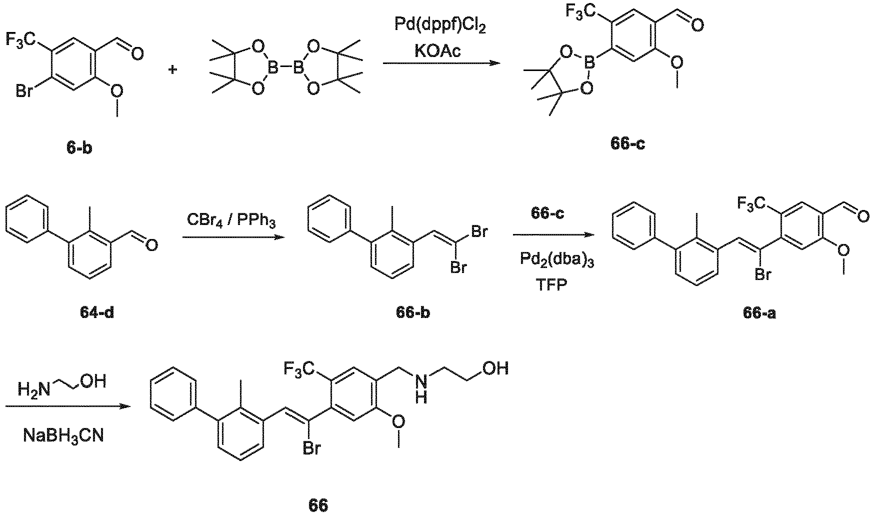

### Synthesis of compound 66-c

Compound **6-b** (1.42 g, 5.0 mmol) and compound bis(pinacolato)diboron (1.90 g, 7.5 mmol) were dissolved in toluene (60 mL), and [1,1'-bis(diphenylphospho)ferrocene]palladium dichloride (366 mg, 0.5 mmol) and potassium acetate (1.47 g, 15.0 mmol) were added to the solution. The reaction solution was heated to 100°C and stirred for 6 hours, then cooled to room temperature. The reaction mixture was concentrated under reduced pressure, and the residue was purified by silica gel column chromatography (petroleum ether: ethyl acetate = 15-3:1) to give light yellow solid **66-c** (1.5 g, yield: 90%).

### Synthesis of compound 66-b

Compound **64-d** (588 mg, 3.0 mmol) was dissolved in anhydrous dichloromethane (40 mL), and carbon tetrabromide (1.48 g, 4.5 mmol) was added, and then cooled to 0°C. A solution of triphenylphosphine (1.56 g, 6.0 mmol) in dichloromethane (10 mL) was slowly added dropwise to the reaction mixture. The reaction solution was raised to room temperature and continued stirring for 1 hour. The reaction mixture was filtered, and the filtrate was washed with saturated sodium bicarbonate solution (20 mL) and saturated brine (20 mL), dried with anhydrous sodium sulfate, concentrated under reduced pressure, and the residue was purified by silica gel column chromatography (petroleum ether: ethyl acetate = 100-20:1) to give colorless oil **66-b** (1.05 g, yield: 99%).
¹H NMR (400 MHz, CDCl₃): *δ* 7.52 (s, 1H), 7.43-7.34 (m, 4H), 7.30-7.27 (m, 2H), 7.23-7.21 (m, 2H), 2.14 (s, 3H) ppm

### Synthesis of compound 66-a

Compound **66-b** (220 mg, 0.625 mmol) and compound 66-c (247.5 mg, 0.75 mmol) were dissolved in a mixture of dioxane (30 mL) and water (10 mL), and then bis(dibenzylideneacetone)palladium (28.6 mg, 0.03 mmol), tris(2-furyl)phosphine (43.5 mg, 0.187 mmol) and cesium fluoride (406.2 mg, 1.25 mmol) were added to the reaction, and the reaction solution was stirred at 65°C for 16 hours. The reaction solution was cooled to room temperature, concentrated under reduced pressure, and the residue was prepared by high performance liquid chromatography to give white solid **66-a** (80 mg, yield: 27%).

### Synthesis of compound 66

Compound **66-a** (80 mg, 0.169 mmol) was dissolved in a mixture of dichloromethane (15 mL) and methanol (5 mL), and ethanolamine (51.5 mg, 0.845 mmol) and a drop of glacial acetic acid were added. The reaction solution was stirred at room temperature for 2 hours, then sodium cyanoborohydride (21.4 mg, 0.34 mmol) was added, and the reaction solution was continued stirring for 16 hours. The reaction solution was concentrated under reduced pressure, and the residue was prepared by high performance liquid chromatography to give white solid **66** (30.9 mg, yield: 35%).

LC-MS (ESI): m/z = 520.1 [M+H]⁺.
¹H NMR (400 MHz, CD₃OD): *δ* 7.71 (s, 1H), 7.51 (d, *J* = 8.0 Hz, 1H), 7.48-7.43 (m, 2H), 7.39-7.37 (m, 1H), 7.34-7.30 (m, 3H), 7.22 (d, *J* = 6.8 Hz, 1H), 7.17 (s, 1H), 7.08 (s, 1H), 4.04 (s, 3H), 3.90 (s, 2H), 3.71 (t, *J* = 6.0 Hz, 2H), 2.76 (t, *J* = 6.0 Hz, 2H), 2.20 (s, 3H) ppm

### Embodiment 67

### (S,Z)-1-(4-(1-Bromo-2-(2-methyl-[1,1'-biphenyl]-3-yl)vinyl)-2-methoxy-5-trifluoromethylbenzyl)piperidine-2-carboxylic acid (compound 67)

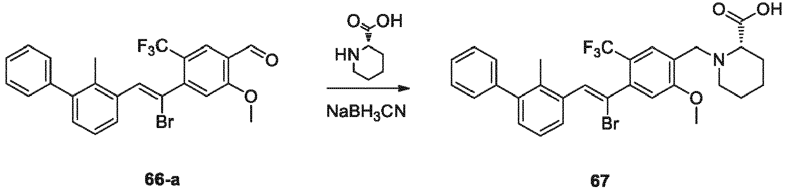

### Synthesis of compound 67

A mixture of compound **66-a** (110 mg, 0.23 mmol), (*S*)-piperidine-2-carboxylic acid (89 mg, 0.69 mmol), sodium cyanoborohydride (28.9 mg, 0.46 mmol) and methanol (20 mL) was stirred at 60°C for 2 h and then cooled to room temperature. The reaction solution was concentrated under reduced pressure, and the residue was prepared by high performance liquid chromatography to give compound **67** (46 mg, yield: 34%).

LC-MS (ESI): m/z = 588.2 [M+H]⁺.
¹H NMR (400 MHz, CD₃OD): δ 8.00 (s, 1H), 7.53 (d, *J* = 7.6 Hz, 1H), 7.48-7.43 (m, 2H), 7.38 (d, *J* = 7.2 Hz, 1H), 7.34-7.30 (m, 4H), 7.23 (d, *J* = 7.2 Hz, 1H), 7.12 (s, 1H), 4.59 (d, *J* = 13.2 Hz, 1H), 4.45 (d, *J* = 12.4 Hz, 1H), 4.05 (s, 3H), 3.57-3.53 (m, 1H), 3.43-3.39 (m, 1H), 3.07-3.01 (m, 1H), 2.33-2.26 (m, 1H), 2.20 (s, 3H), 1.93-1.58 (m, 5H) ppm

### Embodiment 68

### (S,Z)-1-(4-(2-Bromo-2-(2-methyl-[1,1'-biphenyl]-3-yl)vinyl)-2-methoxy-5-methylbenzyl)piperidine-2-carboxylic acid (compound 68)

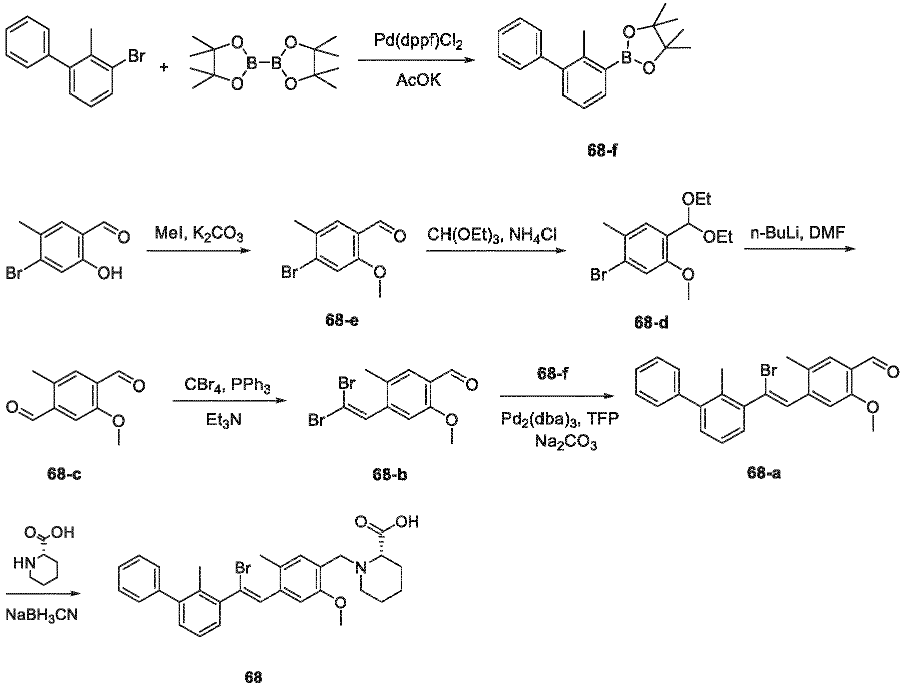

### Synthesis of compound 68-f

A mixture of 3-bromo-2-methyl-1,1'-biphenyl (989 mg, 4.0 mmol), bis(pinacolato)diboron (1.52 g, 6.0 mmol), [1,1'-bis(diphenylphospho)ferrocene]palladium dichloride (293 mg, 0.4 mmol), potassium acetate (1.18 g, 1.2 mmol) and 1,4-dioxane (10 mL) were stirred at 90°C under nitrogen for 16 hours. The reaction solution was cooled to room temperature, concentrated under reduced pressure, and the residue was purified by silica gel column chromatography (petroleum ether: ethyl acetate = 20:1) to give compound **68-f** (1.0 g, yield: 85%).

### ¹H NMR (400 MHz, CD₃OD): δ 7.76 (dd, J = 7.2, 1.3 Hz, 1H), 7.43 - 7.36 (m, 2H), 7.36 - 7.26 (m, 4H), 7.24 - 7.19 (m, 1H), 2.41 (s, 3H), 1.36 (s, 12H) ppm

### Synthesis of compound 68-e

A mixture of 4-bromo-2-hydroxy-5-methylbenzaldehyde (6.45 g, 30.0 mmol), iodomethane (5.11 g, 36.0 mmol), potassium carbonate (8.29 g, 60.0 mmol) and *N,N'-*dimethylformamide (30 mL) was stirred for 16 h at room temperature. The reaction solution was diluted with water (150 mL). The precipitated solid was filtered out and dried, and purified by silica gel column chromatography (petroleum ether: ethyl acetate = 10:1) to give white solid **68-e** (5.59 g, yield: 81%).

### Synthesis of compound 68-d

A mixture of compound **68-e** (5.10 g, 22.3 mmol), triethyl orthoformate (6.60 g, 44.5 mmol), ammonium chloride (119 mg, 2.2 mmol) and ethanol (3.08 g, 66.8 mmol) was refluxed for 1 hour. After cooling to room temperature, the reaction solution was concentrated under reduced pressure and the obtained product **68-d** (6.75 g) obtained after vacuum drying was used directly in the next step.

### Synthesis of compound 68-c

Compound **68-d** (1.52 g, 5.0 mmol) was dissolved in anhydrous tetrahydrofuran (15 mL). *n*-Butyllithium (2.2 mL, 2.5 M *n*-hexane solution) was slowly added dropwise at -78°C. After the dropwise addition was completed, *N,N*'-dimethylformamide (431 mg, 10 mmol) was added dropwise at -78°C with stirring for 0.5 h. After the dropwise addition was completed, the mixture was stirred at -78°C for 2 hours. After raising to room temperature, the reaction solution was quenched with saturated sodium bicarbonate solution (10 mL). The resulting mixture was extracted with ethyl acetate (50 mL). The organic phase was concentrated under reduced pressure and the residue was purified by silica gel column chromatography (petroleum ether: ethyl acetate = 10:1) to give light yellow liquid **68-c** (1.07 g, yield: 85%).
¹H NMR (400 MHz, DMSO-*d₆*): *δ* 10.24 (s, 1H), 7.41 (s, 1H), 7.36 (s, 1H), 5.67 (s, 1H), 3.85 (s, 3H), 3.61 - 3.47 (m, 4H), 2.56 (s, 3H), 1.12 (t, *J* = 7.0 Hz, 6H) ppm

### Synthesis of compound 68-b

A solution of carbon tetrabromide (1.72 g) in dichloromethane (5 mL) was added dropwise to a mixture of triphenylphosphine (2.73 g, 10.4 mmol) and dichloromethane (10 mL) at 0°C. After the dropwise addition was completed, the mixture was continued stirring at 0°C for 10 minutes. A solution of compound **68-c** (1.01 g, 4.0 mmol) and triethylamine (1.21 g) in dichloromethane (5 mL) was then added dropwise. After the dropwise addition was completed, the reaction solution was raised to room temperature and stirred for 4 hours. The reaction solution was concentrated under reduced pressure, and the residue was purified by silica gel column chromatography (petroleum ether: ethyl acetate = 20:1) to give white solid **68-b** (700 mg, yield: 52%).
¹H NMR (400 MHz, DMSO-*d₆*): *δ* 10.31 (s, 1H), 7.83 (s, 1H), 7.55 (s, 1H), 7.21 (s, 1H), 3.91 (s, 3H), 2.20 (s, 3H) ppm

### Synthesis of compound 68-a

A mixture of compound **68-b** (586 mg,1.75 mmol), compound **68-f** (397 mg, 1.35 mmol), bis(dibenzylideneacetone)palladium (62 mg, 0.068 mmol), tris(2-furanyl)phosphine (94 mg, 0.40 mmol), a sodium carbonate solution (2.7 mL, 1 M), and dioxane (7 mL) was reacted at 60°C under nitrogen for 5 h. The reaction solution was cooled to room temperature, diluted with water (10 mL), and the resulting mixture was extracted with dichloromethane (30 mL). The organic phase was concentrated under reduced pressure, and the residue was prepared by high performance liquid chromatography to give compound **68-a** (88 mg, yield: 10%).

LC-MS (ESI): m/z = 421.2 [M+H]⁺.

### Synthesis of compound 68

Sodium cyanoborohydride (25 mg, 0.40 mmol) was added to a mixture of compound **68-a** (42 mg, 0.10 mmol), (S)-piperidine-2-carboxylic acid (26 mg, 0.20 mmol), methanol (1 mL) and tetrahydrofuran (1 mL). The reaction solution was heated to 60°C and stirred for 1 h, and then cooled to room temperature, concentrated under reduced pressure, and the residue was prepared by high performance liquid chromatography to give white solid **68** (21 mg, yield: 39%).

LC-MS (ESI): m/z = 534.2 [M+H]⁺.
¹H NMR (400 MHz, CD₃OD): *δ* 7.47 - 7.25 (m, 9H), 7.22 (dd, *J* = 7.5, 1.4 Hz, 1H), 7.03 (s, 1H), 4.47 (d, *J* = 12.7 Hz, 1H), 4.34 (d, *J* = 12.8 Hz, 1H), 3.94 (s, 3H), 3.50 (d, *J* = 11.3 Hz, 1H), 3.32 (s, 1H), 2.97 (t, *J* = 11.9 Hz, 1H), 2.32 (s, 3H), 2.31 (s, 3H), 2.23 (d, *J* = 10.5 Hz, 1H), 1.98 - 1.63 (m, 4H), 1.61 - 1.46 (m, 1H) ppm

### Embodiment 69

### (Z)-2-((4-(2-Bromo-2-(2-methyl-[1,1'-biphenyl]-3-yl)vinyl)-2-methoxy-5-methylbenzyl)amino)-1-ethanol (compound 69)

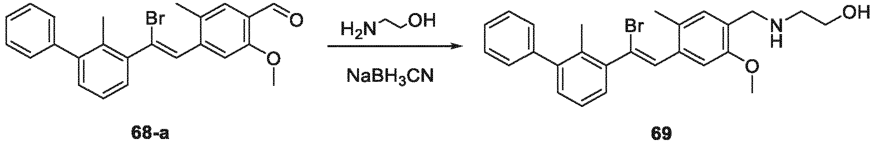

### Synthesis of compound 69

Sodium cyanoborohydride (18 mg, 0.28 mmol) was added to a mixture of compound **68-a** (29 mg, 0.07 mmol), ethanolamine (9 mg, 0.15 mmol), methanol (1 mL) and tetrahydrofuran (1 mL). The reaction solution was stirred at 60°C for 1 h, and then cooled to room temperature, concentrated under reduced pressure, and the residue was prepared by high performance liquid chromatography to give white solid **69** (17 mg, yield: 52%).

LC-MS (ESI): m/z = 466.2, 468.2 [M+H]⁺.
¹H NMR (400 MHz, *δ* 8.53 (s, 1H), 7.47 - 7.20 (m, 10H), 7.03 (s, 1H), 4.20 (s, 2H), 3.96 (s, 3H), 3.84 - 3.77 (m, 2H), 3.13 - 3.06 (m, 2H), 2.32 (s, 3H), 2.31 (s, 3H) ppm

### Embodiment 70

### (S,E)-1-(2-Methoxy-5-methyl-4-(2-(2-methyl-[1,1'-biphenyl]-3-yl)vinyl)benzyl)piperidine-2-carboxylic acid (compound 70)

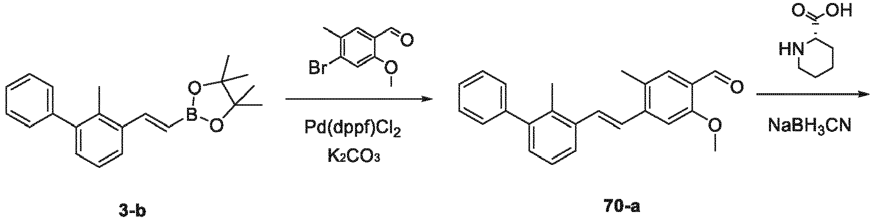

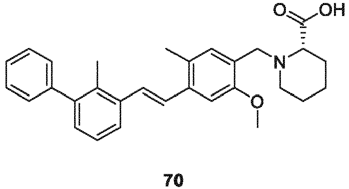

### Synthesis of compound 70-a

A mixture of 4-bromo-2-methoxy-5-methylbenzaldehyde (115 mg, 0.5 mmol), compound **3-b** (192 mg, 0.6 mmol), [1,1'-bis(diphenylphosphino)ferrocene]palladium dichloride (37 mg, 0.05 mmol), potassium carbonate (138 mg, 1.0 mmol), 1,4-dioxane (2 mL) and water (0.4 mL) was stirred for 16 h at 90°C under nitrogen. The reaction solution was cooled to room temperature, diluted with saturated brine (10 mL), and the resulting mixture was extracted with dichloromethane (20 mL×2). The organic phase was combined, dried with anhydrous sodium sulfate, filtered, concentrated under reduced pressure, and the residue was purified by silica gel column chromatography (petroleum ether: ethyl acetate = 5:1) to give yellow solid **70-a** (135 mg, yield: 79%).

### Synthesis of compound 70

Sodium cyanoborohydride (37 mg, 0.60 mmol) was added to a mixture of compound **70-a** (51 mg, 0.15 mmol), (S)-piperidine-2-carboxylic acid (38 mg, 0.30 mmol), methanol (1 mL) and tetrahydrofuran (1 mL). The reaction solution was stirred at 60°C for 1 h, and then cooled to room temperature, concentrated under reduced pressure, and the residue was prepared by high performance liquid chromatography to give white solid 70 (26 mg, yield: 38%).

LC-MS (ESI): m/z = 456.3 [M+H]⁺.
¹H NMR (400 MHz, *δ* 7.60 (d, *J* = 7.5 Hz, 1H), 7.48 - 7.19 (m, 10H), 7.14 (d, *J* = 7.4 Hz, 1H), 4.46 (d, *J* = 12.7 Hz, 1H), 4.33 (d, *J* = 12.7 Hz, 1H), 3.96 (s, 3H), 3.48 (d, *J* = 7.1 Hz, 1H), 3.38 - 3.32 (m, 1H), 2.96 (t, *J* = 11.7 Hz, 1H), 2.40 (s, 3H), 2.29 (s, 3H), 2.23 (d, *J* = 13.4 Hz, 1H), 1.97 - 1.43 (m, 5H) ppm

### Embodiment 71

### (S,E)-1-(4-(2-(2-Chloro-[1,1'-biphenyl]-3-yl)vinyl)-2-methoxy-5-trifluoromethylbenzyl)piperidine-2-carboxylic acid (compound 71)

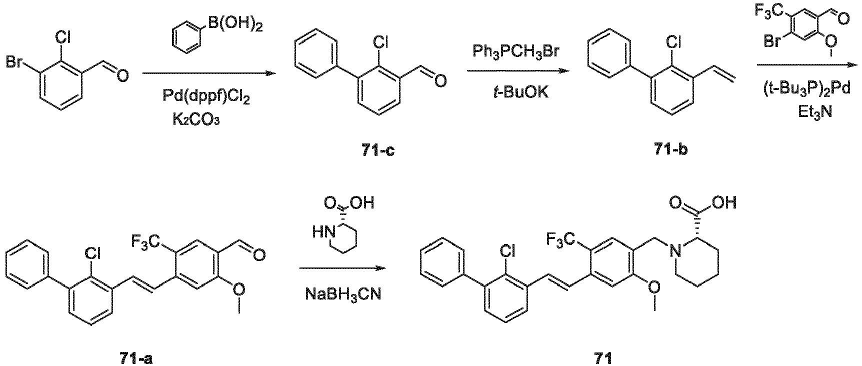

### Synthesis of compound 71-c

A mixture of 3-bromo-2-chloro-benzaldehyde (878 mg, 4.0 mmol), phenylboronic acid (536 mg, 4.4 mmol), [1,1'-bis(diphenylphosphino)ferrocene]palladium dichloride (146 mg, 0.2 mmol), potassium carbonate (1.11 g, 8.0 mmol), 1,4-dioxane (12 mL) and water (4.0 mL) was stirred at 90°C for 16 hours under nitrogen. The reaction solution was cooled to room temperature, diluted with water (20 mL), and the mixture was extracted with dichloromethane (40 mL). The organic phase was separated, dried with anhydrous sodium sulfate, concentrated under reduced pressure, and the residue was purified by silica gel column chromatography (petroleum ether: ethyl acetate = 30: 1) to give colorless liquid **71-c** (764 mg, yield: 88%).

### Synthesis of compound 71-b

Potassium *tert*-butoxide (787 mg, 4.02 mmol) was added to a mixture of methyltriphenylphosphonium bromide (2.51 g, 7.02 mmol) and tetrahydrofuran (20 mL) at room temperature, and the mixture was stirred for 2 h at room temperature. The reaction solution was cooled to -78°C and compound **71-c** (760 mg, 3.51 mmol) was added, then the reaction solution was raised to room temperature and stirred for 16 h. The reaction solution was quenched with saturated ammonium chloride solution (30 mL). The resulting mixture was extracted with ethyl acetate (40 mL×2). The organic phase was combined, washed with saturated brine (20 mL), dried with anhydrous sodium sulfate, concentrated under reduced pressure, and the residue was purified by silica gel column chromatography (eluent: petroleum ether) to give colorless liquid compound **71-b** (360 mg, yield: 48%).

### Synthesis of compound 71-a

A mixture of compound **71-b** (107 mg, 0.50 mmol), 4-bromo-2-methoxy-5-(trifluoromethyl)benzaldehyde (212 mg, 0.75 mmol), bis(tri-*tert*-butylphosphine)palladium (26 mg, 0.05 mmol), triethylamine (506 mg, 5.0 mmol) and toluene (2 mL) was reacted at 80°C under nitrogen for 16 h. The reaction solution was cooled to room temperature, concentrated under reduced pressure, and the residue was purified by silica gel column chromatography (petroleum ether: ethyl acetate = 5:1) to give yellow solid **71-a** (60 mg, yield: 29%).

LC-MS (ESI): m/z = 417.2 [M+H]⁺.

### Synthesis of compound 71

Sodium cyanoborohydride (36 mg, 0.576 mmol) was added to a mixture of compound **71-a** (60 mg, 0.144 mmol), (*S*)-piperidine-2-carboxylic acid (37 mg, 0.288 mmol), methanol (1 mL) and tetrahydrofuran (1 mL). The reaction solution was stirred at 60°C for 1 h, and then cooled to room temperature, concentrated under reduced pressure, and the residue was prepared by high performance liquid chromatography to give white solid **71** (19 mg, yield: 25%).

LC-MS (ESI): m/z = 530.2 [M+H]⁺.
¹H NMR (400 MHz, δ 7.93 (s, 1H), 7.75 - 7.66 (m, 2H), 7.52 - 7.35 (m, 8H), 7.33 (dd, *J* = 7.5, 1.3 Hz, 1H), 4.45 (d, *J* = 13.0 Hz, 1H), 4.30 (d, *J* = 13.1 Hz, 1H), 4.05 (s, 3H), 3.43 (dd, *J* = 10.5, 3.3 Hz, 1H), 3.30 - 3.24 (m, 1H), 2.88 (t, *J* = 10.6 Hz, 1H), 2.21 (d, *J* = 11.9 Hz, 1H), 1.94 - 1.44 (m, 5H) ppm

### Embodiment 72

### (S,E)-1-(2-Methoxy-4-(2-(2-methyl-[1,1'-biphenyl]-3-yl)vinyl-1,2-d₂)-5-trifluoromethylbenzyl)piperidine-2-carboxylic acid (compound 72)

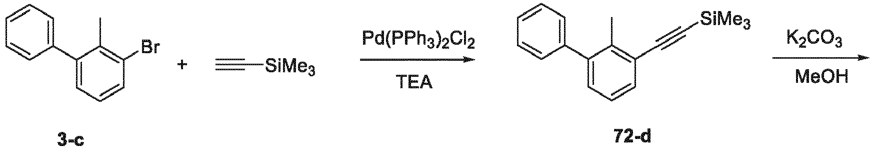

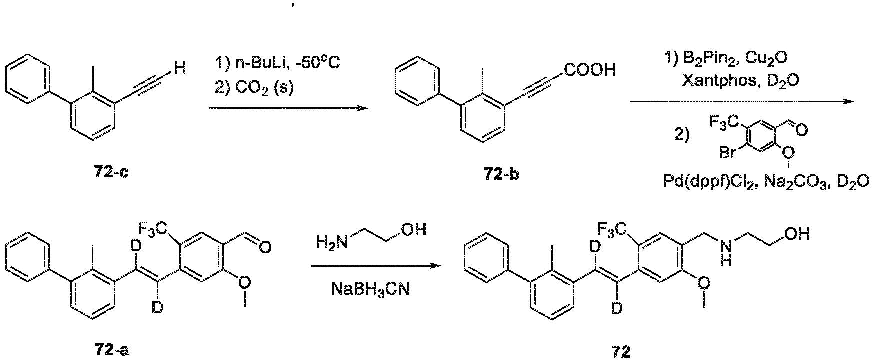

### Synthesis of compound 72-d

Compound **3-c** (1.0 g, 4.05 mmol) and trimethylsilylacetylene (596.2 mg, 6.07 mmol) were dissolved in *N*,*N*-dimethylformamide (15 mL), and then [1,1'-bis(diphenylphosphino)ferrocene]palladium dichloride (142.5 mg, 0.203 mmol) and triethylamine (3.279 g, 32.4 mmol) were added. The reaction solution was heated to 70°C under nitrogen and stirred for 16 hours, cooled to room temperature, diluted with ethyl acetate (15 mL), washed once with water (50 mL) and saturated brine (50 mL), respectively. The resulting organic phase was dried with anhydrous sodium sulfate, concentrated under reduced pressure, and the residue was purified by silica gel column chromatography (eluent: petroleum ether) to give compound **72-d** (551 mg, yield: 51.5%).
¹H NMR (400 MHz, CDCl₃): *δ* 7.56-7.54 (d, *J* = 7.6Hz, 1H), 7.44-7.34 (m, 3H), 7.28-7.26 (m, 2H), 7.17-7.15 (d, *J* = 7.6Hz, 1H), 7.10-7.06 (t, *J* = 7.6Hz, 1H), 2.31 (s, 3H) ppm

### Synthesis of compound 72-c

Compound **72-d** (551 mg, 2.084 mmol) was dissolved in methanol (15 mL), and potassium carbonate (863.9 mg, 6.251 mmol) was added. The reaction solution was stirred at room temperature for 0.5 hours. The reaction solution was concentrated under reduced pressure, and the residue was dissolved with ethyl acetate (50 mL) and washed with water (50 mL) and saturated brine (50 mL) in turn. The resulting organic phase was dried with anhydrous sodium sulfate, concentrated under reduced pressure, and the residue was purified by silica gel column chromatography (eluent: petroleum ether) to give compound **72-c** (361 mg, yield: 90.3%).
¹H NMR (400 MHz, CDCl₃): δ 7.50-7.48 (m, 1H), 7.43-7.35 (m, 3H), 7.30-7.28 (m, 2H), 7.23-7.19 (m, 2H), 3.29 (s, 1H), 2.37 (s, 3H) ppm

### Synthesis of compound 72-b

Compound **72-c** (100 mg, 0.52 mmol) was dissolved in anhydrous tetrahydrofuran (10 mL), then the reaction solution was cooled to -50°C in a dry ice-salt bath, and a solution of 1.56 M *n*-butyllithium (0.4 mL, 0.624 mmol) in *n*-hexane was added dropwise. After the dropwise addition was completed, the reaction solution was continued stirring at -50°C for 0.5 hours.

Solid dry ice was added to the above reaction solution and stirred for 0.5 hours, then, the reaction solution was raised to room temperature and continued stirring for 1 hour. The reaction was quenched with water (10 mL), and then the solution was adjusted to pH=1 to 2 with dilute hydrochloric acid (1N). The mixture was extracted with ethyl acetate (50 mL×2), and the organic phase was combined, dried with anhydrous sodium sulfate, concentrated under reduced pressure, and the residue was slurried with petroleum ether to give compound **72-b** (83 mg, yield: 67.5%).
¹H NMR (400 MHz, DMSO-*d₆*): *δ* 7.62-7.58 (m, 1H), 7.49-7.45 (m, 2H), 7.42-7.39 (m, 1H), 7.37-7.33 (m, 4H), 2.33 (s, 3H) ppm

### Synthesis of compound 72-a

Compound **72-b** (83 mg, 0.351 mmol) and bis(pinacolato)diboron (107.2 mg, 0.422 mmol) were dissolved in 1,4-dioxane (10 mL), then cuprous oxide (5 mg, 0.035 mmol), 4,5-bis(diphenylphosphino)-9,9-dimethyloxanthene (20.3 mg, 0.035 mmol) and deuterium oxide (42 mg, 2.106 mmol) were added. The reaction solution was stirred at room temperature overnight under nitrogen.

Compound **6-b** (99 mg, 0.351 mmol), [1,1'-bis(diphenylphosphino)ferrocene]palladium dichloride (30.3 mg, 0.035 mmol), sodium carbonate (93 mg, 0.878 mmol) and deuterium oxide (0.5 mL, 0.106 mmol) were added to the above reaction solution. The reaction solution was heated to 80°C and stirred overnight under nitrogen. The reaction solution was cooled to room temperature, diluted with ethyl acetate (20 mL) and washed with water (20 mL×2) and saturated brine (20 mL×2) in turn. The resulting organic phase was dried with anhydrous sodium sulfate, concentrated under reduced pressure, and the residue was purified by silica gel column chromatography (eluent: petroleum ether) to give compound **72-a** (41 mg, yield: 29.5%).
¹H NMR (400 MHz, CDCl₃): *δ* 10.442 (s, 1H), 8.16 (s, 1H), 7.60-7.58 (m, 1H), 7.45-7.35 (m, 4H), 7.32-7.28 (m, 4H), 4.07 (s, 3H), 2.33 (s, 3H) ppm

### Synthesis of compound 72

Compound **72-a** (56 mg, 0.106 mmol) was dissolved in a mixture of methanol (5.5 mL) and dichloromethane (5.5 mL), then glacial acetic acid (9 mg, 0.212 mmol) was added, and the reaction solution was stirred at room temperature for 1 hour, then, sodium cyanoborohydride (56 mg, 0.106 mmol) was added, and the reaction solution was continued stirring for 16 hours. The reaction solution was concentrated under reduced pressure. The residue was dissolved with ethyl acetate (20 mL), and washed with water (20 mL) and saturated brine (20 mL) in turn. The organic phase was dried with anhydrous sodium sulfate, concentrated under reduced pressure, and the residue was prepared by high performance liquid chromatography to give compound **72** (11.6 mg, yield: 25.4%).

LC-MS (ESI): m/z = 444 [M+H]⁺.
¹H NMR (400 MHz, *δ* 7.62 (s, 1H), 7.54-7.52 (d, *J* = 7.6Hz, 1H), 7.43-7.40 (m, 3H), 7.36-7.25 (m, 4H), 7.17-7.15 (m, 1H), 4.01 (s, 3H), 3.85 (s, 2H), 3.69-3.67 (m, 2H), 2.74-2.72 (m, 2H), 2.30 (s, 3H) ppm

### Embodiment 73

### (S,E)-1-(4-(2-(2-Fluoromethyl-[1,1'-biphenyl]-3-yl)vinyl)-2-methoxy-5-trifluoromethylbenzyl)piperidine-2-carboxylic acid (compound 73)

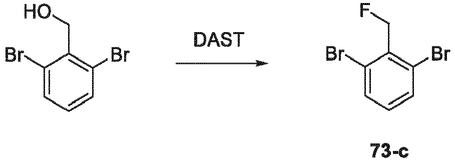

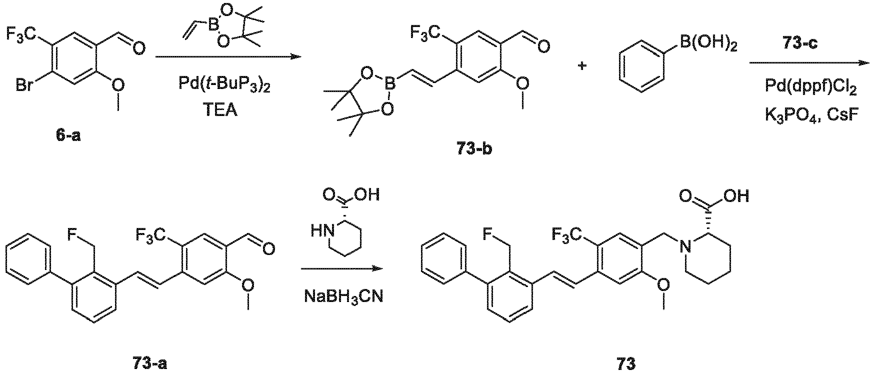

### Synthesis of compound 73-c

Diethylaminosulfur trifluoride (1.90 g, 11.8 mmol) was slowly added dropwise to a solution of (2,6-dibromophenyl)methanol (2.40 g, 9.06 mmol) in anhydrous dichloromethane (60 mL) at -78°C. After the dropwise addition was completed, the mixture was continued stirring at -78°C for 30 minutes. The cooling bath was removed, and the temperature was naturally raised to room temperature, and the mixture was continued stirring at room temperature for 1 hour. The reaction mixture was quenched by slowly adding saturated sodium bicarbonate aqueous solution (50 mL) to the reaction mixture. The organic phase was separated, and the aqueous phase was extracted with dichloromethane (30 mL×2). The organic phase was combined, washed with water (30 mL) and saturated brine (30 mL) in turn, concentrated under reduced pressure, and the residue was purified by silica gel column chromatography (eluent: petroleum ether) to give compound **73-c** (1.86 g, yield rate: 78%).

### Synthesis of compound 73-b

Bis(tri-*tert*-butylphosphine)palladium (255 mg, 0.50 mmol) was added to a mixture of compound **6-b** (1.40 g, 5.0 mmol), pinacol vinylboronate (1.15 g, 7.5 mmol), triethylamine (1.51 g, 15.0 mmol) and toluene (20 mL). The reaction mixture was stirred at 80°C for 16 h under nitrogen. The reaction solution was cooled to room temperature, concentrated under reduced pressure, and the residue was purified by silica gel column chromatography (petroleum ether: ethyl acetate = 6:1) to give compound **73-b** (1.08 g, yield: 61%).

LC-MS (ESI): m/z = 357 [M+H]⁺.

### Synthesis of compound 73-a

Phenylboronic acid (134 mg, 1.1 mmol), [1,1'-bis(diphenylphosphino)ferrocene]palladium dichloride (146 mg, 0.2 mmol) potassium phosphate (848 mg, 4.0 mmol) and cesium fluoride (600 mg, 4.0 mmol) were added to a mixture of compound **73-b** (391 mg, 1.1 mmol), compound **73-c** (268 mg, 1.0 mmol) and toluene (15 mL). The reaction mixture was stirred at 100°C for 16 h under nitrogen. The reaction solution was cooled to room temperature, filtered, and the filtrate was concentrated under reduced pressure, and the residue was purified by silica gel column chromatography (petroleum ether: ethyl acetate = 8:1) to give compound **73-a** (150 mg, yield: 36%).

LC-MS (ESI): m/z = 415 [M+H]⁺.

### Synthesis of compound 73

(S)-Piperidine-2-carboxylic acid (39 mg, 0.3 mmol) and methanol (10 mL) were added to a solution of compound **73-a** (41 mg, 0.10 mmol) in dichloromethane (10 mL). The mixture was stirred for 1 h at room temperature, then sodium cyanoborohydride (16 mg, 0.25 mmol) was added, and stirred for 16 h at room temperature after the addition. The reaction mixture was concentrated under reduced pressure, and the residue was purified by preparative liquid chromatography to give compound **73** (22 mg, yield: 42%).

LC-MS (ESI): m/z = 528 [M+H]⁺.
¹H NMR (400 MHz, J7.92 (s, 1H), 7.70-7.73 (m, 2H), 7.50-7.55 (m, 2H), 7.41-7.48 (m, 4H), 7.38-7.40 (m, 2H), 7.33-7.35 (m, 1H), 5.50 (s, 1H), 5.38 (s, 1H), 4.53 (d, *J*=12.8Hz, 1H), 4.39 (d, *J*=12.8Hz, 1H), 3.49-3.51 (m, 1H), 2.95-3.01 (m, 1H), 2.30 (s, 3H), 2.22-2.26 (m, 1H), 1.70-1.89 (m, 5H), 1.54-1.58 (m, 1H) ppm

### Embodiment 74

### (E)-2-((4-(2-(2-Fluoromethyl-[1,1'-biphenyl]-3-yl)vinyl)-2-methoxy-5-trifluoromethylbenzyl)amino)-1-ethanol (compound 74)

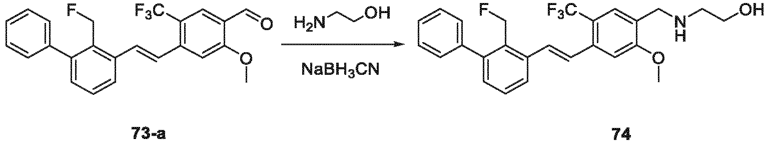

### Synthesis of compound 74

Ethanolamine (31 mg, 0.5 mmol) and methanol (10 mL) were added to a solution of **73-a** (41 mg, 0.10 mmol) in dichloromethane (10 mL). The mixture was stirred for 1 h at room temperature, then sodium cyanoborohydride (20 mg, 0.31 mmol) was added, and stirred for 16 h at room temperature after the addition. The reaction mixture was concentrated under reduced pressure, and the residue was purified by preparative liquid chromatography to give compound **74** (23 mg, yield: 50%).

LC-MS (ESI): m/z = 460 [M+H]⁺.

¹H NMR (400 MHz, *δ* 7.69 (d, *J*=7.6Hz, 1H), 7.64 (s, 1H), 7.62-7.66 (m, 1H), 7.51-7.53 (m, 1H), 7.42-7.46 (m, 4H), 7.38-7.41 (m, 3H), 7.31 (d, *J*=8.0Hz, 1H), 5.48 (s, 1H), 5.36 (s, 1H), 4.01 (s, 3H), 3.84 (s, 2H), 3.68 (t, *J*=4.2Hz, 2H), 2.72 (t, *J*=4.2Hz, 2H) ppm.

### Embodiment 75

### (S,E)-1-(2-((3-Iodobenzyloxy)-5-methyl-4-(2-(2-methyl-[1,1'-biphenyl]-3-yl)vinyl)benzyl)piperidine-2-carboxylic acid (compound 75)

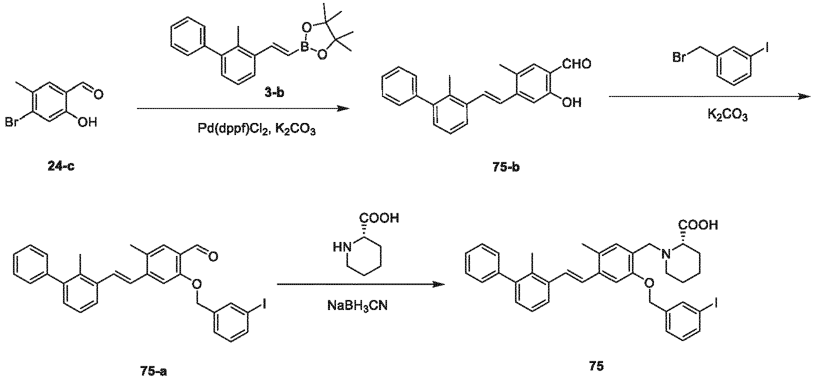

### Synthesis of compound 75-b

Compounds **24-c** (280 mg, 1.30 mmol) and **3-b** (500 mg, 1.56 mmol) were dissolved in 1,4-dioxane (5 mL) and water (1 mL), and then [1,1'-bis(diphenylphosphino)ferrocene]palladium dichloride (48 mg, 0.065 mmol) and potassium carbonate (359 mg, 2.6 mmol) were added. The atmosphere of reaction solution was replaced with nitrogen three times to remove the oxygen in the system. Then, the reaction solution was heated, and stirred at 90°C for 16 hours. The reaction solution was cooled to room temperature, and water (20 mL) was added. The mixture was extracted with dichloromethane (40 mL×2). The obtained organic phase was washed with saturated brine (20 mL×2), dried with anhydrous sodium sulfate, filtered, and the filtrate was concentrated under reduced pressure. The residue was slurried and washed with ethyl acetate, and filtered to give brown solid **75-b** (189 mg, yield: 44%). LC-MS (ESI): m/z = 329.27 (M+H)⁺.

### Synthesis of compound 75-a

Compound **75-b** (33 mg, 0.10 mmol), 3-iodobenzyl bromide (36 mg, 0.12 mmol), and cesium carbonate (65 mg, 0.20 mmol) were dissolved in 1,4-dioxane (0.5 mL), and the reaction solution was heated and stirred at 50°C for 16 h. The reaction solution was cooled to room temperature, and directly separated by reversed-phase chromatography (mobile phase: 0.1% formic acid aqueous solution: acetonitrile = 0%-100%) to give light yellow solid **75-a** (33 mg, yield: 61%). LC-MS (ESI): m/z = 545.09 (M+H)⁺.

### Synthesis of compound 75

Compound **75-a** (33 mg, 0.061 mmol) and (S)-piperidine-2-carboxylic acid (16 mg, 0.12 mmol) were dissolved in methanol (1 mL) and tetrahydrofuran (1 mL), and sodium cyanoborohydride (15 mg, 0.24 mmol) was added. The reaction solution was heated and stirred at 60°C for 1 hour. The reaction solution was cooled to room temperature and prepared by high performance liquid chromatography (mobile phase: 1% formic acid aqueous solution: acetonitrile = 20% - 70%) to give white solid **75** (12 mg, yield: 30%). LC-MS (ESI): m/z = 658.36 (M+H)⁺.
¹H-NMR (400 MHz, MeOD) δ: 7.90 (s, 1H), 7.69 (d, *J* = 7.9Hz, 1H), 7.59 (d, *J =* 7.7Hz, 1H), 7.54 (d, *J* = 7.5Hz, 1H), 7.46-7.39 (m, 2H), 7.36-7.26 (m, 6H), 7.25-7.12 (m, 4H), 5.25 (s, 2H), 4.47 (d, *J* = 12.6Hz, 1H), 4.36 (d, *J* = 12.8Hz, 1H), 3.71-3.53 (m, 1H), 3.45-3.34 (m, 1H), 3.05-2.90 (m, 1H), 2.39 (s, 3H), 2.29-2.19 (m, 4H), 1.98-1.63 (m, 4H), 1.6 -1.47 (m, 1H) ppm

### Embodiment 76

### (S,E)-1-(2-((2-Iodobenzyloxy)-4-(2-(2-methyl-[1,1'-biphenyl]-3-yl)vinyl)-5-(trifluoromethyl)benzyl)piperidine-2-carboxylic acid (compound 76)

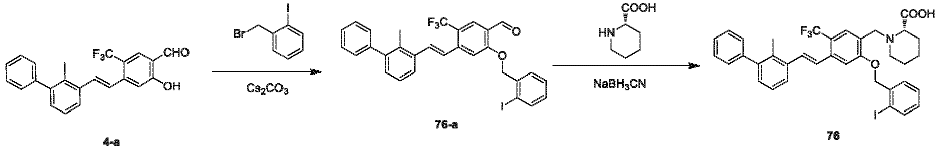

### Synthesis of compound 76-a

Compound **4-a** (38 mg, 0.10 mmol), 2-iodobenzyl bromide (36 mg, 0.12 mmol) were dissolved in *N,N*-dimethylformamide (0.5 mL), then cesium carbonate (65 mg, 0.20 mmol) was added, and the reaction solution was heated and stirred at 50°C for 1 hour. The reaction solution was cooled to room temperature, and the reaction solution was directly separated by reversed-phase chromatography (mobile phase: 0.1% formic acid aqueous solution: acetonitrile = 0%-100%) to give light yellow solid **76-a** (40 mg, yield: 67%).

### Synthesis of compound 76

Compound **76-a** (40 mg, 0.067 mmol) and (S)-piperidine-2-carboxylic acid (17 mg, 0.13 mmol) were dissolved in methanol (1 mL) and tetrahydrofuran (1 mL), and sodium cyanoborohydride (17 mg, 0.27 mmol) was added. The reaction solution was heated and stirred at 60°C for 1 hour. The reaction solution was cooled to room temperature and directly prepared by high performance liquid chromatography (mobile phase: 0.1% formic acid aqueous solution: acetonitrile = 20% - 70%) to give white solid **76** (13 mg, yield: 27%). LC-MS (ESI): m/z = 712.4 (M+H)⁺.
¹H-NMR (400 MHz, MeOD) δ: 8.00-7.91 (m, 2H), 7.61-7.49 (m, 4H), 7.48-7.39 (m, 3H), 7.39-7.23 (m, 5H), 7.18 (d, *J* = 6.8Hz, 1H), 7.12 (td, *J* = 7.7, 1.6Hz, 1H), 5.50-5.37 (m, 2H), 4.56 (d, *J* = 12.9Hz, 1H), 4.41 (d, *J* = 13.0Hz, 1H), 3.61 (d, *J* = 8.7Hz, 1H), 3.47-3.36 (m, 1H), 2.98 (t, *J* = 10.6Hz, 1H), 2.28 (s, 3H), 2.26-2.16 (m, 1H), 1.90-1.48 (m, 5H) ppm

### Embodiment 77

### (S,E)-1-(2-((3-Iodobenzyloxy)-4-(2-(2-methyl-[1,1'-biphenyl]-3-yl)vinyl)-5-(trifluoromethyl)benzyl)piperidine-2-carboxylic acid (compound 77)

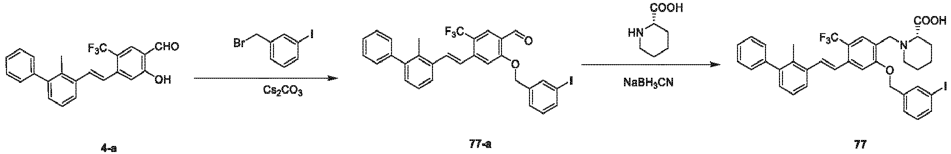

### Synthesis of compound 77-a

Compound **4-a** (38 mg, 0.10 mmol), 3-iodobenzyl bromide (36 mg, 0.12 mmol) were dissolved in *N,N-*dimethylformamide (0.5 mL), then cesium carbonate (65 mg, 0.20 mmol) was added, and the reaction solution was heated and stirred at 50°C for 1 hour. The reaction solution was cooled to room temperature, and the reaction solution was directly separated by reversed-phase chromatography (mobile phase: 0.1% formic acid aqueous solution: acetonitrile = 0-100%) to give light yellow solid **77-a** (50 mg, yield: 84%).

### Synthesis of compound 77

Compound **77-a** (40 mg, 0.067 mmol) and (S)-piperidine-2-carboxylic acid (17 mg, 0.13 mmol) were dissolved in methanol (1 mL) and tetrahydrofuran (1 mL), and sodium cyanoborohydride (17 mg, 0.27 mmol) was added. The reaction solution was heated and stirred at 60°C for 1 hour. The reaction solution was cooled to room temperature, and directly prepared by high performance liquid chromatography (mobile phase: 0.1% formic acid aqueous solution: acetonitrile = 20% - 70%) to give white solid **377** (18 mg, yield: 38%). LC-MS (ESI): m/z = 712.4 (M+H)⁺.
¹H-NMR (400 MHz, MeOD) δ: 7.94 (s, 2H), 7.73 (d, *J* = 7.9Hz, 1H), 7.60-7.33 (m, 7H), 7.33-7.24 (m, 4H), 7.23-7.15 (m, 2H), 5.38 (s, 2H), 4.55 (d, *J* = 13.0Hz, 1H), 4.42 (d, *J* = 13.0Hz, 1H), 3.64 (d, *J* = 8.0Hz, 1H), 3.45-3.34 (m, 1H), 3.01 (t, *J* = 10.4Hz, 1H), 2.29 (s, 3H), 2.28-2.22 (m, 1H), 1.95-1.49 (m, 5H) ppm

### Embodiment 78

### (S,E)-1-(2-((4-Iodobenzyloxy)-4-(2-(2-methyl-[1,1'-biphenyl]-3-yl)vinyl)-5-(trifluoromethyl)benzyl)piperidine-2-carboxylic acid (compound 78)

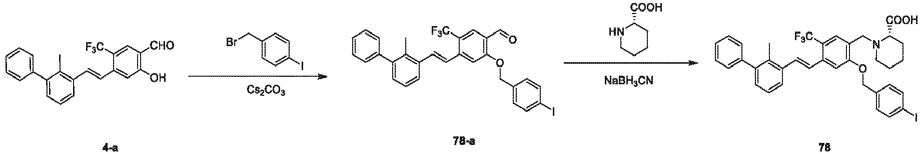

### Synthesis of compound 78-a

Compound **4-a** (38 mg, 0.10 mmol), 4-iodobenzyl bromide (36 mg, 0.12 mmol) were dissolved in *N,N*-dimethylformamide (0.5 mL), then cesium carbonate (65 mg, 0.20 mmol) was added, and the reaction solution was heated and stirred at 50°C for 1 hour. The reaction solution was cooled to room temperature, and the reaction solution was directly separated by reversed-phase chromatography (mobile phase: 0.1% formic acid aqueous solution: acetonitrile = 0-100%) to give light yellow solid **78-a** (50 mg, yield: 84%).

### Synthesis of compound 78

Sodium cyanoborohydride (17 mg, 0.27 mmol) was added to a mixture of compound **78-a** (40 mg, 0.067 mmol), (*S*)-piperidine-2-carboxylic acid (17 mg, 0.13 mmol), methanol (1 mL) and tetrahydrofuran (1 mL). The reaction solution was stirred at 60°C for 1 hour. The reaction solution was cooled to room temperature, and the obtained mixture was directly prepared by high performance liquid chromatography (mobile phase: 0.1% formic acid aqueous solution: acetonitrile = 20% - 70%) to give white solid **78** (20 mg, yield: 42%). LC-MS (ESI): m/z = 712.4 (M+H)⁺.
¹H-NMR (400 MHz, MeOD) δ: 7.93 (s, 1H), 7.82-7.75 (m, 2H), 7.55 (s, 1H), 7.53 (d, *J* = 7.7Hz, 1H), 7.49-7.39 (m, 3H), 7.39-7.23 (m, 7H), 7.18 (d, *J* = 7.5Hz, 1H), 5.38 (s, 2H), 4.55 (d, *J* = 13.0Hz, 1H), 4.42 (d, *J* = 13.0Hz, 1H), 3.66 (d, *J* = 8.3Hz, 1H), 3.45-3.34 (m, 1H), 3.07-2.92 (m, 1H), 2.26 (s, 3H), 2.26-2.20 (m, 1H), 1.98-1.47 (m, 5H) ppm

### Embodiment 79

### (S,E)-1-(2-((4-Iodo-pyridin-2-yl)methoxy)-5-methyl-4-(2-(2-methyl-[1,1'-biphenyl]-3-yl)vinyl)benzyl)piperidine-2-carboxylic acid (compound 79)

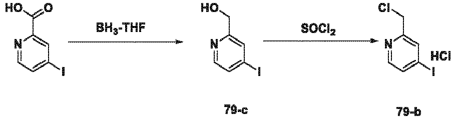

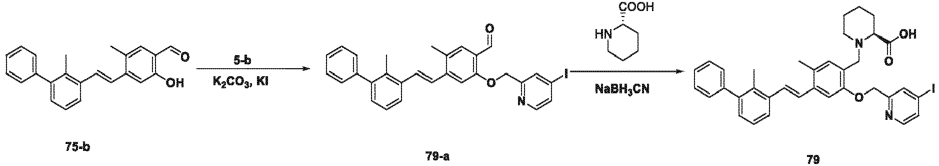

### Synthesis of compound 79-c

4-Iodopicolinic acid (500 mg, 2 mmol) was dissolved in anhydrous tetrahydrofuran (10 mL), and borane tetrahydrofuran solution (4 mL, 4 mmol) was added. The reaction solution was stirred at room temperature for 16 hours. The reaction solution was cooled to 0°C, then methanol (10 mL) was added dropwise to the reaction solution. Then the reaction solution was heated and stirred at 70°C for 1 hour, and cooled to room temperature, concentrated under reduced pressure. The residue was purified by silica gel column chromatography (petroleum ether: ethyl acetate = 1:1) to give **79-c** (300 mg, yield 63.6 %). LC-MS (ESI): m/z = 236 (M+H)⁺.

### Synthesis of compound 79-b

Compound **79-c** (300 mg, 1.2 mmol) was dissolved in dichloromethane (10 mL), and thionyl chloride (400 mg, 3.6 mmol) was slowly added. The reaction solution was stirred at room temperature for 2 hours. After concentration under reduced pressure, **79-b** (355 mg, yield: 96.2 %) was obtained, which was directly used in the next reaction step without purification. LC-MS (ESI): m/z = 254 (M+H)⁺.

### Synthesis of compound 79-a

Compound **79-b** (57 mg, 0.2 mmol) and compound **75-b** (50 mg, 0.13 mmol) were dissolved in *N,N*-dimethylformamide (5 mL), then potassium carbonate (54 mg, 0.39 mmol) and potassium iodide (214 mg, 1.3 mmol) were added, and the reaction solution was heated and stirred at 50°C for 30 min. The reaction solution was cooled to room temperature, and **79-a** (30 mg, yield: 38.4%) was obtained directly by high performance liquid chromatography. LC-MS (ESI): m/z = 546 (M+H)⁺.

### Synthesis of compound 79

Compound **79-a** (20 mg, 0.037 mmol) and *s*-piperidine-2-carboxylic acid (7 mg, 0.054 mmol) were dissolved in methanol (3 mL), and sodium cyanoborohydride (5 mg, 0.075 mmol) was added. The atmosphere of reaction solution was replaced with nitrogen three times to remove the oxygen in the system. Then, the reaction solution was heated at 60°C for 30 minutes. The reaction solution was cooled to room temperature. The reaction solution was concentrated under reduced pressure, and the residue was prepared by high performance liquid chromatography to give compound **79** (6 mg, yield: 29.7%). LC-MS (ESI): m/z = 659 (M+H)⁺.
¹H-NMR (400 MHz, MeOD) δ: 8.33(d, *J*=5.2Hz,1H), 7.99( s,1H), 7.59-7.27(m, 2H), 7.43-7.31(m, 5H),7.28-7.21 (m, 5H), 7.12(d, *J*=7.6Hz,1H), 5.28(s, 3H), 4.45-4.42 (m, 1H), 4.27-4.24(m, 1H),3.42-3.41(m, 1H), 2.90-2.78(m, 1H), 2.28-2.25(m, 1H), 2.39 (s, 3H), 2.24(s, 3H),2.20-2.16(m,1H), 1.92-1.68(m,4H), 1.52-1.50(m,1H), 1.32- 1.28(m, 1H) ppm

### Embodiment 80

### (S,E)-1-(2-((4-Iodopyridin-2-yl)methoxy)-4-(2-(2-methyl-[1,1'-biphenyl]-3-yl)vinyl)-5-(trifluoromethyl)benzyl)piperidine-2-carboxylic acid (compound 80)

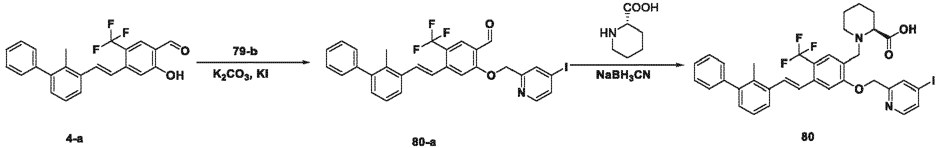

### Synthesis of compound 80-a

Compound **79-b** (57 mg, 0.2 mmol) and compound **4-a** (50 mg, 0.13 mmol) were dissolved in *N,N*-dimethylformamide (5 mL), then potassium carbonate (54 mg, 0.39 mmol) and potassium iodide (214 mg, 1.3 mmol) were added, and the reaction solution was heated and stirred at 50°C for 30 min. The reaction solution was cooled to room temperature, and **80-a** (30 mg, yield: 38.4%) was obtained directly by high performance liquid chromatography. LC-MS (ESI): m/z = 600 (M+H)⁺.

### Synthesis of compound 80

Compound **80-a** (20 mg, 0.037 mmol) and *s*-piperidine-2-carboxylic acid (7 mg, 0.054 mmol) were dissolved in methanol (3 mL), and sodium cyanoborohydride (5 mg, 0.075 mmol) was added. The atmosphere of reaction solution was replaced with nitrogen three times to remove the oxygen in the system. Then, the reaction solution was heated at 60°C for 30 minutes. The reaction solution was cooled to room temperature. The reaction solution was concentrated under reduced pressure, and the residue was prepared by high performance liquid chromatography to give compound **80** (8 mg, yield: 30.7%). LC-MS (ESI): m/z = 713 (M+H)⁺.
¹H-NMR (400 MHz, MeOD) δ: 8.27( d, *J*=5.6Hz, 1H), 8.09( s,1H), 7.91( s, 1H), 7.81( d, *J*=6.8Hz, 1H),7.55-7.41( m, 5H), 7.37-7.25( m, 5H), 7.28(d, *J*=7.2Hz, 1H), 5.48(s, 2H), 4.72-4.64 ( m, 1H), 4.56 ( s,2H),4.40-4.37(m, 1H), 3.61-3.57(m, 1H), 3.04-3.02(m, 1H), 2.28 (s, 3H), 1.88- 1.76(m,4H), 1.59- 1.57(m,1H) ppm

### Embodiment 81

### (S,E)-1-(2-((2-Iodopyridin-4-yl)methoxy)-4-(2-(2-methyl-[1,1'-biphenyl]-3-yl)vinyl)-5-(trifluoromethyl)benzyl)piperidine-2-carboxylic acid (compound 81)

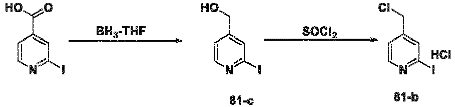

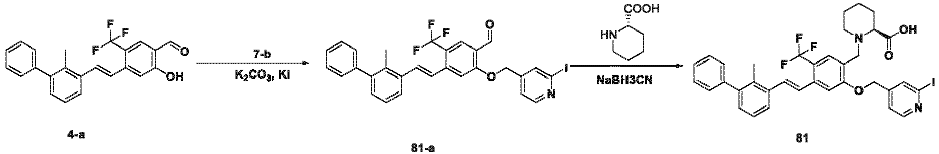

### Synthesis of compound 81-c

2-Iodoisonicotinic acid (500 mg, 2 mmol) was dissolved in anhydrous tetrahydrofuran (10 mL), and borane tetrahydrofuran solution (4 mL, 4 mmol) was added dropwise. The reaction solution was stirred at room temperature for 16 hours. The reaction solution was cooled to 0°C, and methanol (10 mL) was added dropwise to the reaction solution, then the reaction solution was heated and stirred at 70°C for 1 hour. The reaction solution was cooled to room temperature, concentrated under reduced pressure, and the residue was purified by silica gel column chromatography (petroleum ether: ethyl acetate = 1: 1) to give **81-c** (280 mg, yield: 53.9%). LC-MS (ESI): m/z = 236 (M+H)⁺.

### Synthesis of compound 81-b

Compound **81-c** (280 mg, 1.1 mmol) was dissolved in dichloromethane (10 mL), and thionyl chloride (400 mg, 3.6 mmol) was slowly added. The reaction solution was stirred at room temperature for 2 hours. After concentration under reduced pressure, **81-b** (342 mg, yield: 97.0%) was obtained, which was directly used in the next reaction step without purification. LC-MS (ESI): m/z = 254 (M+H)⁺.

### Synthesis of compound 81-a

Compound **81-b** (57 mg, 0.2 mmol) and compound **4-a** (50 mg, 0.13 mmol) were dissolved in *N,N*-dimethylformamide (5 mL), then potassium carbonate (54 mg, 0.39 mmol) and potassium iodide (214 mg, 1.3 mmol) were added, and the reaction solution was heated and stirred at 50°C for 30 min. The reaction solution was cooled to room temperature, and **81-a** (30 mg, yield: 38.4%) was obtained directly by high performance liquid chromatography. LC-MS (ESI): m/z = 600 (M+H)⁺.

### Synthesis of compound 81

Compound **81-a** (20 mg, 0.037 mmol) and s-piperidine-2-carboxylic acid (7 mg, 0.054 mmol) were dissolved in methanol (3 mL), and sodium cyanoborohydride (5 mg, 0.075 mmol) was added. The atmosphere of reaction solution was replaced with nitrogen three times to remove the oxygen in the system, and the reaction solution was heated at 60°C for 30 minutes. The reaction solution was cooled to room temperature. The reaction solution was concentrated under reduced pressure, and the residue was prepared by high performance liquid chromatography to give compound **81** (9 mg, yield: 34.6%). LC-MS (ESI): m/z = 713 (M+H)⁺.
¹H-NMR (400 MHz, MeOD) δ: 8.37 (d, *J*=4.8Hz,1H), 8.03 (s,1H), 7.98 (s,1H), 7.61(d, *J*=4.8Hz,1H),7.55-7.41 (m, 5H), 7.37-7.25 (m, 5H), 7.18 (d, *J*=7.2Hz, 1H), 5.43 (s, 2H), 4.62-4.56 (m, 3H ), 4.45-4.42(m, 1H), 3.61-3.54(m, 1H), 3.01-2.97(m, 1H), 2.28 (s, 3H), 1.89-1.71(m,4H), 1.65- 1.55(m,1H) ppm

### Embodiment 82

### (S,E)-1-(2-((5-Iodopyridin-3-yl)methoxy)-4-(2-(2-methyl-[1,1'-biphenyl]-3-yl)vinyl)-5-(trifluoromethyl)benzyl)piperidine-2-carboxylic acid (compound 82)

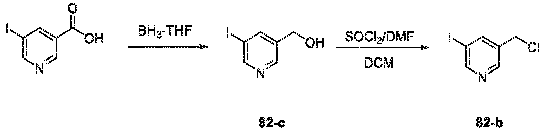

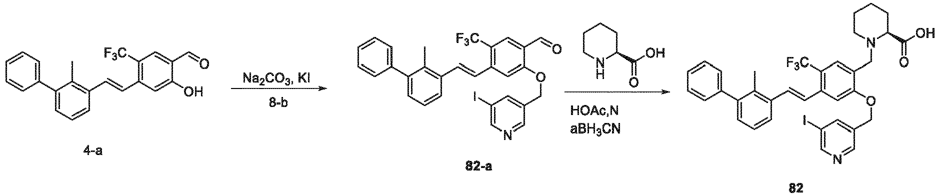

### Synthesis of compound 82-c

5-Iodonicotinic acid (250 mg, 1 mmol) was dissolved in anhydrous tetrahydrofuran (10 mL), and borane tetrahydrofuran solution (3 mL, 3 mmol) was added dropwise, and the reaction solution was stirred at room temperature for 16 hours. The reaction solution was cooled to 0°C, and methanol (10 mL) was added dropwise to the reaction solution, then the reaction solution was heated and stirred at 70°C for 1 hour. The reaction solution was cooled to room temperature, concentrated under reduced pressure, and diluted with ethyl acetate (10 mL). The organic phase was washed with water (10 mL×3), and saturated brine (10 mL×1) in turn. The obtained organic phase was dried with anhydrous sodium sulfate, filtered, and concentrated under reduced pressure to give crude compound **82-c** (141 mg, yield: 59.7%), which was directly used in the next reaction step without purification. LC-MS (ESI): m/z = 236.0 [M+H]⁺.

### Synthesis of compound 82-b

Compound **82-c** (141 mg, 0.6 mmol) was dissolved in dichloromethane (5 mL), and thionyl chloride (214 mg, 1.8 mmol) was slowly added. The reaction solution was stirred at room temperature for 2 hours. After concentration under reduced pressure, **82-b** (146 mg, yield: 96.2%) was obtained, which was directly used in the next reaction step without purification.

### Synthesis of compound 82-a

Compound **82-b** (43 mg, 0.170 mmol) and compound **4-a** (50 mg, 0.131 mmol) were dissolved in a *N,N*-dimethylformamide solution (2 mL), then sodium carbonate (41.7 mg, 0.393 mmol) and potassium iodide (65.2 mg, 0.393 mmol) were added. The reaction solution was stirred at 80°C for 16 hours. The reaction solution was cooled to room temperature, diluted with ethyl acetate (10 mL), and the organic phase was washed with water (10 mL×3) and saturated brine (10 mL×1) in turn. The obtained organic phase was dried with anhydrous sodium sulfate, filtered, concentrated under reduced pressure, and the residue was purified by silica gel column chromatography (petroleum ether=100%) to give **82-a** (48.8 mg, yield: 62.2%). LC-MS (ESI): m/z = 600.0 [M+H]⁺.

### Synthesis of compound 82

Compound **82-a** (48.8 mg, 0.082 mmol) and s-piperidine-2-carboxylic acid (21.2 mg, 0.164 mmol) were dissolved in a mixture of methanol and tetrahydrofuran (10 mL, 1:1 in *ν:ν*), then glacial acetic acid (9.8 mg, 0.164 mmol) and sodium cyanoborohydride (25.8 mg, 0.41 mmol) were added. The atmosphere of reaction solution was replaced with nitrogen three times to remove the oxygen in the system, and the reaction solution was heated at 60°C for 30 minutes. The reaction solution was cooled to room temperature. The reaction solution was concentrated under reduced pressure, and the residue was prepared by high performance liquid chromatography to give compound **82** (16.4 mg, yield: 28.2%). LC-MS (ESI): m/z = 713.0 [M+H]⁺;
¹H-NMR (400 MHz, CD₃OD) δ: 8.81 (s, 1H), 8.73 (s, 1H), 8.44 (s, 1H), 7.97 (s, 1H), 7.62-7.53 (m, 3H), 7.45-7.41 (m, 2H), 7.37-7.26 (m, 5H), 7.20 (m, 1H), 5.43 (s, 2H), 4.56-4.53 (d, *J* = 12.8Hz, 1H), 4.42-4.39 (d, *J* = 12.8Hz, 1H), 3.60 (m, 1H), 3.01-2.95 (m, 1H), 2.31 (s, 3H), 2.27-2.19 (m, 1H), 1.87-1.28 (m, 6H) ppm

### Embodiment 83

### (S,E)-1-(2-(4-Fluorobutoxy)-5-methyl-4-(2-(2-methyl-[1,1'-biphenyl]-3-yl)vinyl)benzyl)piperidine-2-carboxylic acid (compound 83)

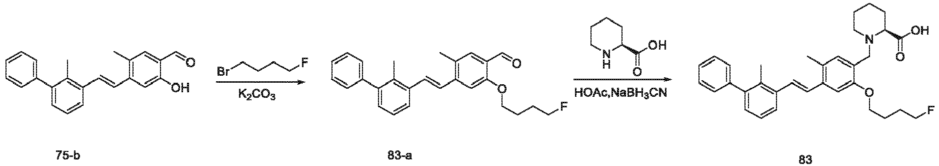

### Synthesis of compound 83-a

Compound **75-b** (200 mg, 0.609 mmol) and 1-bromo-4-fluorobutane (141.7 mg, 0.914 mmol) were dissolved in *N,N'*-dimethylformamide (5 mL), and potassium carbonate (252.5 mg, 1.827 mmol) was added. The reaction solution was stirred at 60°C for 16 hours. The reaction solution was cooled to room temperature, diluted with ethyl acetate (3 mL), and washed with water (3 mL×3) and saturated brine (3 mL) in turn. The obtained organic phase was dried with anhydrous sodium sulfate, filtered, concentrated under reduced pressure, and the residue was purified by silica gel column chromatography (petroleum ether: ethyl acetate = 5:1) to give **83-a** (180 mg, yield: 73.5%). LC-MS (ESI): m/z = 403.0 [M+H]⁺.

### Synthesis of compound 83

Compound **83-a** (180 mg, 0.447 mmol) and *s*-piperidine-2-carboxylic acid (115.5 mg, 0.894 mmol) were dissolved in a mixture of methanol (5 mL) and tetrahydrofuran (5 mL), then glacial acetic acid (53.7 mg, 0.894 mmol) was added. The reaction solution was heated and stirred at 60°C for 3 h, then, sodium cyanoborohydride (140.5 mg, 2.235 mmol) was added, and the reaction solution was stirred for 0.5 h. The reaction solution was cooled to room temperature, concentrated under reduced pressure and the residue was prepared by high performance liquid chromatography to give compound **83** (61.9 mg, yield: 26.9%). LC-MS (ESI): m/z = 516.0 [M+H]⁺;
¹H-NMR (400 MHz, CD₃OD) δ: 7.61-7.59 (d, *J =* 7.6Hz, 1H), 7.46-7.40 (m, 3H), 7.36-7.21 (m, 7H), 7.15-7.13 (d, *J* = 7.2Hz, 1H), 4.61-4.58 (t, *J* = 5.6Hz, 1H), 4.49-4.46 (t, *J* = 5.6Hz, 2H), 4.34-4.31 (d, *J* = 12.4Hz, 1H), 4.22-4.19 (t, *J* = 5.6Hz, 2H), 3.56-3.54 (m, 1H), 3.37-3.35 (m, 1H), 3.01-2.96 (m, 1H), 2.40 (s, 3H), 2.28 (s, 3H), 2.25-2.19 (m, 1H), 2.04-1.55 (m, 9H) ppm.

### Embodiment 84

### (S,E)-1-(2-(3-Fluoropropoxy)-5-methyl-4-(2-(2-methyl-[1,1'-biphenyl]-3-yl)vinyl)benzyl)piperidine-2-carboxylic acid (compound 84)

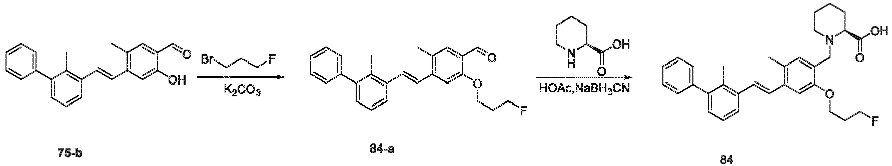

### Synthesis of compound 84-a

Compound **75-b** (200 mg, 0.609 mmol) and 1-bromo-3-fluorobutane (128.9 mg, 0.914 mmol) were dissolved in *N,N'*-dimethylformamide (5 mL), and potassium carbonate (252.5 mg, 1.827 mmol) was added. The reaction solution was heated and stirred at 60°C for 16 hours. The reaction solution was cooled to room temperature, diluted with ethyl acetate (3 mL), and washed with water (3 mL×3) and saturated brine (3 mL) in turn. The obtained organic phase was dried with anhydrous sodium sulfate, filtered, concentrated under reduced pressure, and the residue was purified by silica gel column chromatography (petroleum ether: ethyl acetate = 5:1) to give **84-a** (200 mg, yield: 84.7%). LC-MS (ESI): m/z = 389.0 [M+H]⁺;

### Synthesis of compound 84

Compound **84-a** (200 mg, 0.515 mmol) and s-piperidine-2-carboxylic acid (133 mg, 1.03 mmol) were dissolved in a mixture of methanol (5 mL) and tetrahydrofuran (5 mL), then glacial acetic acid (61.9 mg, 1.03 mmol) was added. The reaction solution was heated and stirred at 60°C for 3 h, then, sodium cyanoborohydride (161.8 mg, 2.575 mmol) was added, and the reaction solution was stirred for 0.5 h. The reaction solution was cooled to room temperature, concentrated under reduced pressure and the residue was prepared by high performance liquid chromatography to give compound **84** (60.5 mg, yield: 23.4%). LC-MS (ESI): m/z = 502.0 [M+H]⁺;
¹H-NMR (400 MHz, CD₃OD) δ: 7.61-7.60 (d, *J* = 7.6Hz, 1H), 7.47-7.40 (m, 3H), 7.36-7.21 (m, 7H), 7.15-7.13 (d, *J* = 7.2Hz, 1H), 4.77-4.74 (t, *J* = 5.6Hz, 1H), 4.65-4.62 (t, *J* = 5.6Hz, 2H), 4.52-4.49 (d, *J* = 12.8Hz, 1H), 4.32-4.27 (m, 3H), 3.54-3.52 (m, 1H), 3.00-2.95 (m, 1H), 2.41 (s, 3H), 2.33-2.30 (m, 1H), 2.28 (s, 3H), 2.26-2.22 (m, 2H), 1.94-1.55 (m, 5H) ppm.

### Embodiment 85

### (S,E)-1-(2-(4-Fluorobutoxy)-4-(2-(2-methyl-[1,1'-biphenyl]-3-yl)vinyl)-5-(trifluoromethyl)benzyl)piperidine-2-carboxylic acid (compound 85)

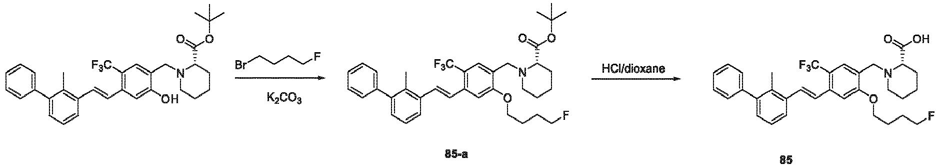

### Synthesis of compound 85-a

*tert*-Butyl (*S*,*E*)-1-(2-hydroxy-4-(2-(2-methyl-[1,1'-biphenyl]-3-yl)vinyl)-5-(trifluoromethyl)benzyl)piperidine-2-carboxylate (50 mg, 0.091 mmol) and 1-bromo-4-fluorobutane (21 mg, 0.136 mmol) were dissolved in *N,N-*dimethylformamide (3 mL), then potassium carbonate (37.3 mg, 0.273 mmol) was added, and the reaction solution was stirred at 60°C for 16 hours. The reaction solution was cooled to room temperature, diluted with ethyl acetate (3 mL), and washed with water (3 mL×3) and saturated brine (3 mL) in turn. The obtained organic phase was dried with anhydrous sodium sulfate, filtered, concentrated under reduced pressure, and the residue was purified by silica gel column chromatography (petroleum ether: ethyl acetate = 5:1) to give **85-a** (37 mg, yield: 65.1%). LC-MS (ESI): m/z = 626.0 [M+H]⁺.

### Synthesis of compound 85

Compound **85-a** (37 mg, 0.059 mmol) was dissolved in a solution of hydrochloric acid/dioxane (4.0 M, 10 mL), and the reaction solution was stirred at room temperature for 16 h, then the reaction solution was concentrated under reduced pressure, and the residue was purified by prep-HPLC to give **85** (17.1 mg, yield: 51.5%). LC-MS (ESI): m/z = 570.0 [M+H]⁺;
¹H-NMR (400 MHz, CD₃OD) δ: 7.94 (s, 1H), 7.64-7.60 (d, *J*=16.0Hz, 1H), 7.55-7.53 (m, 2H), 7.44-7.41 (m, 2H), 7.37-7.26 (m, 5H), 7.19-7.17 (d, *J*=7.6Hz, 1H), 4.62-4.59 (t, *J*=5.6Hz, 1H), 4.57-4.54 (d, *J*=13.2Hz, 1H), 4.50-4.47 (t, *J*=5.6Hz, 1H), 4.40-4.32 (m, 3H), 3.58-3.56 (m, 1H), 3.05-2.99 (m, 1H), 2.30 (s, 3H), 2.24-2.23 (m, 1H), 2.07-1.57 (m, 9H) ppm.

### Embodiment 86

### (S,E)-1-(2-(3-Fluoropropoxy)-4-(2-(2-methyl-[1,1'-biphenyl]-3-yl)vinyl)-5-(trifluoromethyl)benzyl)piperidine-2-carboxylic acid (compound 86)

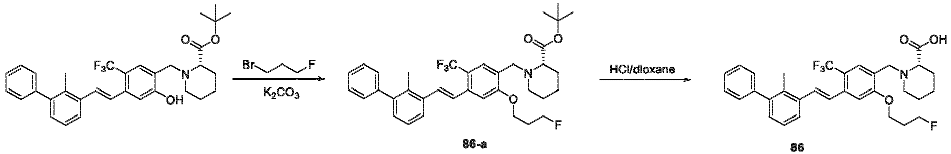

### Synthesis of compound 86-a

*tert*-Butyl (*S*,*E*)-1-(2-hydroxy-4-(2-(2-methyl-[1,1'-biphenyl]-3-yl)vinyl)-5-(trifluoromethyl)benzyl)piperidine-2-carboxylate (50 mg, 0.091 mmol) and 1-bromo-3-fluoropropane (19.2 mg, 0.136 mmol) were dissolved in *N*,*N-*dimethylformamide (3 mL), then potassium carbonate (37.3 mg, 0.273 mmol) was added, and the reaction solution was stirred at 60°C for 16 hours. The reaction solution was diluted with ethyl acetate (3 mL), and washed with water (3 mL×3) and saturated brine (3 mL) in turn. The obtained organic phase was dried with anhydrous sodium sulfate, filtered, concentrated under reduced pressure, and the residue was purified by silica gel column chromatography (petroleum ether: ethyl acetate = 5:1) to give **86-a** (47 mg, yield: 84.7%). LC-MS (ESI): m/z = 612.0 [M+H]⁺.

### Synthesis of compound 86

Compound **86-a** (47 mg, 0.077 mmol) was dissolved in hydrochloric acid/dioxane solution (4.0 M, 10 mL), and the reaction solution was stirred at room temperature for 16 hours, concentrated under reduced pressure, and the residue was purified by high performance liquid chromatography to give **86** (19.2 mg, yield: 45.2%). LC-MS (ESI): m/z = 556.0 [M+H]⁺;
¹H-NMR (400 MHz, CD₃OD) *δ*: 7.92 (s, 1H), 7.65-7.61 (d, *J*=16.4Hz, 1H), 7.56-7.53 (m, 2H), 7.44-7.41 (m, 2H), 7.37-7.26 (m, 5H), 7.19-7.17 (d, *J*=7.6Hz, 1H), 4.78-4.75 (t, *J*=5.6Hz, 1H), 4.66-4.64 (t, *J*=5.2Hz, 1H), 4.60-4.57 (d, *J*=12.8Hz, 1H), 4.44-4.35 (m, 3H), 3.56-3.52 (m, 1H), 3.04-2.99 (m, 1H), 2.36-2.34 (m, 1H), 2.30 (s, 3H), 2.28-2.24 (m, 2H), 1.89-1.56 (m, 5H) ppm.

### Embodiment 87

### (S,E)-1-(2-(2-Fluoroethoxy)-4-(2-(2-methyl-[1,1'-biphenyl]-3-yl)vinyl)-5-(trifluoromethyl)benzyl)piperidine-2-carboxylic acid (compound 87)

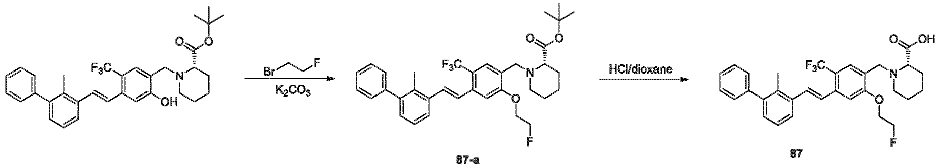

### Synthesis of compound 87-a

*tert*-Butyl (*S*,*E*)-1-(2-hydroxy-4-(2-(2-methyl-[1,1'-biphenyl]-3-yl)vinyl)-5-(trifluoromethyl)benzyl)piperidine-2-carboxylate (50 mg, 0.091 mmol) and 1-bromo-2-fluoroethane (17.3 mg, 0.136 mmol) were dissolved in *N*,*N*-dimethylformamide (3 mL), then potassium carbonate (37.3 mg, 0.273 mmol) was added, and the reaction solution was stirred at 60°C for 16 hours. The reaction solution was diluted with ethyl acetate (3 mL), and washed with water (3 mL×3) and saturated brine (3 mL) in turn. The obtained organic phase was dried with anhydrous sodium sulfate, filtered, concentrated under reduced pressure, and the residue was purified by silica gel column chromatography (petroleum ether: ethyl acetate = 5:1) to give **87-a** (27 mg, yield: 50.0%). LC-MS (ESI): m/z = 598.0 [M+H]⁺.

### Synthesis of compound 87

Compound **87-a** (47 mg, 0.077 mmol) was dissolved in hydrochloric acid/dioxane solution (4.0 M, 10 mL). The reaction solution was stirred at room temperature for 16 hours, concentrated under reduced pressure, and the residue was purified by high performance liquid chromatography to give **87** (14.7 mg, yield: 60.0%). LC-MS (ESI): m/z = 542.0 [M+H]⁺;
¹H-NMR (400 MHz, DMSO-*d*₆) *δ*: 7.79 (s, 1H), 7.66-7.62 (d, *J*=16.0Hz, 1H), 7.57-7.52 (m, 2H), 7.48-7.45 (m, 2H), 7.40-7.31 (m, 4H), 7.23-7.18 (m, 2H), 4.88-4.86 (s, 1H), 4.76-4.74 (s, 1H), 4.54-4.53 (s, 1H), 4.47-4.46 (s, 1H), 3.82-3.79 (d, *J*=14.8Hz, 1H), 3.71-3.67 (d, *J* = 14.8 Hz, 1H), 3.22-3.20 (m, 1H), 2.93-2.91 (s, 1H), 2.29 (s, 4H), 1.81-1.77 (m, 2H), 1.50-1.41 (m, 4H) ppm.

### Embodiment 88

### (S,E)-1-(2-((5-Fluoropyridin-3-yl)methoxy)-4-(2-(2-methyl-[1,1'-biphenyl]-3-yl)vinyl)-5-(trifluoromethyl)benzyl)piperidine-2-carboxylic acid (compound 88)

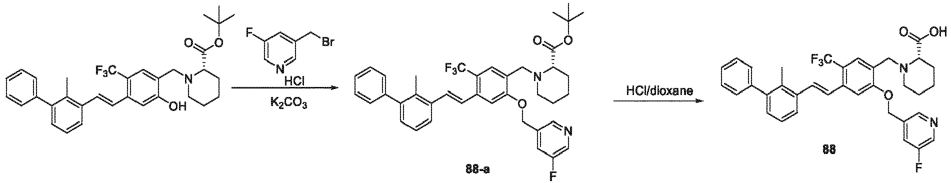

### Synthesis of compound 88-a

*tert*-Butyl (*S*,*E*)-1-(2-hydroxy-4-(2-(2-methyl-[1,1'-biphenyl]-3-yl)vinyl)-5-(trifluoromethyl)benzyl)piperidine-2-carboxylate (100 mg, 0.181 mmol) and 3-(bromomethyl)-5-fluoropyridine hydrochloride (34.44 mg, 0.181 mmol) were dissolved in *N*,*N*-dimethylformamide (3 mL), then potassium carbonate (75.16 mg, 0.544 mmol) was added, and the reaction solution was stirred at 60°C for 12 h. The reaction solution was diluted with ethyl acetate (3 mL), and washed with water (3 mL×3) and saturated brine (3 mL) in turn. The obtained organic phase was dried with anhydrous sodium sulfate, filtered, concentrated under reduced pressure, and the residue was purified by silica gel column chromatography (petroleum ether: ethyl acetate = 5:1) to give **88-a** (119 mg, yield: 99.35%). LC-MS (ESI): m/z = 661.0 [M+H]⁺.

### Synthesis of compound 88

Compound **88-a** (119 mg, 0.180 mmol) was dissolved in hydrochloric acid/dioxane solution (4.0 M, 10 mL). The reaction solution was stirred at room temperature for 12 hours, concentrated under reduced pressure, and the residue was purified by high performance liquid chromatography to give **88** (43.89 mg, yield: 40.31%). LC-MS (ESI): m/z = 605.0 [M+H]⁺.

### Embodiment 89

### (S,E)-1-(2-((4-Fluorobenzyl)oxy)-4-(2-(2-methyl-[1,1'-biphenyl]-3-yl)vinyl)-5-(trifluoromethyl))benzyl)piperidine-2-carboxylic acid (compound 89)

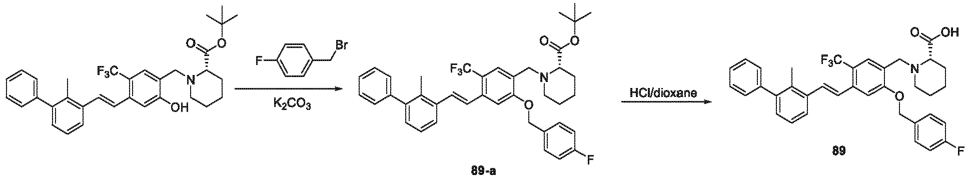

### Synthesis of compound 89-a

*tert*-Butyl (*S*,*E*)-1-(2-hydroxy-4-(2-(2-methyl-[1,1'-biphenyl]-3-yl)vinyl)-5-(trifluoromethyl)benzyl)piperidine-2-carboxylate (100 mg, 0.181 mmol) and 1-(bromomethyl)-4-fluorobenzene (34.21 mg, 0.181 mmol) were dissolved in *N,N-*dimethylformamide ( 3 mL), then potassium carbonate (75.16 mg, 0.544 mmol) was added, and the reaction solution was stirred at 60°C for 12 h. The reaction solution was diluted with ethyl acetate (3 mL), and washed with water (3 mL×3) and saturated brine (3 mL) in turn. The obtained organic phase was dried with anhydrous sodium sulfate, filtered, concentrated under reduced pressure, and the residue was purified by silica gel column chromatography (petroleum ether: ethyl acetate = 10:1) to give **89-a** (118 mg, yield: 98.81%). LC-MS (ESI): m/z = 660.0 [M+H]⁺.

### Synthesis of compound 89

Compound **89-a** (118 mg, 0.179 mmol) was dissolved in hydrochloric acid/dioxane solution (4.0 M, 10 mL). The reaction solution was stirred at room temperature for 12 hours, concentrated under reduced pressure, and the residue was purified by high performance liquid chromatography to give **89** (45.48 mg, yield: 42.12%). LC-MS (ESI): m/z = 604.0 [M+H]⁺.

### Embodiment 90

### (S,E)-1-(4-(2-(2-Methyl-[1,1'-biphenyl]-3-yl)vinyl)-2-(pyridin-3-ylmethoxy)-5-(trifluoromethyl)benzyl)piperidine-2-carboxylic acid (compound 90)

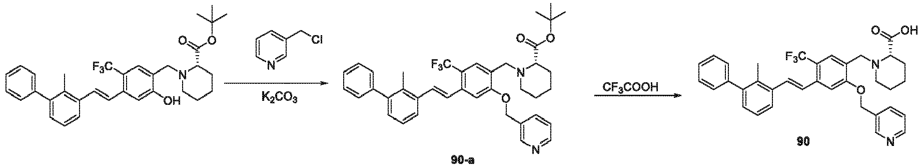

### Synthesis of compound 90-a

*tert*-Butyl (*S*,*E*)-1-(2-hydroxy-4-(2-(2-methyl-[1,1'-biphenyl]-3-yl)vinyl)-5-(trifluoromethyl)benzyl)piperidine-2-carboxylate (55.1 mg, 0.1 mmol) and 3-(chloromethyl)pyridine (25.51 mg, 0.2 mmol) were dissolved in *N*,*N*-dimethylformamide (3 mL), then potassium carbonate (69.0 mg, 0.5 mmol) was added, and the reaction solution was stirred at 50°C for 5 h. The reaction solution was diluted with ethyl acetate (3 mL), and washed with water (3 mL×3) and saturated brine (3 mL) in turn. The obtained organic phase was dried with anhydrous sodium sulfate, filtered, concentrated under reduced pressure, and the residue was purified by silica gel column chromatography (petroleum ether: ethyl acetate = 10:1) to give **90-a** (50 mg, yield: 77.01%). LC-MS (ESI): m/z = 642.2 [M+H]⁺.

### Synthesis of compound 90

Compound *tert*-butyl (*S*,*E*)-1-(4-(2-(2-methyl-[1,1'-biphenyl]-3-yl)vinyl)-2-(pyridin-3-ylmethoxy)-5-(trifluoromethyl)benzyl)piperidine-2-carboxylate (50 mg, 0.078 mmol) was dissolved in dichloromethane (10 mL), and trifluoroacetic acid (5.0 mL) was added. The reaction solution was stirred at room temperature for 12 h, concentrated under reduced pressure, and the residue was purified by high performance liquid chromatography to give **90** (45.64 mg, yield: 48.20%). LC-MS (ESI): m/z = 587.3 [M+H]⁺.
¹H-NMR(CD₃OD-d*₄*) *δ*: 8.77(s, 1H), 8.59(s, 1H), 8.11(d, *J*=8.0 Hz,1H), 8.00(s, 1H), 7.66(s, 1H), 7.61-7.55(m, 3H), 7.61-7.55(m, 3H), 7.47-7.44(m, 2H), 7.40-7.29(m, 5H), 7.21(d, *J*=7.2 Hz,1H),5.52(s, 2H),4.58(d, *J*=12.8 Hz, 1H),4.42(d, *J*=12.8 Hz,1H), 3.58-3.55(m, 1H),3.43-3.38(m, 3H),3.01-2.95(m, 1H),2.32(s, 3H),2.30-2.24(m, 1H), 1.81-1.54(m, 5H) ppm.

### Embodiment 91

### (S,E)-1-(2-((5-Cyanopyridin-3-yl)methoxy)-4-(2-(2-methyl-[1,1'-biphenyl]-3 - yl)vinyl)-5-(trifluoromethyl)benzyl)piperidine-2-carboxylic acid (compound 91)

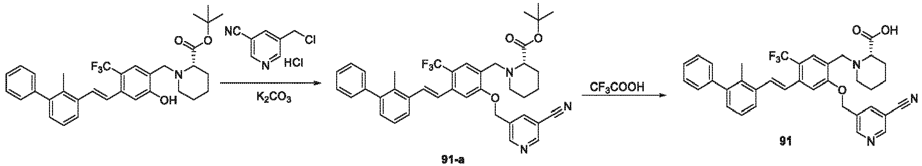

### Synthesis of compound 91-a

*tert*-Butyl (*S*,*E*)-1-(2-hydroxy-4-(2-(2-methyl-[1,1'-biphenyl]-3-yl)vinyl)-5-(trifluoromethyl)benzyl)piperidine-2-carboxylate (55.1 mg, 0.1 mmol) and 5-(chloromethyl)nicotinonitrile hydrochloride (37.81 mg, 0.2 mmol) were dissolved in *N,N-*dimethylformamide (3 mL), then potassium carbonate (69 mg, 0.5 mmol) was added, and the reaction solution was stirred at 50°C for 12 h. The reaction solution was diluted with ethyl acetate (3 mL), and washed with water (3 mL×3) and saturated brine (3 mL) in turn. The obtained organic phase was dried with anhydrous sodium sulfate, filtered, concentrated under reduced pressure, and the residue was purified by silica gel column chromatography (petroleum ether: ethyl acetate = 10:1) to give **91-a** (110 mg, yield: 82.36%). LC-MS (ESI): m/z = 667.2 [M+H]⁺.

### Synthesis of compound 91

Compound *tert*-butyl (*S*,*E*)-1-(2-((5-cyanopyridin-3-yl)methoxy)-4-(2-(2-methyl-[1,1'-biphenyl]-3-yl)vinyl)-5-(trifluoromethyl)benzyl)piperidine-2-carboxylate (110 mg, 0.165 mmol) was dissolved in dichloromethane (10 mL), and trifluoroacetic acid (5.0 mL) was added, then the reaction solution was stirred at room temperature for 12 h, concentrated under reduced pressure, and the residue was purified by high performance liquid chromatography to give 91 (58.7 mg, yield: 58.26%). LC-MS (ESI): m/z =612.4 [M+H]⁺.

¹H-NMR(CD₃OD-d*₄*) *δ*: 9.04(s, 1H), 8.95(s, 1H), 8.48(s, 1H), 8.02(s, 1H),7.67(s, 1H), 7.65-7.56(m, 2H), 7.47-7.44(m, 2H), 7.40-7.29(m, 5H), 7.21(d,*J*=8.0 Hz, 1H), 5.55(s, 2H), 4.60(d, *J*=12.8 Hz, 1H),4.44(d, *J*=12.8 Hz, 1H),3.58-3.55(m, 1H),3.35-3.30(m, 1H),3.02-2.96(m, 1H), 2.32(s, 3H), 2.28-2.24(m, 1H), 1.89-1.54(m, 5H) ppm.

### Effect embodiment 1: Homogenouse time-resolved fluorescence

Homogeneous time-resolved fluorescence (HTRF) binding assay was used to determine the binding ability of the compound of the present disclosure to PD-1/PD-L1.

The purchased kit (CisBio, #64CUS000C-1) contained reagents required for assays such as PD-1, PD-L1, anti-tag1-Eu, Anti-tag2-XL665, dilute buffer, and detection buffer.

### Experimental procedure

1. The compound was formulated to 10 concentrations with a 3-fold gradient with 100% DMSO.
2. The DMSO solution of the compound was added to the dilute buffer, mixed thoroughly, then transferred to a 96-well plate.
3. PD-L1 was diluted with dilute buffer, then added to the above 96-well plate.
4. PD-1 was diluted with dilute buffer and added to the above 96-well plate, which was then incubated at room temperature for 30 minutes.
5. A portion of anti-tag1-Eu and a portion of anti-tag2-XL665 were added to the detection buffer, mixed thoroughly and transferred to the above 96-well plate.
6. The mixture in the above 96-well plate was incubated at room temperature for 1 to 24 hours.
7. HTRF values were read with Envision.

### Experimental results

The biological activity of the compound of the present disclosure was determined by the above assay, and the results are shown as follows (Table 1):

**Table 1 IC₅₀ of partial compounds of the present disclosure binding to PD-1/PD-L1**

| Compound | IC₅₀ (µM) | Compound | IC₅₀ (µM) |
|---|---|---|---|
| **1** | 0.0102 | **2** | 0.0263 |
| **3** | 0.0871 | **4** | 0.0107 |
| **5** | 0.0065 | **6** | 0.0016 |
| **7** | 0.0019 | **8** | 0.0016 |
| **9** | 0.0258 | **10** | 0.0042 |
| **11** | 0.0101 | **12** | 0.0034 |
| **13** | 0.0460 | **14** | 0.0852 |
| **15** | 0.0021 | **16** | 0.0145 |
| **17** | 0.0044 | **18** | 0.0222 |
| **19** | 0.0062 | **20** | 0.0037 |
| **21** | 0.0576 | **22** | 0.0114 |
| **23** | 0.1857 | **24** | 0.0068 |
| **25** | 0.0091 | **26** | 0.0047 |
| **27** | 0.0572 | **28** | 0.1348 |
| **29** | 0.0620 | **30** | 0.0077 |
| **31** | 1.1605 | **35** | 1.1471 |
| **38** | 0.298 | **39** | 0.1725 |
| **40** | 1.437 | **41** | 0.2814 |
| **42** | 0.7058 | **43** | 0.0839 |
| **44** | 0.2653 | **48** | 0.008 |
| **49** | 0.0186 | **50** | 0.1413 |
| **51** | 0.0022 | **54** | 0.160 |
| **55** | 0.0295 | **57** | 0.0044 |
| **58** | 0.0068 | **59** | 0.0089 |
| **33** | >10 | **34** | >10 |
| **36** | >10 | **37** | >10 |
| **45** | >10 | **46** | >10 |
| **47** | >10 | **52** | >10 |
| **60** | 0.00059 | **61** | 0.00073 |
| **62** | 0.00742 | **63** | 0.03053 |
| **64** | 0.02946 | **65** | 0.04802 |
| **66** | 0.04167 | **67** | 0.01279 |
| **68** | 0.169 | **69** | 0.2603 |
| **70** | 0.00931 | **71** | 0.00068 |
| **72** | 0.0020 | **73** | 0.00399 |
| **74** | 0.00709 | **75** | 0.02839 |
| **77** | 0.00591 | **78** | 0.06553 |
| **79** | 0.00531 | **80** | 0.00397 |
| **81** | 0.00213 | **82** | 0.00185 |
| **83** | 0.00699 | **84** | 0.00803 |
| **85** | 0.00434 | **86** | 0.00358 |
| **87** | 0.00280 | | |

### Effect embodiment 2: Pharmacokinetic experiment in mice

Reagents: Acetonitrile, formic acid, methanol (HPLC grade) were purchased from Sigma-Aldrich (USA.), and pure water was purchased from Hangzhou Wahaha Group Co., Ltd. (Hangzhou, China). Other chemical reagents were all analytically pure reagents.

Experimental instruments: liquid chromatograph mass spectrometer (UPLC-MS/MS, composed of AB SCIEX TRIPLE QUAD 6500 triple quadrupole mass spectrometer, Shimadzu high performance liquid phase system. The Analyst 1.6.2 data acquisition and processing system was adopted).

Other instruments: Mettler electronic balance Mettler-ToledoXP26 and XS60025 (USA); Thermo Fisher -70°C ultra-low temperature refrigerator (USA); Thermo HERAEUS Multifuge X3R low temperature refrigerated centrifuge (Germany); IKA VIBRAX VXR basic shaker (Germany); IKA disperser (Germany); Xinzhi SCIENTZ-48L frozen high-throughput tissue grinder (Ningbo, China), etc.

Experimental animals: 6 CD1 male mice, 29-30 g before administration, were purchased from Shanghai Jihui Experimental Animal Breeding Co., Ltd., and the animal certificate number is SCXK(SH) 2017-0012 20170012001403. Before the experiment, the animals should be kept for at least 3 days to adapt to the environment. Throughout the experiment, the animals had free access to food and water.

### Experimental procedure:

1) Preparation of a mother liquor: A certain amount of the test drug was weighed in a clean sample bottle, then dimethyl sulfoxide was added, vortexed thoroughly for 1 min, and sonicated for 5 min to give a 4 mg/mL solution.

Drug preparation for intravenous administration: A certain amount of the above solution was taken and placed in a clean sample bottle, then polyethylene glycol-15 hydroxystearate was added, vortexed thoroughly for 1 min, then normal saline was added, and the mixture was continued vortexing for 1 min to give 4 mg/mL solution A.

Drug preparation for oral administration: A certain amount of the test drug was weighed and placed in a clean sample bottle, then a pre-prepared vehicle "10% (w/v) polyoxyethylene 40 hydrogenated castor + 20% (w/v) sulfobutyl ether β-cyclodextrin + 70% water" was added, vortexed thoroughly for 1 min, magnetically stirred for 20-25 min, and sonicated for 10 min to give 1 mg/mL solution B.

2) Intravenous administration: 3 mice were injected with 2 mg/kg (5 mL/kg) solution A through the tail vein.

Oral administration: 3 mice were given 10 mg/kg (10 mL/kg) solution B by gavage.

3) Collection and storage of plasma samples: Blood samples were collected from the orbital venous plexus of mice according to predetermined time points (5 minutes, 15 minutes, 30 minutes, 1 hour, 2 hours, 4 hours, 8 hours and 24 hours). 25 µL of blood was placed in EDTA-K2 anticoagulant tubes (placed on wet ice), and 20 µL of blood was immediately diluted with 60 µL of water and vortexed until evenly mixed. Plasma was stored in a -70°C refrigerator for long-term storage until sample analysis.

4) Analysis of plasma samples: 200 µL of acetonitrile (containing 100 ng/mL internal standard) was added to 25 µL of plasma sample, vortexed for 10 minutes, and centrifuged at 5800 rpm for 10 minutes. 70 µL of supernatant was transferred to a 96-well injection plate. 1 µL of plasma sample was analyzed by LC-MS/MS.

5) The pharmacokinetic parameters were calculated according to the test results.

### Experimental results

Certain pharmacokinetic parameters can be obtained by some of the compounds of the present disclosure and their control compounds from the above tests, and the results are shown in Table 2 below:

**Table 2: Pharmacokinetic parameters of some compounds of the present disclosure and their control compounds**

| Compound | Cₘₐₓ (ng/mL) | AUC_{INF} (hr ng/mL) | F (%) |
|---|---|---|---|
| 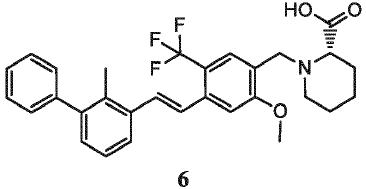 | 1870 | 40577 | 72.4 |
| 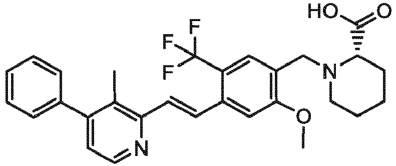 | 673 | 2297 | 23.6 |
| 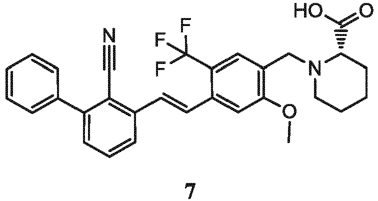 | 1403 | 7618 | 69.7 |
| 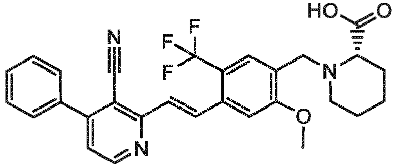 | 254 | 1003 | 16.4 |
| 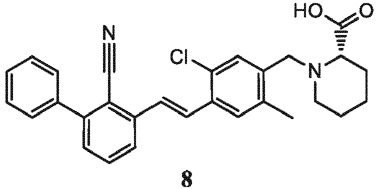 | 2130 | 16799 | 80.6 |
| 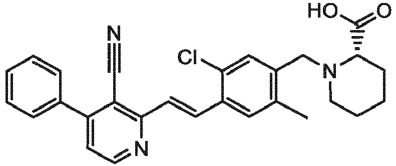 | 1257 | 8582 | 74.1 |

It can be seen from Table 2 that: Compared with the control compound, the compound of the present disclosure has significantly higher drug peak concentration, larger area under the curve and better oral bioavailability in the pharmacokinetic study of mice.

## Claims

1. An aromatic vinyl compound of formula I-0, a tautomer thereof, a stereoisomer thereof, a racemate thereof, an isotopic derivative thereof, or their pharmaceutically acceptable salts; 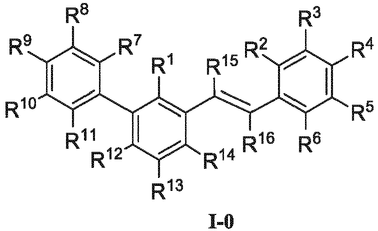 wherein:
R¹ is cyano, C₁-C₄ alkyl, C₁-C₄ alkyl substituted by one or more than one deuterium, halogen, C₁-C₄ alkyl substituted by one or more than one halogen;
R³, R⁶, R¹², R¹³ and R¹⁴ are independently H or deuterium;
R² is hydroxyl, halogen, C₁-C₄ alkyl, C₁-C₄ alkyl substituted by one or more than one R^{A-1}, C₁-C₆ alkoxy or C₁-C₆ alkoxy substituted by one or more than one R^{A-2};
R^{A-1} and R^{A-2} are independently deuterium, hydroxyl, halogen, cyano, C₁-C₄ alkoxy, C₁-C₄ alkoxy substituted by one or more than one deuterium, 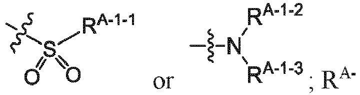 ¹⁻¹ is C₁-C₄ alkyl or C₁-C₄ alkyl substituted by one or more than one deuterium; R^{A-1-2} and R^{A-1-3} are independently H, deuterium, C₁-C₄ alkyl or C₁-C₄ alkyl substituted by one or more than one R^{A-1-1-1}, and R^{A-1-1-1} is deuterium, hydroxyl or COOR^{A-1-1-2};
R⁴ and R⁵ are independently hydroxyl, halogen, C₁-C₄ alkyl, C₁-C₄ alkyl substituted by one or more than one R^{B-1}, C₁-C₆ alkoxy, or C₁-C₆ alkoxy substituted by one or more than one R^{B-2};
R^{B-1} and R^{B-2} are independently deuterium, hydroxyl, halogen, cyano, C₆-C₁₀ aryl, C₆-C₁₀ aryl substituted by one or more than one R^{B-1-3}, 3- to 12-membered heteroaryl, 3- to 12-membered heteroaryl substituted by one or more than one R^{B-1-4}, C₁-C₄ alkoxy, C₁-C₄ alkoxy substituted by one or more than one deuterium, 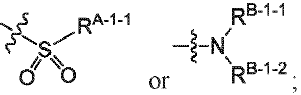 in the heteroaryl, the heteroatom is selected from one or more of N, O and S, and the number of the heteroatom is 1 to 4;
R^{B-1-3} and R^{B-1-4} are independently cyano, halogen, C₁-C₄ alkyl or C₁-C₄ alkoxy;
R^{B-1-1} and R^{B-1-2} are independently H, deuterium, C₁-C₄ alkyl or C₁-C₄ alkyl substituted by one or more than one R^{B-1-1-1}, and R^{B-1-1-1} is deuterium, hydroxyl or COOR^{B-1-1-5};
or, R^{B-1-1}, R^{B-1-2} together with the nitrogen atom to which they are attached form a 5- to 7-membered carbon heterocycle or a 5- to 7-membered carbon heterocycle substituted by one or more than one R^{B-1-1-2}; in the carbon heterocycle, the heteroatom is selected from one or more of N, O and S, and the number of the heteroatom is 1 to 4; R^{B-1-1-2} is deuterium, C₁-C₄ alkyl, C₁-C₄ alkyl substituted by one or more than one deuterium, COOR^{B-1-1-6} or C₁-C₄ amido;
R^{A-1-1-2}, R^{B-1-1-5} and R^{B-1-1-6} are independently H, deuterium, C₁-C₄ alkyl or C₁-C₄ alkyl substituted by one or more than one deuterium;
R⁷, R⁸, R⁹, R¹⁰ and R¹¹ are independently H or deuterium;
R¹⁵ and R¹⁶ are independently H, deuterium or halogen.

2. The aromatic vinyl compound of formula I-0, the tautomer thereof, the stereoisomer thereof, the racemate thereof, the isotopic derivative thereof, or their pharmaceutically acceptable salts according to claim 1, wherein, R^{B-1} and R^{B-2} are independently deuterium, hydroxyl, halogen, cyano, C₁-C₄ alkoxy, C₁-C₄ alkoxy substituted by one or more than one deuterium,

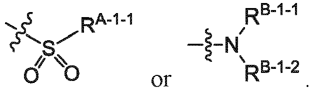

3. The aromatic vinyl compound of formula I-0, the tautomer thereof, the stereoisomer thereof, the racemate thereof, the isotopic derivative thereof, or their pharmaceutically acceptable salts according to at least one of claims 1 to 2, wherein,
the aromatic vinyl compound of formula I-0 is an aromatic vinyl compound of formula I: 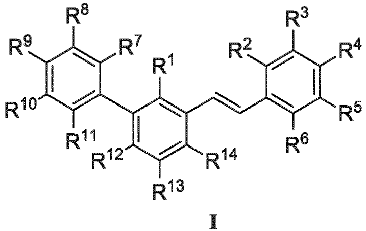 wherein:
R¹ is cyano, C₁-C₄ alkyl, C₁-C₄ alkyl substituted by one or more than one deuterium;
R³, R⁶, R¹², R¹³ and R¹⁴ are independently H or deuterium;
R² is hydroxyl, halogen, C₁-C₄ alkyl, C₁-C₄ alkyl substituted by one or more than one R^{A-1}, C₁-C₆ alkoxy or C₁-C₆ alkoxy substituted by one or more than one R^{A-2};
R^{A-1} and R^{A-2} are independently deuterium, hydroxyl, halogen, cyano, C₁-C₄ alkoxy, C₁-C₄ alkoxy substituted by one or more than one deuterium, 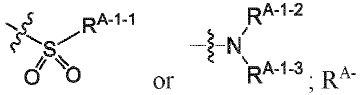 ¹⁻¹ is C₁-C₄ alkyl or C₁-C₄ alkyl substituted by one or more than one deuterium; R^{A-1-2} and R^{A-1-3} are independently H, deuterium, C₁-C₄ alkyl or C₁-C₄ alkyl substituted by one or more than one R^{A-1-1-1} , and R^{A-1-1-1} is deuterium, hydroxyl or COOR^{A-1-1-2};
R⁴ and R⁵ are independently hydroxyl, halogen, C₁-C₄ alkyl, C₁-C₄ alkyl substituted by one or more than one R^{B-1}, C₁-C₆ alkoxy, or C₁-C₆ alkoxy substituted by one or more than one R^{B-2};
R^{B-1} and R^{B-2} are independently deuterium, hydroxyl, halogen, cyano, C₁-C₄ alkoxy, C₁-C₄ alkoxy substituted by one or more than one deuterium, 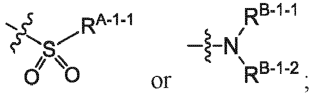
R^{B-1-1} and R^{B-1-2} are independently H, deuterium, C₁-C₄ alkyl or C₁-C₄ alkyl substituted by one or more than one R^{B-1-1-1}, and R^{B-1-1-1} is deuterium, hydroxyl or COOR^{B-1-1-5};
or, R^{B-1-1}, R^{B-1-2} together with the nitrogen atom to which they are attached form a 5- to 7-membered carbon heterocycle or a 5- to 7-membered carbon heterocycle substituted by one or more than one R^{B-1-1-2}; in the carbon heterocycle, the heteroatom is selected from one or more of N, O and S, and the number of the heteroatom is 1 to 4; R^{B-1-1-2} is deuterium, C₁-C₄ alkyl, C₁-C₄ alkyl substituted by one or more than one deuterium, COOR^{B-1-1-6} or C₁-C₄ amido;
R^{A-1-1-2}, R^{B-1-1-5} and R^{B-1-1-6} are independently H, deuterium, C₁-C₄ alkyl or C₁-C₄ alkyl substituted by one or more than one deuterium;
R⁷, R⁸, R⁹, R¹⁰ and R¹¹are independently H or deuterium.

4. The aromatic vinyl compound of formula I-0, the tautomer thereof, the stereoisomer thereof, the racemate thereof, the isotopic derivative thereof, or their pharmaceutically acceptable salts according to at least one of claims 1 to 3, wherein, when R¹ is C₁-C₄ alkyl, the C₁-C₄ alkyl is methyl, ethyl, *n*-propyl, isopropyl, *n*-butyl, isobutyl or *tert*-butyl;
and/or, when R¹ is C₁-C₄ alkyl substituted by one or more than one deuterium, the C₁-C₄ alkyl substituted by one or more than one deuterium is C₁-C₂ alkyl substituted by one or more than one deuterium;
and/or, when R¹ is halogen, the halogen is fluorine, chlorine, bromine or iodine;
and/or, when R¹ is C₁-C₄ alkyl substituted by one or more than one halogen, the halogen is fluorine, chlorine, bromine or iodine;
and/or, when R¹ is C₁-C₄ alkyl substituted by one or more than one halogen, the C₁-C₄ alkyl is methyl, ethyl, *n*-propyl, isopropyl, *n*-butyl, isobutyl or *tert*-butyl;
and/or, when R² is halogen, the halogen is fluorine, chlorine, bromine or iodine;
and/or, when R² is C₁-C₄ alkyl, the C₁-C₄ alkyl is methyl, ethyl, *n*-propyl, isopropyl, *n-*butyl, isobutyl or *tert*-butyl;
and/or, when R² is C₁-C₄ alkyl substituted by one or more than one R^{A-1}, the C₁-C₄ alkyl is methyl, ethyl, *n*-propyl, isopropyl, *n*-butyl, isobutyl or *tert*-butyl;
and/or, when R² is C₁-C₄ alkyl substituted by one or more than one R^{A-1}, each R^{A-1} is the same or different, and the more than one is 2, 3, 4 or 5;
and/or, when R² is C₁-C₆ alkoxy, the C₁-C₆ alkoxy is C₁-C₄ alkoxy;
and/or, when R² is C₁-C₆ alkoxy substituted by one or more than one R^{A-2}, the C₁-C₆ alkoxy is C₁-C₄ alkoxy;
and/or, when R² is C₁-C₆ alkoxy substituted by one or more than one R^{A-2}, each R^{A-2} is the same or different, and the more than one is 2, 3, 4 or 5;
and/or, when R^{A-1} and R^{A-2} are independently halogen, the halogen is fluorine, chlorine, bromine or iodine;
and/or, when R^{A-1} and R^{A-2} are independently C₁-C₄ alkoxy, the C₁-C₄ alkoxy is methoxy, ethoxy, *n*-propoxy or *n*-butoxy;
and/or, when R^{A-1} and R^{A-2} are independently C₁-C₄ alkoxy substituted by one or more than one deuterium, the C₁-C₄ alkoxy substituted by one or more than one deuterium is C₁-C₂ alkoxy substituted by one or more than one deuterium;
and/or, when R^{A-1-1} is C₁-C₄ alkyl, the C₁-C₄ alkyl is methyl, ethyl, *n*-propyl, isopropyl, *n*-butyl, isobutyl or *tert*-butyl;
and/or, when R^{A-1-1} is C₁-C₄ alkyl substituted by one or more than one deuterium, the C₁-C₄ alkyl substituted by one or more than one deuterium is C₁-C₂ alkyl substituted by one or more than one deuterium;
and/or, when R^{A-1-2} and R^{A-1-3} are independently C₁-C₄ alkyl, the C₁-C₄ alkyl is methyl, ethyl, *n*-propyl, isopropyl, *n*-butyl, isobutyl or *tert*-butyl;
and/or, when R^{A-1-2} and R^{A-1-3} are independently C₁-C₄ alkyl substituted by one or more than one R^{A-1-1-1}, the C₁-C₄ alkyl is methyl, ethyl, *n*-propyl, isopropyl, *n*-butyl, isobutyl or *tert-*butyl;
and/or, when R^{A-1-2} and R^{A-1-3} are independently C₁-C₄ alkyl substituted by one or more than one R^{A-1-1-1}, each R^{A-1-1-1} is the same or different, and the more than one is 2, 3, 4 or 5;
and/or, when R⁴ and R⁵ are independently halogen, the halogen is fluorine, chlorine, bromine or iodine;
and/or, when R⁴ and R⁵ are independently C₁-C₄ alkyl, the C₁-C₄ alkyl is methyl, ethyl, *n-*propyl, isopropyl, *n*-butyl, isobutyl or *tert*-butyl;
and/or, when R⁴ and R⁵ are independently C₁-C₄ alkyl substituted by one or more than one R^{B-1}, the C₁-C₄ alkyl is methyl, ethyl, *n*-propyl, isopropyl, *n*-butyl, isobutyl or *tert*-butyl;
and/or, when R⁴ and R⁵ are independently C₁-C₄ alkyl substituted by one or more than one R^{B-1}, each R^{B-1} is the same or different, and the more than one is 2, 3, 4 or 5;
and/or, when R⁴ and R⁵ are independently C₁-C₆ alkoxy, the C₁-C₆ alkoxy is C₁-C₄ alkoxy;
and/or, when R⁴ and R⁵ are independently C₁-C₆ alkoxy substituted by one or more than one R^{B-2}, the C₁-C₆ alkoxy is C₁-C₄ alkoxy;
and/or, when R⁴ and R⁵ are independently C₁-C₆ alkoxy substituted by one or more than one R^{B-2}, each R^{B-2} is the same or different, and the more than one is 2, 3, 4 or 5;
and/or, when R^{B-1} and R^{B-2} are independently halogen, the halogen is fluorine, chlorine, bromine or iodine;
and/or, when R^{B-1} and R^{B-2} are independently C₆-C₁₀ aryl, the C₆-C₁₀ aryl is phenyl;
and/or, when R^{B-1} and R^{B-2} are independently C₆-C₁₀ aryl substituted by one or more than one R^{B-1-3}, the C₆-C₁₀ aryl is phenyl;
and/or, when R^{B-1} and R^{B-2} are independently C₆-C₁₀ aryl substituted by one or more than one R^{B-1-3}, each R^{B-1-3} is the same or different, and the more than one is 2, 3, 4 or 5;
and/or, in the aromatic vinyl compound of formula I-0 or I, when R^{B-1} and R^{B-2} are independently 3- to 12-membered heteroaryl, the 3- to 12-membered heteroaryl is 5- to 7-membered heteroaryl;
and/or, in the aromatic vinyl compound of formula I-0 or I, when R^{B-1} and R^{B-2} are independently 3- to 12-membered heteroaryl, the heteroatom of the 3- to 12-membered heteroaryl is N, the number of the heteroatom is 1;
and/or, when R^{B-1} and R^{B-2} are independently 3- to 12-membered heteroaryl substituted by one or more than one R^{B-1-4}, the 3- to 12-membered heteroaryl is 5- to 7-membered heteroaryl;
and/or, when R^{B-1} and R^{B-2} are independently 3- to 12-membered heteroaryl substituted by one or more than one R^{B-1-4}, the heteroatom of the 3- to 12-membered heteroaryl is N, the number of the heteroatom is 1;
and/or, when R^{B-1} and R^{B-2} are independently 3- to 12-membered heteroaryl substituted by one or more than one R^{B-1-4}, each R^{B-1-4} is the same or different, and the more than one is 2, 3, 4 or 5;
and/or, when R^{B-1-3} and R^{B-1-4} are independently halogen, the halogen is fluorine, chlorine, bromine or iodine;
and/or, when R^{B-1} and R^{B-2} are independently C₁-C₄ alkoxy, the C₁-C₄ alkoxy is methoxy, ethoxy, *n*-propoxy or *n*-butoxy;
and/or, when R^{B-1} and R^{B-2} are independently C₁-C₄ alkoxy substituted by one or more than one deuterium, the C₁-C₄ alkoxy substituted by one or more than one deuterium is C₁-C₂ alkoxy substituted by one or more than one deuterium;
and/or, when R^{B-1-1} and R^{B-1-2} are independently C₁-C₄ alkyl, the C₁-C₄ alkyl is methyl, ethyl, *n*-propyl, isopropyl, *n*-butyl, isobutyl or *tert*-butyl;
and/or, when R^{B-1-1} and R^{B-1-2} are independently C₁-C₄ alkyl substituted by one or more than one R^{B-1-1-1}, the C₁-C₄ alkyl is methyl, ethyl, *n*-propyl, isopropyl, *n*-butyl, isobutyl or *tert-*butyl;
and/or, when R^{B-1-1} and R^{B-1-2} are independently C₁-C₄ alkyl substituted by one or more than one R^{B-1-1-1}, each R^{B-1-1-1} is the same or different, and the more than one is 2, 3, 4 or 5;
and/or, when R^{B-1-1}, R^{B-1-2} together with the nitrogen atom to which they are attached form the 5- to 7-membered carbon heterocycle, in the carbon heterocycle, the heteroatom is N and/or O;
and/or, when R^{B-1-1}, R^{B-1-2} together with the nitrogen atom to which they are attached form the 5- to 7-membered carbon heterocycle, in the carbon heterocycle, the number of the heteroatom is 1 or 2;
and/or, when R^{B-1-1}, R^{B-1-2} together with the nitrogen atom to which they are attached form the carbon heterocycle substituted by one or more than one R^{B-1-1-2}, in the carbon heterocycle, the heteroatom is N and/or O;
and/or, when R^{B-1-1}, R^{B-1-2} together with the nitrogen atom to which they are attached form the carbon heterocycle substituted by one or more than one R^{B-1-1-2}, in the carbon heterocycle, the number of the heteroatom is 1 or 2;
and/or, when R^{B-1-1}, R^{B-1-2} together with the nitrogen atom to which they are attached form the carbon heterocycle substituted by one or more than one R^{B-1-1-2}, each R^{B-1-1-2} is the same or different, and the more than one is 2, 3, 4 or 5;
and/or, when R^{B-1-1-2} is C₁-C₄ alkyl, the C₁-C₄ alkyl is methyl, ethyl, *n*-propyl, isopropyl, *n*-butyl, isobutyl or *tert*-butyl;
and/or, when R^{B-1-1-2} is C₁-C₄ alkyl substituted by one or more than one deuterium, the C₁-C₄ alkyl substituted by one or more than one deuterium is C₁-C₂ alkyl substituted by one or more than one deuterium;
and/or, when the R^{B-1-1-2} is C₁-C₄ amido, the C₁-C₄ amido is 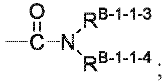 R^{B-1-1-3} and R^{B-1-1-4} are independently H, deuterium, C₁-C₄ alkyl or C₁-C₄ alkyl substituted by one or more than one deuterium;
and/or, when R^{A-1-1-2}, R^{B-1-1-5} and R^{B-1-1-6} are independently C₁-C₄ alkyl, the C₁-C₄ alkyl is methyl, ethyl, *n*-propyl, isopropyl, *n*-butyl, isobutyl or *tert*-butyl;
and/or, when R¹⁵ and R¹⁶ are independently halogen, the halogen is fluorine, chlorine, bromine or iodine.

5. The aromatic vinyl compound of formula I-0, the tautomer thereof, the stereoisomer thereof, the racemate thereof, the isotopic derivative thereof, or their pharmaceutically acceptable salts according to claim 4, wherein, when R¹ is C₁-C₄ alkyl, the C₁-C₄ alkyl is methyl;
and/or, when R¹ is C₁-C₄ alkyl substituted by one or more than one deuterium, the C₁-C₄ alkyl substituted by one or more than one deuterium is monodeuterated methyl, dideuterated methyl, trideuterated methyl, monodeuterated ethyl, dideuterated ethyl, trideuterated ethyl, tetradeuterated ethyl or pentadeuterated ethyl; for example trideuterated methyl;
and/or, when R¹ is halogen, the halogen is chlorine;
and/or, when R¹ is C₁-C₄ alkyl substituted by one or more than one halogen, the halogen is fluorine;
when R¹ is C₁-C₄ alkyl substituted by one or more than one halogen, the C₁-C₄ alkyl is methyl;
and/or, when R² is halogen, the halogen is chlorine;
and/or, when R² is C₁-C₄ alkyl, the C₁-C₄ alkyl is methyl;
and/or, when R² is C₁-C₄ alkyl substituted by one or more than one R^{A-1}, the C₁-C₄ alkyl is methyl;
and/or, when R² is C₁-C₆ alkoxy, the C₁-C₆ alkoxy is methoxy, ethoxy, *n*-propoxy or *n-*butoxy; for example methoxy;
and/or, when R² is C₁-C₆ alkoxy substituted by one or more than one R^{A-2}, the C₁-C₆ alkoxy is methoxy, ethoxy, *n*-propoxy or *n*-butoxy;
and/or, when R^{A-1} and R^{A-2} are independently halogen, the halogen is fluorine;
and/or, when R^{A-1} and R^{A-2} are independently C₁-C₄ alkoxy, the C₁-C₄ alkoxy is methoxy;
and/or, when R^{A-1} and R^{A-2} are independently C₁-C₄ alkoxy substituted by one or more than one deuterium, the C₁-C₄ alkoxy substituted by one or more than one deuterium is monodeuterated methoxy, dideuterated methoxy, trideuterated methoxy, monodeuterated ethoxy, dideuterated ethoxy, trideuterated ethoxy, tetradeuterated ethoxy or pentadeuterated ethoxy, for example trideuterated methoxy;
and/or, when R^{A-1-1} is C₁-C₄ alkyl, the C₁-C₄ alkyl is methyl;
and/or, when R^{A-1-1} is C₁-C₄ alkyl substituted by one or more than one deuterium, the C₁-C₄ alkyl substituted by one or more than one deuterium is monodeuterated methyl, dideuterated methyl, trideuterated methyl, monodeuterated ethyl, dideuterated ethyl, trideuterated ethyl, tetradeuterated ethyl or pentadeuterated ethyl; for example trideuterated methyl;
and/or, when R^{A-1-2} and R^{A-1-3} are independently C₁-C₄ alkyl, the C₁-C₄ alkyl is methyl, ethyl or isopropyl;
and/or, when R^{A-1-2} and R^{A-1-3} are independently C₁-C₄ alkyl substituted by one or more than one R^{A-1-1-1}, the C₁-C₄ alkyl is methyl, ethyl or isopropyl;
and/or, when R⁴ and R⁵ are independently halogen, the halogen is chlorine;
and/or, when R⁴ and R⁵ are independently C₁-C₄ alkyl, the C₁-C₄ alkyl is methyl;
and/or, when R⁴ and R⁵ are independently C₁-C₄ alkyl substituted by one or more than one R^{B-1}, the C₁-C₄ alkyl is methyl;
and/or, when R⁴ and R⁵ are independently C₁-C₆ alkoxy, the C₁-C₆ alkoxy is methoxy, ethoxy, n-propoxy or n-butoxy, for example methoxy;
and/or, when R⁴ and R⁵ are independently C₁-C₆ alkoxy substituted by one or more than one R^{B-2}, the C₁-C₆ alkoxy is methoxy, ethoxy, *n*-propoxy or *n*-butoxy;
and/or, when R^{B-1} and R^{B-2} are independently halogen, the halogen is fluorine;
and/or, when R^{B-1-3} and R^{B-1-4} are independently halogen, the halogen is fluorine or iodine;
and/or, when R^{B-1} and R^{B-2} are independently C₁-C₄ alkoxy, the C₁-C₄ alkoxy is methoxy;
and/or, when R^{B-1} and R^{B-2} are independently C₁-C₄ alkoxy substituted by one or more than one deuterium, the C₁-C₄ alkoxy substituted by one or more than one deuterium is monodeuterated methoxy, dideuterated methoxy, trideuterated methoxy, monodeuterated ethoxy, dideuterated ethoxy, trideuterated ethoxy, tetradeuterated ethoxy or pentadeuterated ethoxy, for example trideuterated methoxy;
and/or, when R^{B-1-1} and R^{B-1-2} are independently C₁-C₄ alkyl, the C₁-C₄ alkyl is methyl, ethyl or isopropyl;
and/or, when R^{B-1-1} and R^{B-1-2} are independently C₁-C₄ alkyl substituted by one or more than one R^{B-1-1-1}, the C₁-C₄ alkyl is methyl, ethyl or isopropyl;
and/or, when R^{B-1-1-2} is C₁-C₄ alkyl, the C₁-C₄ alkyl is methyl;
and/or, when R^{B-1-1-2} is C₁-C₄ alkyl substituted by one or more than one deuterium, the C₁-C₄ alkyl substituted by one or more than one deuterium is monodeuterated methyl, dideuterated methyl, trideuterated methyl, monodeuterated ethyl, dideuterated ethyl, trideuterated ethyl, tetradeuterated ethyl or pentadeuterated ethyl, for example trideuterated methyl;
and/or, when R^{B-1-1-2} is C₁-C₄ amido, the C₁-C₄ amido is 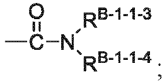 R^{B-1-1-3} and R^{B-1-1-4} are independently H, deuterium, C₁-C₄ alkyl or C₁-C₄ alkyl substituted by one or more than one deuterium; the C₁-C₄ alkyl is methyl, ethyl, *n*-propyl, isopropyl, *n*-butyl, isobutyl or *tert-*butyl; the C₁-C₄ alkyl substituted by one or more than one deuterium is trideuterated methyl;
and/or, when R^{A-1-1-2}, R^{B-1-1-5} and R^{B-1-1-6} are independently C₁-C₄ alkyl, the C₁-C₄ alkyl is methyl;
and/or, when R¹⁵ and R¹⁶ are independently halogen, the halogen is fluorine or bromine.

6. The aromatic vinyl compound of formula I-0, the tautomer thereof, the stereoisomer thereof, the racemate thereof, the isotopic derivative thereof, or their pharmaceutically acceptable salts according to at least one of claims 1 to 3, wherein, R³, R⁶, R¹², R¹³ and R¹⁴ are independently H;
and/or, R² is halogen, C₁-C₄ alkyl or C₁-C₄ alkyl substituted by one or more than one R^{A-1};
and/or, in the aromatic vinyl compound of formula I-0 or I, R^{A-1} and R^{A-2} are independently hydroxyl, halogen, cyano, C₁-C₄ alkoxy, 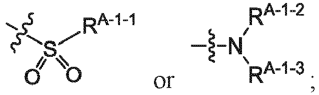
and/or, R^{A-1-1} is C₁-C₄ alkyl;
and/or, R^{A-1-2} and R^{A-1-3} are independently H or C₁-C₄ alkyl substituted by one or more than one R^{A-1-1-1};
and/or, R^{A-1-1-2}, R^{B-1-1-5} and R^{B-1-1-6} are independently H or C₁-C₄ alkyl;
and/or, the C₁-C₄ alkyl substituted by one or more than one R^{A-1-1-1} is 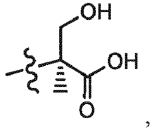 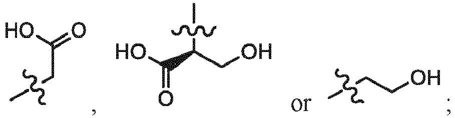
and/or, R⁴ and R⁵ are independently halogen, C₁-C₄ alkyl, C₁-C₄ alkyl substituted by one or more than one R^{B-1}, C₁-C₆ alkoxy or C₁-C₆ alkoxy substituted by one or more than one R^{B-2};
and/or, R^{B-1} and R^{B-2} are independently deuterium, hydroxyl, cyano, C₆-C₁₀ aryl, C₆-C₁₀ aryl substituted by one or more than one R^{B-1-3}, 3- to 12-membered heteroaryl, 3- to 12-membered heteroaryl substituted by one or more than one R^{B-1-4}, C₁-C₄ alkoxy, C₁-C₄ alkoxy substituted by one or more than one deuterium, 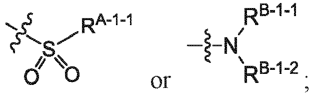
and/or, R^{B-1-3} and R^{B-1-4} are independently cyano or halogen;
and/or, R^{B-1-1} and R^{B-1-2} are independently H or C₁-C₄ alkyl substituted by one or more than one R^{B-1-1-1}; or, R^{B-1-1}, R^{B-1-2} together with the nitrogen atom to which they are attached form the 5- to 7-membered carbon heterocycle substituted by one or more than one R^{B-1-1-2};
and/or, the C₁-C₄ alkyl substituted by one or more than one R^{B-1-1-1} is 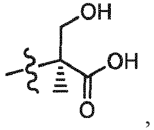 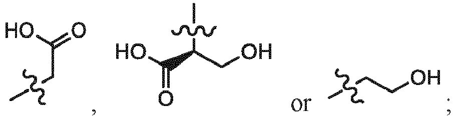
and/or, R^{B-1-1-2} is C₁-C₄ alkyl, COOR^{B-1-1-6} or C₁-C₄ amido;
and/or, the carbon heterocycle is 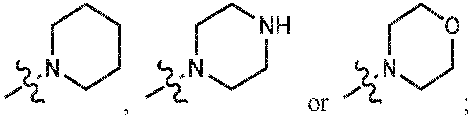
and/or, at least one of R¹⁵ and R¹⁶ is H or deuterium.

7. The aromatic vinyl compound of formula I-0, the tautomer thereof, the stereoisomer thereof, the racemate thereof, the isotopic derivative thereof, or their pharmaceutically acceptable salts according to claim 6, wherein, R² is halogen or C₁-C₄ alkyl;
and/or, R^{A-1} is halogen or 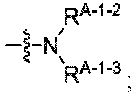
and/or, one of R^{A-1-2} and R^{A-1-3} is H, and the other is C₁-C₄ alkyl substituted by one or more than one R^{A-1-1-1};
and/or, R^{A-1-1-2} , R^{B-1-1-5} and R^{B-1-1-6} are independently H;
and/or, R^{B-1} is hydroxyl, C₁-C₄ alkoxy, C₁-C₂ alkoxy substituted by one or more than one deuterium or 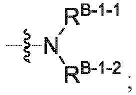
and/or, R^{B-2} is deuterium, cyano, hydroxyl, C₆-C₁₀ aryl, C₆-C₁₀ aryl substituted by one or more than one R^{B-1-3}, 3- to 12-membered heteroaryl, 3- to 12-membered heteroaryl substituted by one or more than one R^{B-1-4} or C₁-C₄ alkoxy;
and/or, the C₆-C₁₀ aryl substituted by one or more than one R^{B-1-3} is 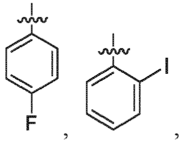 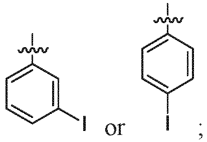
and/or, the 3- to 12-membered heteroaryl substituted by one or more than one R^{B-1-4} is 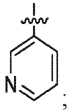
and/or, the 3- to 12-membered heteroaryl substituted by one or more than one R^{B-1-4} is 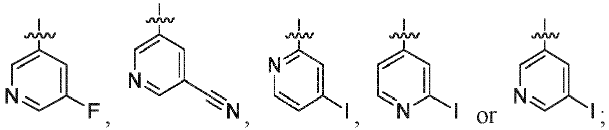
and/or, one of R^{B-1-1} and R^{B-1-2} is H, or and the other is C₁-C₄ alkyl substituted by one or more than one R^{B-1-1-1}; or, R^{B-1-1}, R^{B-1-2} together with the nitrogen atom to which they are attached form the 5- to 7-membered carbon heterocycle substituted by one or more than one R^{B-1-1-2};
and/or, R^{B-1-1-2} is methyl, carboxyl or -CONH₂;
and/or, the carbon heterocycle substituted by one or more than one R^{B-1-1-2} is 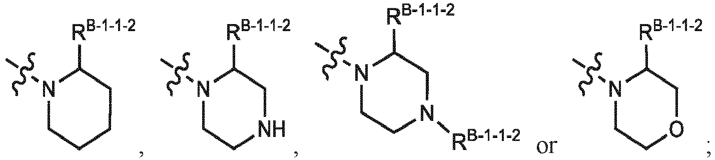
and/or, 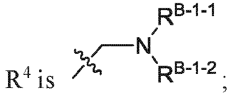
and/or, R⁵ is halogen, C₁-C₄ alkyl, C₁-C₄ alkyl substituted by one or more than one R^{B-1}, C₁-C₆ alkoxy or C₁-C₆ alkoxy substituted by one or more than one R^{B-2}; R^{B-1} and R^{B-2} are independently deuterium, hydroxyl, cyano, C₁-C₄ alkoxy, C₁-C₂ alkoxy substituted by one or more than one deuterium or 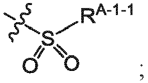
and/or, R¹⁵ is H or deuterium; R¹⁶ is H, deuterium or halogen.

8. The aromatic vinyl compound of formula I-0, the tautomer thereof, the stereoisomer thereof, the racemate thereof, the isotopic derivative thereof, or their pharmaceutically acceptable salts according to claim 7, wherein, R^{B-1} is hydroxyl or 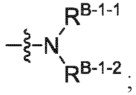
and/or, 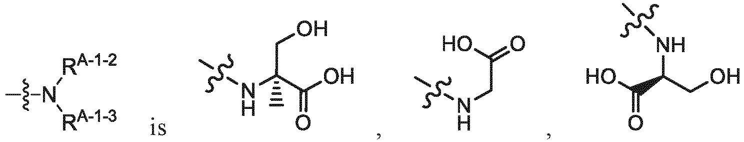 or 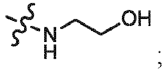
and/or, 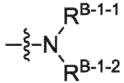 is

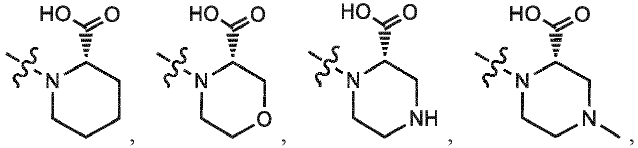

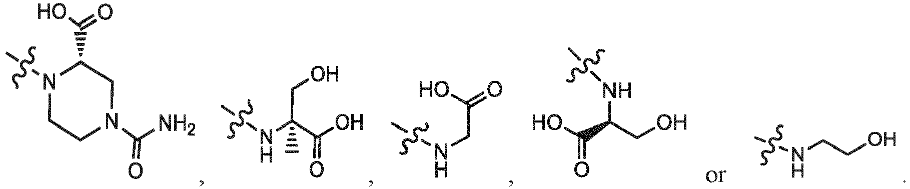

9. The aromatic vinyl compound of formula I-0, the tautomer thereof, the stereoisomer thereof, the racemate thereof, the isotopic derivative thereof, or their pharmaceutically acceptable salts according to at least one of claims 1 to 3, wherein, R¹ is halogen or C₁-C₄ alkyl substituted by one or more than one halogen;
and/or, R¹⁵ is deuterium or halogen;
and/or, R¹⁶ is deuterium or halogen.

10. The aromatic vinyl compound of formula I-0, the tautomer thereof, the stereoisomer thereof, the racemate thereof, the isotopic derivative thereof, or their pharmaceutically acceptable salts according to at least one of claims 1 to 3, wherein, the aromatic vinyl compound of formula I-0 or I is selected from any one of the following schemes,
scheme 1: R² is 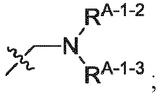 R⁴ and R⁵ are independently halogen, C₁-C₄ alkyl, C₁-C₄ alkyl substituted by one or more than one R^{B-1}, C₁-C₆ alkoxy or C₁-C₆ alkoxy substituted by one or more than one R^{B-2}; R^{B-1} and R^{B-2} are independently deuterium, hydroxyl, cyano, C₁-C₄ alkoxy, C₁-C₂ alkoxy substituted by one or more than one deuterium or 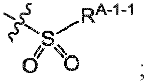 R³, R⁶, R¹², R¹³ and R¹⁴ are H;
scheme 2: R² is halogen, C₁-C₄ alkyl or C₁-C₄ alkyl substituted by one or more than one R^{A-1}; R^{A-1} is halogen; R⁴ is 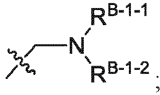 R⁵ is halogen, hydroxyl, C₁-C₄ alkyl, C₁-C₄ alkyl substituted by one or more than one R^{B-1}, C₁-C₆ alkoxy or C₁-C₆ alkoxy substituted by one or more than one R^{B-2}; R^{B-1} and R^{B-2} are independently deuterium, hydroxyl, cyano, C₁-C₄ alkoxy, C₁-C₂ alkoxy substituted by one or more than one deuterium or 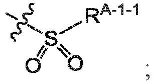 R³, R⁶, R¹², R¹³ and R¹⁴ are H;
scheme 3: R² is halogen, C₁-C₄ alkyl or C₁-C₄ alkyl substituted by one or more than one R^{A-1}; R^{A-1} is halogen; R⁴ is halogen, C₁-C₄ alkyl, C₁-C₄ alkyl substituted by one or more than one R^{B-1}, C₁-C₆ alkoxy or C₁-C₆ alkoxy substituted by one or more than one R^{B-2}; R^{B-1} and R^{B-2} are independently deuterium, hydroxyl, cyano, C₁-C₄ alkoxy, C₁-C₂ alkoxy substituted by one or more than one deuterium or 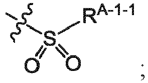 R⁵ is 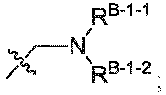 R³, R⁶, R¹², R¹³ and R¹⁴ are H;
scheme 4: R² is 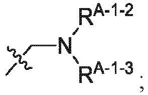 R⁴ and R⁵ are independently halogen, C₁-C₄ alkyl, C₁-C₄ alkyl substituted by one or more than one R^{B-1}, C₁-C₆ alkoxy or C₁-C₆ alkoxy substituted by one or more than one R^{B-2}; R^{B-1} and R^{B-2} are independently deuterium, hydroxyl, cyano, C₁-C₄ alkoxy, C₁-C₂ alkoxy substituted by one or more than one deuterium or 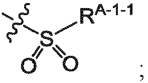 R³, R⁶, R¹², R¹³ and R¹⁴ are H; at least one of R¹⁵ and R¹⁶ is H or deuterium;
scheme 5: R² is halogen, C₁-C₄ alkyl or C₁-C₄ alkyl substituted by one or more than one R^{A-1}; R^{A-1} is halogen; R⁴ is 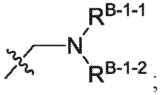 R⁵ is halogen, hydroxyl, C₁-C₄ alkyl, C₁-C₄ alkyl substituted by one or more than one R^{B-1}, C₁-C₆ alkoxy or C₁-C₆ alkoxy substituted by one or more than one R^{B-2}; R^{B-1} and R^{B-2} are independently deuterium, hydroxyl, cyano, C₁-C₄ alkoxy, C₁-C₂ alkoxy substituted by one or more than one deuterium or 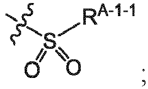 R³, R⁶, R¹², R¹³ and R¹⁴ are H; at least one of R¹⁵ and R¹⁶ is H or deuterium;
scheme 6: R² is halogen, C₁-C₄ alkyl or C₁-C₄ alkyl substituted by one or more than one R^{A-1}; R^{A-1} is halogen; R⁴ is halogen, C₁-C₄ alkyl, C₁-C₄ alkyl substituted by one or more than one R^{B-1}, C₁-C₆ alkoxy or C₁-C₆ alkoxy substituted by one or more than one R^{B-2}; R^{B-1} and R^{B-2} are independently deuterium, hydroxyl, cyano, C₁-C₄ alkoxy, C₁-C₂ alkoxy substituted by one or more than one deuterium or 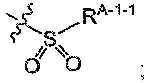 R⁵ is 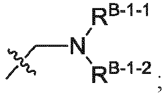 R³, R⁶, R¹², R¹³ and R¹⁴ are H; at least one of R¹⁵ and R¹⁶ is H or deuterium.

11. The aromatic vinyl compound of formula I-0, the tautomer thereof, the stereoisomer thereof, the racemate thereof, the isotopic derivative thereof, or their pharmaceutically acceptable salts according to at least one of claims 1 to 3, wherein, R¹ is cyano, CH₃, CD₃, Cl, CH₂F or C₁-C₄ alkyl substituted by one or more than one halogen; and/or, 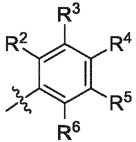 is 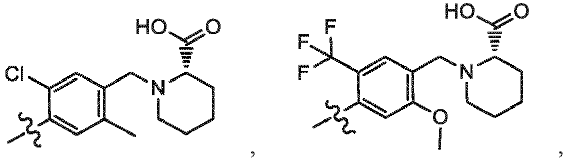 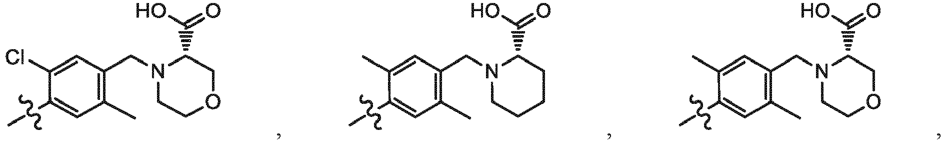 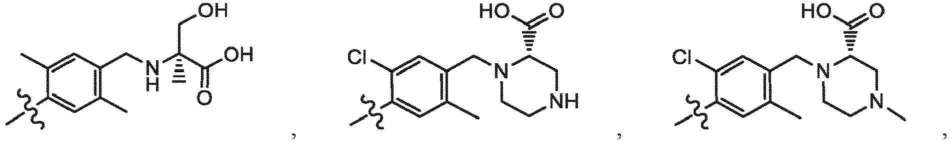 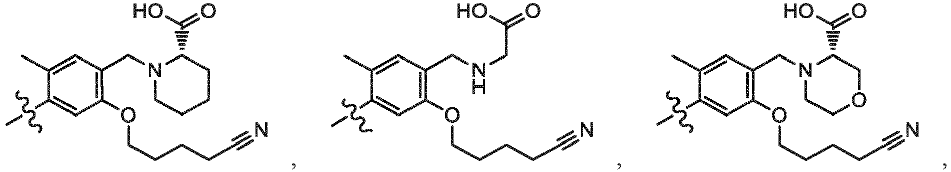 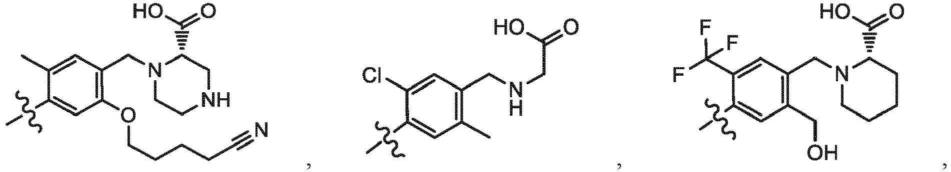 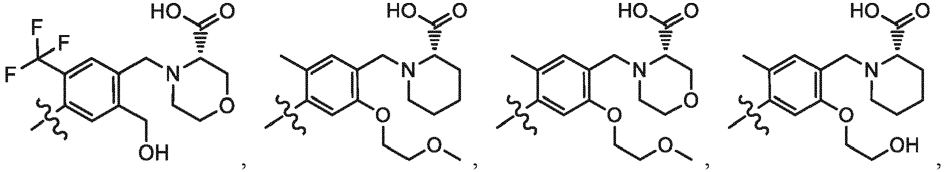 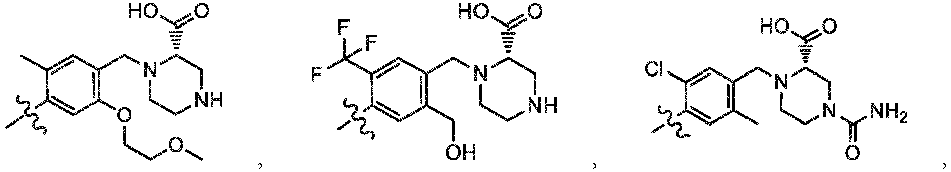 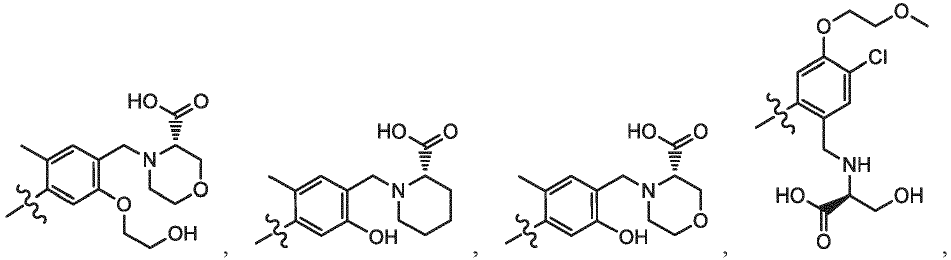 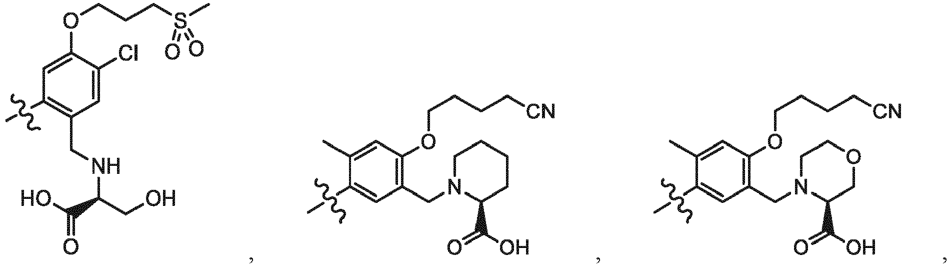 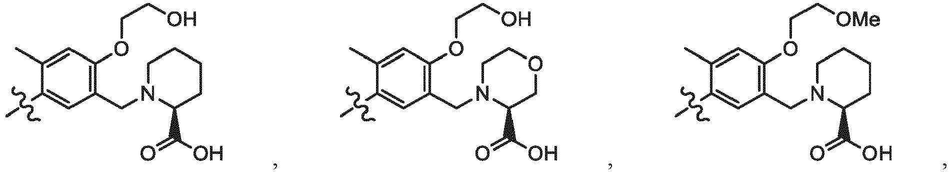 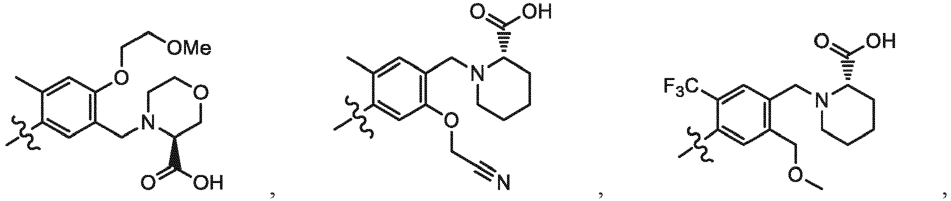 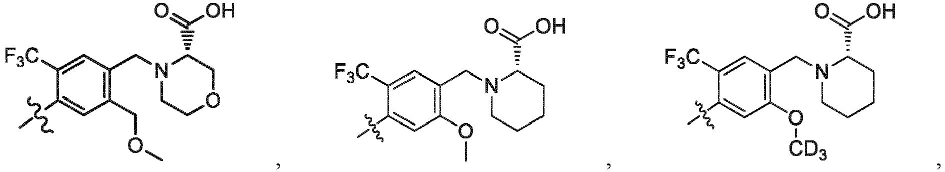 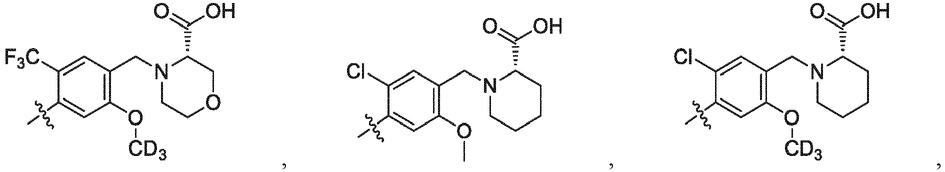 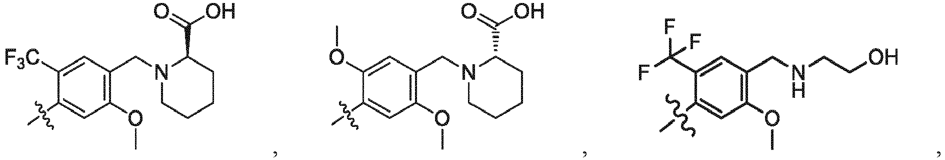 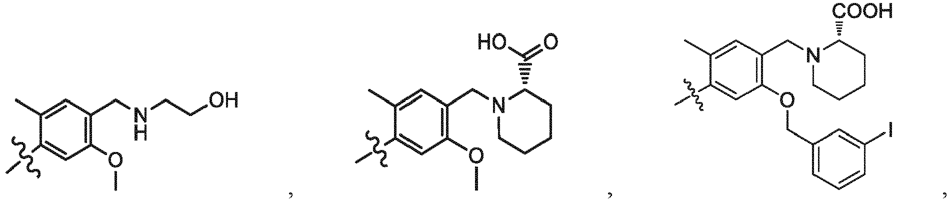 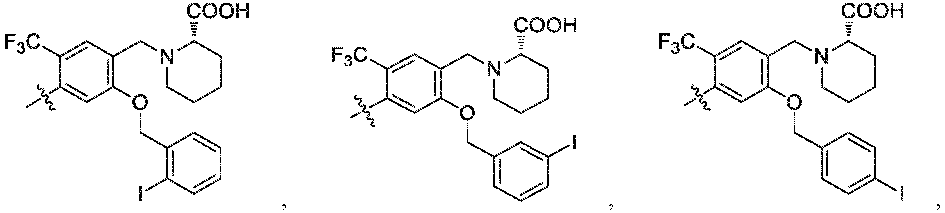 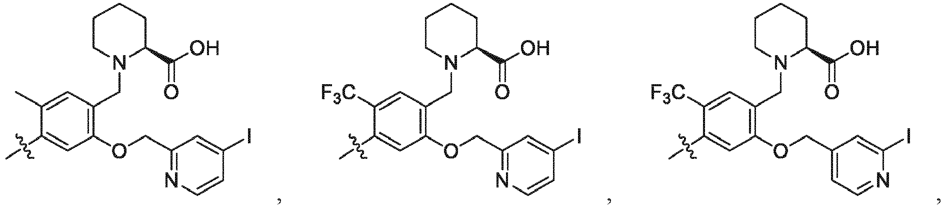 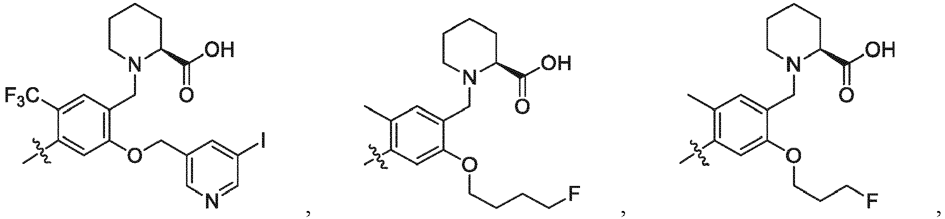 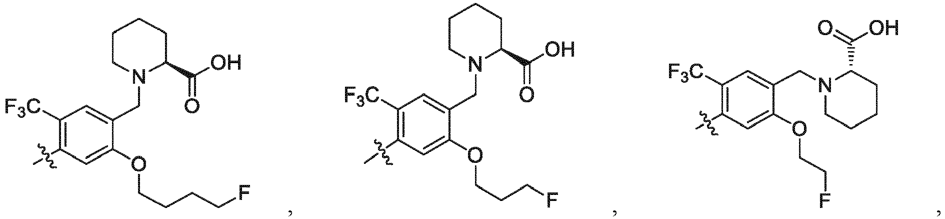 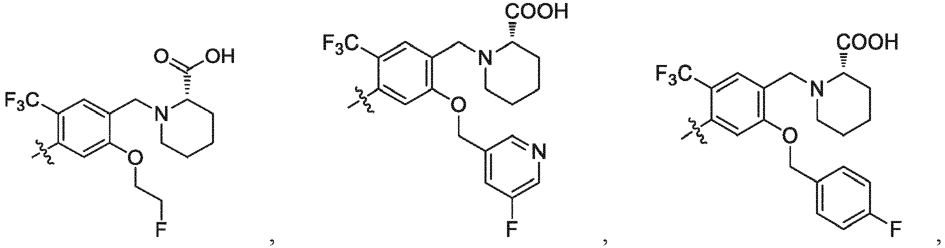 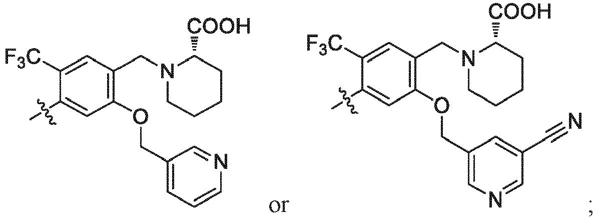 and/or, R¹⁵ and R¹⁶ are independently H, deuterium, Br or F.

12. Any one of the following aromatic vinyl compounds, tautomers thereof, stereoisomers thereof, racemates thereof, isotopic derivatives thereof, or their pharmaceutically acceptable salts,

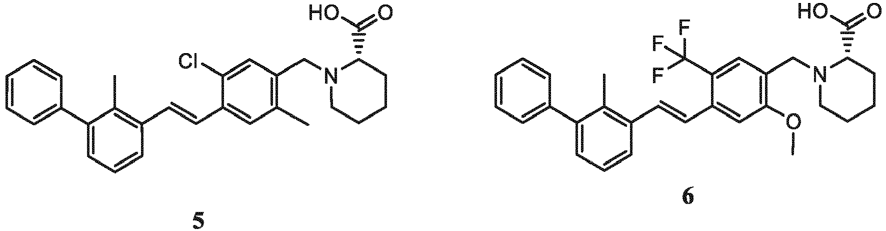

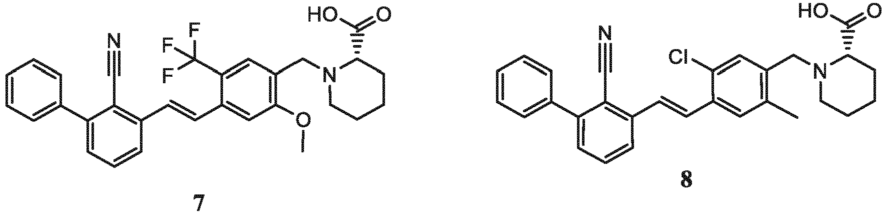

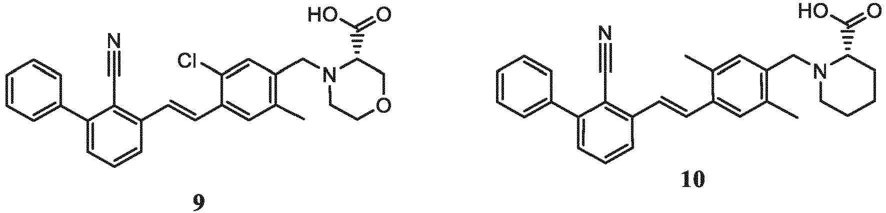

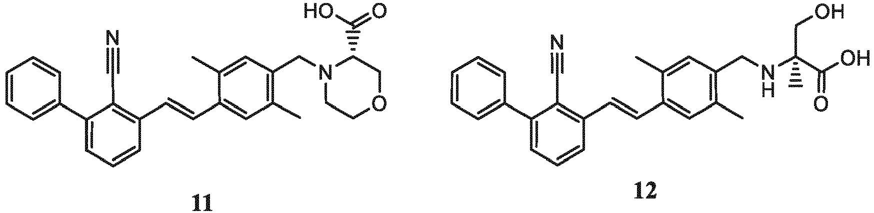

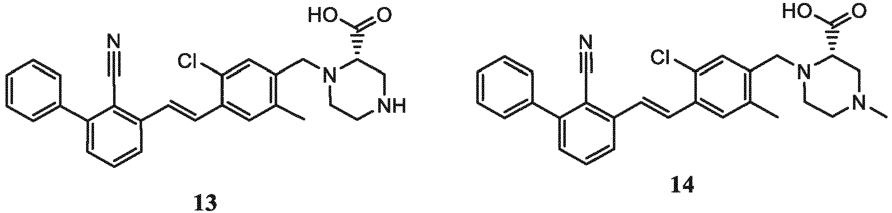

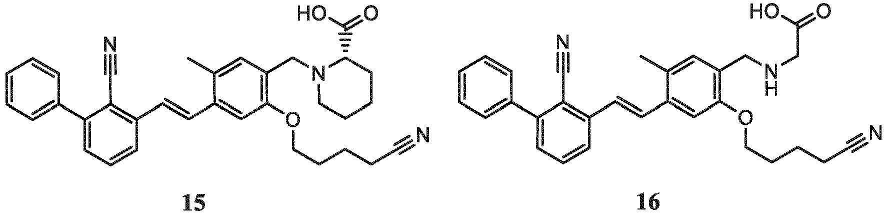

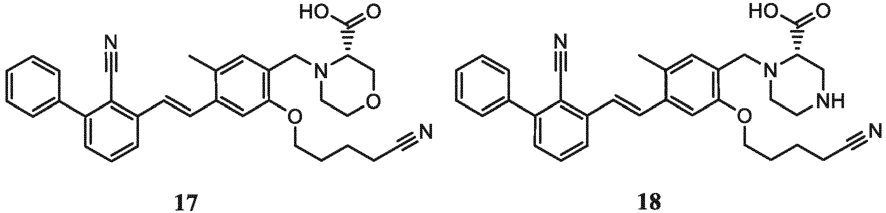

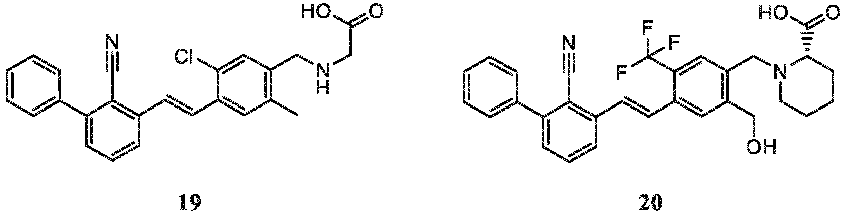

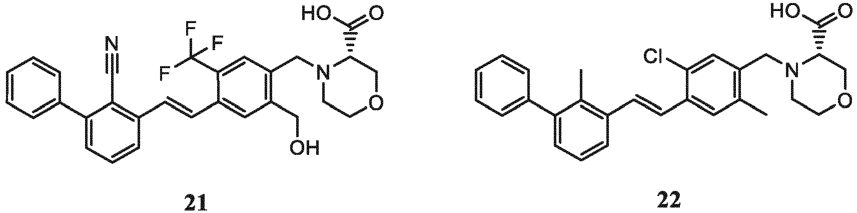

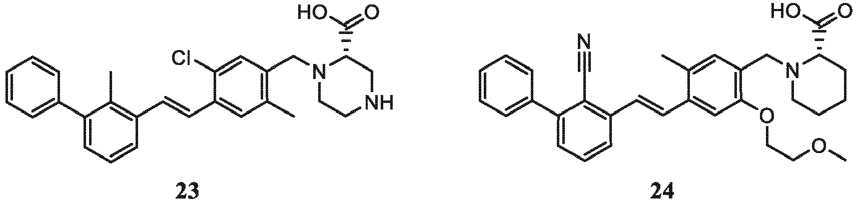

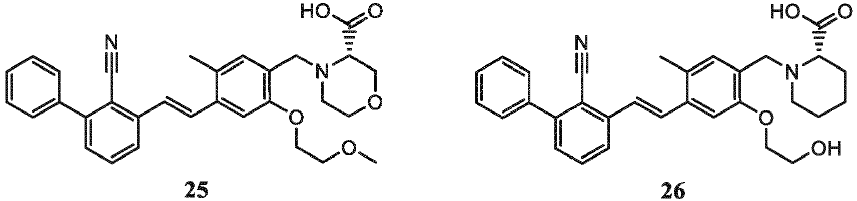

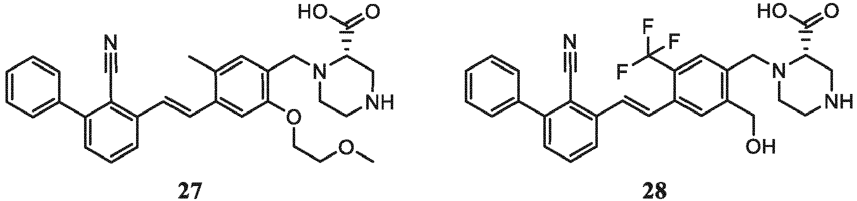

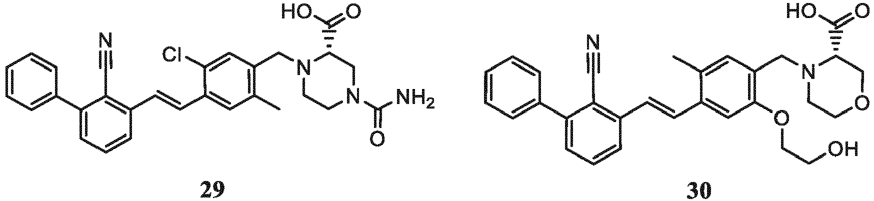

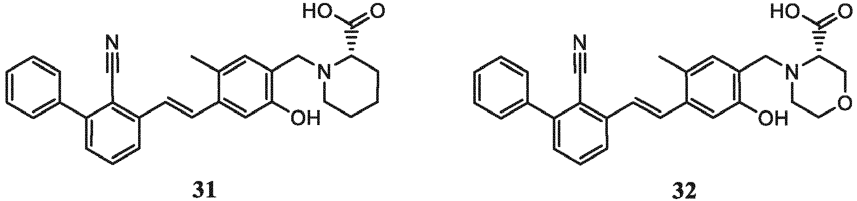

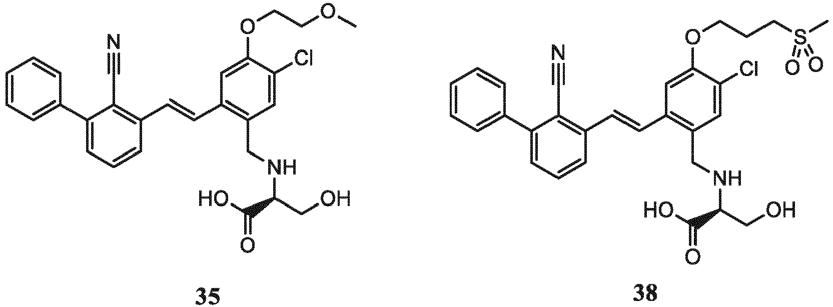

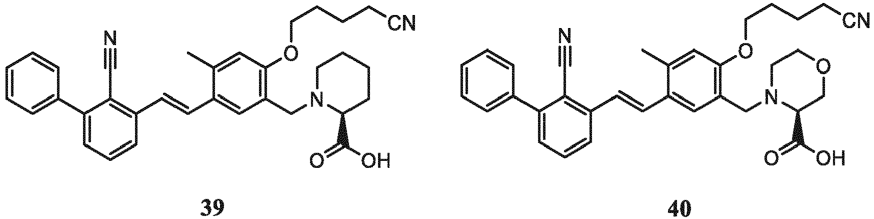

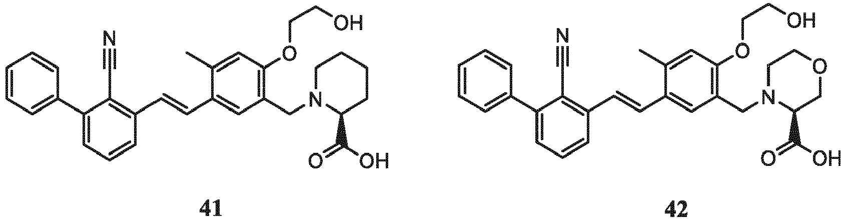

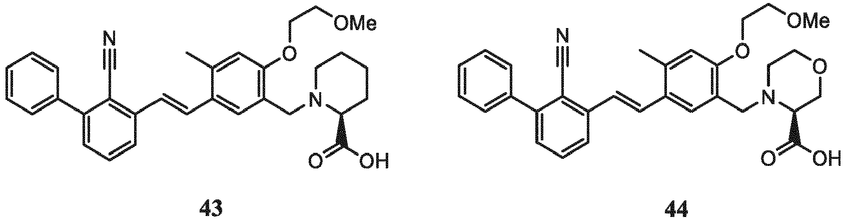

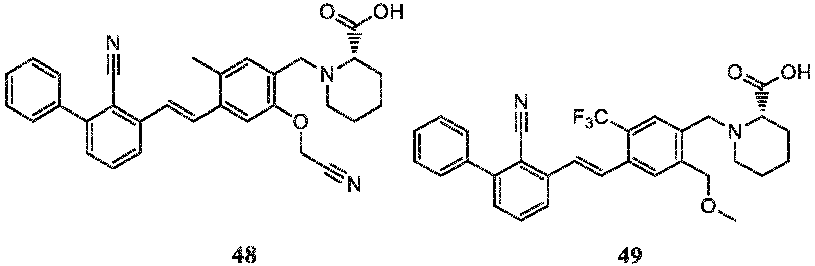

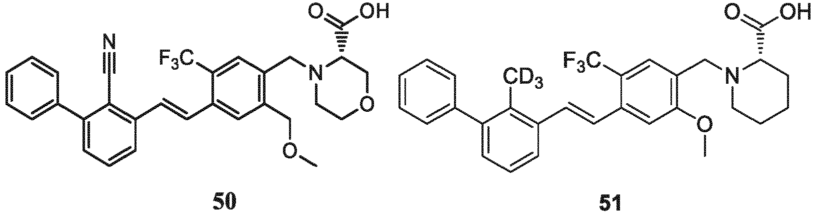

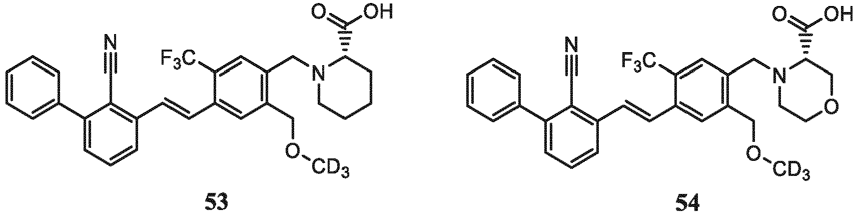

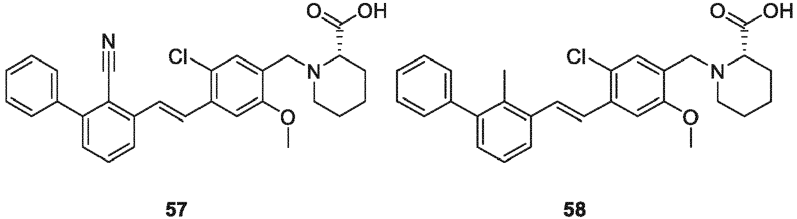

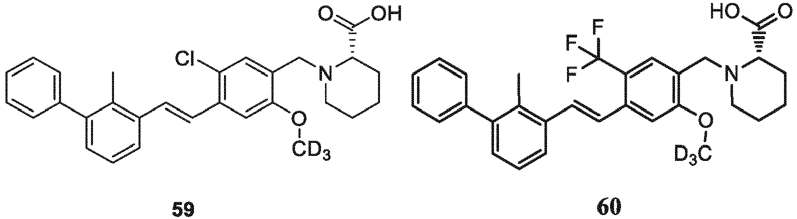

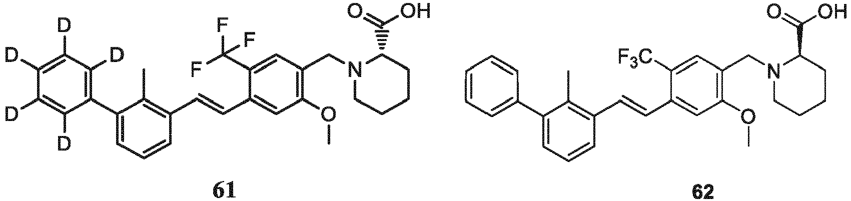

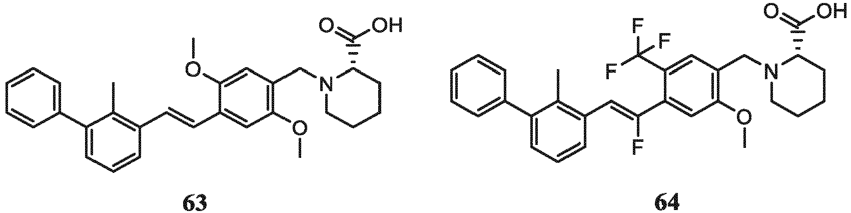

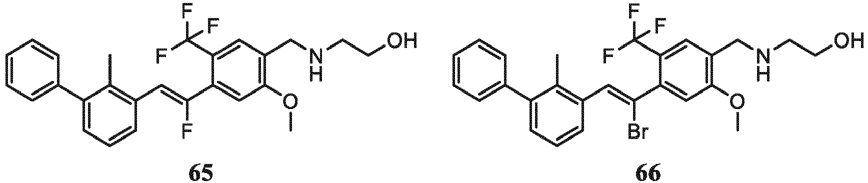

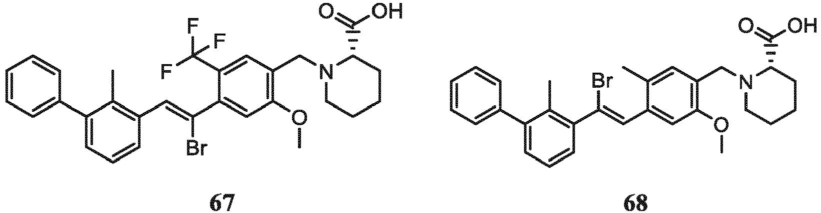

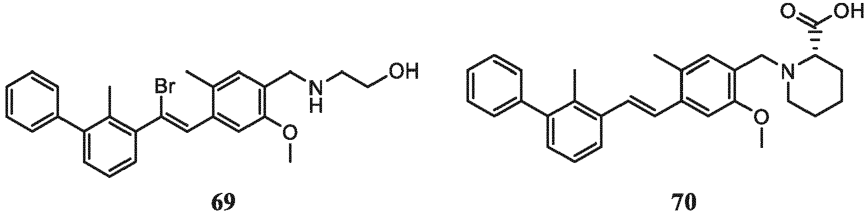

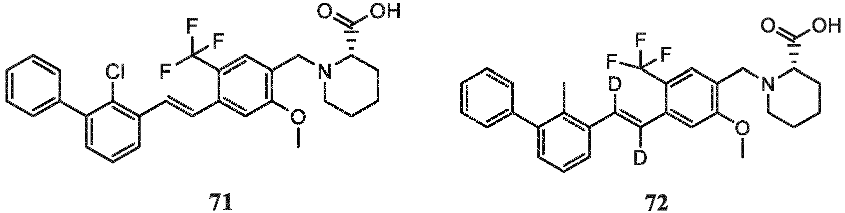

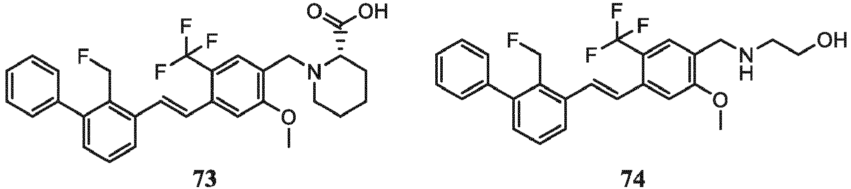

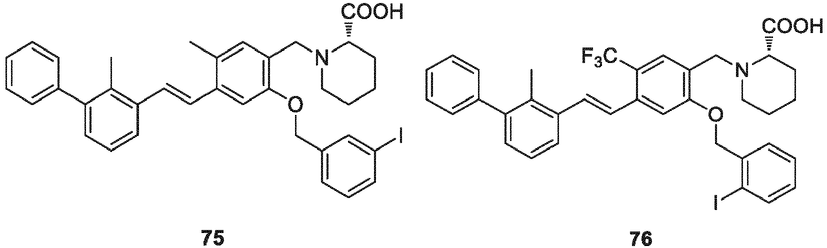

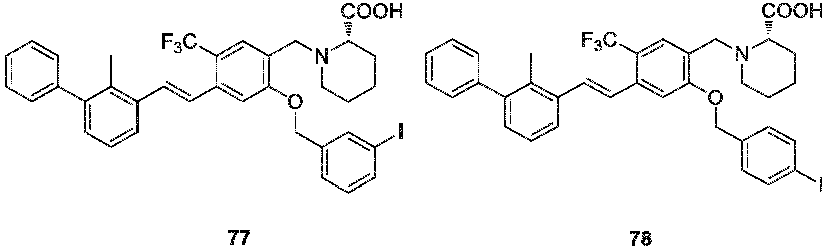

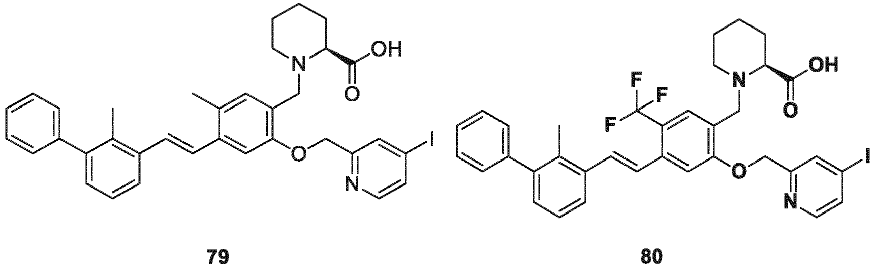

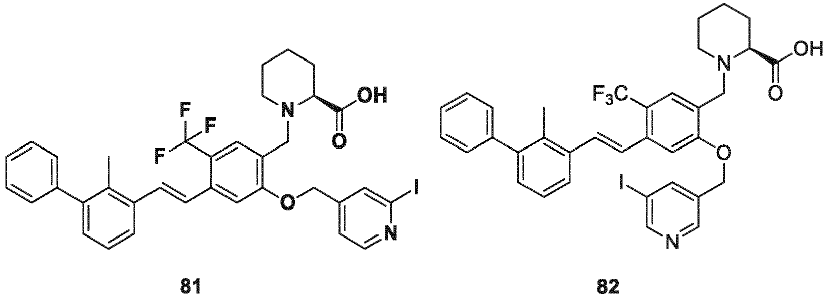

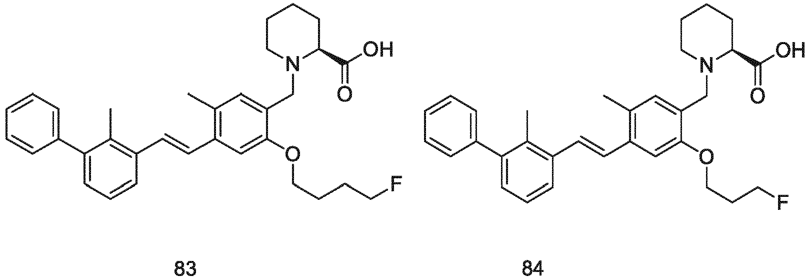

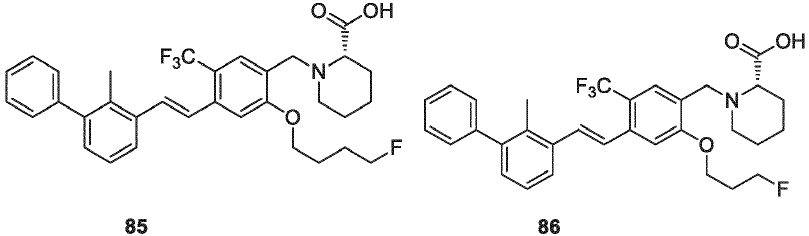

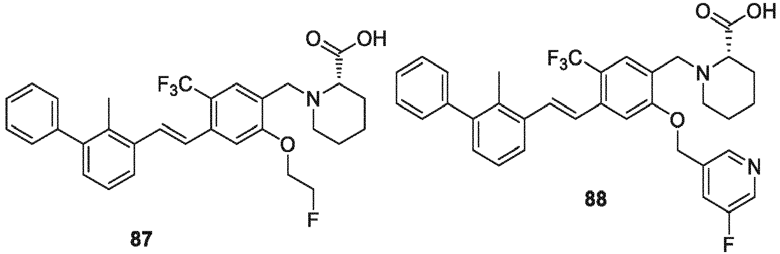

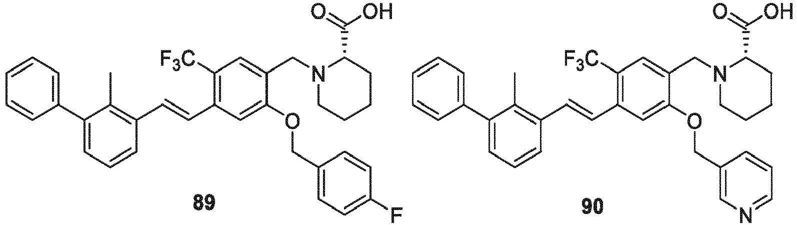

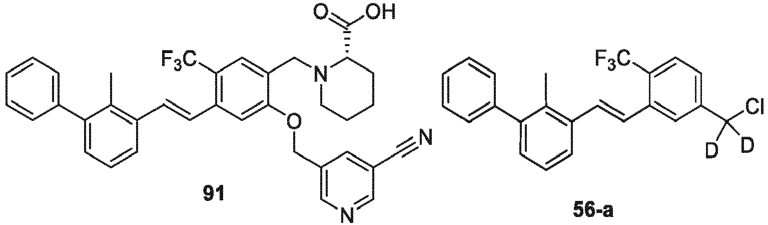

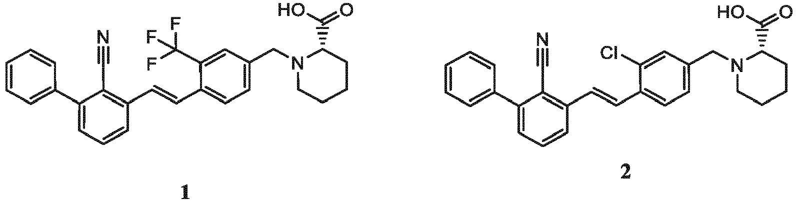

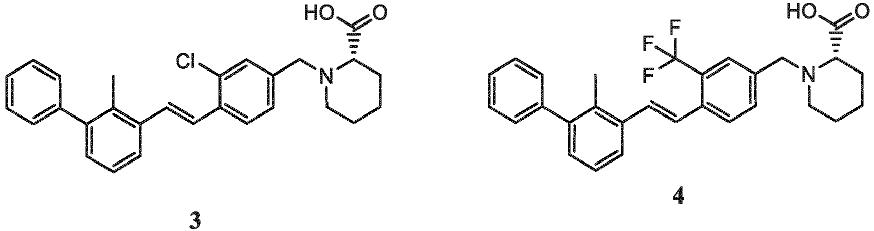

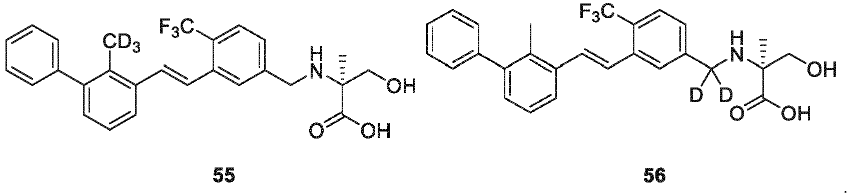

13. A preparation method for the aromatic vinyl compound according to at least one of claims 1 to 12, wherein, the preparation method comprises the following method 1, method 2, method 3, method 4, method 5 or method 6:
method 1 comprises the following steps: in a solvent, in the presence of a reducing agent, subjecting a compound of formula II-a-0 with a compound of formula III-a to a reductive amination reaction as shown below to give the aromatic vinyl compound of formula I-0; 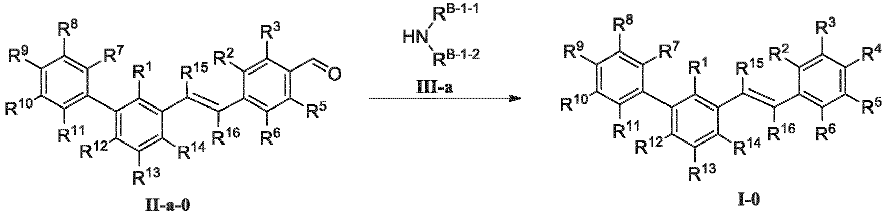
in method 1, R⁴ is 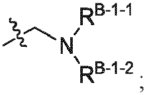 R¹, R², R³, R⁵, R⁶, R⁷, R⁸, R⁹, R¹⁰, R¹¹, R¹², R¹³, R¹⁴ R¹⁵, R¹⁶, R^{B-1-1} and R^{B-1-2} are defined in at least one of claims 1 to 12;
method 2 comprises the following steps: in a solvent, in the presence of a base, subjecting a compound of formula II-b-0 with a compound of formula III-a to a substitution reaction as shown below to give the aromatic vinyl compound of formula I-0; 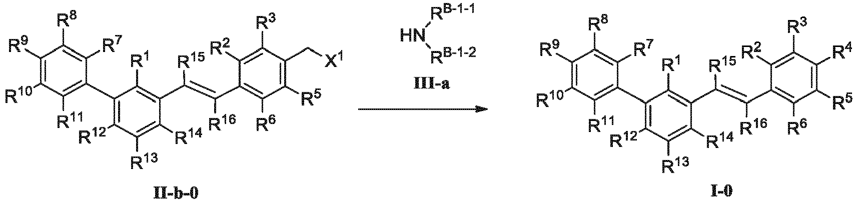
in method 2, R⁴ is 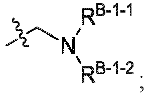 R¹, R², R³, R⁵, R⁶, R⁷, R⁸, R⁹, R¹⁰, R¹¹, R¹², R¹³, R¹⁴ R¹⁵, R¹⁶, R^{B-1-1} and R^{B-1-2} are defined in at least one of claims 1 to 12, and X¹ is halogen;
method 3 comprises the following steps: in a solvent, in the presence of a reducing agent, subjecting a compound of formula II-c-0 with a compound of formula III-a to a reductive amination reaction as shown below to give the aromatic vinyl compound of formula I-0; 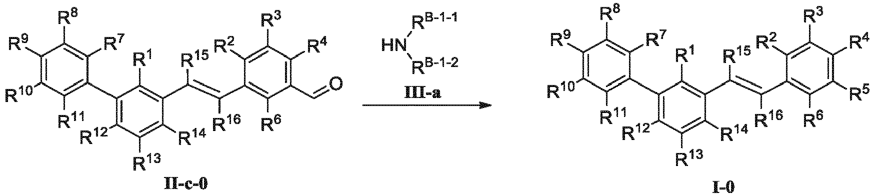
in method 3, R⁵ is 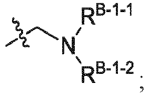 R¹, R², R³, R⁴, R⁶, R⁷, R⁸, R⁹, R¹⁰, R¹¹, R¹², R¹³, R¹⁴ R¹⁵, R¹⁶, R^{B-1-1} and R^{B-1-2} are defined in at least one of claims 1 to 12;
method 4 comprises the following steps: in a solvent, in the presence of a base, subjecting a compound of formula II-d-0 with a compound of formula III-a to a substitution reaction as shown below to give the aromatic vinyl compound of formula I-0; 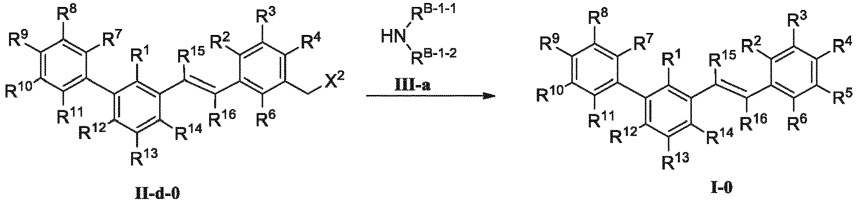
in method 4, R⁵ is 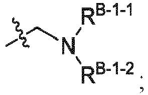 R¹, R², R³, R⁴, R⁶, R⁷, R⁸, R⁹, R¹⁰, R¹¹, R¹², R¹³, R¹⁴, R¹⁵, R¹⁶, R^{B-1-1} and R^{B-1-2} are defined in at least one of claims 1 to 12, and X² is halogen;
method 5 comprises the following steps: in a solvent, in the presence of a reducing agent, subjecting a compound of formula II-e-0 with a compound of formula III-b to a reductive amination reaction as shown below to give the aromatic vinyl compound of formula I-0; 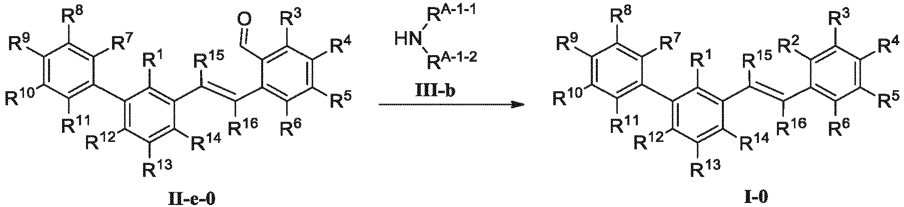
in method 5, R² is 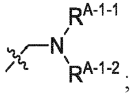 R¹, R³, R⁴, R⁵, R⁶, R⁷, R⁸, R⁹, R¹⁰, R¹¹, R¹², R¹³, R¹⁴, R¹⁵, R¹⁶, R^{A-1-1} and R^{A-1-2} are defined in at least one of claims 1 to 12;
method 6 comprises the following steps: in a solvent, in the presence of a base, subjecting a compound of formula II-f-0 with a compound of formula III-b to a substitution reaction as shown below to give the aromatic vinyl compound of formula I-0; 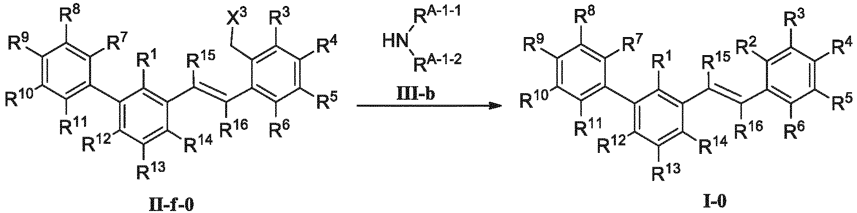
in method 6, R² is 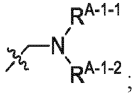 R¹, R³, R⁴, R⁵, R⁶, R⁷, R⁸, R⁹, R¹⁰, R¹¹, R¹², R¹³, R¹⁴, R¹⁵, R¹⁶, R^{A-1-1} and R^{A-1-2} are defined in at least one of claims 1 to 12, and X³ is halogen.

14. A compound of formula II-a-0, II-b-0, II-c-0, II-d-0, II-e-0 or II-f-0, 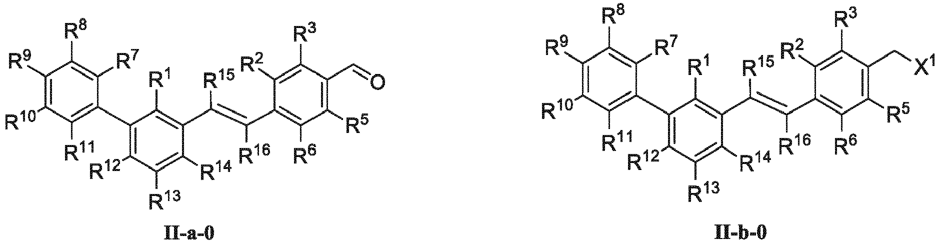 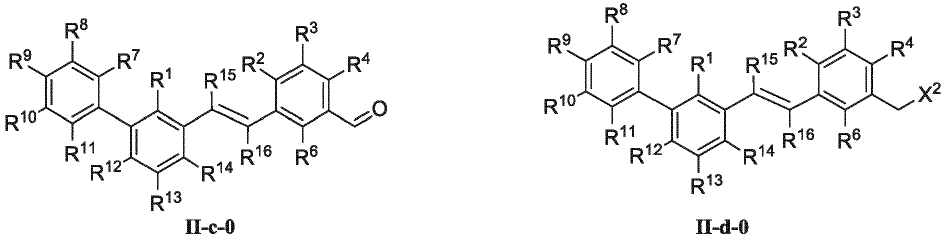 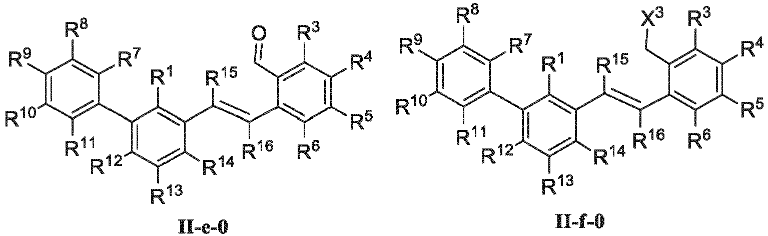 in the compound of the above formulas, R¹, R², R³, R⁴, R⁵, R⁶, R⁷, R⁸, R⁹, R¹⁰, R¹¹, R¹², R¹³, R¹⁴, R¹⁵ and R¹⁶ are defined in at least one of claims 1 to 12, and X¹, X² and X³ are independently halogen.

15. Any one of the following compounds, wherein,

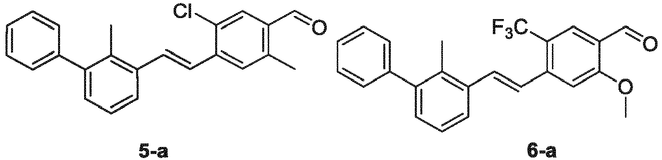

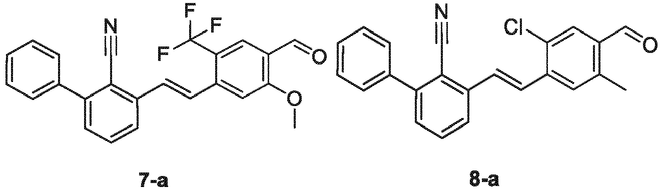

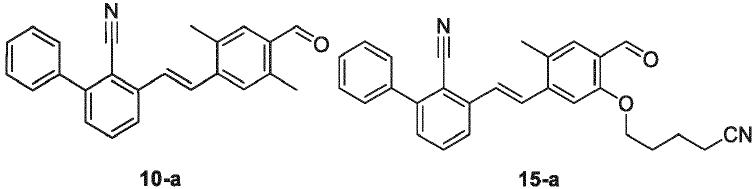

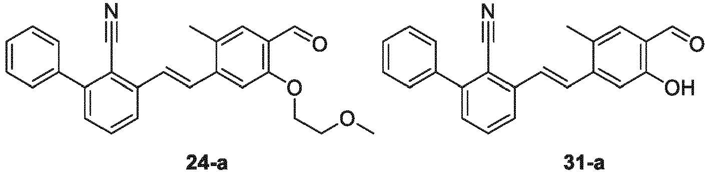

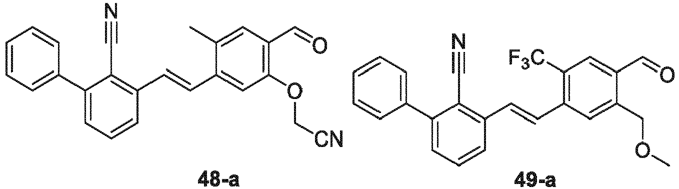

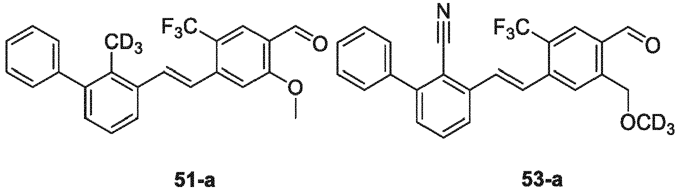

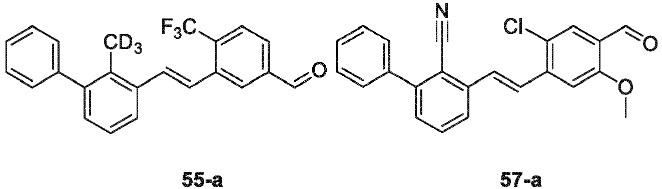

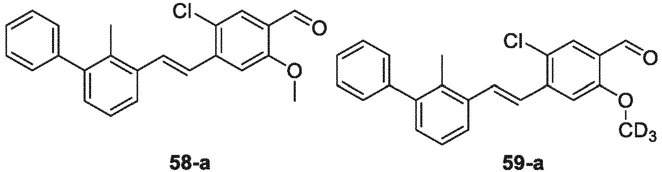

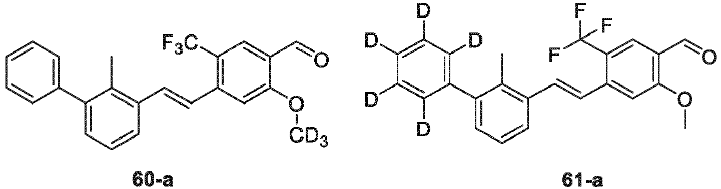

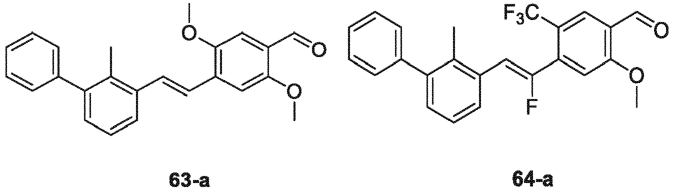

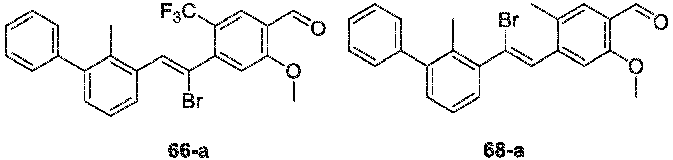

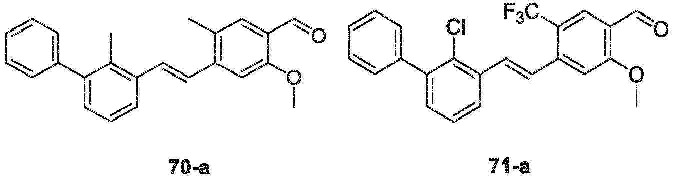

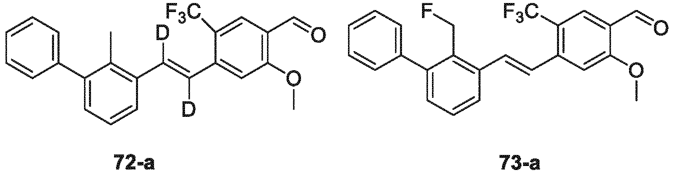

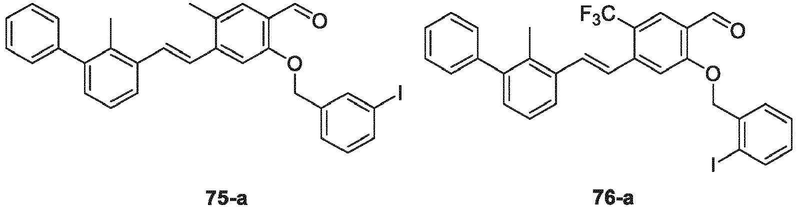

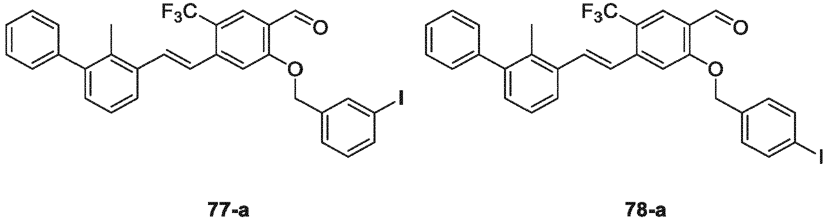

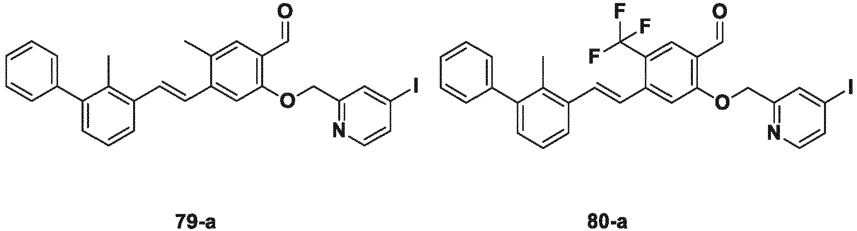

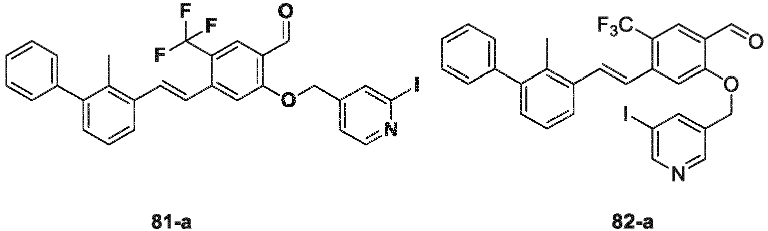

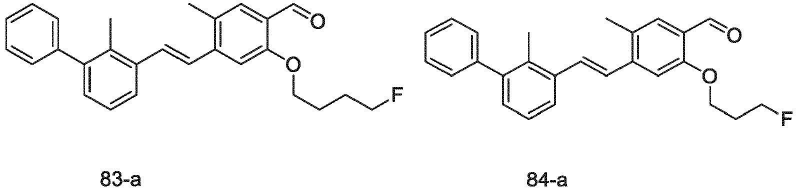

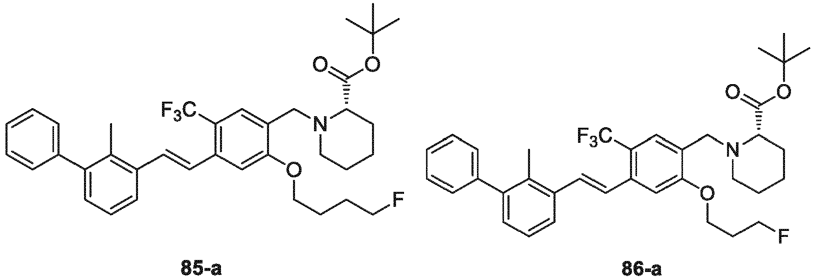

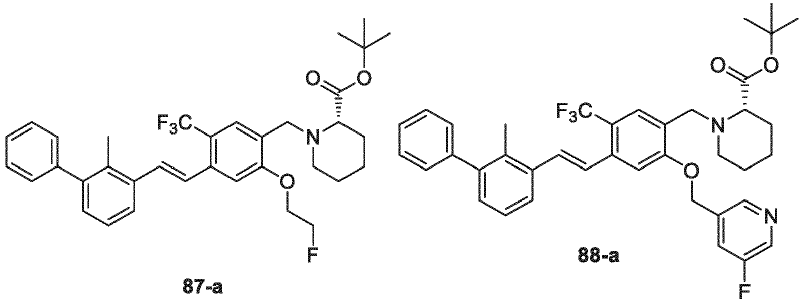

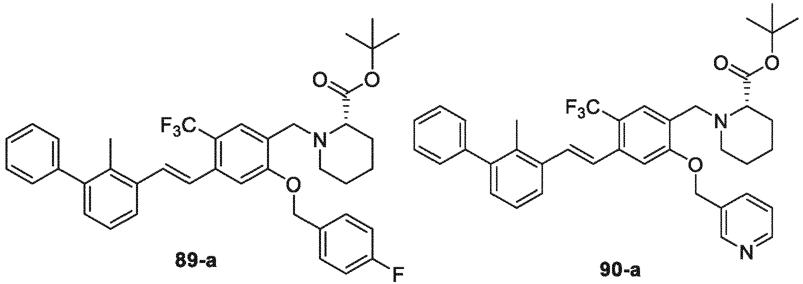

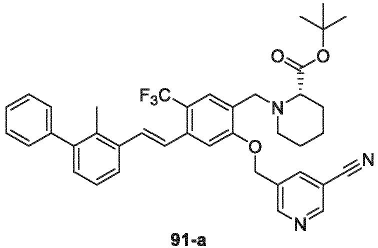

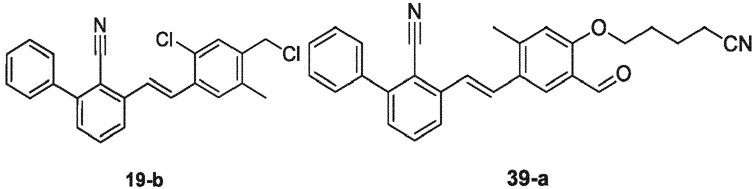

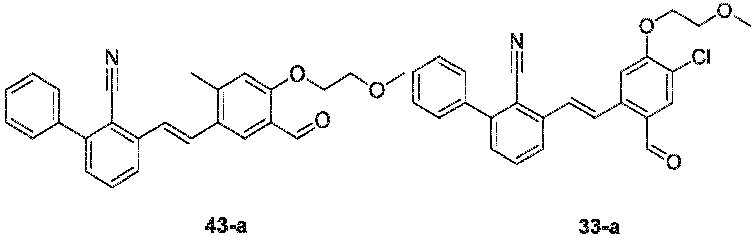

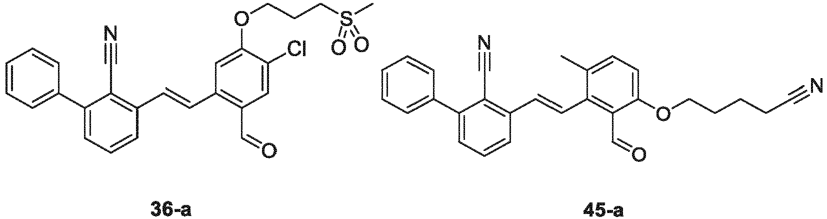

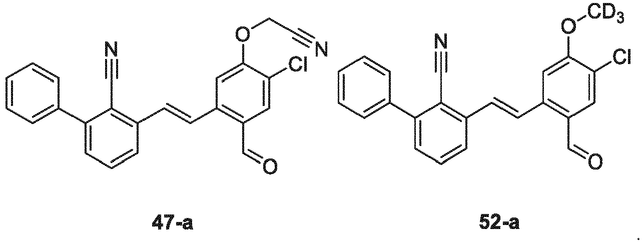

16. A pharmaceutical composition, wherein, the pharmaceutical composition comprises the aromatic vinyl compound, the tautomer thereof, the stereoisomer thereof, the racemate thereof, the isotopic derivative thereof, or their pharmaceutically acceptable salts according to at least one of claims 1 to 12, and a pharmaceutical excipient;
and/or, the pharmaceutical composition comprises the aromatic vinyl compound, the tautomer thereof, the stereoisomer thereof, the racemate thereof, the isotopic derivative thereof, or their pharmaceutically acceptable salts according to at least one of claims 1 to 10, and at least one other drug; wherein, the other drug is a chemotherapy drug or a targeted drug.

17. The pharmaceutical composition according to claim 16, wherein, the targeted drug is one or more of COX-2 inhibitors, DPP4 inhibitors, CSF-1α inhibitors and A2a antagonists.

18. A use of the aromatic vinyl compound, the tautomer thereof, the stereoisomer thereof, the racemate thereof, the isotopic derivative thereof, or their pharmaceutically acceptable salts according to at least one of claims 1 to 12, or the pharmaceutical composition according to claim 16 or 17 in the manufacture of a PD-1 inhibitor and/or PD-L1 inhibitor.

19. A use of the aromatic vinyl compound, the tautomer thereof, the stereoisomer thereof, the racemate thereof, the isotopic derivative thereof, or their pharmaceutically acceptable salts according to at least one of claims 1 to 12, or the pharmaceutical composition according to claim 16 or 17 in the manufacture of a drug for the prevention and/or treatment of diseases related to PD-1/PD-L1 signaling pathway.

20. The use according to claim 19, wherein, the diseases related to PD-1/PD-L1 signaling pathway are cancer, infectious disease, autoimmune disease or related disease.

21. The use according to claim 20, wherein, the cancer is one or more of lung cancer, esophageal cancer, gastric cancer, colorectal cancer, hematological tumor, lymphoma, head and neck cancer, liver cancer, nasopharyngeal cancer, brain tumor, breast cancer, cervical cancer, blood cancer and bone cancer;
and/or, the infectious disease is bacterial infection and/or viral infection;
and/or, the autoimmune disease is one or more of rheumatoid arthritis, systemic lupus erythematosus, systemic sclerosis, systemic vasculitis and relapsing polychondritis.
